# EUROPEAN PATENT APPLICATION

(11) **EP 2 327 793 A1**
(43) Date of publication of application: **01.06.2011**
(21) Application number: 09177117.0
(22) Date of filing: 25.11.2009
(51) Int. Cl.: C12Q 1/68

(54) **Pyrosequencing method for predicting the response of a patient towards anti cancer treatment**

(71) Applicant: UNIVERSITÄT ZU KÖLN, 50923 Köln (DE)
(72) Inventor: Nürnberg, Peter Prof. Dr., 16818 Wuthenow (DE); Altmüller, Janine Dr., 51399 Burscheid (DE); Querings, Silvia Dr., 50670 Köln (DE)
(74) Representative: Michalski Hüttermann & Partner Patentanwälte

(57) **Abstract**

The present invention is directed to a method for predicting the response of a patient suffering from or at risk of developing a neoplastic disease towards a given therapy agent, said method comprising at least the steps of obtaining a biological sample from said patient; determining, in said sample, the presence or absence of at least one mutation affecting the response to a anti-cancer treatment by means of pyrosequencing, and predicting the therapeutic success of a given anti-cancer treatment.

## Description

### FIELD OF THE INVENTION

The present invention is related to methods for predicting the response of a patient towards anti-cancer treatment.

### BACKGROUND OF THE INVENTION

The epidermal growth factor receptor is the cell-surface receptor for members of the epidermal growth factor family (EGF-family) of extracellular protein ligands. The EGFR is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). Mutations affecting EGFR expression or activity are implicated in cancer development and progression. Thus research on the EGFR is ongoing and a variety of mutations have been described for EGFR-dependend tumors, like NSCLC (non small cell lung cancer) tumors. The occurrence of some of the mutations, which reduce the susceptibility of the tumor against respective treatments, already impact therapy as the detection of these variants allows choosing the most suitable tumor therapy, and helps to reduce side effects in patients, which would not receive any benefit from, e.g., a given anti-EGFR treatment. However such a detection is not trivial, since many of the said mutations are not merely simple SNPs (Single Nucleotide Polymorphims) at defined positions; but rather a multitude of possible variations including deletions, insertions and substitutions within mutation hot-spot regions is observed.

Additionally, genotyping approaches are often hampered because tissue obtained by biopsy comprises only a small amount of raw material, which contains sometimes only a few tumor cells surrounded by predominantly healthy tissue. The perfect diagnostic procedure would require a highly specific, highly sensitive and fast and cost effective technology. These goals are not met by current techniques, like SNP-genotyping strategies, Sanger-sequencing, or next generation sequencing (NGS) technology e.g. 454's Pyrosequencing method, microfluidic Sanger sequencing and sequencing by ligation.

### The EGFR signalling pathway

EGFR (epidermal growth factor receptor, also termed ErbB1) is a cell surface tyrosin kinase receptor, which is activated by binding of its specific ligands, including epidermal growth factor (EGF) and transforming growth factor α (TGFα).

Upon activation by its growth factor ligands, EGFR undergoes a transition from an inactive monomeric form to an active homodimer. In addition to forming homodimers after ligand binding, EGFR may pair with another member of the ErbB receptor family, such as ErbB2, to create an activated heterodimer. There is also evidence to suggest that clusters of activated EGFRs form, although it remains unclear whether this clustering is important for activation itself or occurs subsequent to activation of individual dimers.

EGFR dimerization stimulates its intrinsic intracellular protein-tyrosine kinase activity. As a result, autophosphorylation of several tyrosine residues in the C-terminal domain of EGFR occurs. This autophosphorylation elicits downstream activation and signaling by several other proteins that associate with the phosphorylated tyrosines through their own phosphotyrosine-binding SH2 domains. These downstream signaling proteins initiate several signal transduction cascades, principally the **MAPK**, Akt and JNK pathways, leading to DNA synthesis and cell proliferation. A major downstream signalling protein is GRB (Growth factor receptor-bound protein), which is associated to another downstream protein, SOS (Son of sevenless). Upon phosphorylization of GRB, the latter is thus activated and activates KRAS by catalysing the exchange of GDP by GTP in KRAS. KRAS is tethered to cell membranes because of the presence of an isoprenyl group on its C-terminus. KRAS is a member of the Ras family of GTPases, and is, upon activation, responsible for second messenger responses, which for example promote cell proliferation or angiogensis.

### Relevance of EGFR in tumor formation

Anti EGFR-treatment has turned out to be a promising approach in tumor i.e. neoplastics disease therapy. The reason being that many tumors exhibit an overexpression of wild-type EGFR, which seemingly overactivates the cell and thus supports tumor proliferation, cell mitosis and formation of metastases. These tumors generally correlate with a poor clinical outcome. Moreover, in some cases EGFR overexpression coincides with an increased production of EGFR ligands, such as TGF-α, thus leading to autocrine stimulation.

In additions to this, some tumors have mutations in proteins of the EGFR signalling pathway. These mutations may lead to an autoactivation of the EGFR pathway, and hence promote tumor proliferation, cell mitosis and formation of metastases.

### Therapeutic approaches

The EGFR signalling pathway is thus subject of therapeutic approaches in anti-cancer therapy. One approach is to block the EGFR against binding of its respective ligands extracellularly. For this purpose, receptor antagonists have been developed, for example monoclonal antibodies (mAb), like cetuximab (Erbitux). Another approach is to inhibit the intracellular tyrosin kinase domain, e.g., by means of small molecules, like Gefitinib (Iressa) or Erlotinib (Tarceva). Other approaches involve the use of antisense nucleotides to reduce expression of EGFR, and Anti-EGFR-based immunoconjugates, like immunotoxins, immunoradionucleotides and immunoliposomes.

### The role of mutations

Mutations in the EGFR signalling pathway may affect the efficacy of anti-cancer treatment, especially of anti-EGFR treatment. It has, for example, been reported that tumors having an EGFR mutation are less responsive to extracellular EGFR antagonists, while tumors without an EGFR mutation are less responsive to tyrosine kinase inhibition.

Other studies have demonstrated that mutations in the KRAS gene also play a role for responsiveness towards anti-EGFR-treatment. Patients with wild-type KRAS tumors have been shown to benefit from a high response when treated with the EGFR antagonist Erbitux. In tumors having a KRAS mutation, however, extracellular EGFR antagonists have turned out to show little effect.

The following table summarizes data from different sources related to susceptibility of different EGFR-dependent tumor genotypes to different anti-EGFR therapy approaches

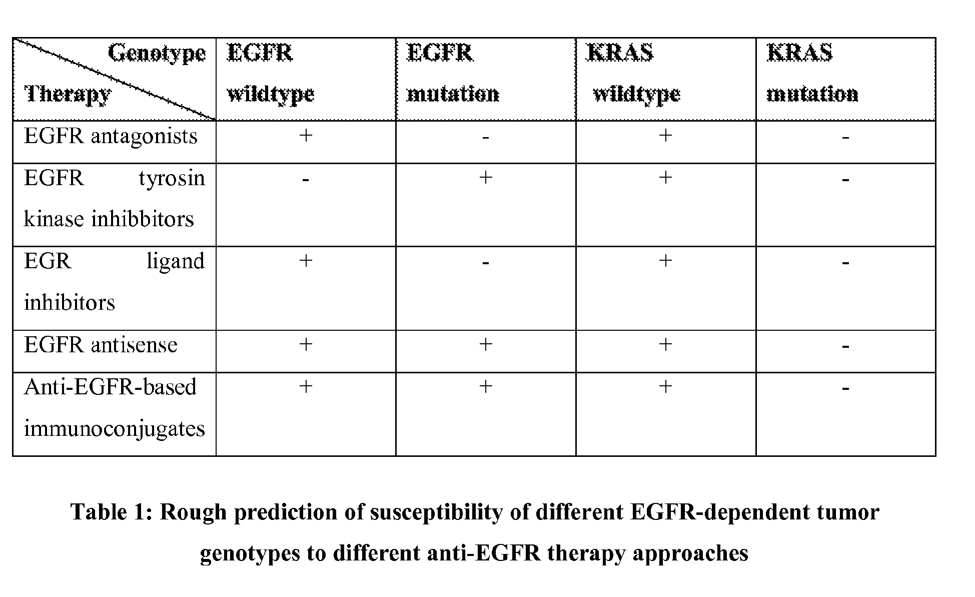

### Further roles of KRAS

In addition thereto, KRAS is also involved in EGFR-independent signalling pathways, e.g. the Hedgehog signalling pathway or the Integrin signalling pathway. For this reason, KRAS mutations may also play a role in EGFR-independent cancer genesis.

### Need for mutation genotyping prior to anti EGFR therapy

For the above reasons, it has been considered to carry out a mutation analysis prior to anti-cancer-therapy, especially to anti-EGFR-therapy, in order to be able to select a therapy, which meets the patient's specific needs i.e. has the best anti-tumor effect and avoids unnecessary side effects.

In W02007001868, mutations of the EGFR gene and the KRAS gene are described, and their impact on anticancer therapy is discussed. However the main focus of this application is directed to co-detecting in one sample a KRAS mutation by means of Big Dye Terminator sequencing, a Sanger sequencing method, and the presence of a possibly mutated EGFR protein by immunohiostchemistry.

Thus a significant drawback of this method is that it is quite laborious, involving many steps, especially laser capture micro-dissection and electrophoresis that render the whole process very time-consuming. Therefore the process can not be readily integrated into a modem clinical setting, which requires fast decisions, on which therapy is the most suitable one for a patient.

A kit for the detection of EGFR mutations affecting anti-EGFR cancer therapy is provided by Qiagen under the trademark "Therascreen". The kit detects a number of deletions, insertions and SNPs in exons 19 and 20 of the EGFR gene. The kit uses a combination of allele specific PCR and real-time PCR technology to test for several EGFR mutations.

However, this method has the disadvantages that the probes used in the real-time PCR, termed "Scorpion-primers" are expensive to produce and that the signal generated needs to be quantitated using the ΔCt-Method. In contrast pyrosequencing reagents are more cost-effective, and the generated signal is the sequence itself. So unlike a fluorescent signal, the sequence information is intelligible; therefore it is easy to verify and communicate the pyrosequencing results.

### SUMMARY OF THE INVENTION

It is an object of the present invention to provide a method for predicting the response of a patient towards anti-cancer treatment, like anti-EGFR treatment, which is faster, more specific and more cost-effective that methods know from the art.

It is another object of the present invention to provide a method for predicting the response of a patient towards anti-cancer treatment, like anti-EGFR treatment, which has a considerably higher sensitivity than methods know from the art and hence requires less sample material and/or less elaborate preparation techniques.

It is yet another object of the present invention to provide a method for predicting the response of a patient towards anti-cancer treatment, like anti-EGFR treatment, the outcome of which is easy to understand for the people using the method.

It is a further object of the present invention to provide a method for predicting the response of a patient towards anti-cancer treatment, like anti-EGFR treatment, which is highly reliable.

These objects are achieved by the method as set forth in the independent claims. The dependent claims indicate preferred embodiments. In this context it is noteworthy to mention that all ranges given in the following are to be understood as that they include the values defining these ranges.

### BRIEF DESCRIPTION OF THE DRAWINGS

Additional details, features, characteristics and advantages of the object of the invention are disclosed in the subclaims, the figures and the following description of the respective figures and examples, which, in an exemplary fashion, show preferred embodiments according to the invention. It is to be understood that the examples are by no means meant as to limit the scope of the invention.
Fig. 1 shows linearity test diagrams for the hot-spot region in exon 19 of the EGFR and the mutation variant V2 (a deletion). I is demonstrated how easy it is with the method according to the invention to find out whether a sample contains mutated DNA.
Fig 2 shows a summary of the frequently occurring deletions in exon 19 of the EGFR, wherein WT is the wild-type and V1 - V18 are known deletions.
Fig 3 shows the same summary of the frequently occurring deletions in exon 19 of the EGFR as shown in Figure 2 except for V 18. This time, however, the dispensing order of nucleotides has been changed to the "mutation-detection-insert" dispensing order of nucleotides.
Fig. 4 shows linearity test diagrams for the fragment L858R in exon 21 of the EGFR. The samples comprised a wild-type cells line (L_H1975_WT) and/or a cell line (1975) containing mutated cells.
Fig 5 shows parts of some of the linearity test diagrams for the mutation T790M in exon 20 of the EGFR. The samples comprised DNA from a wild-type cell line and/or a cell line containing mutated cells.
Fig. 6 shows diagrams of the pyrosequencing results of several mutation variants in the hot-spot region of the KRAS gene in exon 2. Even if just a small percentage of the sample was made up of tumor cells a determination of the mutation is easily possible.
Fig. 7 shows linearity test diagrams for the mutation variant G12V in exon 2 of the KRAS gene. The samples comprised DNA from a wild-type cell line and/or a cell line containing mutated cells.
Fig. 8 shows part of some of the linearity test diagrams for the mutation variant Q61 H in exon 3 of the KRAS gene. The samples comprised DNA from a wild-type cell line and/or a cell line containing mutated cells.

### DETAILED DESCRIPTION OF EMBODIMENTS

The invention is directed to a method for predicting the response of a patient suffering from or at risk of developing a neoplastic disease towards a given therapy agent, said method comprising at least the steps of:
a) obtaining a biological sample from said patient;
b) determining, in said sample, the presence or absence of at least one mutation affecting the response to a anti-cancer treatment by means of pyrosequencing, and
c) predicting the therapeutic success of a given anti-cancer treatment.

As used herein, the term "a neoplastic disease" refers to an abnormal mass of tissue as a result of neoplasia i.e. the abnormal proliferation of cells. The growth of these cells exceeds, and is uncoordinated with, that of the normal tissues around it. It usually, but not always, causes a lump or tumor. Neoplasms may be benign, pre-malignant or malignant.

As used herein, the term "biological sample" refers to any sample that will allow the implementation of the subsequent analysis steps. Thus the biological sample will usually be a biopsy or a blood sample i.e. its preparation will involve the removal of cells or tissues from their original environment for examination, but depending on the type of neoplastics disease the biological sample might also be a body fluid.

The term "mutation" as used herein, refers to changes in the base pair sequence of the genetic material of an organism. Examples of mutations are insertions, where one or more extra bases are added into the base pair sequence, deletions, where one or more bases are removed from the base pair sequence and substitutions, where one or more bases are exchanged for one another. In addition the term mutation might also refer to large scale alterations such as chromosome changes e.g. translocations and inversions.

As used herein, the term "anti-cancer treatment" refers to a treatment that inhibits or stops neoplastic phenomena, e.g. tumor proliferation, and/or formation of metastases, wherein said neoplastic phenomena are in a causal relationship with at least one mutation. Preferably the "anti-cancer treatment" even eliminates mutated cells.

As used herein, the term "pyrosequencing" refers to a method of nucleic acid sequencing based on a "sequencing by synthesis" principle, where a single strand of the DNA to be sequenced is taken and its complementary strand is synthesized enzymatically. Moreover pyrosequencing relies on the detection of pyrophosphate release upon nucleotide incorporation, rather than chain termination with dideoxynucleotides like the Sanger sequencing method.

Essentially, the method allows sequencing of a single strand of DNA by synthesizing the complementary strand along it, one base pair at a time, and detecting which base was actually added at each step. Light is produced only when the nucleotide added ― each nucleotide is added in turn ― complements the unpaired base of the template. The sequence of solutions, which produce chemiluminescent signals, allows the determination of the sequence of the template.

In order to obtain a single strand of nucleic acid, typically DNA, for pyrosequencing usually a PCR is performed prior to pyrosequencing, in which the reverse primer is biotin labelled on its 5' end. Using this method, the single nucleic acid strand can be isolated after completion of the PCR through exploiting the interaction of biotin and streptavidin or avidin. For example streptavidin coated sepharose beads are added to the PCR reaction product. After a sufficient time for the biotin and streptavidin moieties to interact with each other the beads and hence the single stranded nucleic acid are isolated.

The actual pyrosequencing method then basically has four steps:
1. A single stranded template e.g. single stranded DNA is hybridized to a pyrosequencing primer and incubated with the enzymes DNA polymerase, ATP sulfurylase, luciferase and apyrase, and with the substrates adenosine 5' phosphosulfate (APS) and luciferin.
2. The addition of one of the four deoxynucleotide triphosphates (dNTPs) initiates the second step, in which the DNA polymerase catalyzes the incorporation of the deoxyribonucleotide triphosphate into the DNA strand, if it is complementary to the base in the template strand. Each incorporation event is accompanied by release of pyrophosphate (PPi) in a quantity equimolar to the amount of incorporated nucleotide.
3. ATP sulfurylase quantitatively converts PPi to ATP in the presence of adenosine 5' phosphosulfate. This ATP in turn acts as fuel to the luciferase-mediated conversion of luciferin to oxyluciferin that generates visible light in amounts that are proportional to the amount of ATP. The light produced in the luciferase-catalyzed reaction is detected by a camera and analyzed with help of a software i.e. it is seen as peak in the raw data output. The height of each peak is proportional to the number of nucleotides incorporated.
4. Unincorporated nucleotides and ATP are degraded by the apyrase, and the reaction can restart with another nucleotide.

Since the addition of dNTPs is performed sequentially the sequence of the complementary strand can be determined.

As the DNA polymerase can add free nucleotides only to the 3' end of the newly-forming strand, the elongation and hence the sequencing will proceed in 5'-3' direction. Due to the preparation of the single stranded DNA using the biotin labelled primer as described above only one primer, termed sequencing primer, is needed for starting the sequencing reaction in step 1 of the protocol.

In this way only one strand is generated, in contrast to conventional PCR where two primers that are complementary to the 3' ends of each of the sense and anti-sense strand of the DNA target are added, generating both the sense and the antisense strand i.e. two strands in each cycle.

Pyrosequencing has several advantages over conventional sequencing methods particularly the Sanger sequencing method. For one the inventors could show in linearity tests that the assay is sensitive enough to detect a mutation if only 5% of cells of a sample where carrying the mutation. This is a sensitivity unreached by comparable studies using the Sanger sequencing method (Ogino et al. 2005). Furthermore using their method the inventors could analyse a multitude of possible mutations variants in a mutation hotspot. Whenever such a multitude of possible mutations exists, it is especially important to have a method, which works reliably with as little material as possible, since the quantity of biological material available for testing is almost always limited. In addition using the method according to the invention has the great advantage that only one analysis method - pyrosequencing - is needed to assess all relevant mutations affecting the response to an anti-cancer treatment like an anti-EGFR treatment. In methods known from the art often different analysis methods are used (in parallel), which adds to the cost, the possible error sources and reduces the speed of analysis.

Moreover the pyrosequencing results of the inventors were highly reproducible and quantifiable, while the whole process was very cost-effective, fast, and flexible regarding the number of samples.

Thus the method according to the invention is extremely well suited for a clinical setting where the response of a patient suffering from or at risk of developing a neoplastic disease to a given anti-cancer treatment, like a given anti-EGFR treatment, needs to be predicted accurately and correctly, but as quickly as possible in order to reach a decision as to which anti-cancer treatment strategy is the most promising one for that patient.

In a preferred embodiment the anti-cancer treatment is an anti-EGFR treatment.

As used herein, the term "anti-EGFR treatment" refers to a treatment that inhibits or stops an over activation of the EGFR and/or its signalling cascade in order to arrest neoplastic i.e. tumor proliferation and prevent formation of metastases. Preferably the "anti-EGFR treatment" even eliminates the mutated cells.

The advantages of the method according to the invention in cases of anti-EGFR treatment are especially:
- In most cases it was not only possible to determine whether a mutation was present or not, which is the conclusion required by clinicians to decide whether an anti-cancer treatment like anti-EGFR treatment is suitable for a patent, but it was also possible to determine the specific variant.
- Cases which were unclear following Sanger sequencing could be unambiguously genotyped and could be proven in a second test using fragment analysis for deletions.
- The linearity tests showed for all samples containing at least 5% mutated cells the difference between wild-type and mutated cells.
- For the deletion in the EGFR gene in exon 19 a further, so far unknown, mutation
   variant was found in one cell line and one tumor sample.

In a preferred embodiment the anti-EGFR treatment is at least one treatment selected from the group consisting of
- EGFR antagonist treatment,
- EGFR-tyrosine kinase inhibitor (EGFR-TKI) treatment,
- EGFR ligand inhibitors,
- RNA interference, e.g. microRNA or small interfering RNA
- antisense nucleotide treatment to reduce expression of EGFR, and/or
- anti-EGFR-based immunoconjugates.

As used herein the term "EGFR antagonist" refers to entities which bind to the EGFR without evoking a receptor response. Such antagonist can be, for example, EGFR specific antibodies, like cetuximab (Erbitux), panitumumab (Vectibix), Matuzumab/EMD72000 and/or ABX-EGF. By binding the EGFR receptor these entities thus at the least prevent any signalling from the EGFR as the binding site occupied by them cannot bind to other entities that might elicit a downstream signalling cascade that plays a role in neoplastics disease progression. Moreover some EGFR antagonist also seem to lead to a down-regulation of EGFR expression.

As used herein the term "EGFR-tyrosine kinase inhibitor" in most cases refers to small molecular drugs, which intracellularly bind to the tyrosine kinase domain of EGFR. Such inhibitors are, for example, gefitinib/ZD1839 (Iressa) or erlotinib/OSI-774 (Tarceva). As one of the first critical steps in EGFR signaling is the activation of intracellular tyrosine kinases, blocking this kinase activation effectively blocks the down-stream signaling cascade usually initiated when the EGFR is stimulated. Thus EGFR-tyrosine kinase inhibitors ultimately achieve the same effect as EGFR antagonists.

As used herein the term "EGFR ligand inhibitors" refers to entities which bind to EGFR ligands, like epidermal growth factor (EGF) or transforming growth factor α (TGFα). By binding the EGFR ligand and thus prohibiting the ligand from binding the EGFR, the ligand can no longer activate the EGFR. Potential candidates are for example anti-EGFR ligand antibodies e.g. antibodies directed against EGF, TGFα, HB-EGF, amphiregulin, betacellulin, epigen and epiregulin.

As used herein the term "anti-EGFR-based immunoconjugates" refers, for example, to antibody-based immunotoxins, immunoradionucleotides and immunoliposomes. These conjugates bind cells having an EGFR (wild-type and/or mutant) on their surface, and support the elimination, or apoptosis, of such cells.

Other preferred anti cancer treatments may involve
- anti-Integrin treatments, e.g. Integrin antagonist treatment (e.g. with the Integrin receptor antagonist Abciximab (ReoPro), Integrin ligand inhibitors, RNA interference, e.g. microRNA or small interfering RNA, antisense nucleotide treatment to reduce expression of Integrin, anti- Integrin-based immunoconjugates or small molecular drugs like Tirofiban and Eptifibatide, and/or
- anti Hedgehog treatments, e.g. Hedgehog antagonist treatment, Hedgehog ligand inhibitors, RNA interference, e.g. microRNA or small interfering RNA, antisense nucleotide treatment to reduce expression of Hedgehog, anti- Hedgehog-based immunoconjugates or small molecular drugs like Cyclopamine (11-deoxojervine),
as it has been shown that KRAS is also involved in EGFR-independent signalling pathways, e.g. the Hedgehog signalling pathway or the Integrin signalling pathway.

Other preferred anti cancer treatments relate to treatments interfering in cancer-related pathways in which KRAS is involved.

Therefore the preferred anti-cancer treatments, e.g. anti-EGFR treatments, all have the goal to circumvent effects that mutations affecting EGFR expression or activity have on neoplastics disease especially cancer development and progression i.e. the anti-EGFR treatments have the overall goal to inhibit DNA synthesis and cell proliferation and if possible/suitable even the destruction of the mutated cells.

In a preferred embodiment the at least one mutation affecting the response to anti-cancer treatments, e.g. anti-EGFR treatments, is a mutation in a gene selected from the group consisting of EGFR and/or KRAS.

The human EGFR gene is located on Chromosome 7 it has the SEQ ID NO: 1. Its longest isoform (transcript variant 1 of 12) has 5616 base pairs (bp) and is listed in GenBank under the sequence identifier NM_005228. Its cDNA has the SEQ ID NO: 2.

The human KRAS gene, on the other hand, is located on Chromosome 12. It has the SEQ ID NO: 3. Its longest isoform (transcript variant a) has 5436 bp and is listed in GenBank under the sequence identifier NM_033360. Its cDNA has the SEQ ID NO: 4. However, the predominant isoform of KRAS (transcript variant b) has only 5312bp and the GenBank sequence identifier NM_004985. Its cDNA has the SEQ ID NO: 5.

As described above mutations in the EGFR and/or KRAS genes have been correlated with the responsiveness towards anti-EGFR-treatment, therefore it is likely that, if the pattern of mutations in these genes is known for a given patient, the predicted therapy outcome of anti-EGFR-treatment will be consistent with the actual outcome.

In a preferred embodiment the at least one mutation affecting the response to anti-cancer treatments, e.g. anti-EGFR treatments, is a mutation in a position selected from the group consisting of:
- EGFR, exon19 (SEQ ID NO: 6),
- EGFR, exon 21 (SEQ ID NO: 7),
- EGFR, exon 20 (SEQ ID NO: 8),
- KRAS, exon 2 (SEQ ID NO: 9) and/or
- KRAS, exon 3 (SEQ ID NO: 10)

The protein sequence of EGFR corresponds to SEQ ID NO: 11, whereas the protein sequences of the two KRAS isoforms correspond to SEQ ID NO: 12 (isoform a) and SEQ ID NO: 13 (isoform b), respectively.

These sequences are especially preferred, since it is known that they contain regions with possible mutations that play a role in anti-cancer treatment success, e.g. anti-EGFR treatment success. For example on the EGFR exon 19 alone, 18 possible deletion variations, which are relevant to anti-cancer treatment outcome, have been described up to this point.

In a preferred embodiment at least one mutation affecting the response to anti-cancer treatment is a mutation selected from the group consisting of
- single nucleotide polymorphism (SNP),
- single or multiple nucleotide substitutions,
- single or multiple nucleotide deletions, and/or
- single or multiple nucleotide insertions.

In a further preferred embodiment the biological sample is at least one sample selected from the group consisting of
- fresh sample,
- frozen sample and/or
- fixed sample.

Fresh biological samples in the meaning of the present invention are samples that have been taken so recently from a patient that no methods of conservation were needed prior to analysis with the method according to the invention. However, fresh samples might have been stored temporarily in some way e.g. on ice.

Frozen samples, on the other hand, were frozen down prior to analysis with the method according to the invention. In the case of frozen samples the whole biological sample or parts of it are frozen, optionally using protective medium. Moreover it is possible that the biological sample or parts of were processed in some way e.g. DNA extraction, protein isolation etc. prior to freezing.

The term "fixed sample" as used herein refers to a chemical process by which the biological sample tissue is preserved from decay in order to keep it as close to its natural state as possible. There are several methods using different substances known in the art for achieving fixation of a sample for example alcohols, especially ethanol and methanol, acetic acid, oxidizing agents, such as potassium permanganate, mercurials such as B-5, Hepes-glutamic acid buffer and picrates, such as formalin and paraformaldehyde.

In another preferred embodiment at least one gene section of interest is amplified by means of a nucleic acid amplification technique prior to the pyrosequencing step.

By carefully choosing the gene section to be amplified, it is possible to directly target the regions, in which mutations are most likely to occur and hence those regions which are most relevant in the pyrosequencing step. In other words the selection of the correct primers for nucleic acid amplification is very important, as it determines, which gene section is pyrosequenced later on. In case unsuitable primers are used, the amplicons produced may turn out useless for the subsequent detection of the mutations of interest.

In a preferred embodiment the nucleic acid amplification technique is a polymerase chain reaction technique (PCR).

In molecular biology, the polymerase chain reaction (PCR) is a technique to amplify a single or few copies of a piece of nucleic acid across several orders of magnitude, generating thousands to millions of copies of a particular nucleic acid sequence. Numerous variations of the basic PCR technique including allel-specific PCR, assembly PCR, asymmetric PCR, hot-start PCR, inverse PCR, ligation-mediated PCR, methylation specific PCR, multiplex ligation PCR, nested PCR, quantitative PCR, RT-PCR, single-cell PCR and TAIL-PCR are well known in the art.

In a preferred embodiment the neoplastic disease is at least one selected from the group consisting of
- non small cell lung cancer (NSCLC),
- metastatic colorectal cancer,
- head and neck cancer,
- pancreatic cancer,
- prostate cancer,
- ovarial cancer,
- breast cancer (BRC) and/or
- hepatocellular cancer (HCC).

All of these cancers are though or have been demonstrated to show a response to anti-EGFR-treatment and thus are especially relevant for the method according to the invention. However, should other caners e.g. leukemia show a response to anti-EGFR-treatment in the future the inventive method can also be used to predict the response of a patient (probably) suffering from this additional neoplastic disease to anti-EGFR-treatment.

In an alternative embodiment the invention relates to a nucleic acid molecule, selected from the group consisting of
a) the nucleic acid molecule presented as SEQ ID NO: 14-28
b) a nucleic acid molecule comprising the nucleic acid molecule presented as SEQ ID NO: 14-28
c) a nucleic acid molecule that is capable of hybridizing to any of the nucleic acid molecules of a) - b) under stringent conditions, and/or
d) a complement of any of the nucleic acid molecules of a) - c).

As used herein the term ,,nucleic acid molecule" refers to a macromolecule composed of chains of monomeric nucleotides e.g. in DNA, the purine bases are adenine and guanine, while the pyrimidines are thymine and cytosine. RNA uses uracil in place of thymin. The most common nucleic acids are deoxyribonucleic acid (DNA) and ribonucleic acid (RNA). Artificial nucleic acids include peptide nucleic acid (PNA), Morpholino and locked nucleic acid (LNA), as well as glycol nucleic acid (GNA) and threose nucleic acid (TNA). Each of these is distinguished from naturally-occurring DNA or RNA by changes to the backbone of the molecule.

As used herein, the term "complement" refers to the complementary nucleic acid of the used/known/discussed nucleic acid. This is an important concept since in molecular biology, complementarity is a property of double-stranded nucleic acids such as DNA and RNA as well as DNA:RNA duplexes. Each strand is complementary to the other in that the base pairs between them are non-covalently connected via two or three hydrogen bonds. Since there is in principle - exceptions apply for thymine/uracil and the tRNA wobble conformation - only one complementary base for any of the bases found in nucleic acids, one can reconstruct a complementary strand for any single strand. This is essential for DNA replication. For example, the complementary strand of the DNA sequence
5' A G T C A T G 3'
is
3' T C A G T A C 5'

However, for double stranded DNA the term "complement" can also refer to the complementary DNA (cDNA). cDNA can be synthesized from a mature mRNA template in a reaction catalyzed by the enzyme reverse transcriptase.

Sequences SEQ ID NO: 14-28 relate to oligonucleotide molecules, preferably primers for nucleic acid amplification or for providing a sequence-specific 3' terminus, which serves as initiation point for the pyrosequencing process, i.e., a pyrosequencing primer (see table 2).

In a preferred embodiment the nucleic acid is selected from the group consisting of DNA, RNA, PNA, LNA, GDA und TNA and/or Morpholino.

As discussed above the most common nucleic acids are deoxyribonucleic acid (DNA) and ribonucleic acid (RNA). A DNA backbone is made from alternating phosphate and 2-deoxyribose, whereas the sugar in an RNA molecule is ribose.

Peptide nucleic acid (PNA) on the other hand is an artificially synthesized polymer similar to DNA or RNA, whose backbone is composed of repeating N-(2-aminoethyl)-glycine units linked by peptide bonds.

Furthermore a locked nucleic acid (LNA) is an altered RNA nucleotide, whose ribose is modified with an extra bridge connecting the 2' and 4' carbons. The bridge "locks" the ribose in the 3'-endo structural conformation, which is often found in the A-form of DNA or RNA. LNA nucleotides can be mixed with DNA or RNA bases whenever desired.

In addition glycol nucleic acid (GNA) is a polymer similar to DNA or RNA but differing in the composition of its backbone, as GNA's backbone is composed of repeating glycerol units linked by phosphodiester bonds. The glycerol molecule has just three carbon atoms and still shows Watson-Crick base pairing.

Besides threose nucleic acid (TNA) differs from DNA and RNA as it does not have a deoxyribose or ribose sugar backbone, but a backbone composed of repeating threose units linked by phosphodiester bonds. The threose molecule is easier to assemble than ribose.

Moreover the structural difference between Morpholinos and DNA is that while Morpholinos have standard nucleic acid bases, those bases are bound to morpholine rings instead of deoxyribose rings and linked through phosphorodiamidate groups instead of phosphates.

In a preferred embodiment of the inventive method at least one nucleic acid selected from the group consisting of
a) the nucleic acid molecule presented as SEQ ID NO: 14-23
b) a nucleic acid molecule comprising the nucleic acid molecule presented as SEQ ID NO: 14-23
c) a nucleic acid molecule that is capable of hybridizing to any of the nucleic acid molecules of a) - b) under stringent conditions, and/or
d) a complement of any of the nucleic acid molecules of a) - c)
is used as primer for nucleic acid amplification prior to the pyrosequencing step.

As used herein the term "primer" refers to a strand of nucleic acid that serves as a starting point for an enzyme e.g. DNA polymerase. They are required because the enzymes that catalyze nucleic acid elongation, can only add new nucleotides to an existing strand of nucleic acid. The polymerase starts elongation at the 3'-end of the primer, and copies the opposite strand. Most laboratory techniques of biochemistry and molecular biology that involve nucleic acid elongation use primers that are short, chemically synthesized oligonucleotides, with a length of about twenty bases.

The inventors of the present invention have surprisingly found that the amplification primers, whose core sequences are set forth under Sequences SEQ ID NO: 14-23 (see table 2), are best suited for the above requirements, especially the detection of the mutations of interest. This is the case, as with these primers help amplicons can be produced, which allow a sensitive and reproducible detection of the mutations of interest in the subsequent pyrosequencing step.

In a preferred embodiment at least one nucleic acid selected from the group consisting of
a) the nucleic acid molecule presented as SEQ ID NO: 24-28
b) a nucleic acid molecule comprising the nucleic acid molecule presented as SEQ ID NO: 24-28
c) a nucleic acid molecule that is capable of hybridizing to any of the nucleic acid molecules of a) - b) under stringent conditions, and/or
d) a complement of any of the nucleic acid molecules of a) - c)
is used as pyrosequencing primer.

As described above only one primer is needed for the pyrosequencing reaction. Thus in the context of this invention the term "primer for nucleic acid amplification" always refers to a primer pair as it is known in the art for nucleic acid amplification.

The term "pyrosequencing primer", on the other hand, refers to the single primer needed for initiating the pyrosequencing reaction. This primer provides a sequence-specific 3' terminus, which serves as initiation point for the pyrosequencing process, which runs in 5'-3' direction. The sequence of a pyrosequencing primer should therefore be designed in such way that it is within short distance of the 5' end of the mutation, or mutation hot spot, in order to facilitate the pyrosequencing process.

The inventors of the present invention have surprisingly found that the pyrosequencing primers having, as core sequences, SEQ ID NO: 24-28 (see table 2) are best suited for the above requirements, since with their help a sensitive and reproducible detection of the mutations of interest can be achieved.

It is to be understood that, instead of the primers mentioned above, the reverse complements can be used as well.

The term "reverse complement" as used herein refers to the complement of a nucleic acid sequence but in reverse order. This type of depicting a given nucleic acid sequence is used in order to keep the 5' and 3' ends properly oriented (see example below):

| | |
|---|---|
| Original Sequence | 5 ' ATGCAGGGGAAT 3 ' |
| Complement | 3' TACGTCCCCTTT 5' |
| (Pairs with original sequence) | |
| Reverse Complement | 5' TTTCCCCTGCAT 3' |
| (Complement written 5' to 3') | |

While it is easy to manually find out the reverse complement with short sequences, for longer sequences computer programs are available.

In an alternative embodiment the invention relates to a nucleotide dispensing order, selected from the group consisting of at least one nucleotide dispensing order presented as SEQ ID NO: 29-34.

As used herein the term "nucleotide" refers to a molecule composed of a nucleobase (nitrogenous base), a five-carbon sugar and one to three phosphate groups. Together the nucleobase and sugar comprise a nucleoside. The phosphate groups form bonds with either the 2, 3, or 5-carbon of the sugar, with the 5-carbon site most common.

Thus the term "nucleotide" also refers to nucleotide triphosphate (NTP). Natural nucleotide triphosphates include adenosine triphosphate (ATP), guanosine triphosphate (GTP), cytidine triphosphate (CTP), thymidine triphosphate (TTP) and uridine triphosphate (UTP). The nucleotide triphosphates containing deoxyribose take the prefix deoxy- in their names and small d- in their abbreviations: deoxyadenosine triphosphate (dATP), deoxyguanosine triphosphate (dGTP), deoxycytidine triphosphate (dCTP), deoxythimidine triphosphate (dTTP) and deoxyuridine triphosphate.

As used herein the term "nucleotide dispensing order" refers to a sequential manner in which nucleotides are added during the pyrosequencing reaction in order to determine the sequence of the complementary strand, and hence the presence or absence of at least one mutation affecting the response to anti-cancer treatment, e.g. anti-EGFR treatment.

It should be understood that, in contrast to the sequences given for the preferred pyrosequencing primers, the nucleotide dispensing order is not an oligonucleotide molecule i.e. a short nucleic acid polymer that can bind to a complementary strand of nucleic acid. On the contrary it is an order, in which the individual nucleotides are added during the pyrosequencing reaction, but the individual nucleotides do not form a separate molecule but are used individually by the enzyme for synthesizing the new strand of nucleic acid.

In a further preferred embodiment of the method according to the invention at least one nucleotide dispensing order selected from the group presented as SEQ ID NO: 29-34 is used during pyrosequencing.

The inventors of the present invention have developed a number of nucleotide dispensing orders, which enable the simultaneous detection of different mutations in a number of mutation hotspots. These mutations might affect the response of a patient to anti-cancer treatment, e.g. anti-EGFR treatment. The nucleotide dispensing orders account at every time point during the pyrosequencing reaction for all known variants in a given region, thereby allowing for the determination of the presence or absence of a specific mutation from the pool of variants ideally in just one single pyrosequencing reaction.

Thus the inventive dispensing orders overcome the drawback of methods known in the art for genotyping a pool of variants, since in the known methods either a lot more pyrosequencing runs are necessary to identify a specific mutation variant or different methods have to be used simultaneously in order to identify a specific mutation. In contrast, in the method according to the invention, ideally just a single pyrosequencing run will determine whether a mutation exists and if yes which one of a pool of variants the specific mutation is.

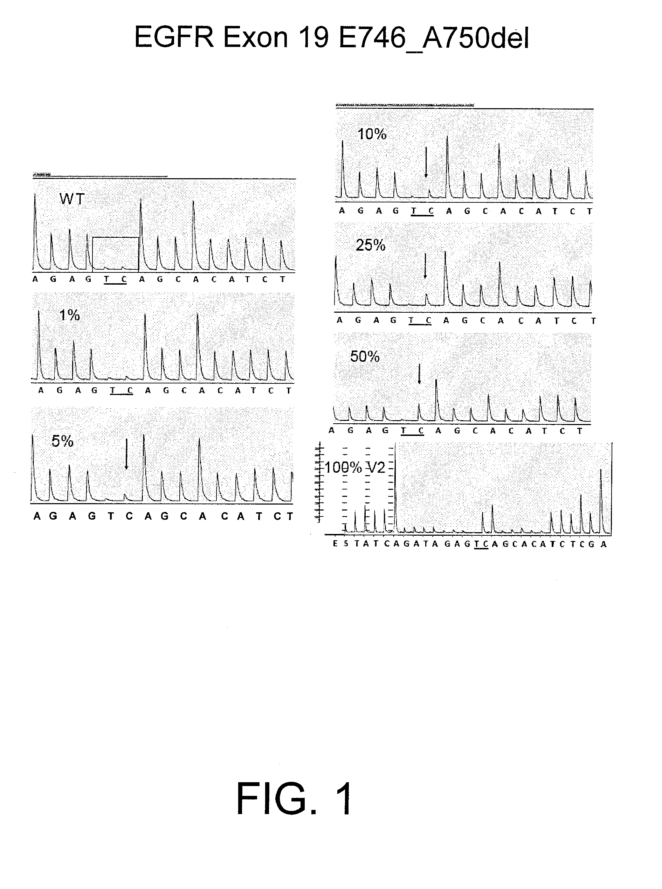

Furthermore, these dispensing orders allow detection of a mutation even when only a small number of mutated cells are present in a sample comprising predominantly wild-type cells e.g. up to 95 % see Fig.1.

In a further preferred embodiment of the method according to the invention the nucleic acid selected from the group consisting of
a) the nucleic acid molecule presented as SEQ ID NO: 14-28,
b) a nucleic acid molecule comprising the nucleic acid molecule presented as SEQ ID NO: 14-28,
c) a nucleic acid molecule that is capable of hybridizing to any of the nucleic acid molecules of a) - b) under stringent conditions, and/or
d) a complement of any of the nucleic acid molecules of a) - c).
that is used as primer for nucleic acid amplification prior to the pyrosequencing step or as pyrosequencing primer, is characterized in that it is labelled with at least one detectable marker.

As used herein the term "detectable marker" refers to a moiety that the nucleic acid can comprise in any way, which will not hamper the function of that nucleic acid, but which will allow detection of the nucleic acid. An especially preferred detectable marker is biotin. In the context of the present invention it is preferred that the reverse primer SED ID NO: 15, 17, 19, 21 and/or 23 employed in the nucleic acid amplification step is biotinylated.

In an alternative embodiment the invention is related to a pyrosequencing pipetting method in which different nucleotides are added to a pyrosequencing reaction mixture in at least one nucleotide dispensing order, wherein said nucleotide dispensing order comprises at least one sequence as set forth under SEQ ID NO: 29-34.

As already mentioned above it is important to understand that the said nucleotide dispensing orders relate to the sequential addition of nucleic acids, preferably deoxynucleitide triphosphates (dNTPs, namely dATP, dGTP, dTTP and dCTP), to the reaction mixture. These nucleotides are used by the enzyme e.g. DNA polymerase throughout the pyrosequencing procedure. It is furthermore important to understand that the said dispensing orders as recited under SEQ ID NO: 29-34 are not oligonucleotides, but, as mentioned earlier, indicate a dispensing order of nucleotides, preferably dNTPs. SEQ ID NO: 29-34 are thus in sharp contrast to SEQ ID NO: 14-28, which relate to oligonucleotides, preferably primers for nucleic acid amplification or for providing a sequence specific 3' terminus, which serves as initiation point for the pyrosequencing process.

The inventors have surprisingly found that the object of the present invention can in some cases be solved best with nucleotide dispensing orders termed "mutation-detection-insert".

As used herein, the term "mutation-detection-insert" refers to a nucleotide dispensing order in which nucleotides are added at a specific location in order to detect a mutation causing a deletion. The basic concept is that for pyrosequencing a gene section the sequence of which is roughly known, one would use a nucleotide dispensing order which reflects the expected nucleic acid sequence. The introduction of a "mutation-detection-insert", however, is for example accomplished by adding one or more nucleotides to a nucleotide dispensing order which has been designed to sequence, or detect, a wildtype. In case the sequenced gene section belongs to a wild type genotype, the respective pyrogram would not show any peak with respect to either of the two nucleotides in the dispensing order. In case the sequenced gene belongs to a mutated genotype, however, one or more peaks would appear.

Such mutation-detection-insert in a given nucleotide dispensing order can for example look as follows (insert underlined):
agatagagtcagcacatctcga

In order to differentiate a wildtype sequence for a deleted sequence, the following programs are obtained:
nucleotide dispensing order: a g a t a gagtca gca catctc ga
wildtype sequence: aaggaattaagag aagcaacatctccgaaagccaacaaggaaatc
pyrogram peak height: 202020202011100201120111112011
nucleotide dispensing order: agatagagtcagcacatctc ga
deleted variant: aaaaca tctccgaaagccaacaaggaaatc
pyrogram peak height: 00000000011000001112011

While upon dispension of the mutation mutation-detection-insert, no peaks are generated in the wildtype sequence, in the deleted variant a peak is generated once the C base of the mutation mutation-detection-insert is dispensed, while no signal ("flatline") is generated while the t base of the mutation mutation-detection-insert is dispensed. Therefore, it is easy to differentiate the mutant from the wildtype even in samples which have only a small portion of mutated cells, while the major sample fraction comprises wildtype cells.

In a preferred embodiment, said "mutation-detection-nucleotides which are introduced into the nucleotide dispensing order which has been designed to sequence, or detect, a wildtype.

The above idea is demonstrated, for example, in exon 19 of the EGFR gene, which is a region where at least 18 mutation variants are known. Here, two additional nucleotides are added at a specific point during the pyrosequencing reaction. The first nucleotide is in this case a thymidine triphosphate (dTTP). Since only one very infrequently occurring mutation requires a dTTP at this point, there will almost always be no peak in the raw data output, but the measured value is used as base line. The second nucleotide is in this case a cytidine triphosphate (dCTP). Again the wild-type sequence does not required this nucleotide triphosphate and hence gives no peak, however, all deletion variants require at least one cytidine triphosphate at this point of enzymatic chain elongation and hence a peak will be observed in the raw data output, if the level of mutated cells is ≥ 5% in the biological sample. Thus this approach enables in this specific example the distinction between the wild-type sequence and the mutations V1 to V18.

Furthermore the invention relates to a kit of primers, comprising at least one of the nucleic acids mentioned above.

The nucleic acids and dispensing orders according to preferred embodiments of the present invention are summarized in the following table:

**Table 2: Preferred oligonucleotide sequences and dispensing orders used according to the present invention. Note that there is a fundamental difference between the two sequence types. The dispensing order 1 of E746_A750del represents a "mutation-detection-insert" dispensing order.**

| **gene/exon [mutation example)** | **Type** | **NA sequence** | **SEQ ID NO** |
|---|---|---|---|
| EGFR, exon19 [E746_A750del] | forward PCR primer | taaaattcccgtcgctatcaa | 14 |
| | reverse PCR primer | agctgccagacatgagaaaag | 15 |
| | pyrosequencing primer | ttcccgtcgctatcaa | 24 |
| | dispensing order 1 | agatagag**tc**agcacatctcga | 29 |
| | dispensing order 2 | agatagagagcacatctcga | 30 |
| EGFR, exon 21 [L858R, L861Q] | forward PCR primer | aaacaccgcagcatgtcaa | 16 |
| | reverse PCR primer | tctttctcttccgcaccca | 17 |
| | pyrosequencing primer | catgtcaagatcacagatt | 25 |
| | dispensing order | ctgcgtgtcactacg | 31 |
| EGFR exon 20 [T790M] | forward PCR primer | ctgggcatctgcctcacct | 18 |
| | reverse PCR primer | tgtgttcccggacatagtcca | 19 |
| | pyrosequencing primer | accgtgcagctcatca | 26 |
| | dispensing order | actagcagc | 32 |
| KRAS exon 2 [G12S] | forward PCR primer | ctgaatataaacttgtggtagttg | 20 |
| | reverse PCR primer | tgtatcgtcaaggcactct | 21 |
| | pyrosequencing primer | aaacttgtggtagttgga | 27 |
| | dispensing order | gctgacgatgagtcgta | 33 |
| KRAS exon 3 [Q61H/K/L/R] | forward PCR primer | gatggagaaacctgtctcttgg | 22 |
| | reverse PCR primer | ccctccccagtcctcatgta | 23 |
| | pyrosequencing primer | tctcgacacagcaggt | 28 |
| | dispensing order | cgatgcatgag | 34 |

### DISCUSSION OF THE FIGURES

Fig. 1 shows parts of the linearity test diagrams for the hot-spot region in exon 19 of the EGFR and the mutation variant V2 (a deletion). As dispensing order of nucleotides the "mutation-detection-insert" dispensing order, i.e. agatagagtcagcacatctcga was used, in which the underlined nucleotides t and c represent the said mutation-detection-insert. Most diagrams do not show the first four nucleotides, i.e., only the section agagtcagcacatctcga is shown. The wild-type DNA can be distinguished from the DNA of mutated cells in that no peak is observed at the nucleotide dCTP, i.e., at nucleotide 10 of the "mutation-detection-insert"dispensing order of nucleotides.

In the first diagram no mutant DNA is present in the sample. Hence no peak is detected at the first dCTP of the dispensing order of nucleotides. In the second diagram 1% of mutant DNA is present. However, this ratio is too low to be detected, thus there is still no peak detected at the first dCTP of the dispensing order of nucleotides. However, as soon as 5% of mutant DNA is present (diagram 3) a peak at the first dCTP of the dispensing order of nucleotides can be detected. With each subsequent increase in the ratio of mutant to wild-type DNA the peak height increases in a linear fashion, as shown in the other diagrams.

Thus using the inventive method it is very easy to distinguish between samples containing only wild-type DNA and those containing mutant DNA, if at least 5% mutant DNA is present in the sample.

The "es" indicates the start of the dispensing order of nucleotides.

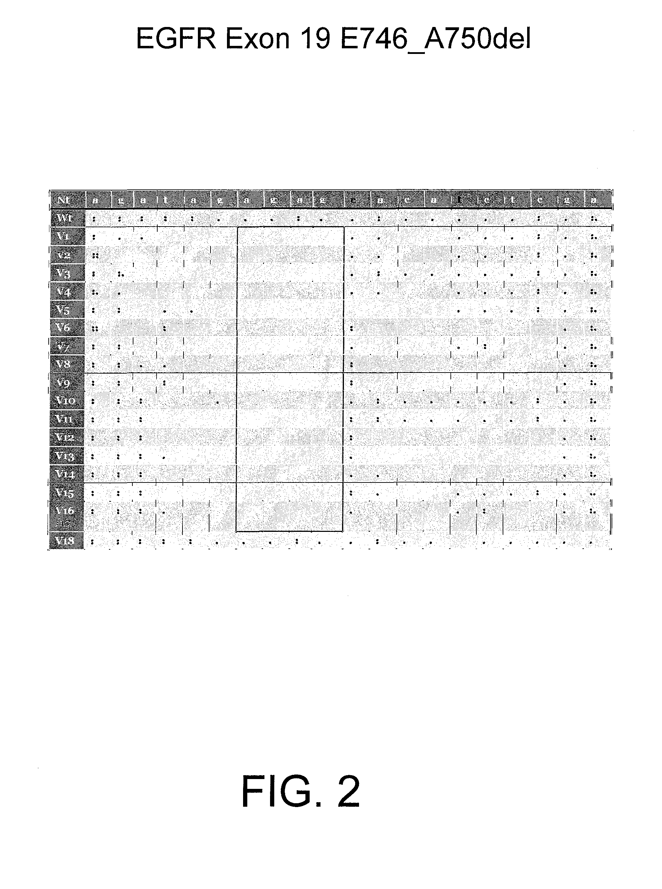

Fig 2 shows a summary of the frequently occurring deletions in exon 19 of the EGFR, wherein WT is the wild-type and V1 - V18 are known deletions.

In the top row of the diagram a conventional dispensing order of nucleotides is shown. Marked by the dots in the shaded areas are the signals that the various mutants give in a pyrosequencing diagram when using the shown dispensing order of nucleotides.

For example when comparing Fig. 2 to Table 3 of the present text - the dispensing order of nucleotides start at the first dATP of the wild-type sequence - it become clear, that all sequences require at least two dATPs as first nucleotides for chain elongation in the pyrosequencing reaction. Therefore all sequences will not only give a peak but the peak will be at least twice as high as a "normal" peak, since two dATPs are incorporated and not only one. This is symbolized with two dots. Moreover some sequences even incorporate more than two dATPs i.e. V2 incorporates four dATPs, V4 incorporates three dATPs and V6 incorporates four dATPs. Thus the peaks of these mutants will be even higher and hence are symbolized by three and four dots, respectively.

In the next round of the pyrosequencing reaction a dGTP is added. The wild-type sequence requires two dGTPs at this point and hence will again give a higher than normal peak, symbolized with two dots.

The frequent deletion V1, on the other hand, only requires one dGTP, therefore its peak will only be of normal height and thus is symbolized with one dot. The other frequent deletion V2 does not require a dGTP at all (its waiting for a dCTP, see table 3) and hence gives no signal.

In the case of the third nucleotide added, the dATP, the wild-type DNA again incorporates two dATPs as it requires a dATP at position 5 and 6 of the assayed sequence. Thus the wild-type sample will not only give a peak but the peak will be twice as high as a "normal" peak, since two dATPs are incorporated and not only one. This is again symbolized with two dots.

The deletion V1, on the contrary, only requires one dATP, therefore its peak will only be of normal height and thus symbolized with one dot. The other frequent deletion V2 does not require a dATP at all (its still waiting for a dCTP, see table 3) and hence again gives no signal etc.

The box covering rows 7-10 of the dispensing order of nucleotides illustrates that all sequences except for V5 are waiting for a dCTP to continue the pyrosequencing reaction at these positions of the dispensing order of nucleotides and hence give no peaks.

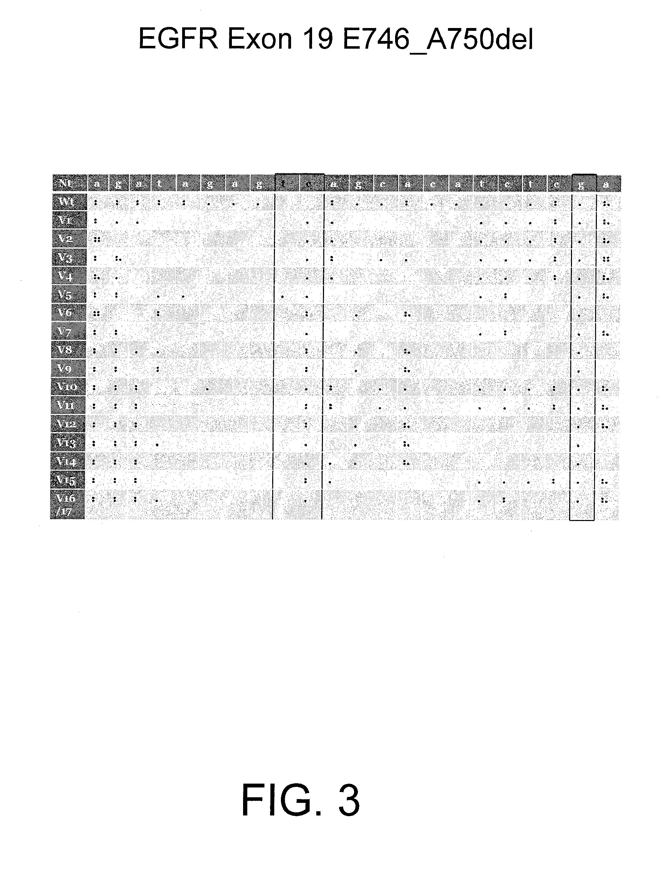

Fig 3 shows the same summary of the frequently occurring deletions in exon 19 of the EGFR as shown in Figure 2 above, except for V18. However, this time the dispensing order of nucleotides has been changed to the "mutation-detection-insert" dispensing order of nucleotides.

This means that between nucleotides 8 and 9 of the dispensing order of nucleotides of Fig. 2 two extra nucleotides are added (see the black box surrounding these rows). The first nucleotide, dTTP, can be used for calibrating the baseline of the pyrosequencing diagram, which is needed to distinguish the background signal from a real peak. This is the case as none of the possible sequences except V5 will generate a signal at this point of the dispensing order of nucleotides, if a dTTP is added. Since V5 is rare, it will almost always be possible to calculate the baseline of the pyrosequencing diagram through adding a dTTP at this position. Through adding the next nucleotide, the dCTP, it is possible to distinguish between the wild-type sequence, that does not require a dCTP but a dATP, and hence gives not signal, and all deletions, since all deletions require at least one dCTP and hence show a peak.

As the baseline of the pyrosequencing diagram was calculated one base before at the dTTP, it is even possible to detect a mutation when only 5% of the cells in a sample carry the mutation, Therefore calibrating the baseline of the pyrosequencing diagram is crucial. Moreover the second last nucleotide of the dispensing order of nucleotides, the dGTP, can be used to normalize all values for a given pyrosequencing reaction. In this way it is possible to distinguish between all the mutations and the wild-type sequence in a single reaction.

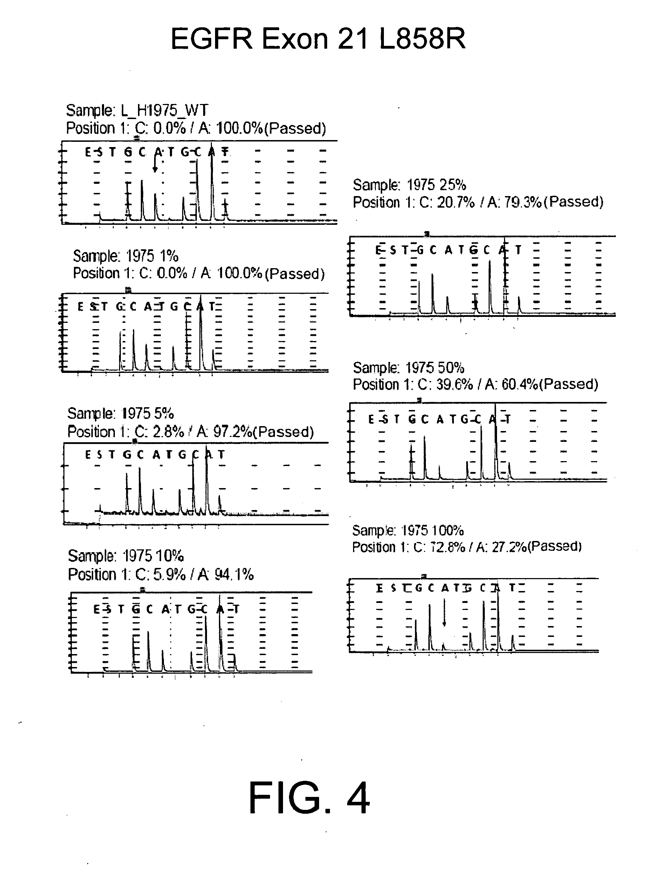

Fig. 4 shows linearity test diagrams for the fragment L858R in exon 21 of the EGFR. The samples were a wild-type cell line (L_H1975_WT) and a cell line (1975) containing mutated cells. The dispensing order of nucleotides used in this example was: tgcatgcat. The first diagram shows the pyrosequencing result for the wild-type cells. In the following diagrams a mixture of wild-type DNA and mutated DNA was assayed. The percentage of mutant cell DNA is given for each diagram behind the sample specification e.g. "Sample: 1975 1%" indicates that 1% mutant cell DNA is present in the sample. The term "position" refers to the nucleotides of the nucleotide dispensing order, whose relative peak high is monitored in the shaded area of the diagram. So for diagram 1, where a 100% wild-type DNA is present the nucleotide dATP gives a 100% signal, since the wild-type sequence requires a dATP at this point. When a mixture of 99% wild-type and 1% mutant DNA is assayed, the peak height of the dATP and hence the pyrosequencing result will not change, since too little mutated material is present to show an effect.

However, in the next diagram, where 5% mutated DNA is present an effect is visible and the relative height of the dATP peak decreases to 97,2%, thus indicating that mutated DNA was included in the sample. As the ratio of mutated DNA in the sample rises which each following diagram, the peak height generated by the dATP of the nucleotide dispensing order decreases.

This decline is linear in respect to the change in ratio of wild-type to mutant DNA.

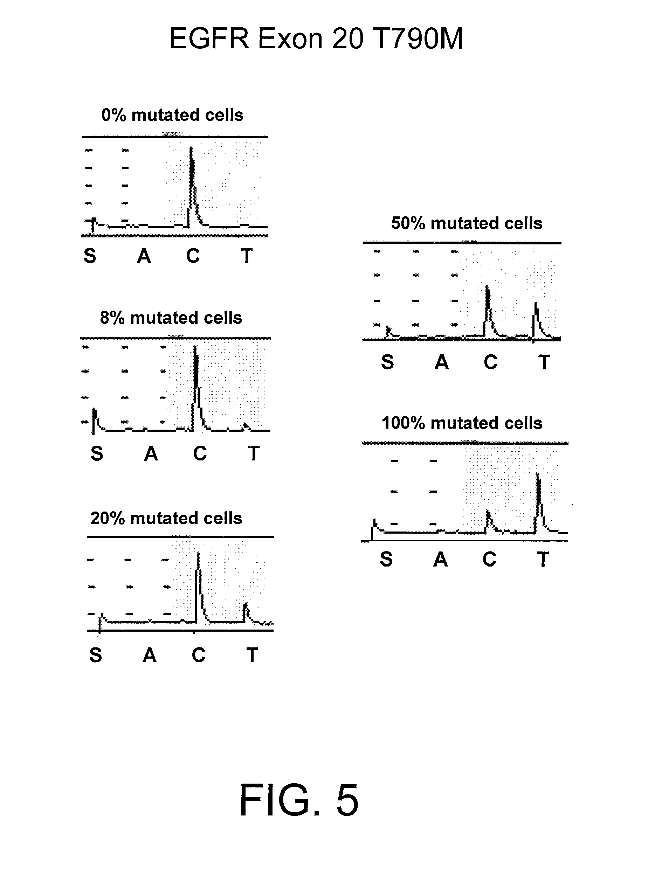

Fig. 5 shows parts of some of linearity test diagrams for the mutation T790M in exon 20 of the EGFR. The samples were a wild-type cell line and a cell line containing mutated cells. A part of the dispensing order of nucleotides used is shown in the graph i.e. act. The "s" marks the start of the pyrosequencing reaction.

The first diagram shows the pyrosequencing result for the wild-type cells. In the following diagrams a mixture of wild-type DNA and mutated DNA was assayed. The percentage of mutant cell DNA is given over each diagram.

So for diagram 1, where 100% wild-type DNA is present the nucleotide dCTP gives a 100% signal, since the wild-type sequence requires a dCTP at this point. The next nucleotide - a dTTP - however, gives no signal as the wild-type DNA does not require a dTTP at this point of the pyrosequencing reaction.

When, as shown in the second diagram, a mixture of 92% wild-type and 8% mutant DNA is assayed, an effect is visible and the relative height of the dCTP peak decreases to 93,2%, whereas the relative height of the dTTP peak increases to 6,8%. This indicates that mutated DNA was included in the sample. As the ratio of mutated DNA in the sample rises with each following diagram, the peak height generated by the dCTP of the nucleotide dispensing order decreases and the height of the dTTP peak increases.

The decrease and increase, respectively, are linear compared to the change in ratio of wild-type to mutant DNA.

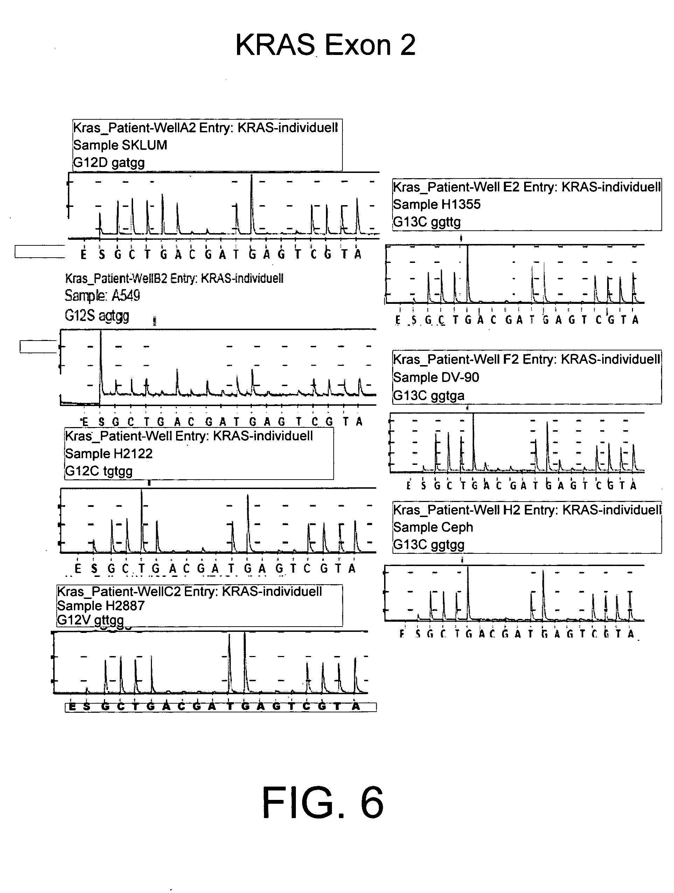

Fig. 6 shows diagrams of pyrosequencing results of several mutation variants in the hot-spot region of the KRAS gene in exon 2. Even if just a small percentage of cells carry the mutation a determination of the mutation is easily possible.

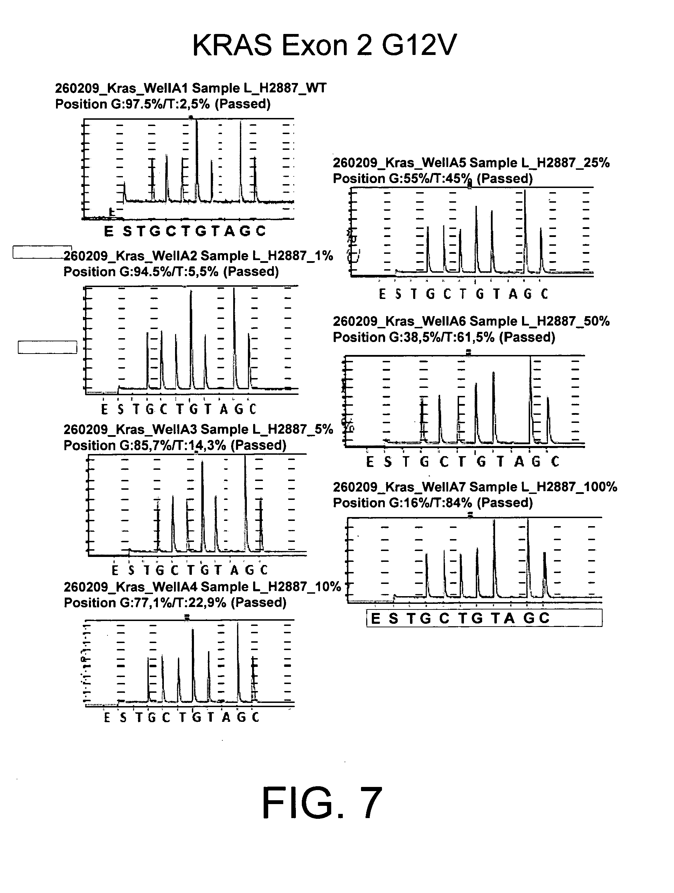

Fig. 7 shows linearity test diagrams for the mutation variant G12V in exon 2 of the KRAS gene. The samples were a wild-type cell line and a cell line containing mutated cells. A part of the used dispensing order of nucleotides is shown in the graph i.e. tgctgtagc. The "es" marks the start of the pyrosequencing reaction. The percentage of mutant cell DNA is given for each diagram behind the sample specification e.g. "L_H2887_1%" indicates that 1% mutant cell DNA is present in the sample. In the grey shaded area it can be clearly seen that as the percentage of mutated material in the sample increases, the peak height of the signal generated by adding the 3rd dTTP increases and the peak height of the signal generated by adding the 2^{nd} dGTP decreases in a linear fashion.

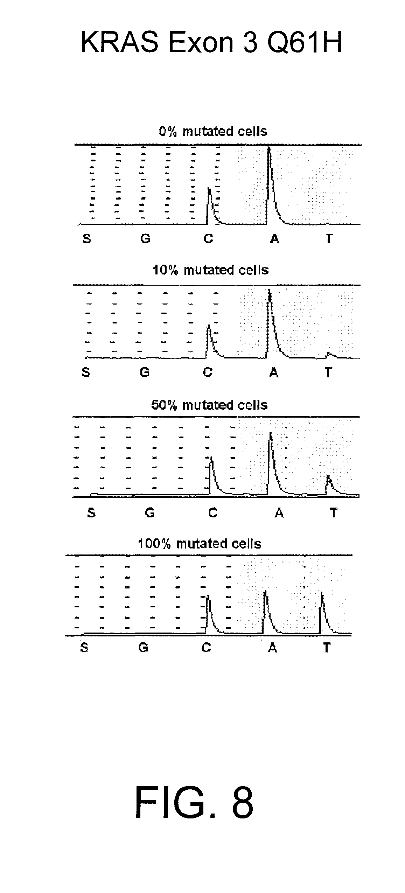

Fig. 8 shows parts of linearity test diagrams for the mutation variant Q61H in exon 3 of the KRAS gene. The samples were a wild-type cell line (H460) and a cell line containing mutated cells. A part of the dispensing order of nucleotides used is shown in the graph i.e. gcat . The "s" marks the start of the pyrosequencing reaction. The percentage of mutant cell DNA is given on top of each diagram. In the grey shaded area it can be clearly seen that as the percentage of mutated material in the sample increases, the peak height of the signal generated by adding a dTTP increases and the peak height of the signal generated by adding dATP decreases in a linear fashion.

### EXAMPLES

For each experiment a linearity test was carried out. In these test, mixtures of a PCR product of a sample containing 100% mutated cells and a PCR product of wild-type cells were employed usually in the following ratios: 0%, 1%, 5%, 10%, 25%, 50% and 100% PCR product of a sample containing 100% mutated cells. When a hot-spot containing multiple variants was assessed more than one linearity test was carried out.

The drawn conclusions were based on:
1) all genotypes known from Sanger sequencing could be verified
2) all wild-type samples e.g. negative controls from genomic DNA extraction from blood, were always classified as wild-type, even though blind testing was usually carried out.

### 1. The different assays

### 1.1. EGFR, exon19, deletion E746-A750

For this assay 7 patient samples with deletions, 9 patient samples with wild-type phenotype and 5 tumor cell lines with deletions were used.

The assay detects a variety of deletions in exon 19 (about 15 bp), which occur between the glutamic acid at position 746 of the EGFR protein and the alanine at position 750 of the EGFR protein. The relevant sequences of the respective mutation hot spot are as follows (the deletion area is underlined and indicated by an asterisk).

155341 catcttcgtt tgcttcttct agatcctctt tgcatgaaat ctgatttcag ttaggcctag
155401 acgcagcatc attaaattct ggatgaaatg atccacacgg actttataac aggctttaca
155461 agcttgagat tcttttatct aaataatcag tgtgattcgt ggagcccaac agctgcaggg
155521 ctgcgggggc gtcacagccc ccagcaatat cagccttagg tgcggctcca cagccccagt
155581 gtccctcacc ttcggggtgc atcgctggta acatccaccc agatcactgg gcagcatgtg
155641 gcaccatctc acaattgcca gttaacgtct tccttctctc tctgtcatag ggactctgga
155701 tcccagaagg tgagaaagtt aaaattcccg tcgctatcaa ggaattaaga gaagcaacat
155761 ctccgaaagc caacaaggaa atc* ctcgatg tgagtttctg ctttgctgtg tgggggtcca

155821 tggctctgaa cctcaggccc accttttctc atgtctggca gctgctctgc tctagaccct
155881 gctcatctcc acatcctaaa tgttcacttt ctatgtcttt ccctttctag ctctagtggg
155941 tataactccc tccccttaga gacagcactg gcctctccca tgctggtatc caccccaaaa

The following primers and dispensing orders were used:

| | |
|---|---|
| forward primer (bold print): | taaaattcccgtcgctatcaa |
| pyrosequencing primer (bold underlined): | ttcccgtcgctatcaa |
| reverse primer (bold italics): | cttttctcatgtctggcagct |
| dispensing order during pyrosequencing: | agatagag**tc**agcacatctcga or |
| | agatagagagcacatctcga |

It should be noted that the dispensing order listed first corresponds to the above described "mutation-dectection-insert" dispensing order of nucleotides in which nucleotides are added a specific location in order to detect a mutation causing a deletion. It is the same sequences as listed in table 2, wherein the position of the specific location is marked in bold.

As can be seen, forward primer and pyrosequencing primer have a large overlap. The following table summarizes frequently occurring deletions in the above mentioned deletion area, wherein WT is the wild-type and V1 and V2 are the most frequent deletions. Bold printed nucleotides are SNPs:

**Table 3: Deletions occurring in the hot-spot region E746_A750 in exon 19 of the EGFR.**

| **Variant** | **Sequence** | |
|---|---|---|
| Wt | aaggaattaaga gaagcaacat ctccgaaagc caacaaggaa atc | |
| V1 | aag | acat ctccgaaagc caacaaggaa atc |
| V2 | aa | aacat ctccgaaagc caacaaggaa atc |
| V3 | aagg | gcaacat ctccgaaagc caacaaggaa atc |
| V4 | aa | a**g**cat ctccgaaagc caacaaggaa atc |
| V5 | aagg | **t**at ctccgaaagc caacaaggaa atc |
| V6 | aa | a**a t**tccgaaagc caacaaggaa atc |
| V7 | aagg | ctccgaaagc caacaaggaa atc |
| V8 | aagga | tccgaaagc caacaaggaa atc |
| V9 | aagg | **t**tccgaaagc caacaaggaa atc |
| V10 | aaggaa | gcaacat ctccgaaagc caacaaggaa atc |
| V11 | aaggaa | **c**caacat ctccgaaagc caacaaggaa atc |
| V12 | aaggaa | ccgaaagc caacaaggaa atc |
| V13 | aaggaat | cgaaagc caacaaggaa atc |
| V14 | aaggaa | c**a**gaaagc caacaaggaa atc |
| V15 | aaggaa | **c**cat ctccgaaagc caacaaggaa atc |
| V16 | aaggaa | t ctccgaaagc caacaaggaa atc |
| V17 | aaggaat | ctccgaaagc caacaaggaa atc |
| V18 | aaggaattaaga gaagcaacat | |

Thus when sequencing this mutation hot-spot in exon 19 using the inventive method, one can determined which of the many possible variants is present in the sample. This is the case as only those mutations that still have a given nucleotide at a given position will give the DNA polymerase a template to elongate the complementary base by one nucleotide.

In order to do so the correct region was amplified by PCR using the forward and reverse primer given above. In the next step the matching pyrosequencing primer was employed. Then the actual pyrosequencing reaction was initiated. In this example, which corresponds to Fig. 3 the "mutation-detection-insert" nucleotide dispensing order (agatagag**tc**agcacatctcga) as described above was used. Hence a dATP was added as first base to the pyrosequencing reaction. Therefore in the case of all sequences (see table 3) the DNA polymerase was able to incorporate this dATP and hence a light signal was emitted.

In the second round of adding a nucleotide to the pyrosequencing reaction according to the "mutation-detection-insert" nucleotide dispensing order a dGTP was added. The wild-type sequence as well as all variants except mutants V2 and V6 required a dGTP at this point and generated a signal.

In the third round of adding a nucleotide to the pyrosequencing reaction according to the "mutation-detection-insert" nucleotide dispensing order a dATP was added. The wild-type sequence as well as mutations V1, V8, V10, V11, V12, V13, V14, V15, V16, V17 and V18 (see table 3) required a dATP at this point and generated a peak.

In the given example the next 5 rounds of adding a nucleotide to the pyrosequencing reaction according to the "mutation-detection-insert" dispensing order of nucleotides generated peaks when assaying the wild-type sequence, but did not generate peaks when assaying the frequent mutations V1 and V2. This was the case as the mutants require a dCTP but the mutation-detection-insert nucleotide dispensing order was tagag .

In order to distinguish clearly between a sample containing only wild-type DNA and one containing V 1 and/or V2 mutant DNA a dTTP was added in round nine of the "mutation-detection-insert" nucleotide dispensing order.

Since neither one of the mutations V1 or V2 nor the wild-type sequence required a dTTP at this point, there was no peak in the raw data output, but the measured value was used as base line (see Fig 1). Upon adding the tenth nucleotide, dCTP, the wild-type sequence again did not require this nucleotide triphosphate and hence gave no peak, however, the mutations V1 and V2 required a cytidine triphosphate at this point and hence a peak was observed in the raw data output, whenever the level of cells carrying the mutation was ≥ 5% in the biological sample. Thus this approach enabled in this specific example the distinction between samples containing only the wild-type sequence and those containing also the mutations V1 and V2.

### 1.2. EGFR, exon 21 L858R, L861Q

For this assay 1 patient sample with the L858R amino acid exchange mutation and multiple wild-type samples were used.

The mutation at base pair 858 of exon 21 leads to an amino acid exchange at protein level from arginine (R) to leucine (L) whereas the mutation at base pair 861 of exon 21 leads to an amino acid exchange on protein level from glutamine (Q) to leucine (L).

Thus the said SNPs cause either of the following amino acid substitutions:
839 AARNVLVKTPQHVKITDFG**L**AK**L**LGAEEKEYHAEGGKVPI
wherein the 2573 G SNP causes L858R and the 2582 A causes L861Q and the numbers 2573 and 2582 indicate the position of the SNP in the DNA sequence of exon 21 of the EGFR.

The relevant sequences of the respective mutation hot spot are as follows (the two relevant SNPs are indicated by an asterisk):

cccgccaccc cccaccccca ctttgcagat aaaccacatg caggaaggtc agcctggcaa
gtccagtaag ttcaagccca ggtctcaact gggcagcaga gctcctgctc ttctttgtcc
tcatatacga gcacctctgg acttaaaact tgaggaactg gatggagaaa agttaatggt
cagcagcggg ttacatcttc tttcatgcgc ctttccattc tttggatcag tagtcactaa
cgttcgccag ccataagtcc tcgacgtgga gaggctcaga gcctggcatg aacatgaccc
tgaattcgga tgcagagctt cttcccatga tgatctgtcc ctcacagcag ggtcttctct
gtttcagggc atgaactact tggaggaccg tcgcttggtg caccgcgacc tggcagccag
gaacgtactg gtgaaaacac cgcagcatgt caagatcaca gatttgggc t/g* ggccaaac t/a*
gctgggtgcg gaagagaaag aataccatgc agaaggaggc aaagtaagga ggtggcttta
ggtcagccag cattttcctg acaccaggga ccaggctgcc ttcccactag ctgtattgtt
taacacatgc aggggaggat gctctccaga cattctgggt gagctcgcag cagctgctgc
tggcagctgg gtccagccag ggtctcctgg tagtgtgagc cagagctgct ttgggaacag
tacttgctgg gacagtgaat gaggatgtta tccccaggtg atcattagca aatgttaggt
ttcagtctct ccctgcagga tatataagtc cccttcaata gcgcaattgg gaaaggtcac
agctgccttg gtggtccact gctgtcaagg acacctaagg aacaggaaag gccccatgcg
gacccgagct cccagggctg tctgtggctc gtggctggga caggcagcaa tggagtcctt

The following primers and dispensing orders were used:
forward primer (bold print) aaacaccgcagcatgtcaa
pyrosequencing primer (bold underlined): catgtcaagatcacagatt
reverse primer (bold italics): tctttctcttccgcaccca
dispensing order during pyrosequencing: ctgcgtgtcactacg

As can be seen, forward primer and pyrosequencing primer have a large overlap.

Thus when sequencing these mutations in exon 21 using the inventive method and especially the inventive nucleotide dispensing orders as described above in detail for example 1, one can determine, which if mutated DNA is present in the sample (see for example Fig. 4).

### 1.3. EGFR exon 20 T790M

For this assay 1 patient sample and multiple wild-type samples were used.

This assay detects mutation T790M in exon 20 of the EGFR. The relevant sequence of the respective mutation hot spot is as follows (the relevant SNP is indicated by an asterisk):

atcctcgatg aagcctacgt gatggccagc gtggacaacc cccacgtgtg ccgcctgctg
ggcatctgcc tcacctccac cgtgcagctc atca c/t* gcagc tcatgccctt cggctgcctc
ctggactatg tccgggaaca caaagacaat attggctccc agtacctgctcaactggtgt

The following primers and dispensing orders were used:

| | |
|---|---|
| forward primer (bold print) | ctgggcatctgcctcacct |
| pyrosequencing primer (bold underlined): | accgtgcagctcatca |
| reverse primer (bold italics): | tgtgttcccggacatagtcca |
| dispensing order during pyrosequencing: | actagcagc |

As can be seen, forward primer and pyrosequencing primer have no overlap.

Thus when sequencing this mutation in exon 20 using the inventive method and especially the inventive nucleotide dispensing orders as described above in detail for example 1, one can determine, whether the sample contains mutant DNA (see also Fig. 5).

### 1.4. KRAS exon 2, e.g. G12S

For this assay 8 tumor cells lines and a negative control were used.

This assay detects a variety of mutations in exon 2. One of these, for example, leads to the substitution of a glycine (G) by a serine (S), but a whole variety of substitutions at positions 12 and 13 of the amino acid sequence of the KRAS protein are known (see table 4). The relevant sequences of the respective mutation hot spot are as follows (the mutations are indicated by an asterisk):

5101 aataagagtg gagatagctg tcagcaactt ttgtgagggt gtgctacagg gtgtagagca
5161 ctgtgaagtc tctacatgag tgaagtcatg atatgatcct ttgagagcct ttagccgccg

5221 cagaacagca gtctggctat ttagatagaa caacttgatt ttaagataaa agaactgtct
5281 atgtagcatt tatgcatttt tcttaagcgt cgatggagga gtttgtaaat gaagtacagt
5341 tcattacgat acacgtctgc agtcaactgg aattttcatg attgaatttt gtaaggtatt
5401 ttgaaataat ttttcatata aaggtgagtt tgtattaaaa ggtactggtg gagtatttga
5461 tagtgtatta accttatgtg tgacatgttc taatatagtc acattttcat tatttttatt
5521 ataaggcctg ctgaaaatga ctgaatataa acttgtggta gttggagct
g/t/a/c* g/a/t/c* t g/t/a* g/a/t* cgtagg
5581 caagagtgcc ttgacgatac agctaattca gaatcatttt gtggacgaat atgatccaac
5641 aatagaggta aatcttgttt taatatgcat attactggtg caggaccatt ctttgataca
5701 gataaaggtt tctctgacca ttttcatgag tacttattac aagataatta tgctgaaagt
5761 taagttatct gaaatgtacc ttgggtttca agttatatgt aaccattaat atgggaactt
5821 tactttcctt gggagtatgt cagggtccat gatgttcact ctctgtgcat tttgattgga
5881 agtgtatttc agagtttcgt gagagggtag aaatttgtat cctatctgga cctaaaagac
5941 aatcttttta ttgtaacttt tatttttatg ggtttcttgg tattgtgaca tcatatgtaa
6001 aggttagatt taattgtact agtgaaatat aattgtttga tggttgattt ttttaaactt
6061 catcagcagt attttcctat cttcttctca acattagaga acctacaact accggataaa

The following primers and dispensing orders were used:

| | |
|---|---|
| forward primer (bold print) | ctgaatataaacttgtggtagttg |
| pyrosequencing primer (bold underlined): | aaacttgtggtagttgga |
| reverse primer | tgtatcgtcaaggcactct |
| dispensing order during pyrosequencing: | gctgacgatgagtcgta |

As can be seen, forward primer and pyrosequencing primer have a large overlap. The different SNPs and the resulting amino acid substitutions are shown in the following table:

**Table 4: Variety of substitutions at positions 12 and 13 of the amino acid sequence of the KRAS protein**

| **Variant** | **SNP** |
|---|---|
| Wt | none |
| G12A | 35 g<c |
| G12C | 34-36 ggt>tgc |
| G12D | 35 g>a |
| G12R | 34 g>c |
| G12S | 34 g>a |
| G12V | 35-36 gt>tc |
| G12L | 34-35 gg>cc |
| G12N | 34-35 gg>aa |
| G12E | 35-36 gt>aa |
| G12F | 34-35 tt |
| G12G | 36 t>a |
| G12Y | 34-35 gg>ta |
| G13A | 38 g>c |
| G13C | 37 g>t |
| G13D | 38-39 gc>at |
| G13G | 39 c>g |
| G131 | 37-38 gg>at |
| G13R | 37-39 ggc>cgt |
| G13S | 37 g>a |
| G13V | 38-39 gc>tg |

Thus when sequencing this mutation in exon 2 of the KRAS using the inventive method and especially the inventive nucleotide dispensing orders as described above in detail for example 1, one can determine whether cells in the given sample are mutated and if this is the case, which of the possible mutants is present in the sample (see for example Fig. 6 and 7). Brieftly, the nucleotide dispension order is: gctgacgatgagtcgta and in the fourth and fifth nucleotide of the nucleotide dispension order (dGTP and dATP) clear differences in peak presence or absence or peak height can be seen between the possible mutation variants, allowing for a distinction between them.

### 1.5.Kras Exon 3, Q61H/K/L/R

For this assay 3 tumor cell lines and a negative control were used.

This assay detects a variety of mutations in exon 3, wherein the wild-type amino acid glutamine (Q) is substituted with histidine, lysine, leucine, proline, glutamic acid or argenine. The relevant sequences of the respective mutation hot spot are as follows (the mutations are indicated by an asterisk):

gattcctaca ggaagcaagt agtaattgat ggagaaacct gtctcttgga tattctcgac
acagcaggt c/a/g* a/t/g/c* a/t* gaggagta cagtgcaatg agggaaggag tacagtgcaa
tgagggacca gtacatgagg actggggagg gctttctttg tgtatttgcc ataaataata
ctaaatcatt tgaagatatt caccattata g

The following primers and dispensing orders were used:

| | |
|---|---|
| forward primer (bold print) | gatggagaaacctgtctcttgg |
| pyrosequencing primer (bold underlined): | tctcgacacagcaggt |
| reverse primer | ccctccccagtcctcatgta |
| dispensing order during pyrosequencing: | cgatgcatgag |

As can be seen, forward primer and pyrosequencing primer have no overlap. The different SNPs and the resulting amino acid substitutions are shown in the following table 5, where for example g indicates that a the more common SNP is c and when the less common variant g is present the amino acid will change from glutamine (Q) to glutamic acid (E).

**Table 5: Variety of substitutions at position 61 of the amino acid sequence of the KRAS protein**

| **Variant** | **SNP** |
|---|---|
| WT | none |
| Q61E | 181 c>g |
| Q61H | 183 a>t |
| Q61K | 181 c>a |
| Q61L | 182 a>t |
| Q61P | 182 a>c |
| Q61R | 182 a>g |

Thus, just as in the examples discussed before, when sequencing this mutation in exon 3 of the KRAS gene using the inventive method and especially the inventive nucleotide dispensing order, as described above in detail for example 1, one can determine, whether cells are mutated and if this is the case, which of the possible mutants is present in a sample.

### REFERENCE

Ogino et al: Sensitive Sequencing Method for KRAS Mutation Detection by pyrosequencing. Journal of Molecular Diagnostics 2005, Vol 7(3), 413

## Claims

1. A method for predicting the response of a patient suffering from or at risk of developing a neoplastic disease towards a given therapy agent, said method comprising at least the steps of:
a) obtaining a biological sample from said patient;
b) determining, in said sample, the presence or absence of at least one mutation affecting the response to an anti-cancer treatment by means of pyrosequencing, and
c) predicting the therapeutic success of a given anti-cancer treatment.

2. The method according to claim 1, wherein the anti-cancer treatment is an anti-EGFR treatment.

3. The method according to claim 2, wherein the anti-EGFR treatment is at least one treatment selected from the group consisting of
• EGFR antagonist treatment,
• EGFR-tyrosine kinase inhibitor (EGFR-TKI) treatment,
• EGFR ligand inhibitors,
• RNA interference, e.g. microRNA or small interfering RNA
• antisense nucleotide treatment to reduce expression of EGFR, and/or
• anti-EGFR-based immunoconjugates.

4. The method according to any of claims 1-3, wherein the at least one mutation affecting the response to anti-cancer treatment is a mutation in a gene selected from the group consisting of EGFR and/or KRAS.

5. The method according to any of claims 1-4, wherein the at least one mutation affecting the response to anti-cancer treatment is a mutation in a position selected from the group consisting of:
• EGFR, exon 19,
• EGFR, exon 21,
• EGFR, exon 20,
• KRAS, exon 2, and/or
• KRAS, exon 3.

6. The method according to any of claims 1-5, wherein the neoplastic disease is at least one selected from the group consisting of
• non small cell lung cancer (NSCLC),
• metastatic colorectal cancer,
• head and neck cancer,
• pancreatic cancer,
• prostate cancer,
• ovarial cancer,
• breast cancer (BRC), and/or
• hepatocellular cancer (HCC).

7. A nucleic acid molecule, selected from the group consisting of
a) the nucleic acid molecule presented as SEQ ID NO: 14-28,
b) a nucleic acid molecule comprising the nucleic acid molecule presented as SEQ ID NO: 14-28
c) a nucleic acid molecule that is capable of hybridizing to any of the nucleic acid molecules of a) - b) under stringent conditions, and/or
d) a complement of any of the nucleic acid molecules of a) - c).

8. The nucleic acid according to claim 7, **characterized in that** the said nucleic acid is selected from the group consisting of DNA, RNA, PNA, LNA and/or Morpholino.

9. The method according to any of claims 1-6, wherein at least one nucleic acid according to claim 10 is used as primer for nucleic acid amplification.

10. The method according to any of claims 1-6, wherein at least one nucleic acid primer according to claim 10 is used as pyrosequencing primer.

11. A nucleotide dispensing order, selected from the group consisting of at least one nucleotide dispensing order presented as SEQ ID NO: 29 - 34.

12. The method according to any of claims 1-6, wherein at least one nucleotide dispensing order according to claim 14 is used during pyrosequencing.

13. A pyrosequencing pipetting method in which different nucleotide are added to a pyrosequencing reaction mixture in at least one sequential manner ("dispensing order"), wherein said sequential manner comprises at least one sequence as set forth under SEQ ID NO: 29-34.

14. A kit of primers, comprising at least one of the nucleic acids according to any of claims 10-13.

15. Method for the detection of at least one mutation in a given gene, or exon, by means of pyrosequencing, **characterized in that**, during pyrosequencing, a nucleotide dispensing order comprising a mutation-detection-insert is used.


SEQUENCE LISTING

<110>  University of Cologne / Provendis

<120>  Method for predicting the response of a patient using
       pyrosequencing

<130>  UD40196

<160>  34

<170>  PatentIn version 3.3

<210>  1
<211>  188307
<212>  DNA
<213>  Homo sapiens

<400>  1
ccccggcgca gcgcggccgc agcagcctcc gccccccgca cggtgtgagc gcccgacgcg      60

gccgaggcgg ccggagtccc gagctagccc cggcggccgc cgccgcccag accggacgac     120

aggccacctc gtcggcgtcc gcccgagtcc ccgcctcgcc gccaacgcca caaccaccgc     180

gcacggcccc ctgactccgt ccagtattga tcgggagagc cggagcgagc tcttcgggga     240

gcagcgatgc gaccctccgg gacggccggg gcagcgctcc tggcgctgct ggctgcgctc     300

tgcccggcga gtcgggctct ggaggaaaag aaaggtaagg gcgtgtctcg ccggctcccg     360

cgccgccccc ggatcgcgcc ccggaccccg cagcccgccc aaccgcgcac cggcgcaccg     420

gctcggcgcc cgcgcccccg cccgtccttt cctgtttcct tgagatcagc tgcgccgccg     480

accgggaccg cgggaggaac gggacgtttc gttcttcggc cgggagagtc tggggcgggc     540

ggaggaggag acgcgtggga caccgggctg caggccaggc ggggaacggc cgccgggacc     600

tccggcgccc cgaaccgctc ccaactttct tccctcactt tccccgccca gctgcgcagg     660

atcggcgtca gtgggcgaaa gccgggtgct ggtgggcgcc tggggccggg gtcccgcacg     720

tgcgccccgc gctgtcttcc cagggcgcga cggggtcctg gcgcgcaccc gaggggcggg     780

cgctgcccac ccgccgagac tgcactgttt agggaagctg aggaaggaac ccaaaaatac     840

agcctcccct cggaccccgc gggacaggcg gctttctgag aggacctccc cgcctccgcc     900

ctccgcgcag gtctcaaact gaagccggcg cccgccagcc tggccccggc ccctctccag     960

gtccccgcga tcctcgttcc ccagtgtgga gtcgcagcct cgacctggga gctgggagaa    1020

ctcgtctacc accacctgcg gctcccgggg aggggtggtg ctggcggcgg ttagtttcct    1080

cgttggcaaa aggcaggtgg ggtccgaccc gccccttggg cgcagacccc ggccgctcgc    1140

ctcgcccggt gcgccctcgt cttgcctatc caagagtgcc ccccacctcc cggggacccc    1200

agctccctcc tgggcgcccg cgccgaaagc cccaggctct ccttcgatgg ccgcctcgcg    1260

gagacgtccg ggtctgctcc acctgcagcc cttcggtcgc gcctgggctt cgcggtggag    1320

cgggacgcgg ctgtccggcc actgcagggg gggatcgcgg gactcttgag cggaagcccc    1380

ggaagcagag ctcatcctgg ccaacaccat ggtgtttcaa aatggggctc acagcaaact    1440

tctcctcaaa acccggagac tttctttctt ggatgtctct ttttgctgtt tgaagaattt    1500

gagccaacca aaatattaaa cctgtcttac acacacacac acacacacac acacacacac 1560
accggattgc tgtccctggt tcaagtgtgc caagtgtgca gacagaacat gagcgagtct 1620
ggcttcgtga ctaccgacca taaacccact tgacagggga aacatgcctt ggaaggttta 1680
attgcacaat tccaaccttg agctgcgcgg gttccaagag ccaggcccgt acttgctgtt 1740
gatgtcattg gcttggggag ttggggtttg gtgcccagcg cggtcgttgg gggaggggca 1800
aggcatagaa cagtggttcc cagaccttgc tgcacattgg aattacctgg gattaaaaaa 1860
aaaaaaatca aaacaaaaac cagtgtctgg ctcccgcccc cagacattct gatttaattg 1920
gcatggggca agacctggac ttgggatttt ttttaatgct cttcatgtga tctgttgggc 1980
agccagattt ggggatcact agacggaaga aggattgtta aagtctccgg agatgttact 2040
tgccaatgct aagagctctt tgaggacatc tggaattgtt acaatattgc caaatatagg 2100
aaagagggaa aaggtagagt gtgattccaa taataaagga ttccgctttt cattgaagga 2160
actggtggaa aggtttcttc tctgctgagc ctgcaggccc gtcctgcctg cctggggtgc 2220
ccgggagacg cgggcctgct ccggagactg ctgactgccg gtcctgttag tcaggtgtca 2280
gccctgtctc tgccgaagag actcttctct ttattttaaa ttaaaccctc agagcaccac 2340
caaagcatca cttttctccc tccattggtg ttctcattct ttgatgttac ttgtttgaac 2400
accactatta gtagttggag atttgttcct gagaaaaata taaataccac ttaatttgcc 2460
tgtttgtccc gcattcactc aaaacagaat gctcctgaag acaagagaga gagtaggaga 2520
acagacgcta ttccattaca gtaacataaa agactggatt ttcaggggca aattattaaa 2580
ataggagatg agctctttta acagaaattt gtttaaggcc tgtgtctatc aaattcagtg 2640
gattttattc aagatgcact ttgtttagtg ggagttttgt ttggttctgg gacatgctaa 2700
cttctagact tgctgctctt agaggtaatg actgccagac accatttcat gagtcctaat 2760
ccccacatta agcataagag gtgcacactc tcctcctatg ggggaaactg aggtacgaag 2820
aactaaagtg actttcccac agctggtggg aggcagacgg gaaattcaca ccaggggctt 2880
ccaactccag atccctctct caacttccaa actccactgc cttgtccgag ttctggtttc 2940
aggagatcca aatcaggtgt gtgcaaatgt ctaatgtcag agctggcaag gggaaagggc 3000
ccagggagcc ggctcatgac gatgagcctg tctgaagctt caacgcgggc tgtccggcag 3060
tctgcattcc tgccgagttc ctcagccctc tgttgggtca ccttccatag aggcagctta 3120
gtcctcagtt cagtgagcat ggagtggaga ctgcttgagg ggtgctgagc aaagccctgc 3180
ctcttacagg atgaaggtgc tctccagaag ggacactgga aagtattcca aggcgagtcg 3240
aattcccaac tgagggagct ttgtggaaat aagcccgccc agccccactt ctggagacgt 3300
tcccattcag taggtccgag ctgtcttaaa gagaaaccaa agtggggata ttaatggtat 3360
ccaaagtgag atctacccca ccctccctcc tcaaaggagg tcagatcaag aaagcccaag 3420
cccggcctgg caattgggac ctttcttctc actccagccc agggtgaagg tggacaagtc 3480
actttgaccc ttcaggcttc tgagctgttg tttctgaatt cagtgaatat ttactgagtg 3540
catagaatat gctagatatt ctgggctaaa ggttgaaggg ggggtgagtt ttaagggttt 3600

ctgctcttgc ttccagattg ctttcaaatc tggaaaggac accagtggtt tgtgtgttag 3660
acccacactg ccgtagcaca gaatacaaga aactggctga gagctccaat aggcttttaa 3720
cagtaatttc tggcttcacg tatttagttt cataactcat gatttttcaa aaacttctgg 3780
tttgaagaca ccgattgccg aaagtccatt gtgctgcata attacacttg gtccacgtga 3840
cagcactaac atgttctgaa atgtttttag aagtagtctc agcaaagatg aaggattcct 3900
ccctgtttga aaagaaaata ttctttgttt tttctttgat ctaagctcta agactagcag 3960
ctagcatctg aaactttttt gacgagagtg acaaaccaac tctaatatta aaggcaattg 4020
atgattatgg gcactgaagg gaaggtaacc ccaggctggt gccccggaat agggatgggt 4080
cacaatgttg aggacatttc gcctgttgca gaacccacct gcaacacagt gtggcccttg 4140
ccatgtgact tgtgtgtgtg cctgtgtgtc tgtgtgtgcg tgttttaatt ttgacttcat 4200
aagtactcta gttatgagct tatttaacat tgggttttac taataggggt atgtgttgag 4260
aaaatttcaa agttttagaa tatggttcac ccacatgttg cttccctgta aatataattt 4320
ttaaaaccag attctgggcc gggcatggtg gctcacctct ataatcccaa aacgttggga 4380
ggccgaggca ggcgaatcat gaagccagga gtttgagacc aggctgacca acacggtgaa 4440
acccagtctc tactaaaaat acaaaaaaaa ttagctgggc gtggtggcag gtgcctgtaa 4500
tcccagctac tcaggaggct gaggcaggag aattgcttga acccaggagg cagaggttgc 4560
agtgagccaa gatcgcacca ttgcactcca gcccgcgcga cagtgtgaga ctccatctca 4620
aaaaaaaaaa aaaaaaaaca gattctgttc ctcagatcca ttccattttt gttttccttt 4680
atcacttatg gacatttgaa attatggtaa taaacattgt tagtctcagt taattattac 4740
tggtttattc ttgaaccact aatccataga gaatagagtg taaatcttaa cttgttcctg 4800
taggccatcc ccattaaaca tcatagtgtt ttctcattcg ttctttttcg ttttcctcct 4860
acaggaatga attttctaag aaaattccag cagttggctc tttggacgac atctctagat 4920
tgtcctccat tgggcccata ggcacaagct ggccagtttg aatttgggca agaatccagg 4980
cattggaact tattcaaata actagtttgc ctgtaatttt cactttttca gagtcatctg 5040
ataaagcttt cttgctacac atttagatag atacactcaa tccagttgtc tagaaagttc 5100
cctgagccag ctgggagcag gaggggtagt tggggccagg aatattgggg gtgtgtttac 5160
tgagccccta gaaagtaagt gctagatttg acatttcaat ccctgaaggc cctgaagttc 5220
agtatcaaat gactggtcct gtggactgag catctgtgaa ttgcatatgc ttagagtaaa 5280
ttttactcct accagtttca gcagcttgct ttagcaagca gtatggaaac actaacatgg 5340
gggagtagaa tttctctctc tgatccaagt tttatctcat tctggtgggt tttcaaggag 5400
agactcggag tccaagtgtc ctttctgaat atatctggaa cttctcatta acaaaagact 5460
caagttataa tttaggggac aaggcaccca atgagaatgc cttgcaggca gccctaagta 5520
cacctgcaat tacaccatta ctagcgcggc agcacacatg gccctgactt agtttaaata 5580
attacgtaag tcaaccatga ttgtttgccc tttgcataga agggcaagta ttggtacctg 5640

ttacaactta ggcttttttt tctttatgtt tgagccatga tgagtgattt acactgttgc 5700
atccatatgt tgagatgtaa gaataaatta gacttggtaa ttgcccttaa gtgtctggaa 5760
gtcaactggg gaaagagagc tagagataat aagtgtgaaa caatgtcaca gaatcaatga 5820
cggaactctt cccaggacaa aggatgactt ttgagttcag tctttgcctt taattctaca 5880
tggggaggag agcacgttta gccacaaatg gaagggatta ctcatttgag ctatttggtt 5940
atatgattat ttccccagag aataggatgt gcagggcatt acacaagcag tgccaatagc 6000
agcaaagttc ttgagagtgc tagtaattca aatggcagga agagaaggaa taaatggtaa 6060
ggctacctac agttcacaga gagctccatc ctcactgtgg ctttggattt tgtcctgtgt 6120
gaaagagaag tgactgtgaa ctgacatgct gtgtttggtg ttttagaaag atggctgcag 6180
cagcggtttg gggaatggac tgcaggagtg gcattggaaa caggaaggtt catgactatt 6240
gccagagaca gaggatgaag caggagcaag gaagattcag gacaggggac tccggggctg 6300
atcaggaggc agaactggtt gataagtata tgtagcagca taagaaagaa agaatcccag 6360
attgacaccc aggcttctca cttggaagcc tggatagata ctgaatgcaa tcacaaaggc 6420
tgggaagtca atgggactgc agggaaggga agggaaggga ggagaagagg aagggcagga 6480
gggtccaata tcaatattca gcttttagat gtgttgagct tgaagtgctc agatggagaa 6540
gtccaggagg cagtagaata cggtggtcca gagcacagga gagcaatgtg gcttgagttg 6600
tcatttgctc acatatttcc gtgtcagtta cttgtcttag atcacagaac aagttctcct 6660
ctcacagttt cctggctcca cctgtctcat gctcaccgtc agcatcgaaa ttgagccaca 6720
ccaggggttc tggataccag cttctctcta ggtgaggctg ctatagtcag cagctgatta 6780
gttgcagtta tcagcaactg gtaatataat atattgtgca tataagtgta ccagaagtca 6840
tgtttatata ttgctgcaaa tactcggaat ggggatctct tgttccctgc ttaagaccac 6900
atcacattac ttggttttgt acgctagtgg ctgaaccaaa aaaagtagga gatgattttt 6960
tttctttttt cttaaagcag tagcttttga accttgacca tgctttctaa ccagctgagg 7020
ggcttttgaa aaagagggtg ccttactgtg ccccagacca ggacaatcag tatttctggg 7080
gaatggagcc tggcacacac acatttctta aagctccctt ggcaattctg aggagtggat 7140
tacatgttgt atgtagctcg taacgaaaga aatcttgtct ttgctctcag acccccattt 7200
cttactcatc tcatgagctc cttcgagatc cagaaacagt tgcatatttc attagtaaat 7260
cagttccaga gtcacatttt atttcacaag ttagtccatt aaaagtttcc tgcagtgagg 7320
aaatagccag aaagaacact ccacccctcc tcctttttat aactataggg tctggctcga 7380
cagagcagga gcatcgccat cttggacaag cccctcattc taaagttcac cttaataaaa 7440
aactgcctaa attcaaactg catcagccta atggctaagg tcagcatgac cataaaccac 7500
aaataacatc tccaaccgga aacattcgaa actcctcctc gaccagagac atgctagtcc 7560
cgagataacc cccctccagc agggaagatg ccagtctcgg gataacctct ctctggccgg 7620
aaagatgcct gccccaagat aaacttgcct cctcccagag atattccaac cctgccataa 7680
aacttctccc tcaaacagga acattccaaa attctgataa tctccctcac cctaaaacca 7740

atatatactc ctagtctgta agagaaagcg ctcttgacca aaattcacca ggagtgcctc 7800
ccaggtttta actaaagaaa acctctcttt aactgccaaa aaaaaaaagg gaaaaaaaaa 7860
agctttctgc agtggctttc agcgggccca gcatggcagc agcacctgag aacctgttgg 7920
agatgcacac tcttggaccc caccctggcc tctgagtaag acactggaag ggcaggcccc 7980
ggtctgtgca cacaagtcct cagggagatt ctgactgatg catgccagat tttgagaact 8040
gctgatatac tccaggcaca tcgcatgctg ggatctagat acaccaaggg aacaaaataa 8100
ctgcacttgt cctctgagga ccgacttacc ttttggaagg gctgagaaag agagacacat 8160
acaagatcac tccctgtaat gcaatgtttt ataacagatg tgatttggga tttcagtggg 8220
agcccaaaag agggactgac taattcagcc tctgtgacaa ggggagtttc tcagaaacag 8280
aatgcttagc tgggcctcca ggcacaggga caggaatgag gaaatacttg taggccctgt 8340
gctccttcag caaaaccctc agtttcttgt tatttttata aatgcaaaca tcttattaaa 8400
gtagatgcta aggcattaga atttcctgct ttatttttct aaatgaccat gaggaaacct 8460
ggaatgtcaa agataaagtg caacacattc tgcatttaaa aattaaaatg atccttttta 8520
aaagtagcaa ccagatgtga aaaattggac tggagtccag gttatagttg atagctttaa 8580
ctttctcccc aacagcaaca gcacaatttt ccctaaaatg tgttatgaat aagtaaaatg 8640
actacttcac atcctttaac tcttcctaca gaaatctaag agagaaatga aacaaaagtt 8700
tgcacagttc tagacacgat aaatacatgt gaaatcacac aactcagaaa atgtccctta 8760
aattaattga gccattggta cttgtgaatt agaagagaca tctatgttct gatccactgt 8820
tgaaagctgt acaatgttac ctatttattt gcagacatcc tttggaaaca aataggtaga 8880
tttgcaacaa ataaagagtg gagtacagct gctgacatta ccttgtatat tcatgccttt 8940
atgtaaaaaa aaaaaaaaaa atatatatat atatatatat atatatatat atatacacac 9000
acacacacat atggaggtaa agaccactgc ttgctttgca gttgttttaa gagcattcat 9060
gaaggatttt attttataag cagaaatgtg atatctgacg attttaccac tacatgcttg 9120
caggccagtg cacagcagat gacgtcatga ttgttttagc agtcctatcg ttttacttat 9180
gatgtcatta caaccctttg ctaaaatttc tttcctttac tccaggtttt ggataaaatt 9240
gatgcattgc acatagtctc tctgataaga caaactggca tttgtatgtg aaaaactgtg 9300
catgttttag tgtctctgct gatactcaaa ttatccatta ttttagtgct ggaataaaaa 9360
caaaccactt agtgaatttg tgcaggtcct taaggacagg caaaggtgtc ctgagatttt 9420
ctgatcattg tataccaaat tttagaaact ttttcaaaaa cattttttta atttcaaaaa 9480
cctggttttg tttatttacc agcaatcatt gaatacctga aagctttcag gagattttat 9540
tacaatggtt tctattcact tacaaaatta tctcctagtt cattctcata cactgtaagc 9600
cattgtaaat gcttcaaatt gtgccgaaca agataaacta gacaaactat tttaagtttg 9660
ttctagtgct aacttgcaag atctaatggc tccaactaga tttttaaaat aaagtatatt 9720
ttaatatatt attagaaagt taagcaatta tctgtttata ggtaacaaaa accctggaac 9780

cccaatgtca gatgtcatcc acttttgatt aagtccaaac atatgacaga taaacaaaag 9840
atggttggct gggctcagtg gctcatgcct gtaatctcag cactttcaga ggccgaggcg 9900
ggcggatcac aaggtcagga gtttgagact tgcctgacca acatggtgaa acccgcctct 9960
actaaaaata caaaaaaaac agctgggtgc ggtggcacgt gcctgtagtc ccagctactc 10020
aggaggctga ggcaggagaa tcacttaaac ctggaaggca ggggttgcag tgagctgaga 10080
tcacaccact acactccagc ctaggcgaca gagcaagact cagtcaaaaa acaaaaaaaa 10140
agtggtcatt ggagaattat tgtgtcacct gttgtttttt aatgtactaa ttttgagagg 10200
cttttaaata gagtgcacta tagaactttt tcttggcttc aatttgctac aatgttaata 10260
gagaatcaga aaccttatcc ttatagatgt ttcttgattt ttttaatttc tggtgacatt 10320
tatgagtgag aatagtgtat tgccctgttt tctttcttac tcccctttct tcttccttcc 10380
ttgctttctt tcttcttccc ttccttcttt ctcttcctcg ctccttcttt tttacaagct 10440
gttatgaatt agccttcaca gagaaagaaa aatttttata aataactgga aatgaaactt 10500
tgcaaaggac tgcagatgaa aaactttgtc aaatgactgt aaaaatatac tatataattt 10560
tcaaaagtta gaaagtacca aacacactca gtattcatgg ttatacaagt atgcatacac 10620
atgtattgct ccctgaaaag tggtgttgtt aagggagttt ttcttagtac gcggcttaac 10680
atattttttt ctgtaatttg ttgttagtta taatggggag agaaaacagg ttagagtctc 10740
ccctctcagt ttcaccttcc ataaaacagc taaactagac gatcgtcaga ctccttccag 10800
ctgaaaacat ctgtaaaatt aaaaacaaat ctaaatgtat gcaagatatg tatttaaaca 10860
tgctggtaat aagtgtgctg tccctataat ttagatgcta aaacattgat gtcataataa 10920
taacaacacc tcgcatttgt acagcacctc atagtttaca caatgcctta acattcttct 10980
ctctcagcct cctacaaccc cacaggattg ggatagcttt ccagattggg aggtgaggga 11040
cccaggctca gagcgattct gctgttgtcc gtaatcacca ggctggtgat cagtgggcac 11100
tgggtgctct cctgctacac agcactgtct ctcaacatgc aggtcaaggt tacttattcc 11160
tccttcaaga cgtcattggg ttttttagct atggatgccc catcactttt agttctattt 11220
gtgaatcaaa ggctaaataa agtattcctc aaaatttgtt atacttctgt tactaatgct 11280
taatgtccct cacaatttct gtatatttct gtgtatttct gctctgtttt ggttcctttc 11340
ccaggtttct tttttgttat gaagtagttt ttagactcaa gtctcttctg tatgtgttat 11400
aactgcccat tccataagat acagggcagt gaatttgtga gccttgaaaa tatttacttt 11460
agaaatgaga agtatgactt ttcaacgttg tgtcatcaac ttctgtaaat tttccagacc 11520
tataaatact tgcagaaaaa aaatgaaagg agaaggcaac ttgatttagc agttgggtca 11580
gttagcaatg cctatggcaa gctgtagtaa ttcccttaca tagatttgta agactcattt 11640
ctatgattta aatgaaggca tacacttaac ctctttaggg tgtgaaacag cttttacaaa 11700
aagagacaaa cttaagaaac agtgtggccc tccaagagtg ttcattttcc atatcatacc 11760
atttgtaata agctattctg gctgggattt acttgcaagc attggctttt aagaagagat 11820
ggtttcacac atcaaattat tcacttggag gcactttctg ggttgaagga atggaatgga 11880

gagtgcggca gtgagtagat ctctcagtga cggtgatgtg cctctcccag aagaaatttc 11940
aaaatgcagt gttcattttc ctccacaaga aaggaagaaa ctgttttgtt attgtttatt 12000
cctaacatag tggaaacttt tcagtactct ggcagaaatt tcccaaaagc aattttctat 12060
ttcatgatta taaagtagca aaggaaaaag tcctgcactc cagctgagca atggatctcc 12120
agttgttatc taggtgctgc aggtttagag aggattgcca ggagaacaca tcgatttttc 12180
aggcctgtga tgacgtatct cttgttgaat aagtaaaccc ttccagtaaa cagacagtta 12240
gtatattgat ttcagggtgg ctttagccac tgaacctgta agtcttgcaa aggttacttg 12300
ggcaaaagca tcattatttt accttcagtc aacaaaaatc tacctggcca aggcagaaca 12360
gaaagttcag caatttgatg aagtgggaca acatgaagaa tcaggtgagt tgcctacttt 12420
ttcacttcac tttccacctt tagagattct tgtttagatg cagagtagtg acgtgcctgg 12480
tgtcagggag agagttgaat gagaaaagtc ccagaagggc agaagacttg ggtgattatc 12540
tgagtccatc tttccttatc acatgacaga gttcttgaag tcttggctag gaattctagg 12600
cttttagatt ctttgggcaa tggctactaa atgttcataa tgttgctcag ttgcaaaaac 12660
aagacattca aactatagcc agggagataa gtagtcacga actcaaggcc taaattctgc 12720
tgatggagcc gatgagaatt gggtgctaag gcaaagagag ttgccaatat tatattcttc 12780
ggggtttttt gtttttattc gcattttgga aaaggaaaat attagcattc ctctgactta 12840
atattgagaa gacattgggc actctttttc ctcccacact tgtcttcttt cactaggtga 12900
caagggaaga ggtagcatga ggtggtggtc acaggtgaga ggggctgttg tgagcacagg 12960
catgttgact gcacattggt cacctagtag aagttttgca ggcttggtga cttctgaaca 13020
ctgttttcaa ggttgatttt tagttgagag aacctctagg taccacgtaa tgttattaac 13080
agtagtactg atctcacaat cgccctatgt cccattcaca agatgttctg ccaagccata 13140
aaaggcccag ttaagtttaa gagaagtctc aaaagtaaca gatgataact aattaatacc 13200
cagtgatttt gaaatgtaga catcaaacat accaattcag tggtatcatc cttagaggca 13260
gacagaggat gattaaatca ttcagcccat ctctgtctga ggacgcagct tagcacagca 13320
tggtggaggc taaatgggcc ttaagggaaa aaatgatatc tgaagatgca atttatttca 13380
aaaagagttt gctcccgtga attttcactc tctatgtaga acggcaccag cacacacttt 13440
tcctgagcct ttgcatgtgt ggcaggcagc ggcctggcat cctggggaac tgaatgagga 13500
cgcagatgac ccggacgtgt tcacagtttg acacatctga ctcccagatc agggacagct 13560
agctttgctg gctggttaag ttgatgattc catctttgcc tggttctctg actgtctcat 13620
gctttctgtt attactattt tgcagcagat atttctgctc atttttcaat catatatgca 13680
tcctggatgg catagagttg attctcctaa caaatcagtg tccctttgta tttttttctg 13740
gccataagat agaatatata tgtcatttat taaaaatgga gaaaatgttc aggagtttct 13800
tgactcagag agggaaaagg gatactcagg gcactttttc agccaggaat ttactacctt 13860
tgcagggtaa aggggactca ccacgctgga agtcaaaata agccaccagt gccaagtgtt 13920

caaagccctt agaatcacaa tgctcttaaa gcaaagtctt caacaatgct tgaaaacttc 13980
cactggttct cagtatgtcc aaaattgtca tgtctatgaa tgattttctc aatctgaaaa 14040
tttttatagc aggctaaaga atgagatagg tcagtgtgat tctagaacta atcattaaca 14100
ttcaatagat gactatttta ttctagaaaa agcagcaact ttctatttac tctctatttt 14160
gagggtaaat tctctgtaag tagaaaaagc aaaatgtgga catgggacta acatatgaat 14220
atacaaagca aatgtaccga aaaaatctta agacctgcct tgtggtgttt tttgttttgt 14280
tttgttttca ttaaagtgac ttgttagcct cttgctccct gtgaagcaca gggaggtgac 14340
gtgatgtgca cagggcagac tctgccatat gccctggcct tgaactcagg gccccctggg 14400
gactgcaggg gatgctggcc atgctgagca atgcctgtgg gtgtcagttt cctcatctgc 14460
agaatgaggg taggcctggt gcttatttca tagggtcgca gaggggattc agtgacaggg 14520
tggtgtagag gctggagcgt gccccatgtg tgcacgacag ccttccaact aggggaggcg 14580
ggcctgggct ctcaccagag agcctgtgtt ctccatggct acatgacttt gccccagacg 14640
tccttcccgt ggtctggacc ctgggaagtc gccaagagcc agacaggaga aaggctccac 14700
ttggctctcc tctttggtga ccatcccttg cctccatggc gggactctca ggtgacatcc 14760
caccaaccct cactttgctt ccctggtggg tctcactttc cctcaagagt gttgcttttt 14820
tgtttcctgc atagtcctgg gccagttttg ataaccctct tcatttcact tcagaaaccc 14880
tgatgatttc ttcctgtgct ctttttacct taggactttt actatgacga ctgtgactgg 14940
cccatttctt gttttttttc tcttgctctg ctttctcccc catcatcact aaagcagaca 15000
tggcaatgat ggccatgcac actttccaag ggtccagctg tagatcttca tggttcccca 15060
ggtgcctgga ccatcttgtg aggagggagg caaacacacc ctgcctggag cacttggccc 15120
tttcggcaat gttttggctt cctcaagtga gaaaagaatg gatttgtatt ccccctctgc 15180
attattgttt ttgttttgtt tgtttgtttt gttttgtatt gagacagagt ctcacttttt 15240
tccccaggct ggagtgcagt ggcccgacct cggctcactg caacctccac cttccgggtt 15300
caagtgattc tcctgtctca gccccctgag tagctgggac tacaggtgcc cgccaccaca 15360
cctgactaat ttttgtatgt tttgtagaga cagggtttca ccatgttggc caggtgccca 15420
ttattatttg atctggaatt aactgagcta ctgcaggaat tgcttgattc actgatgact 15480
ggtgttgagc cagtacacac ccacacccaa ggactgtgac tgtcttctga ggtccatcct 15540
cagaaattcc tgtctcttca cctagtgtgt aataaggcct gcgcgtgtta tatggaactg 15600
taaaaaatgc gccaaccatc tgtccttcct ctttatctga ttacttatca ttgttctcta 15660
agttgcaagt taatagactg atcataaatt aatgcatgct ggagacttgc tgtttcctac 15720
tagcagcata taaaagttat ttttaaagtt gttttaaatc tgtgagtaaa aataaattgc 15780
tttgctgcaa gaaacaccaa acatggaaaa gctaacggtt caaagttaat aatttatctt 15840
atggacatca ctagtggcat agttgcttta aacagtgaga ggatttaata gatatttgat 15900
ttgcaagtgg gatgaagggt ggtctaacct ttgtcctgtg tttaccttcc atgagatcct 15960
agaggttgta cagcacagta gtggcatgtg acacacttga gagtgcctgt tctgtttgga 16020

aacctggaaa ctatgaaggg aagtggcctt cgagcttaac acataagact tgggaggcaa 16080
aaccttttat tctctttaaa tattcacttt aggataagca tttttttagg tgttaggaac 16140
agggaaaact gtgtggttag gaaggaagaa agaagaaagt taactgttgt acattcccta 16200
ggtaatgttt ttaagcattg ttattcactt tcaaaacaca ttttatttat ttggacttaa 16260
tattttgatc ttattttttc aatttctttt aatttaacag acaggatgag tttttttata 16320
gttgtattac ttagaaatta tactaaaaat ggccgagtgt ggtggctcac acctgtaatc 16380
ccagcacttt gggaggccaa ggcaggtgga tcacttggat cacttgaggt tgggagttca 16440
agaccagcct ggccaacata gcaaaacccc gtcttcacta aaaaaaaaaa acaaaaaaaa 16500
aactagccac gcatggtggc aggtgtgcct gtaaccctat ctactaggga gactgagaca 16560
taagaatcac ttgaatccag gaagcagagg ttgcagtgag cagagattgc accactgcac 16620
ttcagcctgg gtgacagagc aagactctgt ctcggaaaaa aaaaaaaaaa aggataaaga 16680
aatcatacta aaaacaaaac agaatgctga ccaccttata gaaatagaaa tagtggtttg 16740
ctgtgatagc aaattttctt gttaactttt tatttttaaa gaattgcaca ttcacaggaa 16800
gttgcaaaaa atctactggg aggtcctatc ccccttcccc caacctcctc cagtagtaac 16860
atcttagtag caaagttttg tatatttatt ttgatatcat tatctaagtt tgacatcatt 16920
atctaatatt aacctaagcc aaaagcccac tattttaatt atctagtgat gcagtgttat 16980
agaactcata gcctttcaca gcattatttg gaagttaatt ttcttaagtg aaatgttttt 17040
ggtctttaag gtttggaggc catggaggca tgaggagaaa tgggatgagg gagagagagc 17100
taagatagat aaagacagag atggggagat ccactgattc gttgaacaaa ccagatactt 17160
ccttatagtt tttggattaa cttacatgag ctaagtttat attctgttca gatcacaagt 17220
ggtcaagttt gtgtgtgtgt gggggggggg gggtgggtgt gtgtgtgtac cactctaccc 17280
atcctatatt tattgtcctg tatttggtct gttctgcctt ctttattttc aggataggtg 17340
tcctaaatga gggtctttgg aaagctggtg aggccatgtt gcccgtttca ggtgttccgt 17400
gctcaaatgt attcatttct tgaaaaattc agggagtgca cacttttgta cattttccta 17460
tgtgtatatg ataccattat ataaatctta aaaatatata tggttcacct gaatccccag 17520
ccatttggta gagaagatag aaaacctaca gaggaggcta agattttatt agaaaattca 17580
gcttctcgac ggaggtattg gctttaaagt caaggcaatg catctattct ttcttttgat 17640
ataactagct aaaagatctc ttaaattcaa agtggccctc atcttactgt tactgcaatt 17700
tactcttaat tacaaattat ataaaaatag gttttgaaat actgtagcga caaagtaaca 17760
tacctctgct ccattacaca gataaaacct ctaaggaaca cctcctctct taacaggcat 17820
taaccaactg cagaaactgc agaaggacag ggctatttgg gaataacaca gctcccttcc 17880
ttgtctgttc cctcccattg tcaggcttct gtggagccat attcagagca acatagggag 17940
ggggaagaga aaatcaaccc cttggtgaag gaaagctccc aattcacaga gcaaacatgg 18000
gtactcttgt ttgtgggagc tcccagggcc tcccagctca ccgagcattc tgagccctga 18060

tccttacact aattgtatta tgcaaccata aatgatgtct gctgtaccag cggggacagt 18120
ttattttaat agattggtat aacttggcag aatcttatct gcatgtttca tcttggattt 18180
ttagctcaat tcaactcaat aggcatgtgt caaatgtcta ctgcagactg agcactgaaa 18240
agctgctggg tacagggtta catggataga aaacgtagcc tctgacccct aaggagcctg 18300
taatccagat ccccattctt tccatcccat tctcccaagc aagaatttac ctaatgtggt 18360
ttgcgagaat ttaagagctg gaaaggtggt cacgagaagc cggaatgggt tcgctaaaat 18420
gtgtctatat gattaagcat aacgtagctt tgcagcactc ttcacagctt cctcagagcc 18480
ttccgcacgc ggtgtctcat ttgaatactt gtgtgaggat agcctcatac ccctcagtga 18540
gctcttcatg gagtgatgca gtagacagca agcctcacac ttctatgctc acggaagacc 18600
aaatttgcct tgaaaaatct ttatagtctc ttcacatttc taagttgaca tcaaaaatcg 18660
gttaccataa aatcctaata gttgaagaga tgtaatttca attatttggt aaacctgacc 18720
ttcattgtca aagcaattag tcaactcaga tttactttct cccagataat agattctgac 18780
ttcttttttt ctgattaaaa aacttaacac cttcctcagg agatctatct cagttctgaa 18840
tgctgattct aactaagaag gatatttggc tacatgctgg gaagaggggt actgaggcac 18900
gccgcgattc cactccagca tttccagtta gtcgggtgcc tctgcactcc cggtgttccg 18960
gcgcccagtt agttgtgtac tctgggctgt ccctatactg gagtcctaaa acacttacga 19020
ctgcagatag ggggaggttt ttcaaaacct tggtctgaaa agccatagaa gggagatagg 19080
aaagcggggg ggtggagcca cagtacattc aggtggatcc gtttttggaa atagtacaaa 19140
ctggaggtga aaccctggaa attgatctgt cgttcacatg cttcatgccg agtccttgtg 19200
gacccacaga gacacactcg ccccagtttg aaggctgcta acttgattct gaggacacca 19260
gtgaggtggt agtgtgcaaa tgatgtgtga ggaaactttg gaggagtctc accctgcctg 19320
gagcacgtgg cccctaaaac agcgcagcct cccaaagaca gaagatgtgg actagtgaga 19380
agccaggtat ggtgactgct gctggatgaa gcttgtccca ccagaggctc gcttgtttca 19440
ttgagcacct actgtgtgct tgtgggatgc aaacacacgt gtggtccctg ccctcaggtt 19500
aataggcagg ggtggaacag ttatgaaact gctctaaagt cattttctca aactgggagt 19560
gacaaatgta tccacttgga aaagattgag aattttataa gatttttaaa tttttgttta 19620
ttcacattga ggagaatcta aattcttttg aacttatgta tagatttcac cattttatag 19680
taataaatca gtcctcctgt gtgtgtgtgt gtatgtgtgt gtgtgtgtat gtaaacctca 19740
ccttgcaata ttattatttt aaatagccac ttgcatctta aggaaattaa gaggacaaaa 19800
gaaaagctgc tgttttgtat gtatccacat atttaccagc tgcttccctg ccggcaggtg 19860
ctctggttct gcactgcctg ttgtcccttg cctgaaaatg gttgcctcca atattttgct 19920
cagttttctg attgtttaca gtggcagagg agggtagatc tggtaccagt tagtaattgc 19980
cagaggtgga agtctgtgga tgaaatttgt ataacatgga acgttagttc cacagttaat 20040
gctactcaat tggaacccat ggaaattatt ttttggtgaa aagggcccat gcgttatgaa 20100
atttgagatc catcacttta agtgaatgta ggccctggat acagtgggag ctcagaagag 20160

caaatcagtt ggtcaccttg ctcaacgtat tttactaagg gcatcagtaa ggctttctat 20220
gacctgctcc ttcaatgctt ggttgacatt tggggagcaa agataaacta aggattctaa 20280
gttctgtcct gtgatgctgt aaggggaatc tcaaacctct aggtggagga gtgcagagat 20340
gaccaggatg gtggaagcct gcaggagagc tgaacacctg aagacaccca gtgggaagac 20400
caggaccttt aacgcccata tctgctgctc aagactggca gagagaagag ggtttgtgat 20460
gagaaaaggt ggtgaaaggc acaaggaggc acagagcatg tcaggtccca tatcccaaaa 20520
ggaatgtgct tgggtgaggg agagctcctc catggctgga ggcattcaga gaccaggcag 20580
tcgcttgtgg gtttgtgatt agagtgaggt tcttttataa agggagtgag aagagaaggt 20640
ctgtggatac ttgagtgtat cggtaattaa gaaataaatt gtgtacatcc catttctttc 20700
cacattttcc tgggctgtca cagtggctgc aaagaaagca gtccgtgaac tgaactgtga 20760
tcccagacag gcaagcacac caggaatctc ttctcagctg ttgataatga gggagcgctg 20820
gggagagaaa tggggtcctc tttgagtttc ctctgtgccg atacctttct ctttgttaaa 20880
acagctaatt aaacactgaa gcagtatagc tctcttacta tacactggta gtcatagttc 20940
tcttactgtt ctcttcactg acagttctct tactatacac tgatggtgac gcagaaattc 21000
agaattcccc gcatgtgtcc cggtttgaaa gccactgtgc tttgctgtgg attaggatca 21060
gacagttgag tcttgttcca acaaggaaag ttgcttattg gaaagttttg ctgcagggag 21120
ccttgagttc tgcatcaggc ttggaagtgg gctctgtgga ggtcagaagg aggatccccc 21180
acccgcagcc tcaagaaaaa tatgaaaagt ggattatgcc tctgtagcta tattgcctat 21240
aaactttctg cagaatgaca gtattcatat cctacatttt ttcaaagcga tattaatcct 21300
gagacctgca gctaaagtca agtagaattt agggataatt aataggagga aggtggggtt 21360
ggaagatctg catgattata gtcctctgat ataactggaa aattctttcc attagcaagg 21420
agctttggtt aatataaaat ggacagatta aacctaggca atttatttta ctcattgctg 21480
tatttttatt tcagagctgg ttgaaaatat tacaaagtaa tattttaaag tgcttatcta 21540
aactcttact ctgcatttta tcattgggtt atgaaatgac tggggaaaga cttttcttgc 21600
ttttatttct cagtgtctac ttataaacat gttttttgaa ctactgtttt tgtgacaaca 21660
tgcctttttc ccagaaaatc tcaggttaac attaaatagg cactggatgt ttatctgatc 21720
ttgtttatag aaacacaaga aaattttaac cttgtatata ctttactcaa ttaactaggt 21780
aagaggtcat tgaaacattt agaattccac tctacatttc aataattatc aggtgaaagc 21840
tactgcatct acatcagaag atgtttgtaa tttatttaag aataaaatta gctatgcaag 21900
aaatagtatg tggagtccta tgtggaaatc acagaaaccc tgacaacttg atgatctttc 21960
cgcaagctaa aaatatcact ctggatcaca gcagtagagg actctgtaaa tttaatctgt 22020
gtgtctcctg taaataagtg cattagcagt acacaggtgg tgtcagagtc agtgatgatg 22080
gatagaaatt ctacataaaa tccaggctca gtggctcatg cctttaatcc cagcactttg 22140
ggagtctgag gcgggtggat cacctgaggt caggagttcg agaccagcct ggccaacatg 22200

gcaaaacctc gtctctacta aaaatacaaa aattagctgg atgatggcac atgcctgtaa 22260
tcccagctat tcgggaggcg gaggcaggag aatctcttga acctgggagg tagaggttgc 22320
agtgagccga gatcacgcca ttgcactcca gcctgggcaa aagagcgaca ctccatcgca 22380
aaaaaaaaag aagtaagaag ttttacataa aaacgtggag tgagcccaag gtgccattta 22440
tccagcccat acacatcgta ccatgtacag agtggacacc agataaatac attgactgca 22500
tgccacaaac atatatatgt aggcaccgtt gcattcaaat acacatctgc agccctaaca 22560
catctttatt tgctaacgag catcaatgta tttaaaaaca aacatgttta aactagtgaa 22620
tgattagatt ataatgatct taattcataa gttttctcat tggccttttg tatacttcaa 22680
ttgtaatacc tagaaaaaca gttatgtcca aaggagtgaa taggccttat ctgaaacagg 22740
tgagcgtgac aagtgttttc ttacttattt tacttttcag ataattcatc cttaaagtac 22800
attagtttaa aagtactgtt taaggaaaca gtacttggat taaaacttga atcattgtta 22860
aggaaaacta taccttaact tcatgtaatc acaattaaac ctcttcatat agaaggatct 22920
aagaattttc tgcagcattc accagcacca aaaagctcag agacatatat ttctttctct 22980
gtatatgtat tttaaattca agttagtata aattgacagg caggtcagag taatatatga 23040
tcttctgagt ccccttagta attaaaagaa atgattattt ttgcatgaaa tatgataaag 23100
tgattttaag tgcctgataa aaagtcttaa ccatgacaac cattaaagat tacatcaaag 23160
aaaaataagt ttgactttca tttaccttgg aaacagctat taactggtaa cctcaagaaa 23220
caccatgaag agtcagtttg ctccacacat gtcttgtaaa agtcaaataa ctggtggtta 23280
tccagtaatg acaagaggta gaagttacat ccttgctgtc tgattgaacc ttcccagagc 23340
tggcacaagg ctgggaagac cataggtgct aaatgaggaa ctacttaaag aaagaaaatg 23400
gaatttcacg gacaagaaaa tccatgtcca tttggttctg tgacccacat cctttgtatc 23460
ctatgctttt ttacacttgg tacatggttg caagattgcc cctgttttct acttatagtt 23520
ccatgcagca tggatgtggg aaaaagtctc ctctgcaaag ggggttaatg caggtcactc 23580
tacgtatgtg cacgaggtcg ttataaagct cgaaaatatg ggctcaccaa ccaggtgatt 23640
tttttaatta tccaaccaga agacataaca tataggggaa tcaaaagaaa tctctgagta 23700
aaataatgat aacaggtcaa actttgcggt cccacgtgag gctggagatg cgtattgtct 23760
tgactttgca tctacaagtt taacaaatga tgctttctca gtttacctct ggaaatggaa 23820
attagcattg caaatgactt catgaggagg tagaagctat ctgtgaattt cctttcgctg 23880
tgtttacgat agactctcac gtctagatgt gtcatgtatt atgttaaatt ggtatgtctt 23940
gaagttataa agcacagccc tctataagta tatatattcc acctctttca aatcggatgg 24000
tacctatcct tcaaactgct atttaatgac tgtctgctat gttcaaggca ctgctctcaa 24060
tgttaatact tgatgagatc gggcgcgttc aaggtggcat ggccgtagac tcaatgttag 24120
tatctgaaat atggcctacg agctgagttg tgaatcaagt taatagattt tcggaatgtt 24180
aaggtctaaa ccagtagctc ttaactgaga caatcctgtc ctcatctcac ctgggagaca 24240
tctggcaatg tttggagaac cttttggttg tcacactggg gcatctagtg agtagaggtc 24300

agggatggtg gtaaacaagt ttttttgttt gtttgttttg tttttgagac agagtctcac 24360
tttgtcaccc aggctggagt gcagtggtgt gatctcagct cactgcaacc tctgcctcct 24420
aggttcaagc aattcttatg cctcagcctc ccaagtagta gctgggatta caggtgtgca 24480
ccactacact cagctaattt ttgcattttt agtagagacg gggttttgcc atgttggcta 24540
ggttggtctc gaactcctgg cctcaagaga accgccccct tcttggcctc ccaatatgcc 24600
gggattacag gtgtgagcca ccgtgcccag gctaacattc tttaatgcat aggacagccc 24660
ccaccataca gaggaatccc cagcccagaa tgttaatagt tctaaggttg agaaacccaa 24720
ggttaagcca agtcaactta tctatcttct ttaaaattgc ataagaatgc agtcctgttc 24780
ttcattcctc ttgctttgca gttaatgatc ctttgcctgg actttctaag tgcccagaag 24840
agcaacagcc agcatgcagg atggcattcc tgaccagttg cacttggcct agcattccaa 24900
cctcacctgc ctcagcttgt tcaacctgaa aacctaccaa gtgaaagcaa gagccacgtg 24960
aagacgcctt agttatatgc acccacccag acacttgctc agaaaggaat cagtggggcc 25020
ctggccttag aaactggctc cttcactgct gtagaaacaa cataaattta acataaaaca 25080
cgtgcttttc ttttttcttc ttactttttc ctgtcttggc aatgcaagga tgccattagg 25140
taaagaaatc cttcaccaca ctaatcctgc agagccagaa gagaaaccag cttgttctaa 25200
cccagctttg tcatggagag aaggcagctg ctccagtctg aactattctt tcttttggta 25260
gcagcctgcc caagggtgaa agtgtgttta atagtttgaa ttacacaagt gaacagtaaa 25320
tgtatgcctg tttctgcttt atgggacttt gaaataatgt tgtttgtgcc aaggttttag 25380
attactatac ctaacaacct agaaaaagaa atgaaaagga agccttctgc caggcagagg 25440
tcactacggg cctggagctg ggcacctgac tcagcagctg cccagatccc cagagctgag 25500
aagtcaccat gcatttgtgg tgcttcgagc gagttaccag agtcctggaa cagagcagca 25560
cacctgcggg gtgtcccctt ggcatttggg cagggcaggt gaccaagggt cttgttggaa 25620
ctgaagtcca gcttgaaaag caaatctggt tgtgagctag agtccagtaa cacttgtttc 25680
ccgccgcccc ccgcataact cgtgtgtcct aaaatacaat aatttcttga acttcagtca 25740
cttatgccta taagcgggca tacaacaggg gcacaataaa tgtttgttaa gtgaatgaat 25800
tctttcagaa ctagatggga tcttagtcca actctcttat ttaacgaggt ccacagaggt 25860
tctgcgattg tctaagaaag aaggctgtgt tcatggcctt tgttgtttac gtggccctgt 25920
gattctcttg gctccgtgaa agtcctgatg cagacattcc ggccatctag aaaggcatgc 25980
agacaagcca tccagctggc atgatcctga gtccagcttt ctttaaaaga gcttccaaaa 26040
ctgcttaagc tttgactgca caaaacctgc atcacctcca gttgagaaac tcaagagaat 26100
aagtaagtta tggagttgga gaccccagct taactactag ttttaaaata gtgaaatcaa 26160
cattttcaaa tctttgactt cactaagatt taataaagtt tattaatcat atattatgag 26220
ttattgctct ctctttatgt ctgtaatgca gttgctcctc tctgtataaa ttaataagtt 26280
ttagagatcc aaaatgagaa ttttaaaata aattacgtat attttaatca agtttaattt 26340

gactatatcc agctaaacaa ttgattgaac ttcacttgct tttctatgac aggttttttg 26400
ttcttagtaa aagaccccag ttttctcact tgtgaacaga aggggttaga cttcatgaca 26460
gctaaggttc cttccgtctc taacaaaagt ggcctgaaga gaggcttcta gactatactc 26520
acggtgggtt cttgggacct cagagtcagc tccatcactt aagtggctgt gtgattgagt 26580
ggagacacct caatctcttt gtgcctcagt ttcctcacct gtcgagtgtc aacatgatgg 26640
cacctaaagc tgttgagact tcagaaaggt aatgtgtgaa aagtgaaaag tgcctggcat 26700
ccaggaagta ctcaataaat accaactatt ttattgctgc agctgttctt atagatgtga 26760
tttctagaac attgccttct aatagggtag ccatgggcca caattgttgg ctgttcggtg 26820
tttcacatat ggttagtcca aactaagatg tgttgtgagt ctcaaataca cactggattg 26880
tgaagactta ggacaaggaa aacaatgtta ataaaatctc attgataact tttaaattaa 26940
ttacatgttg aaatgaaaat atttgggaca tattgagtta aataaaacag gagattaatt 27000
tcttctgttt ctttctactt tttttattag tgtggctact caaaaatgtg acattatgta 27060
tgcatctcgt attacatttc tattggacag cagcgctcta gacagtacta tgggtagtat 27120
ctgtggggag gttctcagaa acatgtcgca tgctctttta gaaccttaaa gtattcctag 27180
tctcctctac ttccagccct tggctcttgg gcctcagtct ttttactttt gcggctgtgt 27240
ttctctgaag gcttggcatt agtagattga aaagaataac catctaggga aatgtgaatt 27300
cagtttcttt ctgacattct gctctctaca aggggatatt atgtacacat aaacctactt 27360
ccaaaataat gaagtgaggc ctaattcctt actcttcaga gagcccactg tggaagtgtc 27420
actgaccttg tgtatgggct gcccttcatg gctctgggag tcattataaa gggcagcatt 27480
tggcgtggtg cgtcctaagc cagtgtttct cggctctgtt ccttagacat gtgttagtgt 27540
taatagatgt tcttggaaaa aaaaaaaaaa aacagcattc tgaggtcaaa catgctcaga 27600
aagcttggaa tctgcactac gcttctcgta cacatttcat attaaagatt ttggaaagtc 27660
ctgcaataca gagccctgtc taatattgcc acaacccaca attgctcaaa tgtaaataga 27720
tttgagttta ttcacattca gatcacctct taaggcccca cctcccaatg ctgtcacaat 27780
ggcaattaga tttccacatg agttttggaa gggacattca gaccacagca ggggaaagca 27840
gggtacttgc tgctttgcaa gtgtgtccac atctaattaa tagtacagtt cttactcttg 27900
gtgtgtccgg tgatattaaa aattaatgtg ccttatttag ataagtaaca taaaaatcac 27960
aaaatgtatg ccttagattt atatgtattt ataactagtc tatttcctga aaacagttga 28020
gacaccttgt aaaagttacc ggtacgatag ggccattcca acaaagctgt aaagtggtga 28080
taacacagtc ataaagaaga ggagatagct ctgggagaaa aggtggccca gaaaccagct 28140
ctgagcctca tggctgcagg caaggtctgc aggttcctgg tcctgattgc aggccatttg 28200
ctgccttgag tggtggttac acaaggccag ccctgggggt atcacccaga acacctagta 28260
cacgaatttc agtttagagg acgaagcatt actggagtat tgttatgcag gaaaactttt 28320
tcctaaaaat gccctgaaaa gagagtagcc taatgcattc aatcaaaatg tttttaagtg 28380
gaaaacatat tgtgtgtact tgatctggcc tgctgctttt aaaagattaa aactgggact 28440

gggcatggtg gctcacacct gtaatcccag cactttggga ggcagaggca ggtggatcac 28500
ctgaggtcaa gagttggaga ccagcctgac caagatggtg aaaccccatg cctactaaaa 28560
atgcaaaaag ttagccaggc ttggtggcgc atgccggtaa tcccagctag ttgaggggct 28620
gaggcagggg aatcacttga acctgggagc cggaggttgc agtgagctga gatcgcatca 28680
ttgtactcca gcctgggcaa caagagtgaa actccatctc gaaaacaaac aaacaaacaa 28740
aaaaacactg gggccaaaga actctgtgtg ctgtatcacc taaccacatt tcatgacacg 28800
gctagagaag aatcatgcaa ataaaaattt ccaacatgtt cgtaaactgg gaaagtattt 28860
cactggggag tgagcagaaa agtaatacta taacctctat atctagacaa atgtgaattc 28920
agtttcacat ataaatatat aagtgaaaaa atatataaat ataaataata tgaaataatg 28980
gttatctcac cactttctac atcttttgtg aatattttat agtgctcaaa tatattagtg 29040
cactagtata tgtacattac attaaataac taatcattta ttaggaggat gtgcttgttt 29100
tttgctaata aagatgataa taaaaaaatc cttagacccc ccctcggttt gttttcagtt 29160
aggaattagg gatatttata agaatatctt taaatgacac atgccttgct ctgggacgag 29220
gcatctgcat gggtgacaca tatgtgttgt gtgtacaggc tcccagcatt tccagggccc 29280
tgctcagaat gtaggcctta ctgattctta cagagttaca agcgctggtg aggttggcga 29340
agtttaggta aacacagctg ggaatgcccc atggcctctg ggtgactttg gacatcactg 29400
aactttaccc ttagagatgc atacctgcat cttttttacc ctgatagggc cttccatgat 29460
gctttcaaag tgtttttgtc tgcttttcgg ttaatagact ttcacagtag ccaattgaat 29520
atattggtta aatgcatctc tttatacaca gactggattc aaactgaggt tgtgtctctc 29580
cctggctgtg tgacgttggg tatgatccaa gtgtcagatt actcaacttc aaaatgagga 29640
cagagccttt cccttctagg gctgccagga acattgaatg agagagtgct ggcagcttag 29700
tacaggtgtt cattgctctt gtatggtact gtctgtggca cggctagata aaatacagta 29760
gccactgatt caaatttcaa ctgaggagta aaataaactg aataacttag aaaagttttc 29820
ttcttttgaa tgactctaag aatttaagga gcatgtgagt gttgatggct ctaaaagggt 29880
aacagagccc aactagctca gttctcagca tgaaaatagt catatggcac agactcagtg 29940
gagtgggtgc acttcaataa ctggaagcac agatgcccta cagcagcatc aaagatggca 30000
ctctaaacta ctttcaatcc tttaaaataa atggaaacgc acatttagta tgcatatgac 30060
aacacgaagg acttcgattt tgctgatgca atacagtttt acaggatttt ttatactcaa 30120
attagtaaaa ttctgtattg catccaaatt ataaattata atatcatcta gattggacat 30180
aggaataacg accactggta tctgcccaga aagctctacc gcctgtttat aagctcctgc 30240
aggagacaca aaaagaagag aatttgaata taacttgaaa tgaccgtaat ctcctgcccc 30300
aactcatttc attaccaaac cgcctctttc ttcattattt ctcctgaagc acaaatctat 30360
agagaactca gctgccagtc tctcccactg cactcagcag tgaaagggtt aggcctaggc 30420
ttttcaaaca gaccagtgct tgtatcagcc cttaaacatc tctggagaag gaaatgggat 30480

ccttctttgg taattcattt ttgacagttg gggattaggt gttctgtatc tggggggcct 30540
tgctgtcttc tctcctcctc ctcccactgc agaccctctc ctcccctccc ctctccagct 30600
ctctgatgac tgcttcatgc tccttccacc tgaggactgc cagcacagcc tattgcagga 30660
acagccaatg aggggctggc tgtgctcttt tatttataaa attataaact caagcaaaat 30720
ctagactatg tgtccccaag atcagaggag cacaaatccc ttgcttacag attgcatggg 30780
gggcacattc tttaaaattg gtccctgatc tagactctag cctgagaatc atctttaagt 30840
tcagaatttc cactcatgac ctcacatctg tgggctccca cattgtcttc caaaacacac 30900
atggcatctg gcatcacctt cacccccacc ctcagagcct catctccctg caggtagata 30960
gtcaaggcaa cctcttcact cttctgccaa gcctcctctc ctcagctctt cccttcctct 31020
ctctttttga aaatattttt aattgtggca aaatatacac aacataaaat ttaccatctt 31080
aatcatgtat aaaagtggag ttcagtggca ttaaatacat tcacgttgtt ctatagccat 31140
aaacaccatt catctccaga gctcctttca tcttgcaaag ctgaaactct gtccccatta 31200
agcaatggct ctgttttcct ccgttccccc agcccctggc caccatcctc agttttctgt 31260
ctctgtgagt ttgattactc taagcacctc ttataagtgg atcatacaat gtatctgtct 31320
ttttgtgact ggcttgtttc actttccata atgtcttcaa ggttcatcca cgttgcagca 31380
tatggcagaa catctgtcca tttccaggct gaatggtact cttttgtacg tgtggaccac 31440
atttcattta tccattcatc cacgggaggg cacttgggtt gcttctgctt tttagctatt 31500
gtgaataacg ctgctatgaa catagctgta tgcctttgtc ttttaaagcc caaatctgat 31560
caagtcactc cccagcttaa aaccttccac tgctccccag cagtgggata aaggccagtc 31620
tcccctgtag gtctctcccg ccagccctgc tcagtcttct tgcttgtcat ccttggctag 31680
gccttgcatt gccatagccc tctgcctctg ttcacgctct ctcatcttgg agcatgagcc 31740
ttccatcatc tctaccagat gaactctcat ttcttctttc aaaaaataaa aaacccaaaa 31800
aacccagaga tcccaactgt cctggtgtct gcatagtctg cagcacacgc cccctccatg 31860
gcccttcctc cataagcaga atcactcctc actgttcctg cagcacctcc tgtgtgccca 31920
cacagctgtc ctgcggtggg ctgtgtgtgt gagtgtgccc cctctaggac ctgagctcct 31980
tctggagggt gggcacagca tccattcatt ctgggaatcc tggtcggcac catgctagaa 32040
cttctgcaag tgagtgcctt tggtgctggc ccatgggaga gctgttggta aggcatactt 32100
ttgcagattc cagttgctgc tgaggttgtt gctctttgca caagtttctt ctagtcacca 32160
gtgaagtgac atgtgtggca ggcatggccc agggaggctt tttcataaag aagaggttga 32220
atctttgggg ctgtggtttg aatatgtccc tcaagcttat gtgttggaaa cttaatccca 32280
aatgcaatag tgttaggagg tggggcctaa tcacaggtga ttaggtcata aggctctgcc 32340
ctcatggatg gcttaacatg tttagtgagg cagtgggtta gctattgtga gagtgggctt 32400
gttagaaaat tgagtgcagc cccctcttgc ttgctggcta ccatgctctc ttgcttttct 32460
gccttctgcc gtggggtgac acagcaagaa gaccctcccc agatgctggc accatgccct 32520
gggactttcc agccttcaga accacgagcc agacaaattt cttttcttta taaattaccc 32580

agtctgtggt attctgttat agaaacacaa aatggactaa gacaatcttc tttcatcaag 32640
ttagggtacc aacctttaaa gactgccagt ccaaggttaa aggaaacttt tcaagagcag 32700
tccaaacatg atctggccct cagctactct ccagggtcat gccaccctat cacccactgg 32760
ctcacacaga cgctgaccac tgcttagttt ctcaaactga agttttcctc ctcagagctt 32820
ttgcaaaacc ttttctttgc ctggaaaact ccccccacaa atctttagtt gtaggttcct 32880
tctcatcttg cagaattatt agtttgctct tcaaatagtc tctccagcta gactatcaac 32940
tccaggaggg cagagttctt cttcgcttcc ttcacccatg tgcccactga gtccagaact 33000
gtatagcagt ttgattgaaa aaatccacag ggtggaggat gagaggaccc tggatcccag 33060
cctcacagcc tcttacttca cctgtgtgat tttggtcaag tcctttattc ttcctgggct 33120
ttagttttcc cttatctaaa atatgagaaa agttcccctc tcctgggtat tctgggagac 33180
tcatgtaaaa ggcactgagc cagtgcagca catctatgac caggaagggt cagcttcctg 33240
ccttgcatga gacacacatt cccttcttca tgcacagtta ttcatgagtt aaatatgtat 33300
tgagaagtgg gttctcagga gatgatgcat ccacagcatt gtttgtatgc ctctgtcttt 33360
gatgtccctg cctgagtcgc ccactttaga gcccttctgt tcttcagaaa ccagactttt 33420
ctttcaatag tttcagtaat caatcgatca atcaatcaac caatcaacag tgataataat 33480
catgagtgag cccctgcccg tgctggctgt gtcctgctga aggcacacta agtgctgccc 33540
ttcccagaag cctcaggaag cttgcgaagc tcaggtgcat ggatgcctgg tggaatgagg 33600
aagggatgca gccaggtaga gaaatgccct gccatcactt gcatcagcat ctgtgaagag 33660
ctggccaggc ttttgctcac agtggttgac acagtcaagg agcaagggcc ccgtaggaga 33720
ggggagtcaa gggctccggg tgggaatgga gctgggggct gatgctggct tctggagcac 33780
tgtaatgtga ctgagaaagg tgaaggagcc gttctgaaaa agaagaaggc aggagctcgc 33840
acagctcttg actcatcttg acttcttttt cctgcttcat ccaagcaggt cgactctctc 33900
gtgatctcag agacagagtg aagtcatgag tgggagggga gcacagaaaa taagaccttg 33960
attcccagca ttgggagact ccctgctccc ctgagtctcg gaaaatagca cccttcaaat 34020
gttttaggga tccagatttg atgaagagat gttattttgg cttttagatt cttaggagag 34080
atttgtcttt ctcaggtcag gaagaaaatg ctgcccgctg cacattcttc gggacagact 34140
cttttaatta ttactagttt aatgtatgtt ttgcttagtt aaggaaaacc cctgtggttt 34200
cttgacgtgc ttcagtattc taactcacag ctgattcagt tcagggggct ggggagatgt 34260
cctcgacctc tggaaaggag ggtgcatctc tagaaataag gctaagtatg ccactgacac 34320
tgtctgcata aacgtgtgtg atctcaggtc caaaggatgg ggcctggtct aagccaggga 34380
cgtgggaaat cattttcctg tggcaacttg tgaagaccat tctgtgacct tggtgtctct 34440
gggccttctc ttagattttc taagttggct agtcagtgga gctgccatcc ctcctttgcc 34500
catgttctac tcccagagtt cctccaagaa attgcggagc aatgcctgtt tcatgagagc 34560
tgagtttgct gtgtcttcca cttagaaaca acactgtgga ccaggaggac acacagctcc 34620

cagggccatc accacacaaa gtgaaggctg gtgaatccga ggcttctagc ccttgccggg 34680
ccaggcccgc agcactccgc tccccaaccc agccgctgct ttgtcgcagg aacctcagca 34740
gggcagggtg tttcctagga ggacatccga ttcccagcca ttcctttcag tgaatcacct 34800
gagctcacat tcttttttct tttatttttg aagctcttag ccaatctgct tcgcgatgaa 34860
ccagttttgc ttgaagcaga caaacccgat tgtcaggaga cagtgatgat ttcttcagtc 34920
tctgaggaag agttttcatt ttccccaatt cgcaaaaaaa gtcaggtccc tccctccctc 34980
cctctccgta gaatattttc catgtgtgtt aacaatggct gagcgtggta gatgccagga 35040
atttctgtca accctcaaag aggaaagccc tgcctaatgg tctgcccgtt cttgttcact 35100
ccctgcccca ggctccccac ccgccttctt tctggaaggt ataaaggctc ctgcttatac 35160
ctggcactgc acgcttcgct ccctctgatc tcctgactgt catgcccagt gtctcagcct 35220
atcattctac ctctaactcg accttgagtg accttgagca agtttctcag gattccacct 35280
ccaagtcact ctccctttgg gatatgcagc actaagttaa gcttgcctgg aaaacatcac 35340
ttgaagctgg aaaaccactt ttaacacagc gggaaaagct atttgttcag acaggagtgg 35400
ggtgggtctg ggcagagcac tgctctaact tggccatgcc gtggcagcag ctcctttaat 35460
gccacttttt cctggcgcgc ccgcggggcc tggagctcag aaagagggga acgctccctc 35520
gtctctcaac agttgctcca gacaggtcag caaacatgga attcagaatg ttcattaaac 35580
actggctgtg tcttttgtgt tcaaaagcaa gacactctct ctgaaccatg gccccacaga 35640
gagtgcagaa tgtgtgaaac ctgccgggaa ggtctggacc ccttgcgggg cagtgggcag 35700
caccgtgcct ccgttcacac cactcacatg gctgtgcctc tgcttccttc tggcatggct 35760
gcttcttcct caggtctcaa ccatctccct cagatgctct ttcccatgtt tgtggctaca 35820
ggtccccgtg acctgcagag gcagagcact caccagcagc ccagcctcgt tgcgcaccca 35880
tgtttgcatt tgcaggccct agaaccactc caagctccgt gtggcgagat gcaccctcct 35940
gcccttcact ggggagctgc cctcctgttc acagcggcac ctgagtcaca catctggagc 36000
catcctggac tgcctcattt ccccgatggg gggtttccct gacttcatcc atcctgtctt 36060
ttgggtcccc ataataactg acatgggtcg gcccgtacca gcccctgtga gaagggcttt 36120
aactgccttc ccaccccctg ctcatcttag agtctctcta tagtgctgct gaaagaatct 36180
ctaaatcagt ggttctcaac ctcagccgca cattgagaat cacctgggac ccttaaaaaa 36240
atcttaactc ttggtccaag aattctatta caatcggtct gggatggggc cctacaggta 36300
tttttttaaa gctctccagt tggtaatgca tagctagagt tgagtatcgc tgttctaacg 36360
tgcagatctg gtcatgttac cagcctttta ggtggtcttc tttggctttc tctatctaaa 36420
gttcaaaacc gaacatgtgc gcattcagtg cacccatttt caactgtgca ttaacacatt 36480
cagcccacca gcaagattta tgaaccattt tctgctgttg tatataacat atcatatgca 36540
taatggcata ggttattgtt ttcttcaaaa tatatgagat gtgagtcctt ctacgaactg 36600
actcacactg attgcccaac ttcctctctc gaggtctcat cctctttccc tgcagccgtc 36660
tccctcttgc acgcacacac acacacacac accacacaca cacacacacc acacacacca 36720

gggtcgatgc catctaccct ggacttcatc ttgaactcct tcgagtgtga gtcattactc 36780
ctttgtgcac ctctgctttc tcttctcaag atgttcacct gcttgaggtc agttccttga 36840
gcgtcttcca cttgccatgt tcaccacagt gctcaacatg cctgaatgca tggatggcga 36900
cttctcagat cctcagtctc ctcatctggg taataaggca ttgggttggc gggtccatct 36960
ggtttcttcc agctctgaga gtgcatttgc tctgtgattc attcgttcca caacacttca 37020
ccaattaaag agagggtaca aaaggtgaac atccttggct cccagcagat gctcctcaaa 37080
acctgaaaaa tcagataggt gagggaagat tgaatgaaag gcctcttatg attctgcagc 37140
aattttggtg gtttaagaac tctatggaaa aatcatcagt atttctggaa ttgaagtaaa 37200
atggatagtg agcctctgtg tatgtgaagg cccgcatctg gaacatgaaa gaacctgtct 37260
gatgtgttct agtcaggaaa gcaggtagcc aatactattt atagaattta cagaaactga 37320
agattttgtt tctactgatt ttcaaaatag tattatgtct gatttttttc ctcagaaata 37380
tacttcctgc tcttctcaac aaactcattt gaaaatatga ttagaacatg atagaatttt 37440
actcatttgc caactgcggt tcccatttca catattgtta gaattctgca tggtggcttt 37500
gccctttaac cactaactga taaatgatgt agttagcttt taaatgtgtg gaaaaatata 37560
atttcaggtt caaccatagg tcagaagtac acgtgttttg ttagtctatt tgtctctcag 37620
tcatctcatg gaaaattctc agcttttggt atggaaataa ttttcttgaa ggcaatattt 37680
gttgagtgac tgacggaatg aaaaacgcca gttgcgtaag tgtgaaaaag atctgggtgt 37740
tttcattgga tccaaattcc acatgagcca acaacagcgt ggtgtggagg ctggagcaca 37800
ttaataagaa cagtgtccta aattcaggag gtaatgctct gcccatgccc tgtgcagctc 37860
agacggtgtg tgcagtgcag tatgtaaccc agggcacatt tcaggggccc acagggagct 37920
gcagcttgta aggtggagtg cagccaacag agcagagagt cagaatcccc gcagagtggt 37980
tgaaggcaca aggatgcgca gcaaggaaga cagacttata ggtggtgcga ctgccatcct 38040
ctggtactga aggtgctatc atggagggag ggaagtagat tgaccctcct ggctccagag 38100
tacggaactc agacaaacgg tcagaagctt acagggaggc caattttgga tcaactttaa 38160
gaagaatttt ttaaaagcta gagcaatcct aaaatggaat ttgctcttta taaagttgcg 38220
aatgcctcac cctggaattg cttaagcaaa gttgggacgg gcagttgtga gtaatctcct 38280
ttccaatcca tacccgcaat caccagaaac gtggacttcc ctgacactga gcacctctta 38340
attaagcatc tcataagtga acaaaaccca gcccttcaaa gaagtcactt tatttatgtg 38400
tgggtctgca gcttggattt cttgataatg ttaaataaaa ctccatctac tcttccacaa 38460
acacttcaag aaacctaaga cttttggcca gagtaacacc gaggtttgag agaaaggata 38520
tgtgtgtgag aggtgtggtt tcattagaac atattatttg acttcatgtt gaatcaacac 38580
ttttgtgcaa aatgcagttt taccagcctc tttccttgtt ttggtcacat aatttaactt 38640
aacattctcg gtacttgatt ttctaacata aaatgggatt gagaggggaa ttttgaagtt 38700
cccatggtct gtcctctaca ttctgacagc tcattatctc tgcggtattg ttctcacatt 38760

taagtgaggt tagcggaggc agaggcctct caggcctgaa gatagcctct gttttcaggg 38820
aaatactaga ctgtgagatc tgtgacactg aagcactaag ttcatctcac aaaagcaacg 38880
tgctcttttt aaatggttga tcaaagttac tttcaaaagg aagtgttagt ttttgttatt 38940
agccgaaaca agagctgctt taatgtagta tatttaaaat catatctcaa ttaagatgtt 39000
attcaaatac tatttgaccc accaatctca ttactggata tatacccaaa ggaatagaaa 39060
tcattctatt ataaaaacac atggctgggc acagtggctc acgcctgtaa tcccagcatt 39120
ttgggaggcc gaggcgggtg gatcacgagg tcaggagttc aagaccagcc tggccaagat 39180
ggtgaaacct catctctact aaaaatacaa aaattagcca ggcgcggtgg caggcacctg 39240
taatcccagc tactcggaag gctgaggcag gaaaattgct tgaacgcggg aggcggagtt 39300
tgcagtgaac agagatgaag ccactgcact ttagcctagg tgacagagcg agactctgtc 39360
tcaaaaaaaa aaaaagaacc acttgcatat acactattca caatagcaaa gacgtggaat 39420
caacctaaat gcccatcggt gatagactgc ataaagaaaa tgtggtacat atataccacg 39480
aaatactatg cagccataaa aaagaacaag atcatgtcct ttgcggggac atggatggaa 39540
ctgcaggtca ttatccttag caaacgaata agaaaagaaa acaaaatacc gcatgttatc 39600
acttataagt gggaggtaaa tgatgagaac acaaggatac actggggcct acttgagggt 39660
agagggttga agggagagaa gcagaaaaaa taactattgg ggtactaggc ttagtaccag 39720
ggtgacaaaa taatctgtac aacaaactac tatgacacaa gtttacctgt ttaacatacc 39780
tgcacatgta cccctgaact taaaaaaatt tttaaaaaga tgctatgcaa taaaattctc 39840
aattaagaat ttaacttggt aaatgttcat ttaatgatct aaaaatatgt gtctggatgg 39900
ctctagcaaa aaaataaata ataagtttct cagagatggt aaggctgaaa taaatgggga 39960
aaaatctgaa ttgtaatcct ttttctgttg gacctggtgt tggggtttca cacttgtggg 40020
tgaatgtggg cctcctgtga gcaccagcac aaaagactaa actgaacaaa agattaaatg 40080
tcacctctaa aattctgtgc aacaagactt ccagccacag aatgtgcaac tcagatttcc 40140
aagtaaaaac acaccaggaa gcagatctta gatctctgtt atctccttgg caccagctgg 40200
tattcatcct caatgctagc tagagttgaa ataaagagtg aaagaacttt ctcttttatt 40260
acttaataaa cttccttttt tgagctgttt taggcttaca gaaaaattga gtggcagttt 40320
cagggagttc cagcacggcc cctgtttctt tctcatggtc cctgcaggtt tcccctatta 40380
ttaacgtctg tcattagcat ggcacatttg ttacaattaa tgagccaata ttgatacatt 40440
attcactaaa gcccacaggt tgcgttaggg gtcattcttg gtggtgtacg ttcttcaggt 40500
ctggacaaat ctataatgac atgcattcac cattactata tcacgcagag tcgtctcctg 40560
gccctacaag tcccctcctt ccccacctgc tcactcctcc ttcccaccct ccccaaactg 40620
tggcaaccat ttaacttttg actgaatgga tttattctta ttctgcctta ttgtatgtac 40680
accatatttt aataagataa aataatagtc tatagtagac ttctgtaaat actcaatgaa 40740
taaatacttg catgaatgca ggaaaaatca atcagtcttg caggatttct tatgcgttac 40800
atcgtcctta taagaaagca gtcattctca ccgagatgtg ctgagcagat actggacatg 40860

ttctgaccca gataagggct gggtggaagt agggctggag acacagagac ccagtgccaa 40920
cttccaggac ctcggaagaa ctgaaggcag agaggtcctc tcagtgtgga ctgggcctct 40980
gctggcagcc accagcgggc acagagctga tgtgtgttat gccacgtggg gaaaacctac 41040
agacgattct gagaaaggct cacagggaca ccctctgccc ctaaaagaac aatttaactc 41100
taatttattt ctgtcactct gcattttctg acctttccca agtgtacagt tttatatgca 41160
tttaactgcc aaattgtcat gtgagattat atggttatat ttcattaata tattctagtt 41220
tgttcagctg ttcttactgg gtgaatttgt gtggtttcct gacatttttg tttttagtag 41280
tgcctcagta gttttataca taattacgtt tcccttctgg attatttcct tagtatctag 41340
ttcaagaagt gaaatcgctg gattcttgtg gtaaattttt gaatttcaca gtataatgct 41400
gattttctca aagtctcaca ttctaagaaa gtataatgag gcaaaacaaa caacaaacat 41460
cttaagttga tttttcccta gcatcttttc cttccatctt tgcttgtaga atctagacta 41520
tttcatgaac ccaagatata atcagtatcc ttcttcagta tggccaaagt gagtttctca 41580
ttattttacc tccccttcag gaaatgactt ttcatcttgt gttttgggag ccatagatgg 41640
ttctgggcag gaaactggct ttggatagac ccagcatgta gatggctatt tggccttgct 41700
cccagtataa cgatgcagtt ccctgtgaaa gggtatgagt aggttttggg gctctggata 41760
ccgtgtggcc tgaagagaca agggctcaat gccaactctg cctgtttcca actgtgtaac 41820
catgtgagcg tcaaaaatca tggacgtgct ctggttaaca ctgagtggga gctcaacaaa 41880
ttattatttt taattgttac ttggacatgg ccaagttgac tacactttat gttctgctac 41940
ctgccagtct gaaagtgacg ccacagaagg tgaaccgcat gttgggagat gctcctcatc 42000
tgcttaaatg aggtgcaaac acagcccatg cgcctgctct tcatgactgt atctgtacca 42060
gcaatatttg tattggcaaa tcacatgccc cagtgggaac tacttaaggg gaattcaatg 42120
gatttcattc cttttatgta attggccact tagtaataga cgtgtaggtc tcttgtgtgg 42180
ataaggattc tgccttttat gtaagatatg tgttgcaatt cagctttcag gtcccagccc 42240
cgggaaggct ccaggccttc acaaactggc ccacccacga gaaggaaagc aattgtccaa 42300
atgtgggtag cttttcttcc cactgttgtc agctgcttcc aattagcccc catatacata 42360
atcccagttt gtgtctgtat cagtacaatt ctcccatgtc aatgtgaatt ttaagccaca 42420
gagggaaagg ggacagagaa tatgctttca ttcagctctc ctcgtctcac acctcttgcc 42480
ctgcatgcat ttctttgctc tgattaaacg agcattttat aagccacatt tgctgtgtga 42540
aaggcaaagt cttccctccc acggatgacg gtctccaggg atgtgtgtgt gtgtgtgtgt 42600
gtgtgtgtgt gtgtgtgtga gagagagaga gagagagaga gactgtaaac atatatctct 42660
gtgaaacttc attttccata tgtgaatttt tggaaccgag acaaatggaa cttagctaaa 42720
agatgggaaa ggtagactga ctctgactta atctacttaa cctaccaggc aatttataac 42780
ttgatggcct aatttttgca gcacccagaa gcaagcctgt ttcagcacgg caaaggctca 42840
gctgctaagt gggcagcatt gttggaggtg agcagcttag gctgactgtt catcaaagga 42900

ccaagcgctt gaggttcgct catcgctgga ggccagagtg gggagggcca tttaactgct 42960
caaggccatg gaactctact gtcagtttca gggaaatttg ggaccctgga gcacaaacca 43020
aaactccaat taaccaggag aggaactcga tccccaggag ataagtgaag agtaagaagt 43080
ctatctttag aaacaagaga tgtccaaggc tagaaagatg gggaaggagg gtggaactgt 43140
tctggaagtg ggtctcaatc tcagcaccag cagctctcaa gactttctag agaaggaaac 43200
ttcatttctg aattaaaatt agtcttcaat gacatggcag ggatttcggc acactctctt 43260
gcgtcatagg ccactgtgtt ggaggcagga gtgttggctt tggaggcata gagattaaaa 43320
ttagagtaac acgtgagcac tgaaaaggtt aaacagtaga gacatggagg actcccgacc 43380
cccatgtacc cctttcttaa ccctttaatt aagatcacag ccctagaaat agcttgcaaa 43440
ataattaact actgatcatt tataccttag tgcttctgtg agcatgtttt ctctttcatt 43500
gctgctcatc tgcatggaaa aatgtgcatg ggtttctgaa tataactcca tggtgcttgc 43560
ttccattata tttgtgccat ttggatcata actgataagc aaccaaagag tcccatatta 43620
ctgcacgttc ccatcgctat tttatgtgaa ggtggtcctg ggggctgttc tgaattctca 43680
gtttcctttt ttcccctccc cagttctttg aaaatatcag aaacggactt gtggcatctt 43740
tgaaaagcta cttaaaatgt gctgctgtgc tctgaacttg aaaatgtgct tttaatacaa 43800
agtttgtgca gcccttgctg ctcatacgag atgaatctta ccatgtggtg gatgcccgtc 43860
tcatgccagg cactgtgctc taagcccatt ggtttatttc agtgcttgaa attggctttc 43920
gagagaggca ccacggttcc ctttttacag gagaggaaac accagaggat cagagatgga 43980
gagtctttct ccacaaactc acagacccca aaggcaagct cagggttgtc agcttccaaa 44040
gtctgcctgc tccaggacct catgttgcat ctccattctc ttcactgagg gtcaaatgga 44100
aagaacacat gggggtcaag tttcagaaaa taagagaaat gaagaaatat gtgcccggaa 44160
gcaagaacga ccgacctcat taaactggct cccttcacct cctctcacat ctttttctgc 44220
cttttggcca agttttctct cccccgcatt tcctccttga tctcgtttga atcctcttcc 44280
ctggtgaagt catttaggtt caggctctta ttttactttg gtccataatt tagatcgaac 44340
cacatgtgct gatgtgattg aaacgatgtg gaattctctg gacagagata gaattatgga 44400
ggggttagtg tgtgtgttta agattaaaag accaggtgta tgggaggaaa tataatgaac 44460
aaaaaatagt attttaaatg aatactaaac ttgcactcat ggaaaaagtt ctcttcccat 44520
gaggttctcg caaagcattt taccatcagc acacgcagtt tttctcagtt ttctgagatg 44580
gggccatctt gaatccaaca gacaacacac agcatcagcc agactaacac aaaggacgtc 44640
atgggcatgg acgtaaatac tggtgtcaac actaggtctg cacctcgaga ggagtggagc 44700
aaaaggatgg agtggcagat gaaggtatgc tgttcagaaa ggaggcagaa atgaaaggaa 44760
gaccatcagt gcgctccaca gcttgaggac cgtcctggag ggcaaatgcc agctgctcac 44820
ttctgaaaag aaaaattcca gtgaaatgag tacagtcatt cttaggatta ctcacttgat 44880
actgtgtatg tctcttcttg gcttctcatc tccacacaaa accctcaggt ggtaaaaatc 44940
taattaaaaa aattatataa agtcttgtag atttattagc ctgaacataa tagatttttt 45000

ttaagcacgt taagtcttcc atggactaaa agaaaacttg taaacctaag agaacctcta 45060
tttttgatat acaaaataat acatttcctt aaactatgat cttgatacta gaattttaat 45120
taaaaaatac ctgcagttta tatgcaaagt tatagattaa tgcttaaaaa taggttgtat 45180
gtagtatcca caggtcatgt ttgactgtca aatagatgta attttaattc ataataattg 45240
tgtcgtgttc ttccccacta gaagccaatt atgcaagctt caccattcac acatggaaaa 45300
taatttaatg gagtactcat tgcaatttca cttatccaga attggctgtt gttctcagag 45360
cagcttgtgt tgccttgtta aggagaatat gttagtatcc agacatccag aaaggatcct 45420
ttactgtttc agagtccatt ttccccactt ttgaaataca cacacaaaca cccattcatg 45480
caaaccaaac agagattgta aagtgattcc actgacattt atgcacttct tttttctctt 45540
tggttcttca aactctcagt cagtgcgcat ttactcttaa tttagatacg gtttaaacct 45600
aattagaaac cagaagctct tgtatttcca caaaggatta tgacagcccc aagaaaagat 45660
agtgaaacca ttatataaca agataaaggc ttcttaacaa tacaaggatg gattttctca 45720
ttgatcttag ccttctgaat tttagaaatt gccatttcaa agtctaaaac aaaggaaaat 45780
cagggaataa aagaatggta agtagacaca aacctactgg ctccatcatt tctgttttag 45840
caaataacct gccacatata ccaatagccc aagagatggg catgtccctg catttcctgg 45900
tcaaggtgac aacactgcgt cctcctggaa gaggtctgcc actcaccata ccacaaacca 45960
aatataataa aatcagaagg cacactatag tgaatttttt agaggcatgt attgaaaagc 46020
atctcaaaaa gcattctcga agcttccaga agtcaactca agttatctga aaagtgacac 46080
ttttgatgat tgctcgctta atactgggag agccagatga agattcctcc ccacttcctc 46140
agatgtgcaa ctctggaatt tcttagtgtt actggagatt cctgctgcat tctgggcctt 46200
taatgcataa acactgagat gttctaagga aattactccc tagggaggag aggggtggac 46260
gaggagtaag ctttgctggt gactcatgcg ctgtgtggaa actccctgca caagtgagct 46320
gcgcagggtg agtctaaagg gttaatgcac tttcaaaagc ctctaatttg ttattccaga 46380
agagtaattt actcactaga agtatctggg tggctactaa cacatttgtg tctttaaaaa 46440
gatcagtttt attttaagat taaaaatata aagcaagagc tggaaagtca ctaaaaactg 46500
acagccagtt tcccattttc aagagtattt attaaaaggt tctggttgca gaaggaataa 46560
gaaatggctt gagatcatga cacagtgaat catgttgtaa acatgttagc tatggctgtg 46620
aattcaacca gcgatgagtt caagcgtccc cagaaggtgt tgggggaatt agggacatgg 46680
ctgtgtttcc ccagagaaaa gtggccattt tactttccct cttcactaac atgcttttga 46740
catgcatggc agagctgaag gcaaggggaa ggggacaaca tagtaagtga ctaagtggct 46800
tttttttttt tttttttgcc aagtgaagct gagtcatatg gcctctgtca ttccaaaact 46860
attctctacg gctgcattcc tttcgctctt gccttccttt agaaccctgg agaaggcctc 46920
ctgaagcctg gccctattat gtatcctgac aaagataaac ttttccaaaa agctgcatgt 46980
tgtttctagc acagtttttc ctcgcagtga ctacgtgatg aaagtaccat gcagaggagg 47040

tgtctgactg aggcgttcgt ggtgtgtgac agagtcccct gcacaggaca gccgcactcc 47100
cctcttgcgt cctttcctcc catgtttgca aagcctcttt ccctgtcagc agggggtgtt 47160
ctggcagttg acatttctga aaactacagc ctacattttt aaaaaatcca gtaagtgaaa 47220
actaaaaaat taataccgtg gtcataatag tgtggcattt gataactaat gaggcactgt 47280
cgtgccagct attattttca gacatttaca gtcctttttt aaatacaaag aaatatttgg 47340
tgtgaaatgt tccccgggag ctggtgcaag cagaggcgac agggcaaggg agcttgggtt 47400
gtagcctcga attcctccgg ccagggctac cgtcagcctg cggcacacaa gtaaatcaaa 47460
tataaaacca aaatttctgt aagcaaatca gtttctaact cactgtaacg aattatcttt 47520
cgcacatcac agaggcatct cttttcactg tcgagtttgg tttgcttggt tacaaaaagg 47580
gcagttcaaa agctttggtt gctattgtga aagtcagctg aattccttcc accgtgctgg 47640
ggtggggtgg ggttcacgca ggttctcttt tgtcaccagg ggtgctgtgg attcacaagt 47700
aagcaagagg ctcctcaggt caagcctctg gctgctccct gaggtcagct gcctagcttc 47760
tcctcctctg agatagacgg gaacaaagtc tttgatgtgt gcatttctca agcttgacaa 47820
tgatacagct acataaaaac ccatgatttc atatagatat tccaaaacgt aaaagtaaac 47880
catgcatcca cagagacatg gaattacaga actggatgct gagctggtca cttgggaggc 47940
aggcgtcctt gccattggtt tatgcctcag ccccaccatg cagtggctgg ccaggtgacc 48000
taggccagtc ctgcatcctc ggctcctcac ctgcctggtg ggacagtgac atctctcctg 48060
cagcactgct gtcagggtga gggaggtagg gcgcagtttc agaaaaccat tgggctgcac 48120
ctgcgtgagc acagctgcag gagcaaaagt cagaaaggtc agcaaaggat ttcaggagca 48180
aaggtcagaa gaaaccctca aggtggttgt gtctgcagga aagtgctgtc gtctcctgca 48240
atgctttcaa gactattcag aagcacagtg tgaagggaga gccggagccc atggggaaat 48300
gactccagag tgttccacgt gttggaaggc atctgttgga aaacggacat tcaagcaaat 48360
agttgcctgc atagacaacg cagaatgact gggaaagccc caacaagtta cctactggta 48420
aatgaggtga gaagcttaaa gtgagaaccc cattgctgcc tctttttcac tttaaaaaca 48480
tttaagtttt gaattatggt aaaatacacg taagatttac tactgtaacc atttttaagt 48540
gtacggttca gtagtgttaa gtatattcac attgctaagg aaccaatctg ctacttttgt 48600
ttattaattt tttcctgagg ggaaatattt ttaaatttta aaatatttaa ttgacaaata 48660
aaaattgtgt atattcaagg tgtagaacat gatttcatat gcacgtacat tgtatactca 48720
ttaccacaat caaagaaatt aacacatcca acccacccat agttgccatt gtgtgtgcgc 48780
ggatgtgcgt gtatgtgtgt gtatgtgtgc acgtgtgcgc ctgtgtgtgt ctgtgtgtct 48840
ctgtgtatac gtgtgtgtac atgtgtgtac gtgtgtgttc ctgtgtatgt gtgtctgcgc 48900
acgtgtgtat gcatgtatat gggtatgtgt gtacgtgtgt acgtgtgtgt gcatgtgtgt 48960
atatgtgtgt ctgtgggcac aggtgtgcct gtgtgtatgt gtatatgtgt atgtgtgtac 49020
atgtatgtac gcgtgtgcat acgtgtgtgt gtgtgcacag gtgtgtatgt gtgtgcctgt 49080
gtgtgtgtgt gcatgtgtgg tggggacact aaaaatctct catcaccttt ttagtcaaaa 49140

gaacagttgt tttggtttgg ctcttctgtt ttaaaatatc agaacaataa taatttccca 49200
cagacaaaat cctcaatcct caccatcctt ctatttccta tattcatcat aaacttcatg 49260
cttgatgttg aaattgtttt ctgaaaatag agaatacaaa gaggagattt taaaatgtca 49320
gtggcagccc cacactcctt tttaatctta tttcctgata tcttgagttt acttggacgt 49380
agagttttcc ttgactatgg ttatttctgg tagtagcagc tccagattag gcaatggttt 49440
tcttcagaga tagcttagag tgagccccag aacaaggtca atgcgaagat tgcttgtgtc 49500
tgcgtgtcca gggcacagtg atcctcatca ctagccgggg ggctccgtga ggatctgctc 49560
ctggtcgttt ctgttctgta tcttctctgc agcccttact gaagccgtta ccaactggca 49620
caattcaatt cctactgtac ccatcatgca cagatggctg aagtattgag aacgctccag 49680
tgaccgggag gcaatagtct gtccacatct aagaacacac ttggaataac cttagagaag 49740
agagagagag agagaatgca tggttagtag gttatcaaac tcctatgact tttcacagga 49800
aaagccctca tccacaccaa ctttaggaat gtgtagaaag aagggtcagg gacaggggtg 49860
agtggtgggc agagcagttg gagggcacag ggaaaaggca tctggtcatg tatttggagt 49920
aggaggtctt gctttactat tgaattgcag ggacactttg ggaacagtgt tcacttcttt 49980
ttgcaaccat ttcttcagag aaaagtcatg atactcaagt cttcttacaa agcagtttga 50040
ggctttgagt accagactga ttacagagat gagtatgaag cattattgta gtatttttaa 50100
gtgaaattca ctaaatgcaa ataaacctag caaatgctct atggttaatt tttttctaaa 50160
attcagataa ttaagacaat tcattctcct gaaactgctg ttcatgtaaa aaggaatttt 50220
atcgaggtgg cccttgagtg ccaaacagcc tgtcctcagc tgcaaaatga gtcgttgatg 50280
atcctccagc aagggatact ttttagctcg tgtggtgatt gctgcacacg ggatatgtgc 50340
agcaagtatc tgctgagcta ataataaaca gcctcagaca gaaagacagt gggcacaagg 50400
tcatgcttaa aaagacccct tgttctactg catcccagct ccccaccatg gggcctcaca 50460
ggccctggtg accaagcaca tcagacctgg ttcttgctca gtcctgggag ccacagaacc 50520
cagcacgtac tttaccccca agaccagact ccagcttggc ttttgtcctc ctctccagga 50580
ttggtgacct cctaggtcgt gaagctgtga tgagcaaaga cacactcctc tccattctcc 50640
caacttcagg tccctttgac agtgtcagca ggcatttaaa tagcagacca cccacagcag 50700
ggctggtaga tgcagtgaac tcaggaagat gcctgcatag actctagtgt taaagacaga 50760
atccttacaa ggaaccccca tagttaccta actgctgtct ccagtggtca tagaagtgtg 50820
ataacccact aatcatcatt ctctgtctct ctgtctttct catacacact tacacacaca 50880
tacacacaac cttgttgctt aattttcaga gagtctactt tcagaaaagc cttcaggaat 50940
acatcatgta caaaactgag aaattacctg aagtatcttt aaatttagta aaaagttgca 51000
ttgttttttg aacatcacac ttgaaaagta catgaataca aacatactta ggaaaaaaag 51060
ctttaattaa tttaaaaagg agaacaatgc tatatgctgt atcccacctt tctctgaatg 51120
ttacattttc tcccctatcc caggctgcat ctaagaaaac tcagagggaa tatgctatct 51180

atcttttccg agcaatgaaa gctctgggtt ttttccttgc ttttcagggc acaatacttc 51240
tctttcttcc tggttagaca ggataagttc tgagtcccct ggtatcatca gcttacttct 51300
tctctgttaa atattcacaa aaaatcacta actttcatgc ctcagcaaac ctccactgcc 51360
taaaatatag tgaggtcatt catcttcgga caaattgccc caactacggt gggaaaagaa 51420
ccaatgtgtt ggactattta tctaattttt gtttagttcg gggatacaaa taaatgcata 51480
gatacataca aacatgcgta cataatagca gcagcagcct gtgaaacatt gacaagacct 51540
ggagttggaa gaggactttg ccatcctcca gtccaacagt tgcctgtcac agattagacg 51600
actgggatgt gcgcaggcga ttatttgcaa acggccctga gtcccccagt ttatgtctta 51660
attcgcagcc agggctgatt gtagaagcaa atttgcaaac atgtgcaaga agaaatcaca 51720
catcctagag cttggatttc ctcgtttctt gctatttcta tccgtagaca gaaccattgc 51780
tgagctgtta aatttgtctc cttcccctat accagtcttg aaaaaggaaa ggaagtggag 51840
caaagaaaaa gaaattaata aagccggcag atcctaggag aatcttattt aatccaagct 51900
ttgtaaagtt ttgctttatt ccatggcaac atgggtatac acatcccacc ggctgtttca 51960
gtggctcaga gcaggtaagg cctgtgccaa acgccgctag caggaggaac aacgtggaga 52020
cagccccaga ggtggaacgt tggcccttct gtggctccgg tgtctcagga cctccctaaa 52080
gcccagccct gacactgagc aagtttccac cactgttagg aagaagtaga aaggaatttg 52140
gagggttggt gttactgttc aagagctgga aggcttctgc ccccattccc attccattaa 52200
ttgcgtgagg tagagaactc atagaagata ggaacacata tgctgatttc caaaattgcc 52260
tttgtatatt ttcacgtgaa gactttaggg gcaaaagaaa agaagcaagc attttgaata 52320
tgtgtttcaa tttgccttct gttatataaa attgtatttt gcctattctt ttttcattat 52380
tcggaacctt caagaaataa attaagttct ctcaaaaatg tgttttttga aaagaggact 52440
aaaacagatg gcctggctgt gttaaacaca gggaccagac cagcacccac ctctccacct 52500
gccctgcctt cactggcaga attgtgatcc atcatgttct ctgttcaatg tcatcatccc 52560
tttcagagca tgggtctctt cctttctagg cagtcttacc aggatgcatg ggtgtgcctg 52620
cgtaggcaca cgcacagctc ccaaggactc taaaaaaaga tatttttctg cttatatact 52680
aataatatgt tagagattta tgtttcaaat tagtacagaa tcacatggtt ctctccaaat 52740
tatatttgag agagaaagaa tagaacaaaa tttattttac aaaaatactc agtacattta 52800
gggcatatac aaagatgttc cagaatgtag cttatctctt taaagacaat taacacagtt 52860
tctgggcaag gcaaggcaaa atattcagta acttagcaac accaacagaa gacagccaat 52920
attgcagcac atttttctct tggattgggt cagagagtac tgcagagaaa atggagtaga 52980
gagacctgaa atactttcgc acacactgtg gtcagtgcag cgtccactgt gtgccacagt 53040
aatactagaa actccctggt taggccttgg aatccagctc tcatttcgta tgtgacctgc 53100
agggaagtaa gttaaatgca cacgttttat caagttcaaa tgcaaactta attttaaatg 53160
tatgcaacat cagtttaagc gttgtagcta ttactagcaa ttgtacctat tactagtctg 53220
tactctgcac aactttggag tatactgcct actcaaggtg gattttagag ctctatttgt 53280

ggcattatat cacggacaaa agcacgttca tcagagtcag aggaatgtgg tgcaaatccc 53340
agctgtccca cttaccagct gtgggacttg agtaagctcc tgaagcagct gcacctgcat 53400
tttctggtgg gcaccatgga gctgtcagca gtgctttcct cagagggctg cgggctggat 53460
gaggtttgct ggtgcatgtg aagtgtcaat cattgctctc atgagtggtg atgctgatgc 53520
cgttcccttt tttagggaag tgattttccc ttacaaagtt accaacagtt tcatgttggc 53580
ccatttttct attaattgtt tccactaata ggaccaacag tggtagtccc atcattttat 53640
tactgcttgt cgtagcacaa gcagttgctt cattgtgttt agataaatat tgacggctgc 53700
ttttaacagt ctgctgtttt gtctcctttt gaggtcctta aagtaatcct taaaaagata 53760
gtgcagatgg aaagatgtct ggagtcagtg aacctgcctt ctttcctgtg tgcttgtcag 53820
tttctaaaat gccatacaca aaggactttc atgatttctt tttaggtaca tgattacagt 53880
tcaattcact tcactgtctg gaaaatttcc ttataatcag gatgaaattt ctcatgttag 53940
cctttcacat ttcactactt ttagataagg aattctcagg ctttgctata tctgactgct 54000
cttggaggct gagcttttgg ctaactacct gactactttg tcgtttctct tcccttggaa 54060
tgaagcaaat atctaacttc tcactcattg tttctgctat tttaccattt agtcatctgt 54120
gatttttcta aatactgaaa gacttccctc aattcaaact atgtgccgga tcaaggaaag 54180
ggcagttgga tattgcagac agcatagtgc aattgtgaag agtgtctgct taccagccac 54240
gctgccttgc acaagttatc aagcctctca acccacttcc tcaatctgta aaataggtat 54300
gagtgtagga ccttcccagg ggattttttt gtgactatag aatgattctc agaagacttt 54360
caggcagtat gtgggtgagg cacatgctgg aaaggcttct gcaggtgcag tgatcaatgc 54420
ttttctcagt gtgtacatcc cataatacag acacgttacc agaaactccc tagccaggac 54480
tttgattgca gctcacattt tgtatatggc ccatagggaa atgaagtgtg tattttttat 54540
aaagttcaag tgttaactta atttggaatt tactatcaaa tctcagttgt tatgggcatt 54600
tatagctatt aatacttcgt cccatgtgtc ccatgaggaa accaaggaac agaaattaaa 54660
gttctttctg gagtcccctg aatctcgttc ctgttctttt gcaccctgtt aattacatag 54720
agacattcac agctcttctg accttatcag cgttaaggaa aacagaaaac cagcgtgcta 54780
tttgttctgt cccttagtca agccttctca acatatattt ttcttccaag attttgcatg 54840
tgcacaggga tgcctatcct ctacaagaaa cacattttag gcaaattata attaaaatgc 54900
tgtttacatc tcttcacctt tagaatttaa agaatgatca tttcttagat tgcatctcag 54960
acacaccctt cccctagtct ggagagggcg aggcccatgg gtactgcaaa cagcctgacg 55020
ttgtcagggg cggtctcaac ggctcattca ccacatctgc ctcgcgaagg ctaagccatg 55080
tgctgttacc cctgctgcgc tctggctcat tctaaggtac acgctattaa ccttgtgaga 55140
aaacaaagag gccagcccca cccttcctgc tcactctgag tcacggtgaa aatgtttcag 55200
gatctcgggt tcgaccatga gtcctgtcca ggtccaggag gaaattcgga aggaccacat 55260
gttcactctg agatcccact ttcatttccc tcctggttga gcagcattaa tactctggct 55320

agatttaaat tctggctttc tccagttaga actgaaagtt atgacaatgt aatcaaaata 55380
gaatgtgggt ttacagctgg ccccctggcc tggtttgtga acataaaaca gaaacagaaa 55440
gtgtaagtgg tgacatcata ttctctcatt caatgtgaaa ggccaccgaa gtctttccag 55500
aattattttt gagaataata tgaattttta aaaaatacct aattatttta aatatcgtct 55560
tgcttgctcc ccaaatacct actgttttca acttggatat acgacatgat taaagaatat 55620
ctaatatttg ggaatgcata ctttaacctt ataaactacc actgtaaata gacagactca 55680
ttaaagtgaa aggacatttt aaatcaatta gtaagcaaat caattaggtg gcaaagacaa 55740
gattattttt ccttatggta gttgaagaat aatgcttaac ctgtcattct aattaccaag 55800
cacggtgttc tctttggaag atcatttcaa caaaacatta ttttcatcca gaatttgaac 55860
cttgagattg catggtattt tagaaatcta ttttagaaat ctttggcaaa ggttactatt 55920
aaaacaatca cattcatgga aaatcagtat aagagcaact aaaataactc acaataccag 55980
taaaatcact ttgtcatctt cttaagactt ttaaagagca tttgtaagta actgaataga 56040
aggccaaagg gtgtgtaggt agcccagacc atcagtgggc agccagggcc agggcagggg 56100
ccacggttgc agcctgcatt cttctaaagg gcagagcaaa ttaaagttga agcaggagct 56160
aaaaaaaaaa aaaaaaatgt ttcaaagaat tccaccaacc agaggatact acctaggaca 56220
gtttgggcct aacttatctg tgaaggcctc cagcttcctc cacaccggtg gccacttttc 56280
attcactctg aacccttctt tgtatggagg tcattttatt aattgagctg tgaccaacat 56340
gacagaattt cctgttttag ggcttttata atatagatag tttatatcta atttcagaat 56400
atattcactg gggaatggac ttagcaacca ctaccacaac aatgcaacaa tgtgttttgg 56460
aacaaattta ccaatctgaa tttcccccta gattaggtca caggaacatt gcagctgatg 56520
tacagctatg ttcctcctga aacttggaga cacatcctct tgagctgggt tataatgggc 56580
cacccaaagc tcgagttcct gtaatggata cactcaggca gcagaaccta ccaccgtagt 56640
gaggacagca cccagagccc tcagaggcca tcacaagtgc accacagctg ccttctctgg 56700
cacgctcaga gctacacagt gtactctggg attggaactc tttatttttt tttcagttga 56760
tttgtaaata agattgcaca aaaatccatg cacatcaact ctccaaatca gaatttgctg 56820
agctaaaaag agcattaaat tagatgggct ggctttcaag gggtgggggt gcaatagtgg 56880
aactctgcac aacagttctt tacaaagaga caagcaagca catcgcgtgg aaatttccat 56940
tcaactggaa atgtccaagc ctgtttacct caattaattg tccttgttca cttgtccagc 57000
ctagcaattg tccattagta atttgttata aatgagacat ttggtattaa agcatctctt 57060
tgggatactg gtatggttta ttataacatt ctgttagtag tgttgtacaa gcttgagatg 57120
tattaatacg aaatccaagc tgcatgaggg ctttattttt caagcctaca ccttgctgaa 57180
attctgaatt aaaatatgat tctcagtaca aatgaataaa tcaacagaaa tggtaacgca 57240
tgtcaaatat tcttaaaacc caagaaagcc ttgtaacttc cttcaatcta atgggaaatg 57300
caggcaaata caagactgat gtccttgagt tttattatca agactcaagg gcaccagtaa 57360
aatctagttt cattggttgg aaaaaaaatc ctgataagca ctgttaggca tattaacttt 57420

aatgattaca atttttagga cactctgtgg cctagactta gaaacacaac taatgtccag 57480
aaaaagattc ctctttttat tccatcatct gataggccta tttttacaca tacacaccaa 57540
ccaaaagtag ccaagcaaac aaaacaacat actcacaccc cttcgcctat tatcatctag 57600
gtgattttca atgctcattg caatgaaacc tacttattgt gcatggcacc cacccccact 57660
gaggaatact gtagtttctt tccctttgaa cttcattagt agagcacatg gttcattcac 57720
tcctgaagag ttcttcgtat gtcagaatat atatactaca acataatttc catcagagct 57780
ctgaccaccc gcttatctat tttcataatg cctgccactc catcattagc tgttgtcatg 57840
taggctatca ataaatatat gacaaataaa acagttaggg aatgagggaa attgactagc 57900
agccaaagac ctaagccatc ctctgcttgg acattagaaa actgagttca ctacagtcat 57960
aagatacaca aaggcagaat gtaagccata caaaaatcca tgtcaatccc aatatgtgag 58020
tacaactatt gaacaccatg tactaatgga tgagttggta aatcattcaa tgtcttcatg 58080
aggtcaatta cagattatta tttagacccc aaagattcca aagatggtat ttcggtcaga 58140
tcttcatcct ttgtaagcct agcagaaaat atggcagttt tattgactac tattctttgc 58200
tgggtgtggt atttttaaac tgagacatca gtgtgcctag cacagggcct caagcacaca 58260
gaaaaattcc ttgataataa ttaaataaaa tttcagcaaa aaatatcatc ttaaggctgt 58320
gaaattatct tcctgtgtgg ctaaaatagt gaataaaatt cagcgcaata taaatcatag 58380
tacaatttca tcactaaatt ttctgatctt gatcttgtca ttttacattg gaagtaaaaa 58440
tgtgtcctcc tttttttctc tgacagtgaa aagtgtgtgt gtgttgtgtg cccttttgca 58500
caccctgcct cacacttgct ggtctaattc cttccagcat gattatgata taattaaatg 58560
acagaaatgt ttacttccaa gtggaactaa gccagggtaa ctcagggtag ggcagctgct 58620
tgcaccgaaa gaccaagact gctagagaac taggaaacag gcggtgcaag aactccaggc 58680
tctcatggaa gagcgggagg cttctatggg gctgcagaaa ctctttggtg cttggggaaa 58740
aaatgggtta aatgctctta aaaaagaaac ctgggagagg tagtttccag atgcaggccc 58800
gtcttttctt ttaaacagag gcagctccga agagctggac attgaaccct gagcaggaac 58860
tggaggccgt cagcgcagct ttgtttggcg agcggagctt tgcaagggtg taatgctgca 58920
ccagggagac gctatctgca gggaccggtg acgccgtggg tgtggagggg gaggcagtgg 58980
ctggccctct tggggtaagg tacgcccagg aacagtttag aataacgtgc gcgagtcaaa 59040
gggaagaaga agctcctgca gaccttctgg gcactgtgca gggtttgctc ctgtccaccg 59100
tgccgtgttc ctgtcctggg gtatttgggt gtgtggcgtg tggggagggg agaaggagca 59160
aggcggcagg gaggggatga ggaccaccct gtccatggga caggccctgg gccccgcaca 59220
caccccaagc cccgcgtccc gcgtcctcac tgtcctggga caccccccac cccaccccac 59280
cgccacagcc cagagcggtg ccaggaagcc gcctcgacgc agccgtatct tgaggctcca 59340
gccccatccc cagggtacca cgccacgtag agacactatt tttcacttcg tgtttgtcac 59400
tcctaaagca tgtgtgctag ctgcaccaac cctgggatgc ctcggtgcat agggtttatg 59460

tgcgtcctcc tccttccctc tgagctggtc ccccgtgggg aactgctgcc cagactgacc 59520
tgcgtccttc cgcacgtgca ggaaaatgtc cacgtgcact tgtcagggtg ggggccacac 59580
gggcaccacc actgatcatc tgtgggatcg agttactgcc catgcagatc ccacgtgcag 59640
ggcccagtcg ctttggtgag agagtggacg ctgtggtgac tccacggtct gtggctgtgc 59700
tcaggaggac agagagggga catcctgaga tggtttgggc agcccgcgga tcctgtgcat 59760
gtccccagag cgtccacttt ctccatggag cagtggagtg gcgttgctga gacagaaagt 59820
tcaggttctc cactccccat gcagccccca ctcccctgtc tccggccagg cacgcgtctg 59880
gggtggagac tcccggtgcc cggggccctc cagacctctt tccccacccc agggagcagg 59940
cgggtacttc tattccgttt ggcttcagaa gggaaaagag aacgtaagtt cagggagttc 60000
tcgtccattc ctctcccgtg ggccgggcag gcagcaggga cagccttcag gagccaggag 60060
gggctcgagc tgcgaggccc tggaatgagg caggcatggg ctgaggctgg agggaaagcc 60120
ccgctaaggc tgggcggggg cgggaaaact taccaccagg ggactcgaga tggggaagga 60180
aaggtcagaa gaggagaggc caggcacggg gtgtgggcgg cctgcagagc tggagcaggt 60240
gctccgccca gagccaggca tgcacactca gagtaggtgg cctgtgcagc ggggaagagg 60300
ggcgggtcgg cgtgctgctg aagatgcagg agctgcggcc tgctctgtgc gtgctgaagg 60360
tgtggtgaga agcacttaca aaaagaaatg gactgtgtta ggattgcaca ttttactttg 60420
tttctcccaa atacgtgttc tttgaatttt tttccttcca gggccaggac tggagtgatg 60480
gttgagacag gcacgcactg ggtcttgtct gcatttacat tttgagattt tgttcagcat 60540
ggattttatg gcgttttttt gtttgtttgt ttgttcgttt tcaaaatact gcacggttta 60600
tcgtgaagac agggtccttt gctgccgtct taagttttgg gcccaagaac gtgccccacc 60660
ctaggcccgg gcctgctggc ttcatagctc tcatcattcc cacggaacct taagacctga 60720
ggacagaaag gaaggaaaca agcccagtag tccgtgaaaa tccagggtcc cgccactcca 60780
ggtgtctgca gcagagctga acacacgtag gctcttgcca ggaggggcat ttgtatgtgc 60840
tgagcattcc ttatattctc aatatgacgc ctttgaaaga tctgtggttt gcaaatattt 60900
actctcagtc cataacttat ctttccaacc tcttaccagg ctcttttgct gaataaaagt 60960
tttaaatttt gaagtctaat atatttttaa tttttttatt ttatggatca tactttttgt 61020
gtcaggtttg agaagtctgc accaaagtat gtcctgtggt tttcccttag gtcatcttca 61080
acaagtttca tagtattttg tttagatgta aatctgtggc ccattttgag ttagtttttg 61140
cacaagagtt gaggtcaagg ttcttttttt gcctgtgatg ttcagtggct ctggcaccat 61200
ttgttgaaaa catgatagcc aatgtcaaga cttaatagtt ataataatca ggagcttttg 61260
tttctttttg ttttgttttt agtaactgcc agtcactgct tgtggtatac atacacaatg 61320
gaatactatt cagtcttaaa aaaaaaaaaa gaaggaaatc ctgtcatttg catacctgga 61380
ggacattatg ttaagtgaaa taagccaggc accaaaagaa aaacattgca tgatctcact 61440
ccttcatgga atctaaaaaa ttgtattcag agaagcagag agtggaatgg tggttaccag 61500
gggctgggaa ggtgtgagct tggggagatt tggtgaaagg acatagaatc tcagttagac 61560

aggaggaata agttaaagag atctattgca catcatggta actgtagtta gtgacaatgt 61620
attgtataca tgaaaattgc taagagagta gattttaagt gttctcacca caccaaaaaa 61680
aggtatgtgc agtaatacag tcattaatta gcttgatgta gccattccac aatggataca 61740
tatatcaaaa catcatgttg tataccataa atatatactg tctctttatg taaatttaaa 61800
aataagataa aataaatgtt attcacttgt cgtggatgtg gtggggacag gtgtgggata 61860
gccctccctg tacaactagg acccaggggt gatctagtga cactagccat ttatcaggac 61920
gtatgggtgc cagtcaggat gataaagctt ccttttggcc actatactac ttagaaatgc 61980
cctgcaaaag gtgcacatca aagattgaaa gctcaatcct ggattttaag tgcttcaaaa 62040
gtgcacttaa ttgccacatt tttgtcaaac attttcccag gtagtatttt tcctcatgta 62100
aaacaacagc aatttaattt gaacagaaag cattttgaaa catacttttg gcagggttcc 62160
ttgcagatca gaatggaaat gattaacagg gcaattatca atcatggact tttggcggca 62220
gaaggaactg tattgtttgg tacagtctgg gccagggcca cacaccgtaa cggagatact 62280
ctattctgtg gacggttgga gggggctgtg ctgagcaggg taactgcatc ttttcctaga 62340
ctgttcacac tgctgccacg aaggagtctt gtttagactg gacctggctt tcttcttcgc 62400
aatgagtgtt gcagactccc gacaaaggcc aggtggtaaa gtgtggtgtc tgtgagcgag 62460
agcctgagat gcctgagctg acctgtcctc agccacctgc catcgtgcag aggtgagagc 62520
agcccctgaa ttctgcccct cggtctctcc atagctaaag caaaaccatc cttccgtgct 62580
cccaggacaa gcaggctatt accaaatcac ccactaaccc tgggcgagga ggggccatca 62640
ctgcacaatt catcagtgtc tgtgacagga agagattgtt ttagactggt tttttttttt 62700
ttatttgcaa gcttttttct ctctccaaaa cgtgctgtca gtgtgttcta atttactctg 62760
taaggaattc tggagctaat cataggctca caaaaagcag cacaggaaag tttcccagat 62820
aacatctatt tcagtggctt tcaaacattt ttgaccttac caaagtaaga aatacatttt 62880
aatatcatgg cacacataca gctgtatcta aactttcata atactgcctt tacgatatca 62940
ctctgatatt gtctattctt ttctgtttat ttttcttttt gttccttgtt atgctggttg 63000
tgacccactc cagtgatttc acaatgcagg ctgggtggtg tcccacagtt tgaaatccca 63060
atctagggcc ttcctctcac tgtacaaagt aggtaactgg ggacattagt ggatcagtga 63120
tcaaaccaaa gttatttgat cttaccaagt gatatcagga tgagaaagct gttagagtgt 63180
cagatatgtg aaggaacttg ggtcattcct gatacctcaa agagaaaaaa ggtagtcctt 63240
gaacacctcc tacttgtaaa ggatgcacaa tcctacatgc ccctcccttt cctttcctcc 63300
cctctgtacc ccacccctgc ccacattttc ttcataagca gctttggtgt tttggcttgt 63360
ttgtttccct tgtctcctac ctgtgacttt atagcctttt ggagactcac agcaatagtt 63420
gtatttaaac tcagtgggtg gcatccaagg ctaaaaagga gattgcctag acacaaaacc 63480
acccaaggga gaaagcagga cagcatctta ctatgattgt ttcttgtttc ttcctgtctc 63540
ataaggatta ttacccaggg ttttcatttt tttcatttca tggttcattt tcgctccagt 63600

gtagacatac aatagaccac tcgtccctgt ggctccgggc agcagcctca tctgagaccc 63660
tcctgagaca tctcgtgcag ggcagccgta gtgtgtggct tccccagggc tgctctaaca 63720
gatcaccatc cttgccatgg cttaagaagc tgcagattta tttgcttaca gctctggaag 63780
ccagaagtcc aaaatcaagg tgtcagtaga gtctctctct ctgaaacctg ctgaggatga 63840
tgcccctggc ctctccccag cctctggtgt tcccagcagc ccttggcatt ccttgccttg 63900
tagatgcaaa actccgatct ccacctctat cctcacagtg agttctcctg catgtctgtc 63960
tctgtgcctt cacattcctc tctgtgtgtc tgtgtttcca tctccttatg aggacaccca 64020
tcactgaatc agggcccact ctataccagt aagacctcat ttcaactcca ttacatcttc 64080
aaaaacccca ttctcaaata aggttacttc acaagtgctg gaggttagga cttgaacata 64140
ccttattgaa caatccaact gatgacacat agtaatttat gcactcgttc ttggagacgt 64200
tgactttatt tagtagcatt aaccatggca atgtcaccag catcgctgac agcctgaagc 64260
atatgatctc cagaatgtat ttcaatcatc atgttcactt ccttggtatt ctttagacaa 64320
taactcagcc ttgaactcca gtaaagggtt tccctgggat tttcttcttg actcactcca 64380
ctgtggcctc cctcatccag gactgtaaca gacgcctgac gtcagtggtc tagacctctc 64440
tgctgaatgt catctttggt gaatgtctta tgagaaaaca catggttggt cactcttaga 64500
agggcatgaa agcctgtctg cagtataacc aaaacaggca catggcgagg cacactgtgc 64560
gcatgtgtgt acaattaata tcatggtttt aaattatttt caggccaagg ggagatcttt 64620
gctgcatcta ctgaagaaag cgaatctttt tcttcctgaa aaaaaatggc tacttattag 64680
tcgaatttgt gttttaaaaa tatgtgaact aatataatgc agacatgcat taatgtttaa 64740
atatactgga agtttttggt aaaatgaaac ccattgtctc tgttgattac tttgatgagt 64800
caagaagtaa catcctggga atgattggcc agtttaaatg agtgcctcag gtttttggaa 64860
tacaagaaat caagaggaag ggattagaac atataggtta gcaagattgg gatcctaaaa 64920
tacagaccca aatgaatgga acaaaatcag ggaatttatt aataacaggg tcaaggccaa 64980
atcagtaaca aatatcctga gtggaagaaa ggtggtttaa caaatgcccc tatgaaagat 65040
agagattggc ttaccatgat gagatgtaag cccaagttat gaggttggca cacaaaacca 65100
caaatgtcat agcttaaaac aacacacact tcttatctct gtttctgtgg gtcagggtct 65160
gggttctcag ggactcacaa agtatgtttt catctggagc tccaggtcct cttccaggct 65220
cataagggtt cttggcagaa ttcagtttct tgaggctgta ggactgaggt cctggctcct 65280
agaggccacc ctctccataa gcagttctta gcatggccgc ctgcttctcc aggcccagtg 65340
ggaaagcatg tgcctccagg agggctcagt ccattcttca tggcttttac ctggttaagt 65400
caggcccact caggataact tcattttgta ttaaatcaaa accagctgat ttgggatgtt 65460
aattacatct gcacaacttc aactttgcca tataacctaa ccatgggact gatatttatc 65520
atgcatttgg gtcaagttgc attaagagat ataataaagc tggacaagct tctgttgatt 65580
agaagagttc agttacaagg ctacacttgg gaggaatgtt tacaaactgg aatggtcaga 65640
ggatggggaa gacacttgag aaaagtcaag tgacggatga aggcaaatgt ggatatttat 65700

ctgggagaaa actaagagga gttataatag ctgtcttcaa atatttaaag ggcttttatt 65760
aggaagagga atttggcata ttggattttg ccttcagaga agtggagtcc tgagatgctc 65820
ttagccattc attccagcct ccagggctca cctgctgtct tctgtccagg ttctcggtag 65880
cagggcagta cagccccatc cgtgatcttc catagtcagg catattgtca cactcagtga 65940
gcggagagtc aaccgggagg aaggcacagt ttctctggaa tgacctacgg aatggtacgc 66000
tcaaatgcaa attctccttc ccttccccag tccttgtcct tcagatggta atttaggagc 66060
tgaaggtcag ggcaccagca gcctttggaa gcctacagga caacagtcag cctggctaga 66120
aaaaaaaaca atgtcacagg catgttgtgt ttaatcacat gaaggatatt tgcattgttt 66180
tccaactgat gccagcagac acattgtcag tggtatcatg cctggggtat cagagttgac 66240
attgggttgc cccttctctg aggcattcat gtaaatcctt ttaagtttat aaaacctcca 66300
tgtggctcct gcatgcttca tcatttgcat gtgtctcttt ttccagggga ggcagcatgg 66360
ggagcaggat gctggtgggc tccaggtgca gagagcaggg tgggcgtcag accccaggtc 66420
cactgtgcac gccctcttgt agagcccgtt ccgttgtcca tgagatgagg agtgttctta 66480
tctctaaagt attatcatga aaacctaaca atgtagaaag actaaagcac atgggtggtg 66540
cttcataaat agtatttctc ccactttctg aaaactcctg ctgaagtaac tgcacaagaa 66600
tccttgaaca tttagaattc tggttttagc cataccataa agtcagtagt gcgtggtgga 66660
attctgctaa cgaaaattgc gaaggatcaa ggcagagtac agagctggtg tgtagcgggt 66720
accttctgtc tgctggcact aggtatttta cacattaaat cagctcgttc tcacatcagc 66780
tcttttaaaa ataaggaaat gaggagccac agtggcccaa ctgatgcagt ggcagaagta 66840
gaatttgagc ttgtgcagat gtgcctccgt gttttgtctc ctgagcatgc tgccccaagt 66900
ttgacaatac caagatttgt actggaacat tccctcccat ccccaccccc tagaagcccc 66960
tcttcctccc ttagatttga cacatagttt gaaaccacta ttaactacct tatgagagcc 67020
actgtttgtg aagtgctgac tatgtgccag gtcccgtgcc gtgcaatttt tgtgaattat 67080
ctcgtgtcta cagtgcctca caatttctct gctcaatacc tccatgttac tgccgaggaa 67140
agggaagctc agagagagta agtaatttgc tcgagttaaa gagctggcca ggacagccag 67200
gggcttgcac cccggagcct tcatccacta cactgtcagc tggtatctca accagccatt 67260
acaggctgta aaaaaattat ataagatagt ctatggtaat gcagaaaagt gaggttattt 67320
tgctcccttt ccctttgaag aaaaaagccc tggaaagaca tatcacttga gtatgggaaa 67380
aaatgaagct gtggcttttc tgtgagtcaa ttctttcctg gcagcttctt ggaataagac 67440
caagtatagc agcagagttt tctgttttaa tttgagctgc agggtgactt tttttcttct 67500
atgctttcat ctctctgtgg cttcttttgc ctcgttaatt tcatgccctg cccaggcggg 67560
ctactgtgct gcccagtcac ccgggtctgg ggcggccacc gctggccagc aggcaggccc 67620
tccagaggca gaggtggcca cgcttaggtc gctcccgctg tggaggcggc acacttgggt 67680
ggcagcacag ctgtgatgtg gcggcagctg gcagccccat gggaaagatg tgtgaagtgt 67740

ggggtttgac gacccatggg agaacagact ttcttcctct tcttgttttc ccttcaaagc 67800
cgtgagtcaa cctcaaattc tctgtctttt ttctccaccc cctcgtgcct ctctccctca 67860
cgctctgcat ctctcattgc aagcttgcat ttttttgcac acaacactat cttaatattt 67920
ctcttttctg caggcaggaa atgagaagtc atttttcagg gtcattcagg aagtcatcca 67980
gagttataat ggcccattat ctactggtca gagtttactt aggctttcac tacttccact 68040
gcccacttga aacagggaaa aatattttcc ccccgcgctg tgagtgtgct atttagagct 68100
gaccacaagc ggggggaaga gaggatggct cggatgctgc atttccactg agaacacaag 68160
gctggcaaag cttgtctgct gcccagcaag cacttcaggc tcacaccatt ttaggttcac 68220
tttaagtagt ttctcaattg ttaaaaaaaa aacaaaaaaa aaaaaacctg tactctgagg 68280
atatgcttat aatcccatag ctaacccaga atttcttaga gaactgatca acatcagcag 68340
tggcacttac tgaaaatgca cattctcagg ccctgcgtag ggcctactga gttagaatat 68400
tagagagcag gtctcagaaa cattctatcc ggcagtctta ttctatgcac ccgaagggat 68460
aagagccatg ctttcatgaa acatgggttg tgtgtaaaat gtttaaaagg tatggcaaaa 68520
tgtgtttgat tggcaccaag gatttctggt tcctcctaga atcattaatc aaactttgaa 68580
ggagaaataa gagagtcggc attttcttgc acattctttg tgatgttgtg atgagttgga 68640
aacttcccga ttgggtttat tagagcatga acacccaggc acccagcttc tagccagccc 68700
tgtcaggcag agtctcctcg aagatgtgga aaggactgac caacagctga ggcctacagg 68760
aacctgagca ggcaagggga gaggcacccc ggaaccagga gcaatggcct tcccaccctc 68820
cctcgtcctc tcctcttctc cttttggagt tgcaggccac agaaaggaag tgacatgagt 68880
cactttgggc cttcttaatt ccttcatcaa aggcagcaca ggtgtgtatg tgtgttggtg 68940
gctaattgag gtaggcccac agaggagata acagatggac atactatttc ctttcttcca 69000
ttctgatata attcagggta taaacacaca cacacacaca cacacacatt ctcacttctt 69060
tggcatctac cacacctgcc ccagtgccca tttctctccc acctgaataa aaagccccca 69120
caaagcctga ggtacatgga aaggagcagt ggtctggctc ccaggagtgt gagaagcagc 69180
catgttttca gaggctgtat tccacttgga cttggcccta cgctgaaggt aggagcggat 69240
gggggaggcc cccttcgcac aaagagcccc atgaaagagt gcacagtcca gtctataaaa 69300
cagacgcaga aaatgtgtgt aggacttctt cctgaaaaag agcgtggtgc gtccagtacc 69360
tccatgttca tggaacttcc cagtctgcag tttacccttt tgtgcaactc ccttttggta 69420
aagccctggt cacacttctg gttgttcaga ttatacaggg ataattccag agtgatttta 69480
aagtcaactg ccaggcatcc gcacttgcaa attagatgcc tggcacatgc ttgtgttaag 69540
gtaataattc attacaatac aaattacagg ggagttcctc tgggcatgcg acctttcccg 69600
tcatttggct ttccctgtga ttatcagggg agcttccatc gtgctgctaa tgggacctta 69660
accatgtgtc aacccatggc tgtaatgctg acactgtttt ctttctggaa tgaaaggcct 69720
tcgcaattga aaccaaaatg ttatccaact cagtcctgtc cctttgacga tgaaaacatc 69780
aagttctgga gactggccat ccagcctccc tgcctcatct cccacgccct ccatcatttt 69840

ttgtctctac ttacttattt atttggctgt attttacgta catcatgcaa aaatattcct 69900
ctttgtaaaa agtataatga tttcaggaaa ttagagggta aaaagcaaga accatgcttt 69960
cactccactg tcaagagttg tggaagaatc cttccagcat tttttctgtg tattttacat 70020
acatacaaat atatgtacaa ataaaggtcg atcatttagg ttttgtttat atttttgtat 70080
atatgagctt atgtcattca tacatattgt tttgcctctt gctttttttt aacttaattt 70140
tactttgctt gagagctttt tgaactgaag tacgtgtaag tcagcctatg catgtaatgg 70200
ctccctcatc ttctgtgagg ctgtcactaa aaaggggatt tagcttgttc tgggctttgc 70260
agcccgtaca ctgggcactg ttcatacgta cttctctgtg cacgcaaagg agggcttgct 70320
agggaggcct ggcagagggt gccattcaaa taggattttc aatggaggaa tttttaaatt 70380
ttcagttatt tgaataagtt ttaatatata tccagaaccc caaatcatca agtttgtttt 70440
cttccacatc tgtccttcca tttctgaact attttaaggc cagtcatgtc tcatccaaga 70500
aatcccatcc tttcacacaa cactatctcc gtttcatggt tatgaatctc taaaagcatg 70560
atttttaaaa cataatcaca atgctgtcat cgaacttaaa aattagccat aaatctctta 70620
tgttacccaa caaccagcct actgacacat ctccagttgt ctcaaaaatg tgttttccat 70680
tgtggtttgt ctgaaacatg atccaaaagt cagacccacc tctcaccttt ccctaacctg 70740
ccggagccca tgtttctttc cagccaggct tggagaccac cacacgggat ttgcttcttg 70800
gggcctccct ctaaccagct atgcaggatg ccctctttcc tgtcaataca agctgctcaa 70860
aggactcatt cagttcaaat tcacctatgt gagcctaggt gatgctactt atttatttat 70920
ttatttattt atttatttat ttatttattt attttgagat ggagtctcac tctgttgccc 70980
aggctggagt tcagtggcat aatctgggct cactgcaagc tctgcctccc gggttcaagt 71040
gattctcctg cctcagcctc ctcagtagct gagattacag gcacgtgcca ccacgcccag 71100
ctaattttta tagttttagt agagacaggg tttcaccatg ttggtcaggt tggtctcaaa 71160
ctcctgacct cgtgatccac ccacctcggc ttcccaaagt gcttcatgtt ttcaggagct 71220
gtacgtgcat ttttagtttt gatgaccagg tcctttttct gttttttaaa gaacttcaaa 71280
tgatctccag ggtacacagc gcttgtgtgc tgatgaaaaa gctggcagta caaaggccac 71340
cagccaaggt cacacagcca aaaagcccct gacctcgggc cccttcccag accctgggtc 71400
ttttgctgcc acatgaatct tcttcaaggt cctatgtgta gattttcttg acttggccat 71460
attatttagg attcagatat aataacaaaa tagatgttaa agcataacat gaaggcattt 71520
aaaagggtag aaagcacatg atttactaaa accataaatc ttatgacctg aaagtttcac 71580
ctaatctctt aaaaaatacc gtactaaacc ctgattgaaa atcagagctc agacatacag 71640
cctgagatgc caaaaaatgg ccaggcttgt ctgttgagaa agccatatgt aactaactgt 71700
ttggaaattc aaaatatatc ttatcatttt aaaaacatct ttcttctaaa gacaatcatc 71760
ttggcttcag gaatgaggct agtaaaaagt gaaatactcc tacttgtgga agaaatcctc 71820
attttaacca tgaagaactg aaaaatgcat tctgatgttg atggacccaa cctatatttg 71880

ggtattttat gatgtacaca atatactttt gtatatgaga ttgttattaa atgtgacttt 71940
gctttttcaa gacatacaat gttcctccgg gggtcaggca ctgtgtttag cactttgtcc 72000
tgacctcatc tgacttctca gctgtccctg agaggtacca gtgtgcaaga tcgctgagtt 72060
ggcaagtgat agtgacaata ttttcacccc aatttctaat ttaaagaccc cgatttctag 72120
ttttgttttg tattggattt gcacaatttc acgttctgaa agaggatgcc ctcaactttg 72180
caaaatgggc cttttgaatg aaaaggatca gtcatgtcag gaaaagcgct acaatgatga 72240
aatatgataa ataagtcagt ctttcatctg taattatcta ctatggggta aaaagtgatg 72300
aaaactacca tcttgaaagg ttctggtgat agtggttcct aatgcagtga aagatgtgta 72360
agtcaaagat ttgtaaccag ccagggaatg agaggcgaag ccatagctgg tggcgggggc 72420
cacatctggg tgtggggagg ccacagttgg gttgggggtg gggcctgcag ttatccacac 72480
ccctcccacc tcccttcgac agtacaggct tcctggttac cttccagaga gtaaggccag 72540
ggagagttga ataagttgag aaatgtcatg tcgaagctat tggtggaaag agttccatta 72600
attgacaata caagtcccta ctacattcta aaatctggtc ctgactagtg gcaagccggg 72660
cccaggagta gcacttaaac aatggcaggc ttgtgttgct ggcaggatac ttcagcctca 72720
gaggagctgt gtgcagctgg ggagactcac actcagagga tttcaaagca gagggcatct 72780
cgtagagcaa cttatccaaa ccctgaccca ctgtaaacac acacacacac acacacacac 72840
acacacacac acacacacac cctgagagag agaaagagag agagataact aaagagagag 72900
aactaaagtt tggcaaaata atacatgctc taatgaaggt ttattaatga ttaatctact 72960
cctagcattt cctagtccac tctatctcct taaaaaaaaa ttctggttgc agcccactaa 73020
cttgattgta cagctgctta atggatagca ggctgtaatt ttcagagaac tgtttaatgc 73080
gggctacctc tgttcttcca tgctgcttgt ggttcctgct ctgctcagga cagaatgggg 73140
aggaaaacag gctctgcggc acaatattgg caagtgaaat tttgtaaacc ggccctccct 73200
tccttttgca tttggtctga aaattcaatt agatgctgag tcctacaatg tatttgagaa 73260
gcccaggagt gccctagagg atgagactgg gtggctccct gtcaggttga acatttgcct 73320
taattacttt ggcaagattt gcatcagtgg tattagtccc tgcctcactt ggaggcctgc 73380
acttaagtgg ccacattcag gctccaattt cctggtgatt tcatagtgta gggcacttgc 73440
aatcaaaact aggcttaaag cccaaccctc ttacatttta cccaccccca caaatgcagc 73500
aaataaaatg actctgattt tcattcccta gacctctttt ctatatttat tacattattg 73560
ttaagacagt ttttgaagaa agctgtttta tttaacaaaa tagctttatg gaatcaactt 73620
catatatctt ctccgccaga tcaaaacaag ctcgtagtat tagatgtcac cgagcaccat 73680
gacaggcaga tgaacatcat ccctgtgccc ggctaatgat agctcggcct gccccggcgt 73740
cagccgctcc tggcagggcc agcgggcggt gtgggaccgg caccgtatct ccagcaattc 73800
gcagataaca aatatggttc tgatgatgtt actaaagatc tgtccctttc aagattggat 73860
tagacattag gaatttggag ggctttttat tgctagcatt tttaagaata accaattaga 73920
gtattgattc taaagtctga aagccacatg gacagagttc atgtaattgg ctactttatg 73980

tgcctcttcc tagattgccc tgcattttca aaacaagagc ctttctattt taatcaaaag 74040
aatccagaat gaaatgaggc tttgaaaact cagcctatgt ttgtcttgat ttccttaact 74100
gacatctaga agaaaatatg agctcagggg tccgctgggt tccttccagc gcctaagcct 74160
gtaagctctt cctgctggaa ccaagcttta aatgcacttg tcagtcatgt cccatgagaa 74220
tagatactgc cttccatgtt tttttgttct gatttccgtg tttgaaatga tgaaaatcat 74280
ttttctgtgc tttttaaaaa tggaattgct tttgtgttgg gaattgtgct gttcattttt 74340
actctacctc gttttggaat cactaatgtg gccaatttat agccaaaaat cagtatcgta 74400
gagtgagcaa tgaatggcat ggtgactgtg tgagcgaatt catgccctcc ctccccaccg 74460
ctcgccccgc gtctcagtcc tcagtgatgg taaacagaat gaggaccttc tcccgaccgt 74520
gatgcgcctc agccctactt cccttgtcct ttcctatcat aaaatcttct ttcatagaaa 74580
tggtcatttc tgttcatatc tgtggactgt aaataacaag gaagtcattt ttgaggtgaa 74640
aactgcactt agactcattc caattttgat ggaaactttt agctggtgga tggcattttg 74700
ttttgtctta gttttgcaag gagttatctt aatttaggga gatgaaacta gtctgtgatc 74760
cgaggtctca cttccataca tttctctcgg gcagtgtggc tgcctgaatc atgcctggat 74820
gccacaggtg cttagccagc tggtcctgtc gtaactgtca ctggtagctc agggagtgca 74880
gaggtgccag cagacactat gaaattggcc tcgtaaagca tcagttatgt tgtgatggtg 74940
gcaaagctgc aggcgagatg ggaagtgcag ccactgagaa ctcacagtag agcgtgtgta 75000
acgtaaaaag atgaaaccca ttgtacacag ctgtgtactg cctccttgaa gtcaaatttc 75060
ccccattacc aaggaaaagt tttttctgaa gggggctgct tgacaggatg acatctggtg 75120
atatcattta ttcctttgga aatcaatctg tggaagtgag tttccactga ctgatgagga 75180
gaaaaatgaa ttggcttcac ccagcatcca gcttcttatc ctgggagaga tagctcttgg 75240
tctgtcatcc acgcagctgc ctggtgcaag agccaagttt gtgcagcctg cagagcactc 75300
ttcctgagct gtgggctgcc aggtcggggg gcaggggggg cctcactgtg cagcctcctg 75360
ccacccactg atcatctggg gagactggcc tatcctgtca ggagacgcag ttgcccagac 75420
gttttcaagg gcctaagatg taggcagttg atccacagat ttttggagag tccttgagtt 75480
ggagattaca ggtgacctca gaggagggag tgagaacatc tgggtcatgg gtttctacta 75540
ggagtccaca gtgaaaacaa gaagaggaat ttacgacaag acagtccagc aacttccttt 75600
ctaacttctc ctttcacata tgctggatac tccaagactt tgcatttaca tggacatcac 75660
agatccactt tgagagaagt agggtaaaaa gaaataaata catagtgctt taggtgtatt 75720
tctatacatc ttaattgata tgggattaca ttttcacttg tgtttactgt acagactcta 75780
gacagatcct gctcttttgc aggtaaaaca aatatttctt aaaacctaga aagacccaaa 75840
acaatttaac agaaacattt tggaccattt tggaccttgg cagttaggcc ccagtgcagc 75900
agcggcaacc ataaacctct ccataggtgc tgaacccagg tgatccctgg caccggcagc 75960
cttatgtcag ggctctctta tcgctggttt ttatttctcc taataaaagt gattaaaaga 76020

ttcatctttt aaagaaagca aggacacaga ggtggattct ccctgacgct agcacagctc 76080
atgcccaagc cactcctgca gggctctggt ctaagtgcaa aagctggaaa agctgcaggt 76140
cccgcaagac acagagcaac cctgcaagcc aggtcacctt ccctcttctc tgctgtccga 76200
ctggccctcc accatgtgac attcaaaagc tcaagttact taacctctca aaactcagca 76260
tccttttctg tacagtgggg aagatactgg actgttgtga ggattaagtg aggagagtgg 76320
cccaatgagg ttgacagtta ttactgtcat tgtcattatt tgccttctca caggcaggcg 76380
tgccacagtc attttactga agctgcttca gtgggtcctg aattaggccc tgtcctttgg 76440
gagagacagt cctggttcaa cacacagctc cctgcccagg gcagcttggg agtgtgggcc 76500
agtttcgcct ttagaaccac aattctctga tatgtgcaat gagagaatta attatagact 76560
caaaggattg catgcagaca cacacagata caaacacata cacacaacac acagagttac 76620
acacagacat gctcacaata cacagaaata cacacagaca cacgcacaca gcacacagag 76680
atacacacag acacacacac acacacacac agacatacgc acagatgggc acacacagag 76740
acacactcac agagacacac agatacacac aggcacacac acagagagac atacacacag 76800
cccacaggga tacacacaga cacacagaga catacctaca acacacagag atacacacag 76860
tcacacacag agagacatac atacaataca cagagataca cacagagaca cagatacaga 76920
cacagacaga catacacaca gacacgggca cacacagaga cacacagaca cacacaggca 76980
cacacgtgca gataaggtaa tattagctag ttcaggagga gaaagagata aagataaagt 77040
aatattagct agttcaggag gagtgaaaga agccttgttt ttctccactt tttatagaag 77100
agaaagtgaa gattcgattt gaggtgagtt cagcacaaaa gcgtatccca ggccctctgg 77160
ctccaactgc agccctttct acctcattcc cagaccccac ctaagccttt tctcttcaaa 77220
atcttctcag gcacactgat acacatacct cagattttta attctccggt tgtgttcacc 77280
aggtgcttgg tcatgattaa gaattccgtg atgtgtaccc catgtgttta aatttgctgc 77340
tgagttaact ttgtggcggc ctgtggacta gacctctgca catgcaatgc agaacggcag 77400
ggccagattt gaaatcctgc tatcttttcg gctgccttgt aaaaataaca tcaggcgatg 77460
gggatacgat gccagaggtc acctgtgata agttctgttt atggccattt tacttctagg 77520
aagacaggaa gtgtcaggat ctcagggatc taggaagcca aaatgttttt ccactctgaa 77580
ataaagtgac tgaccaggag ttcccggcca cgcagccctg tgggaactgc cgcacggcca 77640
cttttatgaa gtggacacgt gttggtccca ctgaaaagaa actccccacc catggctccc 77700
tcacgctgca gcagaggccc tgccacagca cctgtcagcc cctgccagct tgcaggggcg 77760
caggcgcaga gcggtttgtg cccttgctgg agccagggaa gggcacaggg tccctcctgg 77820
agtcatggga ggtgcagccg aggttctata ttaaaataca gaggctagca catgtgcttg 77880
gggaatgcag ctacagtagt ggaatgaaag tgctgtccgt tccttacccc cccagctcct 77940
cacctgtcct ccacacgcat atccctggct ccctttccct agtaaggaga ctgaattgaa 78000
attgtggctt gcccgaggct gcatacctgt gctctttctg aagcccaagt cactggctct 78060
agaattctaa cctgtgagga agccactgag gatgtttgtc aaaatacata tttctgtgcc 78120

ttgccccagt tccacggccc aggaatctgc agttttcaca agcaccccca ggtgattctg 78180
gtggtgtctt tgcacttctt caaggcagta ctgcctggaa cgcagaatcc cagcctcctc 78240
tatcctcctt gcctaatggc ctggatgctc tcagatctac aggggaaggg aaggtcacac 78300
agtcatcgca atagtaacct cagctgataa atcctccccc ataaaactta ttccccagtg 78360
ttttttaata ggaaacaata aaactgtaac cagcccaaat atccatcaaa gagaaaatgg 78420
agaagtaaat catcgcacat tcacctggac cagatctatt gtaaagccaa taatactgaa 78480
gccccttcca aggccctggg agtcctaaca gtgcactggc agtgtctata atttatatta 78540
tgaaatttgc ataaggaaaa cattttgtct catttgtgca atttctcctt ctaaatatac 78600
gtgtcacttt gtacctgatt tctataagac ccaggaccta caaaccctgt gtctgcccct 78660
gcagccaccc agggaaggac tgcacagcag caagacagat tgccatggag catgttgtgc 78720
ccaactaggg acagcgcaga tagattctgt aatttgccta acaatgtcta taggatgatc 78780
ccatttgtca aaaaaaaaaa agaactgggc tttattgatg tcacctaaat gcacctaaac 78840
ttcttttttg ccccatgctc ttctgtactc ttgatctttc cccaaatttt taaaaacatg 78900
acactcattc ccttattttt cctacttaga aaagtgtaga tggttttatc ataggaagtt 78960
caaaaaaatt aaaatataat gaaaaatact caaatagtgc ctcacaacag taactactgc 79020
taacataaat aaaatccata tttcctctca tacagacccc agagttgctt tgcctgacag 79080
tgtagttgat ggagaaaata atctttatcc ttagcctcca tctggttgca gaccataaag 79140
acagggaaaa aatgagggtg ttggtagctt cgttagaaac tgaaagctca ctgatttttt 79200
caaaacctaa atagcctgtg tttctccaaa taactaattt gcagccttcg gcagccagga 79260
ctggcaggga tggggctagg gggactgggg agaactgctc tctcctgagg gtggtctgac 79320
ccgacagcac gcatgacctt cccacagtca ggaactgctc agagacgtga tggcaactcc 79380
atagaatgaa atactcttca gccagtaaaa tgtatttttg gataaatatt tgctttaaaa 79440
aactttacta tatgttgtta aatgaaaaaa aaaccttaag gcatcagaaa ttatgtgcag 79500
taaaatctca cttttgtaaa taaatatacc tgtttactac gtatgcataa aaagaatcct 79560
gagaaatata agtactgtat gcatattgtt gttaagtatt ttttctgttt gcttatctat 79620
aattctaatt ttgcttcaaa gaacaagtta ctccggcaat ataaaaataa aataactaat 79680
ttgtcttgtc atcaaacaga tagtaagaac aggcaaacct ggccctccac actgccagcc 79740
ttttgtgatt caaggcttca gtttcctcca cttgttaaaa agattcaaca aagtagttga 79800
aatagtatgt gaaccagtaa accctaaaag gtgtccagtg ttgtctgtga gctaattaag 79860
tgatttgatt ctgactcccc gagtcttctg atttcgaagc agtggggagt cagacaggag 79920
cctcaggtgg cctctcctga gaggccctgg aaagtgatga gaacctggcc tctggcagct 79980
cttcataaac gtccatgttt tccctctact ctctcactct tttcccaggg cctcaaacag 80040
aagatgaaaa tcaatttcta aaacagccct ctgtgtgctc tctcgtatct ctccttttca 80100
cacatcgtgg tggtggcttt ctctgtgttc ctctgttgat tcagtctctg gaattaacgg 80160

atcaggattc catgcccaga atgctacaaa gactgtgctt gagttctccc acatctcact 80220
caattacaca gaagtttcag attatgtaac agatgctgtg ctgggttagg cagagccatc 80280
tgacttgttt tgctttattt tagaccatga gatgggtgag tttttctttt taatgccaca 80340
ttcttttaag aattaaaaac ctccacttgg ctgtcagcat tggaaatcag agtgatggtg 80400
caagccctga tgaggacaat gtccttgtct atgaaaaggt gaaatcattg cttgaaatcg 80460
ctaagcagga catgcagtcc cagatggagg ggggaattcg ggagctggtt ggaaaagagt 80520
atttggcact ttgcagcctt gagaggtgca gaagagacac cgaggggttc accaccagag 80580
ccaccattgt cagagaggcg tccagctgtg tccacctggg actctgcctt cagggcttct 80640
tgcctggctg ggagctgcac aggcagactc ctgggacggt gtgccgacag ctctgggcac 80700
ccccttctag gatctgattc ctgaggaatc acaatgtgga tttcacaatc acttccagtg 80760
tcttttgcca acctctgtga acagatgtgc aattaaaaaa aaaaaaagaa aggggcccaa 80820
ttctcaacac tgtaagtgga aactttttaa tggaaaagga taggctaatg aattgaattt 80880
gaaatctgag acagaaccga tgcatcaaat gtgctggtgt ttacagataa tacaaggggg 80940
gctgcatctt atggtttcaa tcctttttta aatttttgtt ctgagagacc cagccagcag 81000
actgccgcca gtcttgtcag agatgtcagt ggtggccact ctgaatggaa agcagcatct 81060
ctcagcatct ctgaggcact gctcctcagc ggagactgtg gtggctttgc ctttcagcac 81120
gcatcctttc tacgatgcct gacagtgccc agggaatggg cagagctggg agctctgaag 81180
ccctttcacc taaaccaccc tgggtcacct gacctagttt tcctcccaat tttaattatg 81240
tcaggcactt cacaaaggcc tccttgggga caccatgagc tcactgtcat cagattgctc 81300
caatcacagc tgtggcttgc acacaaccgc catctctgcc ccagcagatg ctgtgtgtaa 81360
acagttgtat taattacatc tcaaaaacat ggttcttgcc agatcctcag gatttgggtg 81420
cagcctctga ggtgggtggg aggccctcga gggagaaatg tctgcaggaa attcttcccc 81480
tacgagaggt ctgttttcta agttatctaa gagctactgc agctgtttac tgcagagtga 81540
ccctgctcaa agctgtggtc acccaaggct ttgaaagggg acctccactt ccgccctggg 81600
tggagcaccg tgctggagac ccacgcctgc caaggcctca ttgtcatctc cacacgccgt 81660
ccttggggtg ggccactcct gggacacgca gacaggaagc cggccacctg agccactcgg 81720
aggctctatc cagagtcagc tgccaagcct cacgtcacac atcactgtta gtcttggagg 81780
gctggcgggg ccctgaagtc aattgaacac ttggatgaca gggaacttgc cactgccaga 81840
ggcaatatgc tccatttttt tgacagttcc aacaattttt ctttaaactg tcataaaaaa 81900
ttgctgctgt gaataccagt gtcggcgtcc ctgcctcacc tttacctggt gcttttccac 81960
cacacaaaac tgtttctcct cgtgctggcc ttgggcttgc agacagctga ttcttctcct 82020
cccgcggctg agcagcctcc tccgagcaac cctctgacaa ctctgctcct tctgacaacc 82080
tctgcaaggg ctgccagatg tgaacaaggg gcccgggcag aaggtatcca ggaagactgg 82140
aaactcgagg aagcctgccc tgtcctgtcc accagacttt acgcttgcgt cactgggctt 82200
tgggacctaa gtcctcgtca tttgttcctt ttgcagttcc tactgttctc agcacttcct 82260

tccagcttac tgaggtacac tcagatgtga tatgccatcg gtacagacac agttctgctc 82320
cagcatttcc ccgtgttctt tctgtcgctc tatttactga attaccgtga ggatgtggag 82380
cgaggctgag ttctgtattt taacaccatt ttaattctca cctactgaga aatccatcct 82440
cttatcactg tgcttttttt aacctgtcac gaatccatga aatcctatca gccagcctgc 82500
atacttcctt ttaaggtgca gttgaatcag gagaaacttg ccgcacatgc tgcgtccggg 82560
cacagcattg gctgaggctg ctgccctgac ctgtccgctt tgtagtactg cccagctatg 82620
aaacaggtta gccacacatg acctgcattt aggagtaaca agtctgtctg tacatgcaca 82680
tacagcaact tttttaaact gtctatattt tttcctgaga taggtattta taatatctcc 82740
atcttctttc ccattttgaa acttagaaca agtttgcctg tcaacagttc tccacagcat 82800
actgtgtatt ctaggatttt ctaaggttga gcaacggagg ttcagcaatt ttgacttaat 82860
ttcttcccat cccttttcca cgcagcccag aagccttgga tcacgtggtg aggggaagag 82920
gttgtgctat gtcgggaaac tctgtatcga agctcggctc agatcatgac attctcttga 82980
ctaaaaccct cagtttccat caaacttgtc actctggcat taaagcctgt cactgtgtgg 83040
ctctgaaaac ctctctgaac gtgttccctg cctctgccct gcaggtccct gtgctccaca 83100
gaagcccact tatgtgaccc acccccactc atcaccacct tccctcaccc agagcctcag 83160
ctccccactc ccacctgtaa gacccctact ggaaagattc ccacctgccc ctcaagatta 83220
atctccaagg acatttccaa attcctctcc ccatctctca gccagatggc tttgctccct 83280
ccaggaaccc cagccacctt cgacctccag cagggcactc cactccacat tctcctggtc 83340
tgtctggctc atcttacctg agccatgctc tccaggtgaa ggactatgtc taactcaact 83400
ctgctttaaa agcagctaac acattgctct ttgcatattg ttcactcact aagttgaact 83460
ggacttggac atgcacactg aactgcagcg tctgctgctt cttggtggcc cagctcgtca 83520
aaagaataag atttcagcaa aacaatgtaa caattttttt taccaaaagt aatgttaaca 83580
atatatggtt ttcccctgat gtttgcgtca aaatgctttt tggaaaaaac atttttcaac 83640
tctttagggt cagaattaag caatgaaatt tatataccac atgtataatg tgtatgttta 83700
tctaagtatc tgttcattta tatatcttaa atagaaattt taaaaatttt tttaaaactc 83760
ctgataaaca ttctcaggag gcacactatg taactgttgg ttgatatacc tagctagatg 83820
gtgaaatcag attttgttta aagcatggag gagagggaaa aattaaatct tgcagattct 83880
gcagtcctta acatctttga aagaggaaca tttcagacaa tgtaataaga aggccacgtg 83940
ctttgacttc tgtagatttt aaaaatactt ctgtatagtt tcttcttcct ttgaagaagt 84000
ttggggagtt tgggaagatg gagaaagata taagaataga ctccccatat gggtcatgaa 84060
ttatcttttt gcatcagaac tcttagtgca gtttcagtat tttcttcctc aggagggtga 84120
gctgcttccg aatgtcctcc ccttctttga ggcatcctct gttggtgaac tttgagagca 84180
tccatttatg aagttgatga cctttcccag tctctgcaag cccttcagtg tgtgtcctct 84240
ctgagcaaat ctgaattgtg tgcttaatac atggaaaggg atttgggagg gttgcttttt 84300

aaactgattt cttaattaat attatggttt agttaactag acagtctcat tgcagaagtg 84360
cataaccata atatgtcttc aaatatatct cccttcctaa caccctgtaa tatacttttg 84420
taaagatacc cttacagaat gtgatccacc atttatgaac ctgcagcatt gcattcagag 84480
actaagtgaa aagctggcag attttcattt aaagcacaag ctaaggaaga aagctggtct 84540
agaaggagct acagaagggt aatgcttagg gagggaatga tgtgcctgtg ggtggtggta 84600
gttaaatcta accaaagaat gatgtcgtgg gtgtttggat attggatggt ccacattggg 84660
ccacattctt tcaaacataa gagtctgtag aaatatgacc tgtaaaagac tcttaaatat 84720
tctggaaact gtttcttcct tgtcacatcc ttatatatac ttgaacctat gcctaccaga 84780
catgacatgt gactattcat acagatttca tcatctctgg tttaagaata aaggatgctg 84840
catagaaggc tcacatcttt taattcacaa gactgaaact gttctgaaat gacattgttt 84900
ctaaaaattc attacttgca ttatattcat ttttattttt ccatgccaga agggtagaag 84960
ttcctgtgct catattaaga aacagcaatg tcaatcgagg cccaactcaa atccaattta 85020
taggagttat aaagggcgtg tgcctgtttt gtctagaagc agtgttgggc agcactgagt 85080
aggatagacc acctgttgct accgataaag gagcagcttc tcgaatgctc ctgtctggta 85140
ggcactatcc cgagtgcttt ggcccctcat ccacaatctg tgtggcaaaa ggcattgcag 85200
gcaattcagt gaggagaccg aggcatggag agcaagtgcc atggaattcc ctaaggccgt 85260
gcagggagca ggttgccaag ctgggttgaa accgtcctcc gtaggctccc aactccgccg 85320
tcgctgctac tgtgctggat gatgcctggt agatgcagat gtggagcccc atggattctg 85380
agacaggccg ggtttcagtc ctgccctagc tgcctattgg ctggatgacc ttggcaagtt 85440
gactttcgtg agcctcattt gtctcatctc tcaattaaga aaacctagag cctatctgtg 85500
ggggttatct gaaggattcc agggatgcat atggcactgt ctaccgcatg cggtaactgt 85560
ttcacaaatg atgaggagcg atttatgttc ttagtggaaa tatgtcggcg tgtgaagtcc 85620
caaagctctg ccctgcctgg cttgatccag tgcctaggca ctgcccctct tcccctctct 85680
cccaacccac tgtaagaggc taggctgcct cagtaactct gaggggcatt gactcttttc 85740
atccaaaaat tcatgttact gccccacatt ttttctgttg ttttacaacg cagtaggaag 85800
tgggcagact gtcaggaaaa gtgatttata gtcatgtatt gcttgtgctt tggcttcatt 85860
tgatccaatg cagatcagct gcactcagaa aactactcaa gtgaaagaga aaaagtaact 85920
gaagggggaa atctggatga gtaagaattc cagggatagg aatattaata gcaagctttt 85980
tgcctgatat agtcacttta tgctgcaggg gtgccccttt ataaagtgct tgtacaatgg 86040
atgtttgctt ttgattttgg atttggagtc taatgaatgt tctaaattat tattagagga 86100
gcttgcggtt gttacatgtc tgcctttatt gcttattttt agccatctcc cctgatgtca 86160
aatgctcagg caagaatgat acattcattt ataatgtggc tccttcagaa atataccaca 86220
taccttttgg tgtggtttgt ggctgagaag agtggggaat gcacaagtgg aaaactgcag 86280
aaagattatg ccttcatcac ttcaagtatt tgagatgaaa ctagatcatt tgctgttgct 86340
ttttattctc attctaagtg cttttcaaag tcagcgctaa gattttaaaa tggttttctg 86400

ttgttggcag agagggaatt actctattac tttctgataa aacagagtct ttcatgatca 86460
aagagaacca ggctctagta gttccagtat cctaacgtgg acactaattg tttccctcct 86520
tttcttcatg aaaacagctt ctgcacaaat gatagccttg tgaactagcc atgggcacaa 86580
ctggagaagc atttagggag ctttagtgca aattgagacc acctacacat ctgactctac 86640
agggtttgac aacatccagg gtgaatcaca aaacatcagt ctaatcaggg cttatataga 86700
aagagtgaaa gaactctgat ttcatcctaa agattattta tattaaccat tgttccaaat 86760
gcattaacta ttttaattta gttgttttga ttgttaaaaa aaacacatct gtttggtaga 86820
taagacataa tttaagacaa atgttctatt tgataagctt ttagaaacaa cttattttta 86880
ttctttcctg tgagataact cagatgtgga gaatgtgaca aaattttaag cataacatga 86940
gaagggctga cacacataga tttctgtgtg cttacttgaa aacaacaaaa tttaagaatt 87000
tggtatagga gttgtatcag gtagtgcaga gtccccagga gacctagaga cccaggtctg 87060
ggagcctagc ggcaagggct gaatgtggga tgacatcagc agaaactcac agccactgct 87120
attccaaaaa cccagcagca gctcagtgca gggcagtgct gatagtacag tgcctgcaat 87180
cctggagtgg atttggatgt gtcaggtacg cacacgctca ctgctccccc agcagtacgt 87240
tgaacagtgt gcgtccaggt gtctgtaggg cccctcgccc taactcacaa aaccattctg 87300
ggtcagaagc caccaatatt gtcatcatcc tcccttttct gagaacccta gtaagtccct 87360
ccagtggggc aagcccacct tttcccttca ttctgtggca atatgccttc atttcctaat 87420
cagttttgcc ctgctcattc aatgcaaaat ggatctgctt tccttgggca ccaatatgtc 87480
cagggattgt ttatcaatct tcagttctgt ttcctttaca tatccctcca aaaatcaggc 87540
ctgcactgcc tgtgcactcc acaatccaca ggcctgaagg aaatgttatc tttgatgtag 87600
agacttaaag taaaactctt caaattaatt atttcatgca aaaggctagt cctgactcta 87660
attctaagac atgtctccta aactctggaa gtctgatgta tcctattatc aacatttatc 87720
cttaatgtga tggtttatca tttatcctca aagctgcatt gtaaaatgta cactgtaaag 87780
tgtacatttt aaagtcggtt ttaaaaaatc atatttagag atcctggtaa aaatctatca 87840
agtcaagaca ttaccttatt acccatggaa ttgtcttcaa ctcttacagt tcaaatattc 87900
ctgaattggc tttcacaata aacatcctaa atatgtaagt agaaacatat atattgccaa 87960
ctttgtgcct tcccaagcaa aattaaaata caggaaaagt cagtttgttt tgcccataaa 88020
taaatatatg tgtgtgtgta tgtgtgtgta tacacataca cactcagaaa agatagaagc 88080
agcagcatat tttggcagca tctggtttat tggaactcaa acgttctgat tgtgcataca 88140
gactagttaa tgtggtaaca attatgtatt tcttccctgc tccttgcctt ctttccctcc 88200
ccagtttttt tcttcctgat agtaggtgtg tacttttttc ctatttccat tggcaagcca 88260
catgacaagc aaaacgatca ctcgaagaat attgttccct caatcaagaa aaatgcccat 88320
tgggttttgt tatttgatgt tatttgatga cagagaccta ttgtttttcc atttttcttt 88380
ttttgttttc cgtggcacct atggaattaa gcaatataaa aaatctatta tttcagatgt 88440

tcacgtctaa tgaatttcat gtgaaatact ggcagtataa ccccaaatag aggaaatttg 88500
tgaagagtgg atgctgcagg gcatgagaca tctgcacaga gttcatctct tccagcatct 88560
tgcatgtccc aagcactgcc ctgccaggca gagaatgctg cagatcacgg cagtgaattc 88620
cagttgttca gagcacattt gacttccaaa ttctcaaggc cacagatttg aggacagaac 88680
aatatttgca tttgaaattg gaagattatt ttttgcacaa gtgcctatat gctatataga 88740
gtttgcccac tctgcattat cttccccctg ttcccccgtt atctggcaca agctattcaa 88800
aagacacgcc tacttgtaaa ataaatggtt tgcaaactaa ggaaaatact taaatctcat 88860
gtaaatggta ctatactatg tataaaaatg tgaagaaaca cagaacagct catgaacacc 88920
tccactgctg tataaaagaa ccatcttttt tctggctcct attggatgcc ttagaaaaat 88980
ctgtatttcc tctttagtta ttgtgtttga aagatgaagt tgagacaaaa gttctattct 89040
ttttaagttg gcagaacttc tgaaaggtga tttttagctg cagtgtgact cattccaaat 89100
gcagaaatct ctgaccctga gttagtctat ttgtcatgca agagcctaga aaagccctga 89160
gtgataagaa atggccatag gccattccca cagaattttc aacaaaaata gaatcatgct 89220
tatgttctag tcatgactta gaacttataa ctcatgttcg gaactgtcca tgttcacgca 89280
caggggccgt atcactccgc cagagctgcc ctgggtgccg gtgtgcagag gggtccgaga 89340
gtgactgtct cttcctctgt tgtcgaatgt gtgggttatc tccataaatg gctgccatga 89400
gcatccttgt tcacacattt ttaggtactt gagtgagtgt ctgtggaata attttgggaa 89460
gtgaaatctg tggtcagagg tttgtgagtt ttacatgcta cattttcaga agttgagaaa 89520
tagcagtagg ctgaaggcaa gtcgccatgc ctggaattca tgaacactag ttgaaagaac 89580
tggcgtgagt tagtcatgac aggagagatg gggaagggag ttgcaggtag gagggccatc 89640
ttcaaattct caaagtatag tcactccaaa ccaaaattcg atttaatctg taggactcca 89700
ttctcaaagc acagtcactc caaaccgaaa ttcgatttaa tctgtaggac tccaggtggc 89760
agaataagag gcaatggatg ggtggaagcg aaacagggcc aaagtttgac ttcatgtgca 89820
acttcctaag gagtgatttg aactccacaa acatgaacta agcacctcaa cacaggctgg 89880
gcaagttgct gttcttttgg agcttacatc ttagtgggga aagagaaatg cctatgtaaa 89940
catataaatc agcaggatac attgtgagga cggtcattgc tcagtgagac tgcaatagag 90000
tgatacgctg gagggggctg caagggagaa ggtgggaggg acagcattta gcagaatgag 90060
cagcacagtc ccataggaag aagaatttat tgcctcctta ggcaaataaa ttcccaaacc 90120
ttgaacatca gaaaggaaat agattaatgt gcacagagga ttaaattatg tgatctgcaa 90180
agtcatttaa aatctatttc cacataaaac atattaatgc aacctaaaca aaaggggtct 90240
ggataccctc atcttcttcc caagcatcaa gtctttctat agttaaactg agatgctttt 90300
attcttggaa aattttaagg actatctaca gcaatggaag aatcgggtgt tgggatgtgt 90360
tcccaggtaa taatgactgc aggctgattt ggcccttgag gtgtggcctc atggccctct 90420
ccaaaaaaaa tcaaggacct gctacaaagc acaaagccga ctgcaatgct tgctgcttac 90480
tggttagggc agctcctctt tgccagcgac caagcagaaa gcaagacaag acaggttctg 90540

aagcagtaat tcaaagcctt cctcgctttc ccatgtgagt cattgctagt cagaatatta 90600
cctttgcaga gaggcttaat tccaaatttg ctcttaaagg gatatcctct cctggtttag 90660
gtataaactt ttgactcaca ggacaaattc tatcattcct ttgggcctag gattgcattt 90720
atttccatga caaaagggcc tgtctggtgt ttcagcaaat gaaaacaaaa atataaagcc 90780
catctccttt tgaatgagct ctaaaacagt tctccactgg acttcagaac aagagggagc 90840
tctgggctgc tggctggttg tgcatttgct gtgggttccc tccggcaggc gacctctccg 90900
cgctgagaag gttatccgga taaccaagta agaaagtaca tgaggaggca cagaaagaaa 90960
aatgtgagag ataacagcat aaacacacag tgtatgttgt tatgaggcat cacatgatga 91020
gatactgctg gggagggaag aagtgaggag attcctagga atcttatgag aatttccaga 91080
gacaacaagt tttgagcttt tttttaattt agaaaattta ccttattttt aaaagaatat 91140
gtaacatatc ccatgctata aaattctaga catagtagat ttaaaacagc ataatggaaa 91200
atataaatat ctattttctt ttcctattta tgtattctgt gccagtagga atgtagccaa 91260
aaagagagaa aaggggtctc tgcagacatg gatgtctctg tgacttgatc actgctaacc 91320
caagaagata ataaagcaga agcatgtatc caggttgctg cagccaagcc tgcccggtct 91380
gcggggcgtc ctcacacatg gggcagctct cccaccccac acactgggaa aggcggacag 91440
aggctgggca aagcccccaa ttttcgttgg cactgacccc gatgatttat aggcctttgt 91500
ttcccatgtt aaatgtctta cgatcattaa attatttata gctcaattag catgtgtcca 91560
aaaccaggaa gttcatagga gactgtgtga ctgggaatta aggagcaaag caactttcca 91620
gtctgtgatt tactgggttt ccattctgtt tcctgttcgg atccggaagt agaatttcaa 91680
atattgcttt tcatgcttta tttgggaccg attttagccc cgctctcctt tctcttgcca 91740
ttcgctggcc attagccacc agcctctgca caatgaccag ctggcccctg gcagatcttg 91800
ggcccaggtg tgaagtcgct ggagaagcat ttcagggcca agatgggagt gatttcattt 91860
tccattgaca ctatgcagaa atgaagggga ttcaagtgcc ttcagaaaag cttccttcca 91920
gcgaatggag ttttgggggt tttccagact tgcaactgct tttattcttg gaagcatcat 91980
tgttgctttt tccccccttc catttatatc ccaggaactg attcagaaac catagaaatt 92040
ggatttggaa tcgctgaatg ctagcagaca gctgactgca ctcttcccaa gaaaccctgc 92100
cagctgggtt cgggtatcgc gcggtgtgtg ctctctctgc ctggcccgct gagtcctcta 92160
actctaatgg attccttctt acaccaaagt gcactagaac taaagtgttt tgcttcattc 92220
tttagacatt ttgtggttta gggctcaatc agccagggta tgatttgcaa tccacagtaa 92280
ccggtttcag agcagctgcc cagcgaggca ggtttcatct cgcttgctag acgttttgtt 92340
tttttttttt tctaaacctc acacctttta tttattagac ttggattcca gtttcctgag 92400
cctgtttgtg ccactgatta gacaggcttg aagcagaacc caccaggctt cctgaataaa 92460
atgcagcagt gattgtatta gggggtttta aattgctcaa aatactgtct aaaaaacact 92520
aaaaatcatg ttactttcta gattgaataa aatcctatag aaatgaattc ctggacttga 92580

tatgtagcaa gctggcattg gctcgggagt gagtgggctc agttaagtga gctaagatga 92640
gatggtgcac aggcgagcac ccacctgagg agtgtttgga tgttatgata gccagctcct 92700
ctgtaaagac ctgtccttct atgtcagcag cccagcagat aaatgacgtg taaataccac 92760
atttaggagg gcttatgatg atgccaatta atggagacct ttttgaaaca ggaaggaggt 92820
gaaacatatt cctttgcttc tacatcactg tgtgccaggc actgtttaca gcatctcgtt 92880
taaccagcag tcaccacctg acggatggct gatgtggggt ggggtcccag ggtgggattg 92940
cgtgatgggc ttggggtctc tggctgatgg gtgccagagc tgggactgga actcctggcg 93000
tgactgaggc agacacctgg gctacccagc ctcacccacg acgccctcac taagtgaccc 93060
acaggactca ccggaagcag ggcagcaagg tccccctaca gaggtcccca ctgcaaaccg 93120
atacccagct tagacagcag ttctgcagtc ggcgtctcac cccttcgggt ctcattgtga 93180
ctcactttga tagccacacg atttaagggt ggttcagtag tgatttgatg agtgctgtgg 93240
ctcagggtca ttcccctgcc caagcatttc aaattccaga agttcatgcc ctgcatggtg 93300
ggtgaaaagt ctcaggccaa ccatgagcac acagcagcca ggcgactgag gcagctgccc 93360
ggggtggcac gttgctcaaa cccatcattt ggagtcaaaa caaacagatg attagctggg 93420
gtggtcactt tcaatcaaga gttttcacat cgcctagaca tggcctcaga atcaggcctg 93480
gtgtggccag gggctgatct cacagtagac aggaagtgtg gcccgagggc catggctgcc 93540
ccctcagaag gccctgtgga gtggctggcc gagcctcagc agcctcctgt gaagcgagga 93600
agggtcttcc tgccggcctc tggagatcag tatgggaatg cacaagtagg aaacgctgga 93660
tgggaatccc tctgccctgt gataccaagg cagtgagttt gtagactatg gaattgctgt 93720
cggagggctc tgtaaccggc caaggtcaca caggtagcca ttggtagagc agggactgga 93780
atcccagacc cccaacttcc aggactgtgc acctttcttt atcccataca gccttacagt 93840
caagtgccag tgcaacacct gattcccagg ttccagcctt tgtcttttat aatgggaatc 93900
aaccttatct tgacgatcca gagatagtca tcaaggaaga ttaaattatc cccttagact 93960
cagagtgacc atatcatttt ccctccacac aaggacactt ttgagaatga aaaggaggag 94020
atgtctgtac cagacgctgg atgacaggca ccgacaggct gtctgccagg ggagcagcga 94080
ttcctgtatg ttgtagaaag tttttcaaaa gtcaccttgg aaagaggttt tgttccttaa 94140
ccttctgtta aataggaagc tccgtgaatg aaaacaactc ccttccctaa acattctagt 94200
aatgacccaa cactgccaag cctgccagct ctgcctcatg gtcgtgttga ctgtgtgaga 94260
ctatgtgagt gcctgctaca cagtacgctt tcagtaaaca tggtattgcc tcgataatcc 94320
cacaaaaatg tcctattcaa atcacctggc acccaggaaa tttccttctt ttttttccca 94380
ggtgaaatat acagttgaaa acacctgaca gcaattcccc tctcccatgt gtttgcagga 94440
tggtggtttt ggttcctcca tctttgatgt gtacaagtgt gatgttttcc ccccacagac 94500
aagtaaacca cattctcttc acattcccaa tgttttgtca atgtacctcc ttcaatagag 94560
gatcgataag gaaaaaaatc attgacaatc tcaattagat tcactatttc atccaaaagc 94620
atagcttaga actctagttt ttgttcaaca ctcttgccct atgagtgcac agaactttaa 94680

ttctgataca aacatccctg aatgtttagc tttgacagag attccaaggt gatttgataa 94740
gaagcagggc tgtgtttggg ctctgggagt tttgatatgg gtttcaagcc ccatccaaaa 94800
cccacagacc tctagaaagt aggtgcctgc cttcctgcag cagccctgga gcctgctggg 94860
ggctttgagc agctgctgcc aagccaggcc tcacccgaca ctctgatggg cacggccatg 94920
gtggcagggg cttggacgct gccaggtgac tctaacttgt ggccagggtg ggaagcactg 94980
ctccacagag gtgccaaaac caggttcctt cctgtgttct cacatttcac agcctcaatg 95040
taaaaagtaa gacatgggca ctctggaata ttacaaaaat atagaaaagc atgttatagt 95100
aaataaaagg ctcacagaat tttgtcattt aggaacaatg attattaata tattagtgtg 95160
tgtttttgct cattaacagt atatcctgag atatttccta taccatttaa tattttaaaa 95220
gatgtttaca ctggccacag tagctcatac ctataatccc aacactttag agggcaaggc 95280
aggaggatca cttgaggctt aaaaattagc caggtgtagt ggcacatgcc tgtagtccca 95340
gctactcagg aagctgaggc tggaggatca cttgagccca ggagttcaag gctgcagtga 95400
gctataattg caccattgca ctccagccta ggtgacacag tgagaccctg tttctaaaat 95460
aaataataaa taaattaaaa catttaaaaa tacatgatgt ttaattatta gaggactcaa 95520
ttttatatct atgtatacaa taatttttaa gtttcttaat attggacttt tagtaccttt 95580
ttaaaaatac tatttttaaa aaaatctgta tttctaactt tttataacaa ggaacctttg 95640
gctttgagat gactggggaa tccattcttt cctatagtat ccatgtccaa tggacttaaa 95700
gtattaatca atgtgtttat gttttgttat ttttctggca ttacaaaaaa ttctaaatat 95760
attgttaccg cctgtataaa tatcagcttt tgagagaagg acattgtgta gaaataatga 95820
aacactgcaa cttgtatttg tattattctt tttttttttt ttttttttga gatggagtct 95880
cgccctgtca cccaggctgg agtgcaatgg tgcgatctct gctcactgca agctccgcct 95940
cccaggttca caccattctc ctgcctcagc ctcctgagta gctgggacta caggtgcccg 96000
ccaccgcgcc gggctaattt tttgtatttt tagtagagac ggggtttcac catggtctcg 96060
atctcctgac ctcatgatct gcccgcctca gcctcccaat gcactgggat tacaggcatt 96120
atattattct ttaaattcac atgagaattt agtatggctt caaaaaatac cataagttaa 96180
aatatcacca agactctgtt cagacaaaag tatcagaaaa gtgagccagg cactcacata 96240
gtttatagtt tataaaagtg agacaggcat gatctcttaa cctcactata gtcctgtgaa 96300
taaggtttat ttacatttca ttttacctgc caggattatt gtaaaaacgc caagcacatt 96360
gcctacacaa actaaatatt cagtcaatgg ctgctatttt catgagttcg ttttaacata 96420
tatttattgt cctctactgg atttaagaag ttatatttat tatcatctaa gattttagct 96480
attccttctc ttaaaaatag attttataat caatggcagt aagggagagt aactcgcagt 96540
tctctgaatc tcaaggggtt cctggaagcc ttcctgaagg tatagtgaaa tttcagcttc 96600
acattcccat ccatgagctc cctgcaaata tcccggtctg ctctcaggac ccagtgactt 96660
acctatgcag aggctgtaga tagcacctgg agcttcctgt gtgccctcct caaactcagc 96720

caatgccgtc atacagtagc aggcaggtgt ctttgctggg tagttggact ggatgtccct 96780
gggattgcag aactggaatg gggagtgaca tcaggaaact ataatcatca ggacaacatg 96840
gtttgccata actttaagtt ttaagcgacc gcagattatg cggagagaga tgcatgccca 96900
cagccatgct tcccatgtaa ctggagaggg gtctgaagtt tgaaacaagt gttcctaggc 96960
acgggttaca gtgtttgtta tcatcatact tgatttagaa tggggcacaa catgtggatt 97020
catggtaact gttacaacct tactcatttt aatacctgaa aacatgcttt ccccatgctg 97080
ggaatcgaaa gattctccta ggaaaagaaa ggcttgacaa catcgattca aaaagggcat 97140
gcattttcct catttaaata actctaatgt gcaagtagat cccctgacct caagctcaga 97200
agagtccagg ccttcacacc ttctctgctt ctgctctggg gccagctatt gagattcctg 97260
tgcccacgca atgcgcacat cccacccctg gccgctgtcc acaagaaatc cagttgcacc 97320
aagcacccca ctttttgcac ctctcattta tgtactccta agagcctcac cacaactccc 97380
ttctaaaaac atgagttcct gactgggaat tcgatgctgc ccaggcagct ttgctcagag 97440
ggagcagcct tctagaaatg tttcaagtaa actttcaagt ataactaaat tcaaaaaaaa 97500
cacatacaca cacacacaca cacacaagtc aaaggtgtgt aatttggcca atatcacaaa 97560
ccaattagcc ctttgtaagt ggcacccaga tcaggacagc tgaccatacc agcaccctag 97620
aagcaccccg tgctgcctcc tgggacaggg ctaccaccat cctaaggcca gcacgatggg 97680
ccagctttgc ctgctgttga attttgctta catagaatcc tccagtaggt actcctttgg 97740
gtcaggttct ttcactcaac attatgtgtt gatatttttc catgctgtgc tgcaaaattg 97800
tatttcttgc attccataac tgggcagttc catcatagga gaataccaca ctgcgttcgt 97860
ccattctacc gccaatggac atatgggttc tttctctttt cttgcagtta caagtttatg 97920
aatattgtcc cacgtgtccc tggtgaactt ttgtttgcat ttctgttggg tacctcagag 97980
tggcgttgct gggtcagagg gtactggtcg ctttagtagc tttgaaagat attgccaaaa 98040
cattttccag cgcagttata gcaaattata caccaccagc agtagaaaac atctcctaat 98100
tgctcacagt aaacccccaa agattgccac atacatcttc catatcaatt acttaactat 98160
tcagcaaatt tgaagggaaa tatatttaat ctttttattc aaatagttta taaagtggaa 98220
tagagatgtg ggtaaaagtt gtcttgccac ctttttagat cggtaaaagt ttgttgaatg 98280
caggcaagaa aagatgagaa ataatggtac ccaatgaaag acatagcagt ctacaaggag 98340
gggcatttcc cggggtgggg gggacccaca ctctgtaact cccacattca attagcatgt 98400
tataggtaag ctgcagaaaa cgaggcagct tgtcaaagag gaacggctct tggccatggt 98460
tgctgcccta ggaggatatt tgatactagc agagctgggg caaccctgga ggaaaccacc 98520
tggaatgatg ggagaactcc tccagggaac atggcccttt aatagatctc tgttataaaa 98580
aataatccca aagcagccac cagggcatac tgctgcgatc aagtcctagg cggtattccc 98640
ttctgcgcca tagaccctgt gcagagtgcc ctcaacgaag gagcaaggaa gaccaagtct 98700
cccgagggtt tgcatatgtg tatgtgattc tgcagtcatg gtgaatgaca cagtcagggc 98760
tgcggaaaag cattggtaaa gtgtatattt gaggcttcag aagtttgaaa aggctagatt 98820

tcctaggcca aaacactgaa aatttgcaat tagaacttca gtgctgatgc tgggaagact 98880
ggagttagtt tgagacatgc acctgtgcag aactgggccc ccagaaaagg agaaggaagg 98940
gaatccagac cagagtaggg cctgacacca ctcagactcg gcgtgtctat aaattagaat 99000
tgcgttacaa ttacactttg acattttagt ggtttttaaa gtgcccagca caagttaatt 99060
tttcattaat gaatccttta ttcataaaat gcttagatgg agattaccct tttgagcatt 99120
ttgccagtgc ttctgaaatt aatggggacc tcctgttgga ggacacagtc tgttgcaata 99180
ggtgaccact gctctgaatc tatgtcacct ctccaggacc acgggcacaa ccatcacctg 99240
aggcatgttg gagatgcaga tggtcaggcc ctcctagaat ctcagaatct gcattttagc 99300
aaagtcctgg gtaattccta tgtccattgg agtttgagaa gcactggtaa tctcaaatac 99360
tttaaaagat tactagagta agataggctc agtaggtacc tgaaggcacc atcccaaaga 99420
ccagagtggt agaagcaggt ggaccagcct ctgaacacat ttctccccca ctccccggct 99480
gtgtggaagg ttgccacctt tggggtagtc attcaacaaa cacgtgtcaa ctgtccacta 99540
tgtgtcaggc caccactggg cactggctgt ggctagctgg atagacacca tttctgccct 99600
ccagaaatgt catgtccact ggcacatgac aagtcactaa gtcattcaga gccatgggtg 99660
acagctccag gggccgacaa aggagctgtg atctcacaga tccacagaga agtgtcccag 99720
ggcgggcggg aaccaggact gcacagggag gggtgaagtg acacataaga agtcagccca 99780
tcagcctgaa atgctccccc aaatcttccc attcagtgtt ttctcagtag caaactcgtg 99840
ggaaaattgg ttattttact taaaaaactc atactagaaa gctagtttaa ctttaaaaat 99900
aaattttaaa aacattttta ttaacaaatc ctacctttcc tccaaagtca aggagaaaag 99960
aatagaagtg aacaatggac caagtaagcc taaaactctg ctctttcccc tgctcatttt 100020
acagttcaag tgccattcaa tttatcctgg caagaagagg aaggcatcat caagacctta 100080
attttctaat acatctgatc tgagaagaat gtgaaagcta taaaattaat ttttgatcaa 100140
taactacagg ccttttgaga gagtgccctc ctaatgaatt gagtacctat ttctccatac 100200
acagtgtcta tcatgaccta caaacccttt tcccatgagg tgtaacagag agagattaca 100260
gccttggaac tggatgtcag actctcctgg tttaagacaa taagccatga catagagcct 100320
gaaaccaaca caatcttccg agtggttcca gaaacatata ggggataatg ttggctctga 100380
tgctgtacat ccccaacaac catcaactat ttggaaacta gaatttcagc ataattggag 100440
ttggtgttac cctagcaaat gctgtgggaa gagagtctca ctgtgtatct tctcctgttt 100500
aaagcctgaa tttgttcaga atgtaatatc tctgtttagc cactctactg aaactgatct 100560
aggaaatgtt caaaaaaagg tatcccaagg atccctttgt agctacatct gtgggattcc 100620
cctcgctctg gcgtggcctg gcccctctgc atttgacaat acggtcctat gcttttgtct 100680
tcctgggctg cgtgaaccca ccctgccctg gttcacctct cctcttgacc catccttatc 100740
agtgtcttga aaggtccttc tattggagga cacattctgt tgcagcaggt gaccactgcc 100800
ccaaatctgt ttcacctccc cagggccatg ggcacaacca tccctggagt gtgttagaga 100860

tgcagttggc caggtcctcc aaaatctcag aatctgcatt tttgcaaagt cctgggtaac 100920
tcctatgtcc atgagagttt gagaagtact ggtctcatga gttcctgaca tacaaatagt 100980
gctgaggcca gtatgctgac tgggtagcca gatacaagtg aaaaccttcc tgttttttgc 101040
aaacctggat ggacccgagg ccgctgacgt gggccaggac aagctactct ttttcagtgt 101100
ttctgttgca tcgctgtgtc tctctgtgat caggtgctgc cctccctggc aggaggactg 101160
cagacaggat gaccaagagc actctacaca gcctgctctc cagtgttggg ggacgccacc 101220
caccctcgtg gttcctgttc atctgcctac acgtggaggg cccaagaggg ctaatatgtg 101280
actatctcca cttcctggta ccctgtgtga ataacttcac ttactaaagg gatgttgagc 101340
aactttatta ataatgaaga aagcactttg gtttgacaaa taatcactcc attttttcat 101400
ttgaaagtta actcttgtta gtagagaaag caatgtatta caaccacaag gacgtttaca 101460
tggaaatgaa ccatctgcaa agcatccccc attttccttt taaatcagcc aatgggtggt 101520
ggtgggagaa atattcacca gagtatttaa catctatccc ccttcctaga ctgtcagctc 101580
catccgggcg gagactgttg gtatctccac agcacacaca gggcctggca cacatccggg 101640
gctcagtgag cacttgctga atggtgaaca gattagctct cctgggaacg ttgttgacac 101700
atctcataac actggtttgg agtggagggc attcatcggg ctgcatattc ctatttttaa 101760
ttgtattctc cactggttac agcacctaca gttataaaga cattgttaac attgcttata 101820
ggaagacatt tgatggaaat gagtccaaag gcattacggt tagaaactgg ccaggtgtca 101880
tttttgagag attagataac tgttttccgg tagagtgaat tgcctgtttg ttgcaagttg 101940
ggactttgct gggctggttt acagggccaa ggggaaagag ataagtggat cttctagtga 102000
gaggtcatct gttttgaaag cctggaagat tccatgaact aaatccaagt cttacaacac 102060
agggaagtgt gtcatactgt gcagggatga agtctccaat ttagcatgaa aacaagagct 102120
cctcacactg tcctcttcag aaagcccata caatccaaac ttctgaatgc ttagctgctt 102180
acaaccatac atagattgag ggataaaact ctgatatgga agagaaggta aacatttttt 102240
ggcagacatt cccaggaaaa ggcggctctc ttctctcatt gctgctgctc tttcagaatc 102300
catttcaaca gaggaggagt caatgggagc cccgtgcctc tggcagatat catatggcgt 102360
ttcagtggca ttgtgtgtta cccttcttag gtaacagctc agccattaga agaatgtcct 102420
acacaccttc tcattttctg tgatgagagg aatgtgaggt actgcccttc gagagctgtc 102480
atttgtccta gtagccagca gcgtgactgt gctgtcttct gctctgtctc cctgtcagcc 102540
ttctgcccag ccaccaccac tatagttttg ttctctccat tggaactcct ggttcagaga 102600
attaccataa aaaacagacc cctagacata caacactcta tcacataatg gtgactttgt 102660
cttctatttt ggattactga gctttcttgg gtaacttcca ctaaatcgaa gttaatatta 102720
gaagaacttc ctcttactag aatcgaaaag catttaagtg atgcagtcaa gtttgtacca 102780
taagtaattc agtcatttaa caaatatata tggcctctgt gcgacagtga ccttgactgg 102840
gaatgaagct gtcccatgtg gggcctgttc ttcaaaggca gttccctgct gcccagttca 102900
gtccagtgga tctgggcatc tctctttaat ccgcattagg ggctctttac tgattcttca 102960

ctatccaaaa agacttggag gggagacctg agcccacttc tggaaggaaa tgataacaat 103020
ttatttagat aatctttgtg caacaagtca attcactgaa gagatctgct ctctaggagc 103080
ctctgtgacc ccaccataac tgggaaggct ctacctctcc agtcttcggg ccacatttct 103140
ctctggcctg ctgtcttccc agcactctca gccttgctca tggagcactc tagtcctccg 103200
tcgaccttgg cctttggtaa cgtgattttt cacctggcag ctcccatctg gtctcactcc 103260
ctctttttgt ccagtctgca tgacacagcc tcacatcgtt agtgttccct cactcccctc 103320
ttactgccca acctgcaaag tccatgcctg ggccagtgca gcatgtgtcc tcaatgggct 103380
gctggtggca gtggggggaa ccgcacagcc acgctgtgtg ctgctgaaga aatgcacagc 103440
ctcctaccct cgccctcaag aggcagccat ggctgcgcat ttctgccctt ctgagctccg 103500
ctcacttttg gcagcagccg ttccaacctg catgggatct tcactctctc acagatgtgc 103560
tgactcctcc tgctgcctcc cctctctgtg ccttctcact ctctgttccc tttgcccttt 103620
ctcccctttt ctcctctgcc tacctccaag ccatccatca caggacagct caagcatcag 103680
atcctctggg acactttcct tagttgttca gtctgatgag gtgtccctca tcctctctta 103740
gctgaaaatc agcagctgcc tcaacttctt ttccagcatg tctcatgagt attgccacaa 103800
cagcatctgt cacaatgtgg ggtagtggct gacttgcttt tctgccattc aactgagttc 103860
cctcagtgct ggggccagcg tgcagtgtct tgtattcagt atatagctga ttaattgatg 103920
aattgattaa ttaatggttc acactagcac agtgcaacct tcaatgcaaa gatctcatca 103980
aaataattca catggtggga tattttagaa ggatgaccag gctagtttgt agtaagaaaa 104040
aatcaacaag actaggtcag gaattctttt tttgtctaca ggcttgctat agaagatatt 104100
gaaaatcatc tacctaatta cctttatttt atcaggttgt gtattaaata tcacgtctgg 104160
gggaagaaaa tgtgatatgt gattacagac ctttcctggt acaacatagt acgtttcaga 104220
ttaactcaag gtattgtggt gatattgcgg tcaaagccag gtgattaaag agtcattctt 104280
tgaaacaaat atctgtgcaa tcaattaaga aattaatttg caaattttat ttgcttagag 104340
taattgatat atcattcctt ttacaaacaa atataaagaa aacttaacta aaaatactgc 104400
atatctcttt cagattatat atcccagaaa ggatatattt ttctcctttc tggtcttcct 104460
ttttggtgta gcatctgtag gaaatgcatt tcttcatagc taagtgtacc tccttgtgaa 104520
atatcttcag agtctactgg tgcacataag caattgctgg cagcagcttg agggtctcca 104580
tctcacattt atcatatgcc ttattgcatg aggctttgca agaggaggtc tagagctaca 104640
atatctcatg gatatgaatg tcaattcaaa tcccagtggc agtttatgag ggggaaagcc 104700
tagaagagaa gaaacctaga ggaatcaagc aggaggggag agtaataaaa gactagagca 104760
gcaggttttt cttaactcaa actagaatta aatctctgtg tgtgtgtgca tgtgaatgtg 104820
cccgtatgtg catgcatgca cgtgtgtaaa tggatgtgtg tgtgtgtgca tgtgtgtgca 104880
agtaagtgtg tatacgtgtg tgggcatgta ttgtgtacat gtatgtgtgt tttatgcatc 104940
tgtttgcaag tatgtgtgta tgcacataaa agtgtgaatg tacatgtgtg cttggtgtat 105000

gtgtgtgtat taatgtatgc gtgtagttct agagtctagt tagagaaagt gcataaagaa 105060
atagggaaat taacaagaaa gctatagctt aaattatagg aaaaactttt ctccctatca 105120
gtcatggttt taaaatgttc agacttgata tgtttcccag tgctattgtc agaaaatgtc 105180
cctatgacat tccatactac ttcaatcaaa tctaaaacct ttgttccaac atgttttatt 105240
gatatgagta tatttcaaat ttctaccagg tttttggaga ggtattttgg ccataaaatt 105300
gactaaatta ttcaaaataa aaaatgaata agcctgggcc aaggcttgga gacttgctta 105360
actcagttct taaattttca gattttcaaa attacaaatt taagctctaa aatcatggtg 105420
ctgtgtatga tattctttga ttgcaactta tggttgaaaa actatagagg gctttatgct 105480
aagagttgtg gatcttagga ttttcatgaa atctgcatta tcatcatctg caagtttaga 105540
tggggcataa ctgatccaaa ggatggatcc ctcgggggca attcaactgg ctgattccag 105600
ccaagatgac aacagtcagg atccgttccc ttctgatcat ccattgggtg ccctgatttc 105660
ctctacagcc ctagctgaaa gaccagacac tatctcaggc tggctgcccc acatgccttg 105720
ctccacacca aattcacagt ctataaacct gagcctccag tgctcctact accatactca 105780
ctcgaacatt cccgattctg acctggagat gtcaacagct acttgatgcc actctcttct 105840
atctttctgt agctaagcca tccccaagtt tgtcgattca ccctctttaa cccctgtcgg 105900
ggtgtccatt gtgccccttc accctgccat ctccctggtg cactgttttg caaagttcag 105960
catacatgag cgtcacctgg gaaccttaat aaagtgcaga tgttgattca gcaaatctgg 106020
gatgccctcg ggctgcattt ccagcaggct cctggggatg tccccgctgc tgtgctgcag 106080
atgacactct cagtggtggg actccaggct ctgctgtcgc ctcctagggg tttctccaca 106140
ctccctggag gcctaatggg cccttctcca catggcagta agatctgttt ttgtgtttgt 106200
gtttcaagtt gggagaagga gattatttaa tactaaaatg tgcaacatgg gattgagaaa 106260
actaattatt agtcataagt tgagtatgca acattgaaac cacatgcttt aaaaaattat 106320
aagaaaaaat catagtattt gaaagttaca agctattatg gctaactcca tttatctcag 106380
ttagagaaga agagtcacct gtcaccaggg cactgccaga agccaggctc atttccaaca 106440
gcactgggtg ctccagcttt ggggtgccag ctcctcccat aaagcaaaca catacctagg 106500
gatgatattt ctttgcaagg gctctgccct acagcttgta catctcaaga agttatgtaa 106560
ttaaactgtc tgttttgaga aaattgtaga ttcacacata ctagctgtaa gaaatgatgc 106620
ggataaatcc agcgtaccag ctttccccac ggagacgtct tgcagcgtca cagccaggat 106680
gaggcattga cccaggcgaa gtccagagca cctgtgcgct acagggcccc ttgcactgtg 106740
ctgtcacaga cacgcccact tccagatgcc atctaggacc ccctccaaaa agcagaggca 106800
ttcttaaaaa cacacatctg cacatgttcc tcttcatttg aatctgtcag tggcttctca 106860
gtgcctttca aatgaaatct aaagtcctta caagccttgc agcaggaacc tctccatccc 106920
acttcccctc acactctcag cttcatctct gctaggctct gttcagccag gcagcctttc 106980
acagtccctc tcctcctgcc ctgccaggaa ggtcccctgc ccccaactct tccccacatg 107040
tggcggggcc ccgcttgtcc ttagaagccc agctgaactg cttcctgaag gaacccctcc 107100

agaacctctc agaccaggtc aggtttctgc actcttagat catccccatg gcataatcac 107160
agttgtgatg ttgtgatgat tcagtgaatg tctgtctccc cactggatgg taagcttcct 107220
gagggcagga acagcattgg ttccagtcaa tgctatgtcc caggactgtt cgtttttgca 107280
catactaatc ctaaaaggac gatgacaaca gcaaccactt acatgaccta gatgctcttc 107340
tgggtgttgt gcaaatatta acaatttaat ccttgcaaca atccacgagg gaggcattct 107400
tctactccca cttaacagac aaggacagtg aagctagtaa agagaagtca tttgcccaag 107460
gggaccccac tactgttggc agagctgggt gcaaacgcag gcttgtgaag ccaggaccca 107520
tgcattcaaa gaccatgcca ggtgccccca ctgcacacct catccccaca taccagtgag 107580
ggggagagaa atgctcctgc actgcctctg attaactgct ttcctagaag tcacacatat 107640
aaaagggatt taattctagt gggattgaat ctcaatagtt tccttattag gttgatttct 107700
gttaatagtt taagtactgg atatacatga attagaaaat ctagattatt agcaaatgca 107760
aactataaag tattttataa atgttatctt gtttgtcagg ggatgagtga gatattcatt 107820
atacaaaaag tagtgtggat tttgaggtag aaggtttact aaggatcata ccgtagtatg 107880
aaatagccac aaacattcag tgaaaccaaa cacccccgct taacctcaaa ctaacactaa 107940
ataataagga atagacttgg gggcagtgca agtgtatttc taatggtgaa aaccattccc 108000
cagtgaaaac taatgtacca tctagttaat aagagctcct ctgacccacg cacatcaata 108060
cttacatccc aatggtgatg tgacattttg ggttttgtat ttcttttgca aattgagcta 108120
gcatttttga tgagtggcag ggctctgcta cccaaccttt ggacagtttc caagcataaa 108180
atcacaattc cagataattc tgtcacaaag atctgggtct cattaggaag gagaggaagc 108240
tgggagatga tccagtccaa cctcccccaa accaaacatc acggccttct cagttgtttc 108300
accaaccatc taaatgtttt agtaattcta aaaattgatg cgctttttcc acgaaaggaa 108360
gtgttaccac attttccaag tgggaggcat ctatatcctt actccttcat cctctccttc 108420
ccaccccctc accccccacc acccacacaa catctgcaat tcttaaacta aagcacaaat 108480
tgttacaaaa gttaattgca ctttcaaagg aatgcttgta tagaaacttt ctcggcttca 108540
aggaaaaata atacgctttg aatggctgtt caacagcata gaaattagct gagtagaagg 108600
cactcatata gccattagga ccaatccttt ctgccgccaa cacccccctt ataaagactt 108660
gacagtgggc cagaataaac aacttcagga tgaattcagt tgagacacaa agtacacact 108720
tccagttttt cccttctctg gttactggcc tcaataacca ggcagtcaac ttaaaaagaa 108780
aaacaaaagc ttgcttcaga ttacagattg cagacttctt ataatatgtc catttcacca 108840
ggccccgctc tcagccccgg gaaaggccac tggaaaccac ctcacatggt agggccttgc 108900
gggagccagt aataacctta tctccgtcaa catgttctgt cagattgaat ggggcagcca 108960
gagaagccag agttggcaca ggaaccaaaa caaaggcttc ccatcctcct ggagtgagcg 109020
gttgagcctg gattggtgct tagacctata atgggtgcaa gcagcgttca ttcatagtgg 109080
ctttctagac ccagggactt ggccccagcc ctgctgctcc actcctcttc ttgcttcatt 109140

accacgagtc tcctagacca ccgaacgatg cctgcatttg aaagacactt ctgctgatca 109200
aagcagctga tgtgtccctt tgcggttcat ttctaattgt ccccaaggag gagaaattca 109260
aatagtttat tactgagagt taaagaaatc cactgaaata ttctttggtc taaaattact 109320
gtcatggcgg agcagcttca ccttagtcat tgcccttaaa tatgaaagct atttaagaaa 109380
gtttgccctt aaatatgaaa gctattttaa aaagtttaat gaaagaagag aatcacaaaa 109440
cattttcaaa aagcaaaaga aaacctaaga gaaaagttga aagtaggaat tttttaaaga 109500
atatacgacg tgtgttctgt gactcacccc tgcaagttat ttgtgtgtat tcccttgcat 109560
agtaattaat aatgaagcaa agcatggcaa tgatatcttt tcttgtctag tattctagaa 109620
gactccatgt ttttggaaaa tatcactcta gttagatctc aaatatattc aatcagaaaa 109680
tgggttttct acaagattct atatctgtag tcaatagcaa atataattct attaagctag 109740
taggatgtga taggaaacta aaacctaggg gagaccaaag caaggaaaaa tacttcctca 109800
tccaaacttg agagcaattt accgtcaggc ctactattaa tagatggaat acagattcca 109860
ttttcattac tcaactgcca tattcattat tacactgtac agaaaaggga atcacatctg 109920
ttgaaaactt atatatgatg ttcatgcatg cattccagta attcaacaat ttttatttat 109980
ctttttattg cttgctaatt tttcaaaata ataagctaaa gaaaacaaaa tgtttgtgct 110040
gttctcagat gacatgttat ctctttaaag gacaaaatgt gctgtgaaat aatagaatgc 110100
tttcagcact caagtgtgag tgagtgctca tacatgagag aaagccgtgg ggactacaga 110160
agccaagaag cagatctagc tggggaggcc tttgcagagg atgtagttgt gtggagaggc 110220
cacacacgtg gaattcccag gagggctgtg gaggcgggga atctgcagga aagcactggg 110280
gtgagaaacg tgatgagaaa caattattgt cttaaaatat ctgcagggct gtaaggtaga 110340
gaagcaatac gttgcatctg tgttaagtca aacaaaatta tcaagggact ggtttcagct 110400
taacataagg aacaattatg tgatagggtt gtcaataaca agagtagact gcttcttcac 110460
acactcctag tcactcagaa tggtccagga ggagtggaca accatttggt agagtatggg 110520
aaggcagggg ccctgggtgg gagtggtgag ggtagggagt gagtatccca atctagaagt 110580
aaattgtgcc cagcacggag ctgcaacact gccctgcaca caaacacaca caaataacaa 110640
tccccagccc ctgcatttcc ctctccggtt tcaggacctt gtatcttact tcaattcctt 110700
tatttagctg atgatgaaat aggaagagct tagcactaag aaaatccttt tggagtttgg 110760
ccttggggga aaatgaatca ctccaaccag gtctgtcttc tagaaagtat aggatgaaag 110820
ggctcctcat cacatacttc ctgacctcct gctaggcctt tccctaaaac aggggctggc 110880
aaagcacaac ctgtgggtca cgcctagcct gccacctgtt tttgcaaata aagttttatt 110940
ggagcatgac tatatgtatt tgcttacagt ctgtggctgc gttcacacta tcccagcaga 111000
gttgaataat tgggacaggg accatatgat gggtgaagct gaaaacattt actctctggc 111060
tgtattcaga ggaggtttac tgagcccttc tctgagacat ggcaagcgct gcttcaggct 111120
catgcttcac tagattcagg cctggggcag taaagagcca gctcaggata gcactcccga 111180
ctcactcatt ttttcaggca ggggagccat ctaatgtcaa gtgcctacgt gcaggaactg 111240

gtctgttaat tagcagctct cctcatggaa gggataatat attctagaaa caggagtgcg 111300
gccctattgc aagaatgtcc tgagccaaaa ttaagattct tctatggcag aaacttggct 111360
ggggcttctc ctgagttaac ttggtagttg ttagtgattt ttgagtcagt ttttccttgt 111420
caacgacccc aggaatgagt ttgggattac agggtagcca gggaaaggga aagcttcacg 111480
cccgcccccg ggacaaggtc tgtcttcaca ctgctacatc ccttcaccca ctttaaaatg 111540
aaacttaaaa ggaggatttc agttgagtag gaagtgagaa gagggctcat tttaaaacaa 111600
gcgttaaatg aaaacccaca cacactcaga gcacacaaat ccaaccacgc ttacaaaacc 111660
atcacagagg gtcaggcgag gcccttttct aaatgaaaaa gaacaggggt ggagactgtt 111720
ctgagagcat gctgggttcc ctgaagggaa ttctcagctg tatgtgcccc gcacaggatc 111780
cctgctagac acaaggccag ctgccttcct ttcaagccgc agacgcatcc ctgtgtccag 111840
gcgggctggt cagctgcggt cagcaccagc ttccccgctc catggtgagg tcatcacaac 111900
atgtgagcag gagggcaggc cggcaacctc tgagtgctta gagaaaggga cgggattcct 111960
cctgtgcaac ccctctagtc tcactcagac tcaagtctga ctaaggggcc aggtgctttg 112020
accagggact ctcccctctc acttccctcc caggagtcac aggtacatga gtccttgttt 112080
tacaaatgaa gaaaacagac ccaacatgat taagatgttg ccttcatagg ggtggcacca 112140
ggattccaaa ccatggactc cactgagccc agtgcccact gacatgtgcc agtaacagtg 112200
cagctgcctg tggttctgtc gactaaactg ccggcagagg ctggctttcc accttctttt 112260
tttttttttc actcttcaaa cactttatga catgaacata aactactggc tgcatcgttc 112320
tgctgacaac atgacatgtt tctataactt gaaaaaagca agcagtggac tgctcattgg 112380
taaaattgag tcagtaatct tttaggaagg ttatttttct tccttttact gcttctcatc 112440
tgttccccgc agtaaagagg acaagatgac gacgactcag ggaacacctc cagcctgaag 112500
cagcaccatg cgagcttaga ccttagggtc ggcttagaaa ccacaggcgg ggcggcttgg 112560
gcccctcgga cactccctct cgaagctgct tctccccaag ctaccccaaa ggcactgagc 112620
gccctctgcc ccccagcaat tcaattcact ggctgtcctg ctcctgtcag tactgagagt 112680
tgcatgtttg accctcgggg gaaaagtcca gaggccctgg ggtgtccagc atgctctgag 112740
gtccctgctg ctgacccctt gcgctgtcag cattcagaga cattcacaca gcacagcctc 112800
ccaggctaac agctgtcatg gaacagtgga gcagctagac gtggccattc tgtggcccag 112860
tgctgcagag gtcaaaggga caagcgcagg gagcatcttt gctttcagaa aaaaaaaaaa 112920
aaaaaagaag cacactggtg cactgacctg ctcctggtgt ctttgtgatt gctcttttct 112980
ttcgattttt ggttgtcttt ttttttttga aagaggggct tttatgcttt tttcctaatg 113040
ttcatgggta aaccaatgta aatgtgtgta tgtttataga gatggcttta aatcgcaatt 113100
ctgcagtaga gattgatttt ttaaaaaaca tgggtaaaaa ttgaagaaaa attttaaaag 113160
aacatttaaa ccatcttggg ctaggggtgg atatgcacca ccccacggaa gccaaacaaa 113220
atctctctgc agataaacat ttgcaaaaag aatttccaat cccaattttt gagtcagaga 113280

tcttttattt ccttgcaaat tacatatctg tttcaggatt tttgactata agaagaatga 113340
atgaagatgt gtttcttaca gataactatg aacaaaccag gaaggataat aacttgtatc 113400
ccccaattcg aatccagagg atgggaaggc ataaaaaaaa gaaatggaag aaactttatt 113460
tttagtggta aatggtggga ctatgtattt tacgtatggt gaagtcacca agcccaacac 113520
ttggcacttg taggcaaggt agtcttctaa tctgaatgtg aagtattatg ttttcatttg 113580
cttggtaatg aggaatattg gtgctttcgt cccagttctc gagctgactg acttctcttt 113640
ctgacgtgtg ttcctttagc acacctctac actgcatggc tctgagatgt cctgtgactg 113700
tttcatgtgt aaagttgcct ccccaaagga ctcacatatt ccttcagggc agtgagtact 113760
tctgattcat ccttagcagc taccttcgcg ctactttact agatatgttg tagttgaatt 113820
aatgaacaaa agaacaagca actttggtgc ctggtgtgca tctcagagca gggtggagtg 113880
agcctggcca aagggtcatc atgcaacctc tgtggctgac tccatctggc cacggagctt 113940
ctcagccatg cttggtattc acatgacttc tagggcgaca gctcaaccag caaataaaca 114000
gcttcatatg ggaaatatta ctcagccttt gtcatcaagg agtgagtcac gggcctgaac 114060
tgaatagaag atagaggaga aaaggtgtgt ggactgggtg agacagcgcc cagcgaggtg 114120
aactcccggc agccctgcct gtctttacct gcacatcacc ttgctagggt gccttcggtt 114180
gtgagggcct gtctaggaag agaagagttg caccctggca ggcagcactg agctgtctca 114240
tgcaaagctg aggaagaaag agtgagctgc ccagtgagcc tgctggggtg gtggaggctg 114300
ggctgggctg tgcagtctgc agcccccagc agcccttggc acctttctac tgcctggtgc 114360
tcaccagctc tccagtaaca aagagggacg tgaagtcaga ggggaaggga ggtagcacag 114420
ggcagtcttg actttgaaca aagagctggc ttcctgaagt cagctggccg ggttttgaag 114480
ccgattttcc agcagtgatc tttgatgcca accccattta ggaattctgt atctccccct 114540
accttctacc agatgtctct gagctcacct ttggtgataa tcatgcaatc tccgtcatcc 114600
ccacgtccac actgccccat tctgtcccac cccgggttct gtggtgctgt cggctcccca 114660
gcgagccagg aagggagagg ccagctctgc tggggctcct gccgccctgg ctctgcactg 114720
cccttctctg gcaggtctga ggcgccactg gaggagccac acggccctga agcagcaagg 114780
cagatgccct ggacacagtg gaggcacaga gtgcaagcac cggcctggcc cacagacttt 114840
tggaggggaa gtggtattat tcagttcaaa agtatgcctg tgtgtaaaga gagagcccct 114900
gaacatgagt aagcaaaagt ctcagcgcag agattagaca agtagaatgc tggcccgaga 114960
ggaggcgttt actcaccctc tgtctaggaa ggaaagccag gcccagcacg ctcactgcta 115020
tctatcctct cacacagagg gattttgaat cgaagccagc atcctgtcct ttctccaatg 115080
tcccctgctc aggagtcagg actcagcaag gcccacccca gccacacaca gatacagttc 115140
caggactcag aactcagcga ggcccacccc agccacatgc aggtccagtt ccaggattca 115200
ggacacagtg aggcccaccc gagccacatc caggtccagt tccaggactc aggattcagt 115260
gaggcccacc ccagccacac acaggtccag ttccaggact caggactcag cgaggcccac 115320
cccagccaca tgcaggtcca gttccaggat tcaggacaca gtgaggccca ccccagccat 115380

atccaggttc agttccaggt aaatcatctg ccttcctccg tccaaaagcc ttgtttcctg 115440
tgtgtccttg tgtttaaaat ggaaacgtta tgagaaactg cctgccaggg caaagggtgc 115500
tgcccggcac acagtaggga ctcaaaatga aactattgta ttgaatacat aacagatcaa 115560
cgggtattgc tttctgaaat cttttttagc ccaattttgt ttcttatagt ccaataacag 115620
gtcaaattca tttctgattt actagccatt cagttgccca taaaaaatgg aaagtgattt 115680
aagattatta gtttaaaaac caatgaaggt aaaacagtta tcattgaagg cacataggca 115740
gaaatagatt gcaatagttg ctgccatgtg aagcctcagt gtcatgctcc atatttagag 115800
agatctatga tttctgaggc cctttcatgt ccatgatctc agtactgctc acaactgccc 115860
tgtgaaattc gccgagctgg ccccatgtca atcagagtac actgagcact gagacccagc 115920
atgttgagat aactggctag agatcatccc ataatggtac catcacaatc ttcacactgt 115980
agaagtttga tgatgtcact ggaagcatat tccacagtcc cttgtgaact ggccttcctg 116040
tgatcagaag catcagtgaa ctcccaagag ggtgggaact cccaagaggt attctcactc 116100
tacttagtgt atattttaca aatcacaagc ttggctttgg attcttttaa tggctagaag 116160
gagaatcatg gggttggaag tccaccagtt tgggtattct gttccctaac tcaaaataaa 116220
gagatgttat tttcaagtct tctgcttgtt aacttaatta gagatacatg agtttgcagc 116280
tgtgctgggc atgccgcagc ttggcatgtt tagtccagaa ggcatattat aatgtacatg 116340
gaagattgtc agaaattcaa aaggactttt tgagtatcac atgtgtattt tcaagttcca 116400
atatagattc acattcagtt tgacaggtat ctttggatgc ctatcagtta agaactattt 116460
attagttgtg gaataaaata gggtaaaata aggaacaact gaggaaaaaa cataaaattt 116520
gctttgtgaa taaaagttgt cttcaaaatt atgacttttt ccatcccaca aaagttttga 116580
ttaaacccac aatgaaaatt taaataagtg tatttacttt ggtttaacca cttatttcat 116640
tatgactcac aactataggt tttctagttt ccattattac aaactattgt gtggtttaaa 116700
tcaatttcat agactagtct agttctatag tcacaattta taaaattttt ttatgtggta 116760
aattgagtgt cttcatagat gtacatgatt atttctcaat ttttaaggaa tgtatttttt 116820
aagatagcct tctttagcct tctttaacac tgatttttgt aaatttttta cagatttttt 116880
taaatttttg gtaatttttt agcataaagt aatacatggt cactatggaa aacataaaaa 116940
cacaaaaact atgaagagta aataagaaaa acacccagaa atttaccatt cagaaaaggt 117000
cattgttaac aacacggtgt atcttcctcc tgtcatgctt ccgtgcattt gagcacattt 117060
gagatgtgta tacatgttca ctttgagatt ttagtatagc aaaagaaatg accggtcctg 117120
attcaatgaa acctctggca aactcgctat attttcctta catattttta agttcatcct 117180
ataaatgaac tatccattca tcttatttga gattttctta aatctttcag caagaaagcg 117240
ggaaaaaaat cctcctctgg cctttaaagc ctaattaaat atatgactaa gctagaaata 117300
ttttataatg accaaccaga aagtggcaag gactgtcact cttcccatac agcccacctc 117360
ctcctctatc tccctcaggc acacggaaac gagaaaggca gagaaaccca ggacaagtca 117420

tccaagactt tggtcacatg gccatccatt gctttcacaa caaaaatata aatccaacat 117480
gtgtgtgtgc atttcatacc agtaggtcca ataagctatc tatatataca catatgtgta 117540
cacacacaca cacacatcct tacagacact ccccagctta ctacagtttg acttaagatt 117600
ttttgacttt acgatggtgt gaaagcaatg cacattcaat ggaaaccata cttctaatgt 117660
tgaatttttt atcttttctt gggttagttg atgtctgata tgttactttc ttgcgatgcc 117720
aggcaatggc tgggagccag agctcccagt cagccatgca atcaagaggc taaacagctg 117780
atactataca gtggactgtg tcaccagcat tttggggata ttgtgttttg tgtttttgaa 117840
tcctatcatg tctacaaaat gccattttcg actgctattt tcaatttagg gtgggtttat 117900
caggacataa ccctatggaa agttgaggac catctgtata tctggtaggg aaagatggat 117960
aacaaattca taggcaaata ataatttcat gattattatt aagttattcc tacttaataa 118020
taagtagtga tcactgccag ggagcagaga atgcaggata atgtgacaga tgtaatggtg 118080
ggtacttaag ctaatgtagt tgcagaacag gcttttctag agggtaggcc tttaagcgta 118140
cctcgaagat gcaaaggaag caaagatgcg aagatctggg ctggggatgg aagcagagac 118200
aacttggagg ccaaggggag agactgacaa cagcccagct catacctcag cagcctttaa 118260
tgcatagcta agaaaacaac aaattaaaac aattatagtt tacttagacg attctaagtg 118320
tctaagtgga tttgggcaaa tctggagaaa cttgttctaa tactgtgtct taataagtaa 118380
tatagatttg cccaggcttg tgggcagagt ggtatacacc ccataatagc agaggaaggc 118440
cacagggcct accctacaaa accagaggca tttaaaaact taaaggaggc agattgcttt 118500
tattttcagt taaaataaag tgaggagttt ctcaagaaaa ataataacga gaccaccggc 118560
ccgccctaga tgtccaacaa gaatgcacag ataacttcgt atatccactt tcctgaacct 118620
gcccctgaca gccaagtgga gcacaacaac agagatgaac ctcaaaacta ctgtgctgtg 118680
acataaggct tgctcaagag gacagtgtgg tgtgagtcca tctatgttct aaagcaagca 118740
aagctattct gtagtgaaaa tggatcagga cagcagttgc ctctggtgta tgggggcagg 118800
gatcgactgg gaggggcatg agggatgaca gttagggttt cgatcatgac aggaattcag 118860
attactccag catgtgcatt tgttaaagct catcaaatgc tacacttaag attaatcctc 118920
tcacagtttg tggatgttac cttaaaaaca acaatgatga ctgcaaacta atattgaact 118980
ctggttagtg atataccaat gtgaagtata gtgatatctc tactttactt taaaatgcat 119040
ccaaaggcag actagaggac catatctgac agacagaaaa atagatatgt gataaggtga 119100
atgtagtaaa atgctaacat aaggatgttt gcggtacaat tctttcagct tttctataca 119160
tttataaatc ataataaaat tttaggacaa aaagttagtg ctttgaagtc ctaagtcata 119220
gggcctgctg ctcttgatgc agtagaattt gtcttcagat ttgcaaaggg taaggcaaac 119280
cactagcatt ttgtatggaa cttgatgcaa atacttttaa ttgtctggtt ttcaaatgta 119340
tagacttaaa gtaatatcaa ctctttcttt gaatcaacta ctgaaatacc tagtcttaaa 119400
taaatatttt tatgtaatcc ttaaagtact atgtattcat ttttctttct tctttctttt 119460
ctggtttgat aaatattcta taaagtaact gtgtttaatg gccaacattt gagtaagtcc 119520

atatgcagat ccaaacatct cagtttagac aataacttaa gacaatatag agtggctgac 119580
atcccctaac gtgggtccag atgcatgtta tgttatgttt ctgttgcatt ctcaatagtt 119640
aactttaata aaagaaagtc aaaagcttat atattttttc aatcttcaaa acatttctgg 119700
gaggttgtct tagttaattt tatgttgcta tacccatatc acagactggg taatttataa 119760
agaaaataaa tgtatttggc tcatggttct ggtggctggg aagtccaaga gcatggcatt 119820
ggcatctgct tggcagctgg tgagggcctt catgctgtgt caatctatgg tggaaggtca 119880
agagagcatg catgtgaggt ggtggggaag agaaaaagcg ggtttaactc atccttttat 119940
cagggactca ctcccgtgat agctaaccca ttcttacatg aatggcatta atccattcct 120000
tagggcacag ctctcatgac ctaattataa tacctcttaa agtttccacc tctcaacact 120060
gttgcattgg tgattaagtt tccaataaac gcactttgga aaacacattc aaaccacagc 120120
agagatcaac gttattgtca ccattttcat atttgaggaa agcatggcac agagagcttg 120180
gagaagtact tcaaggtcac ccaatgagga agtggctaaa caaaaacctt atcttaaatt 120240
aattaaaaac ctcttgctct ttgcagtttt gtcttaaatc tacctaattt gtgactgtaa 120300
tttttaagta atttactcat ataagtggtc tcacattaaa ttttctcatt gctttatatt 120360
tctaacatga gatatttggt ataaggatgg aaccaagatc ataccttgtt ttaattagaa 120420
aacctagacc aagtcattgt gatcctcatc ctagatttca gttaaatgct gctgtctcct 120480
tttgggtatg tgacagggga aagcctcaga agaaacaacc ttatgtgttt tcttttgata 120540
ctttagtaat taacccagga tagtattcaa gattgacatg ccttatattg aatcaaatag 120600
catatcaact gccttcttat tctcaagtat agacatgttg ggtaattggg catttaagtt 120660
tctttgcaat tttttccatt attaacaaaa ttaatgagca acattctgca taaggtctgt 120720
ttcctcagaa tacgtttccc aaagtggaat catcatgacg tagaatttaa gcatacttac 120780
ttgtttaaac aaattgtcca gttgcttccc aaaatgtttt gtgaattaag atttacatca 120840
agaatatgta atgttgttac tgtctcccaa atacaggatc tttttctgaa tataaaagtt 120900
atacatgcta attgtagaca atgaagggtc attatcctca tagataatga agtgcttcta 120960
atacttgtgc ttttattcat ttattcaaaa agtgctaaat aagccctgaa ggggcttttg 121020
gggggtcatt tggggcttat ttagcacttt ttgaataaat aaataaaagc acaagtacag 121080
tttttttaaa atactgtttt ctataataga ttaatcttaa atggcatgtt ttcctttatt 121140
ttactgacaa aagttactta ctctgtgatt gaataataaa aattctttgg ttcagctgag 121200
agaaacttgc aagctgacgt ccttgattat ttaaaatgaa agcagctgcc tgttttcatc 121260
tctctgcatc ctgaggaaac tcttctgcaa cgtgttccag ccctaggttc tagctgaccc 121320
tgttcatctg tttggcacga ggggcccaac taacacttgc ggctacctgg acgacagcca 121380
atctagttgg aatgagagtt agaggccata gtctgtcagc tgggaaagca gcttttattc 121440
caaggtgtgc caaccgaaag gccacatgtt attgtcacaa cctggtacct acatcagtgc 121500
tgacatcttt aagaacctta gaattgggaa atcagtttag ccctatctgc atgtgtagcc 121560

gacaaccaca caattgttcc aacttgaggt tgcattcaga gcaacctcat ttcccccata 121620
ctcctgagga aaagcagacc agagacgctg ggtcaatcca gagttatggt tggaaaaatg 121680
atggaataat tctgcccctg gtgataggag agagggactc catcttgtca actgtcatgg 121740
ttcccatgtg aaagctatca ttatcactga aattgaatga gaacacagaa gggaagaaca 121800
gggaaatccc cacagagtta aagaggatgt gaagattgct tcatgtttaa tgtttgtgta 121860
agtgctttgg gttggttatg tgctgtctga acatgtgctc atttccatgg ctcattgaga 121920
gggcagacag tccaatgata ctctttagaa tcattcccat ggggaaggaa caaagaagcc 121980
tgtaaaatag aaatgcacat gtaaaaagca ttgaagaaag tgccagtgta ttgattttgg 122040
ccatggtttg tgctctacca cctggttact gtgattgcag aagtgccttt gcagatgagg 122100
aagaacctgg ccaaggctca atccaacatc caaagccaga ggccatattt cttcactctt 122160
aagataattt gggttcaaat tatagtccct ttacacactc tctgcctcaa aaggcccaag 122220
actctctttt gttatgcttg cctaaacatg cctttcaaag aactagttct gtaaatacaa 122280
ctttattata aacctctcct ttgcttttaa aaatggatca ccacgtccat ttctatggtc 122340
caactttgtc ccttaattta aaattttttc ttggattaag tttgatgcct tgaaacatta 122400
ggaactcaag catacaagat tgtatgctgg tggtgaggga agtaactgtg cctccgcctg 122460
tgctgggtgg atcaacatgg agtgtggacg agcataggga tgtgtgggtt tctcactagc 122520
tgagagtgtt tttaaatgtt gtattttgat gtttgttatt ttctgaatat tctacagtta 122580
gacctttgat ttattctttg atgcattcat ttgaataata tttttaatct ccagccagtt 122640
aggtttttaa tttacacttt tgtccctgat tttaggtgta gtgttgtgta cactactgcc 122700
cagtgtatgt tatgtttgta aacattcatt gcacgcacaa caatgtgact cacaatattt 122760
ttgagaagta aaaagttcat tatatagtta ttaactcaac cctacagtta tattcgtgaa 122820
ataccttgtg aaatttattt tttgcctact ggagctctta caggttaatc ctgtcttcaa 122880
gattttcata gaattttcat ctaccaccca cccctttaaa tttcaacatt tttttatttt 122940
ggcattttaa tgcaattcaa tgcattatag ggacaagcta tctcttatta tgaattgcac 123000
cttatataaa cttaaagatc ttttatcaca aatttctttg ctgtgtcctt tagtgagaat 123060
ttgtattatc agtcactaaa gctcactaag ttagtaagct ttgcgcccag atgacctggg 123120
caggaatggg tgagtctctg tgtggagaga gtgaagaaac tgctaccctt aatacctgga 123180
ccttgaggga ttgttttatt tttagttttc tgcatttctc agtatttcat gtgatatctg 123240
tctttttctt ccagtttgcc aaggcacgag taacaagctc acgcagttgg gcacttttga 123300
agatcatttt ctcagcctcc agaggatgtt caataactgt gaggtggtcc ttgggaattt 123360
ggaaattacc tatgtgcaga ggaattatga tctttccttc ttaaaggttg gtgactttga 123420
ttttcctaca caaataaaat tggagaaaat ctaagtggag aaaggcctgg gcagaattcc 123480
acttgaagtg tgtttatttt tgctatggca atgacaagtc ttacagagct acaaacgaga 123540
gttttatgag aaagccattt taccagctaa tgtcaagtaa taactagaaa aggatatcaa 123600
atagaaacag gctaatctgg agttccatgt catcatagac actgacgttt atccctgacc 123660

attacctcag tcatgatgtg ctgccatact cgctcttaaa aacttttttt aaaagccctg 123720
ctttgcacca tttgcctatt cccttagtgt aaatactcct actatagctg atttcaaggt 123780
accaagtttc actcagctgg tcacagaatt cttatttcac gataggcgct aatgacccca 123840
taggagccag ctctgaaggc ttcagagttt cactgaattt tggatggggt ttacttagcc 123900
ttcttctgtt tttcttttac ctttcctttt taaataagaa ataatgcaag acagatacaa 123960
agtaattctt tttaatttcc attttcactg gagagtgttg aaccccgtga ggcatgagag 124020
cacagtgttc cagaacaatg cttactgctc attatcacag gggtcaaagg ctaacgtgca 124080
gggattgttg cagatcgtgg acatgctgcc tcctgtgtcc atgactgcaa tcgtctacct 124140
attttacagt tgttgagcac tcgtgtgcat tagggttcaa ctgggcgtcc tagggctccc 124200
tggacccatt ttagaccttg agttcttgag ttcctcaaaa gagaaatcac gcatttatgt 124260
tttctcttct tagaccatcc aggaggtggc tggttatgtc ctcattgccc tcaacacagt 124320
ggagcgaatt cctttggaaa acctgcagat catcagagga aatatgtact acgaaaattc 124380
ctatgcctta gcagtcttat ctaactatga tgcaaataaa accggactga aggagctgcc 124440
catgagaaat ttacagggtg agaggctggg atgccaaggc tgggggttca taaatgcaga 124500
cagcagttcc gatggctccc agcgagcttg tcactcaatt ccacctcgga gaaggctttt 124560
atttttaccc agtacacgtg cactgagtgc cggctgtgtg taagatactg caggggaagt 124620
tactgagaag atggcagata ctggaatggg aagatttaag cggggtacca gtgtttacat 124680
ggacatgaaa aaatactgag agatagtaag aaatcgtaaa gattctgagt aaaagagagt 124740
atgaccaaac aagctgagca ggaatcgtga atctatgtgt gtaggcagtg aataaactgc 124800
cagtcttatt acctggacct caaggataaa agacatacag taaaaatcaa cccacattga 124860
ggacagtttc gagagtcgcg ctgctacaca gaaagccctg tgtaagttaa ggatagagaa 124920
tgaggtgttc tagaactttg aatttttgtg agcaggactc gtgaggttcc tgtgagagga 124980
aacaatgaag gatgatagaa aagaagggaa attgatttta aaaaactgga gatagcagtg 125040
attgtgcctc actgtgcagt gggtttgggg ccaggaatgt taaattggta acttcattta 125100
acgcccacaa cctttcttca aagtaggcac tgtacagatg ccccttgact tatgatggca 125160
tcctatctgg ctggaccccg ccgagggtga aggcgtcatt aggtcggatt tcagggctaa 125220
ttgaatgtat attgccttca caccatggca aagtcgaaaa tctgtgttaa atcatgctaa 125280
gccggggact ggctgtgctc tgccatcgta caaataaata aatggaagtc aagtaactcc 125340
cttgagggcc ccagctagtg aatggagagg ccagctatgg ccaccactct ctgccccagg 125400
gcgctcaacg cccctcctgt gccatgcagt tctgacaggg aggcagtgct ggtaggaaag 125460
gggtgtgatg aaaggggtgc ccagcagagg gagtcatatc cggagtgaca ggagcccaac 125520
aggggtgcag cgctggaacc caagccagca cctctggtca tggctcctca gttcaccgcc 125580
tataaaattg tgtggttccc ccacacccct tgctgctcag agcagccgcg cacatgcttg 125640
tgctgtgcgt gcctcctgtg agatggcctg gtacaccggt tcctacagtg cgcctcacac 125700

gctgtctcgg agggaggcag cctgtgcggg tgcctggacc tccgagccag accctctggg 125760
ttcctgcctg gccccgtccc tcagcagcca gatggctcgg gagcacattc tccaatccct 125820
ccgtgtctct gtttcgtcat cttcaaaaat gtggatggca tagctgctaa aaaatggtga 125880
catacttcct aggtggtgca gaaaattaag tgactgtagg aacaggcctc agcagctcct 125940
tccacttcct tggtatgatt gttttttaaa ccaaggctgg gattgtatag atgcagatta 126000
gttaatgtga taccattaat agctaaccta gtgcctgctg cagggtgagc ctcccctaag 126060
ccaccgggaa gcggctcctg cagcctccct cacgtgtgct ggccctcctc tggcagtcat 126120
tgcctgtggt gtgctgaagg cccagctctg actgtgcctc tgtgctctcc tcgccccgcc 126180
ccctgctctc tctcaggtct ttggtctgtt gtccgagctg ccacagcagc ctggacatcc 126240
ctgttggtgt ttccagccct gtcctctcct gagttccatc cacctgtgca tggctttttc 126300
atgagtgttt tcacggatgg ttctgctgtc atctccaacc tgataaacaa agcaccacga 126360
ttcagccctt atgaccccaa gcttccttcc tcagttcctt gcttctgtgc atccactgaa 126420
gaagcctgtt ccactgtttc cctgcactgg gtctcctgtc tgcaggaagc cttcagccct 126480
cacttccaca ctcctctaag atgtgtgcct gtgcccttct ggggaagctc attttcctag 126540
cagcctccag gatcttcagg ggtgaatccc tcctttccca cgttggtact ctgtacacac 126600
aacatgccca ttccctgcct ggggagctgg gcattgcttc atgaatcaga ggtcaatttt 126660
ttctctatta aagtcacaga tgctcattgc accattgtga gaatgaatga agatagtgct 126720
tataaatcag ccagcaaggt acccagcctc actgtgtcag ggtctccctg ggcatgaggt 126780
ggttagagtg tgtgacatgt ctgtccccaa gcctgtcagc tcccagatcg aagccagtgg 126840
atctcattca tcctcgcagc gcccacagca cttgcacagg gttttgtaca cataagtcat 126900
tctgtcaatg ttcatgttta atgtcatcag tggaacactc ccactttgta aagacttgaa 126960
tgtgttcatc cctgactttt ccacatcttg ttagttcttc tttggaaaca gctgtacagt 127020
ttcaccatcc tgtgcatccc tggagtctac ctgtctctgt catacattca gattcttctt 127080
gtttcgtgtc actctcatat ccttttctct aatgaaaagc tccgcctggg catgcaaggt 127140
ggagccctgg atgccagccc ctcacctggc atccagggct gtagcactca ggaactgcct 127200
ccctgccctg cctaccccct acatcatgcg accattccag tccagccaat cagccccttg 127260
ggacccagct taccacatgc atatcattta tgctgtgacc actgactaaa ccattctctt 127320
ccttcctccc catatttcta aatttctaat cattgctcaa agcccaattc agagaaaacc 127380
ctagctcctc catggcacca tcattaacaa ttttatctgg ccgccccccg ggaagttcac 127440
tgggctaatt gcgggactct tgttcgcacc atggcatctc tttagcagaa cataaatgcg 127500
aagagcacat gcatccttca tgggaattta aaggagctgg aaagagtgct caccgcagtt 127560
ccattctccc gcagaaatcc tgcatggcgc cgtgcggttc agcaacaacc ctgccctgtg 127620
caacgtggag agcatccagt ggcgggacat agtcagcagt gactttctca gcaacatgtc 127680
gatggacttc cagaaccacc tgggcagctg taagtgtcgc atacacacta tctctgcctc 127740
cagctcctat gggggacagc tctacagcac tggggcaggg gagagaagcc atgtttagta 127800

agtcacatta atcagaaaca aaaagtagta agcaaaatat ctgaccacta gaaaagcatg 127860
tatttaccac ggacatagag atcgtttttt tgtggcgggt ggcagcccag ctggttggca 127920
gtgcaggcca ccggaggcag atcccctgca gggacagcag agcacttgtg tcctgagaag 127980
agctgctgtt catggggctg gcagcaccag ggcctctcct agcctgccct gctgacactg 128040
gccagactcc tacatgcttc tgagtctcca gaggctaccc ggccctcctg aagcaccagg 128100
gctgaatcca cccccagctg agggcatgaa cactgccaca tggagtcaca cacacagctg 128160
ggcactgcca tggagaggaa gtctgtccat gtttccttga atactggtgg cctggtccct 128220
gtcccattcc ccagtgaggc agcctgtggg gaagcctggc agggaaccag gcgcaggtca 128280
gcgtggcgcc ctgactcagg ccagcactga tgggggactc tgagacgcaa gctcacactc 128340
acccagctcc cctgggctgc gcccgttcct gatcgcttgg actttctgtt ctttagagta 128400
agaagtgatc accatttcct gcttctttgt ttctccacaa ctgtgcagtg gatgcctgtt 128460
tgttttctgc cctcagaaca aaaaaaaaaa aaaatagagc tgacgtgaat cttcaaaatc 128520
atcaactaca gggctttgga tttttgtgta tttgttttat tttcatttta tggatggatt 128580
gtgatgaaat gcccgtaata caagattttc catcttaacc attgtaagtt acaatgtcag 128640
tggcattata catccacatg ggtgtgtggc catcaccacc gtccacacac agaactcttt 128700
tatcttgcaa agctgaaact ctacccatta gacagtaact ctctgctctc ccttccttcc 128760
cagcctctgg ccctggcagg caacagtcca cttgatgtct ctatgaattt gactgctctg 128820
gggctctcat acaggtggaa tcatgtagta tctgtccttt tgtgtctggc ttatttcacc 128880
tagcaaaatg tcccgaaggt ttatccatgc tgtagcacgt gttaagaatg tccttcctct 128940
tcatggctga ataatattcc attgtatgtt gacactacat tttgtttgtc cattcaccta 129000
tctacagaca ctggggttgc ttccatcttt tgactgtttg aataatgctg ctgtgaacat 129060
gggtattgag gctctttgtt ttatagacat attattccac cagataccca tcctgacacc 129120
tactatgttt gcaagaaact gaaagcttta ttttacattg caaaatttca tattatgaga 129180
tcaaggttag catttcctca gctgtctggt ggacaatggg gaggttaaac tgtgcacatt 129240
ttattttttt ttaatgaacc tggaacggtt atggggccag tgtttgccat ggatcaggtc 129300
aggcagccca caatggcagg tctccatgtt ctgtacaaca actgtgggaa agacccacag 129360
agaaagtgct ggaaagggga atgatgggta ggttcatgca gtaaaaagat tcaaatacta 129420
cagggcattg aactataggc caatatagca ttgctttaag aataaacaaa aaataagaca 129480
gtaagaataa gcctagcaaa atcaaaagtc tataaagaac tgacatttca agccaataag 129540
agaataattc cttattcaat aaattgtctg gaatgactta actattaggg gtgaaaatat 129600
caaagtgaga gaactataaa gggtttttaa aaaggaatta ggtatgttgg gttagtcgca 129660
ttggagagtg caaattcacc atcgacctga tacctgaaat ttcctcctta ccatctagag 129720
gcaagttggg aatgctgcca ggctcctgtg gtaaaggaag ctcctctctt gactggtgct 129780
ttatggctac acgttcctgc tcagaatgga tctcatttag tcttcaccaa aaaaaaaaat 129840

ctcatgagat gatttaagtg ttttatggac aagatgtcta aaactcagaa aaatttcaca 129900
gtgtgcctag cttttatgtt tatgttgaag ttgggcatta gaagttagaa tgaatgggtt 129960
tacttcagag aaaattaaat ccatcaccca ctccttgtac tatgaattcc aaatacatat 130020
taaatacata taataaaata tttaatatat atgtaagtgc cagaaggaaa cataaatatg 130080
aatattttgt aatatcaagt tgaagaaaag ccaaaatctg acatcataaa agaaaacttt 130140
caagtaaaat atgttaatgg ctaccaggaa aatattgtgc aatgtctgat tgccatgaag 130200
agggttaata tccttgctat atcactctgt gaagtcatct ttaaaagact aagaaaaaga 130260
tgaatctctt aataaaaacc tggcccagaa catgagcagc ctctctctct cactctcact 130320
gtctctcttt ctgtcacaca cacacacgca cacatacaca cacacacaca aatatggcca 130380
agaaataaag taaaatgtta tttctaatgt aataagtagg tcaaaataga aaaagaaagc 130440
atcacacctt cctttgcaaa gtatttgggt tccttttgct tttaaacacc tgggtcagct 130500
ggggtgtcga gaaacagaaa ttctcacgtt ctgcttgtgg gcatatatgt taataaaacc 130560
aagcttggca atatgcctgc aatatgtatc taaagcttca aagtatgtat agctttgacc 130620
aatcaatatc acatttcgga ataagagaaa aagaaataat gaaagtgaaa atcataagag 130680
atgtagaaac atattcttat acaagaattc cttgcagcct tatttataat aaattttgtg 130740
aacaaattat atatctaaaa ataagagatt ggttgaaaaa attatgcagc agccatgcta 130800
ttgataatca tgttagatag aagcatattt aaaggcatgg aaaaattgcc atgttttata 130860
tgggttttta aggttataac acaatgtata gtgggattcc aattcctgta tatacataga 130920
cttatatgtc tatattgatt aactctggat gagtctcatg tcttcttttt gctttcttct 130980
attatccata ttttatacga tgtgcctgca tttctttttt gtaacagatg gtcaatacta 131040
gaatcataaa cagatcttgt ttgtttattg gcaaatgttt cccgttagaa aaagatgcat 131100
ttttctttta aatattttta ttttatacaa tgattacaag cttataatag aaatttgaaa 131160
attatatgtg agtacagggt aaaaagttga aagaatggga ttgcacgcta cagatctagc 131220
tgcttttagc acgcctgcgt aggaccttgc tttctctaga cctctgttgc agtctctctg 131280
cctacctcct cacaacgtcc atcccccgcg gtcactgtcg tgatgccagc ctccccggcc 131340
ttcatgtctc taaggagcac cagcgcggca attagcgccc tttgccttgg tggtattctg 131400
gcttcacagt cacatgggag atcaatcgtc agcttttctg tttgaaatct aaattcttcc 131460
tgactgcagg ggacctcggg acccatgaac acctctagtt tactatgtct tcacagtaaa 131520
agatatctgc atgactggac tctttaacaa atttggtggt taacctactc tttctatata 131580
gatatagcac ttcgaccttc agacttctca atactgataa aaagaaaaca cgacagatga 131640
caggaaaacc tttgcagcta taatttgtaa tcggccaatt ataaaaactg caaaaattga 131700
ccagatagct aaggttttac acagtcatga aagtgatctg cactgttaac atttcaccct 131760
ctgtgcacca ttctgtgctt ctctctggtt tggagtctag aaggttttat ttacaggcta 131820
tgacttaaca atcccagaac ggctgacaca tgcagtcact caagactgga cacagcaagg 131880
aagtagtggg tccatgccaa aggctcagcc agacgagaca ctctagctgt ggcaggagat 131940

gccagggaat gctccaagcc taagcagatt gtaaacaagg aacctcaaat tcatgaaaaa 132000
ttcttgctta tgtggcccat gtcagtaatt actctctgcc tcagtttccg cagctgacat 132060
gtaaataaaa gcagttcatg gttcatcttc ttttcttatc ggggtctcaa gtgattctac 132120
aaaccagcca gccaaacaat cagagaataa gttgaaaaga ttgtcttcat ttattgaatg 132180
tgcttaactc aggcccggga aagggcgtca tcagtttctc atcatttcac tgagatatgc 132240
atctattact tttacatttc aggccaaaag tgtgatccaa gctgtcccaa tgggagctgc 132300
tggggtgcag gagaggagaa ctgccagaaa cgtaagtcag tgaacagcct cagacccatg 132360
tgtgaccgcc cctctcttcc ttcacttgct taggtgattg gatttgtttt ccctctgaag 132420
actccaaaga gttactttat tacagggtca gatgtgaacc agtaggtgaa ggacagtctt 132480
gcaaatctca ccgcatgcag ttaatccagg gtgggctatt ttgggagctt cagcctatca 132540
caaataagtg aacatcagca ggggctgggc gcggtggctc acccctataa tcccagcact 132600
ttgggaggcg gaggcggtcg gatcacgagg tcaggagatc gagccattct ggttaacaca 132660
gtgaaacctc gtctctacta aaaatacaaa aaattagccg ggcgtggtgg cgggcgcctg 132720
tagtcccagc tactcgggag gctgaggcag gagaatggca tgaacctggg aggcggagct 132780
tgcagtgagc cgagattgtg ccactgcatt ccagcctggg cgacagagcg agactccgtc 132840
tcaaaacaac aacaacaaca acaacaacaa taagtgaaca tcagcaagta ccccagccct 132900
gtcctctgaa cacagcacac tttcccagga atggaagact tgctcctgtt gacagcagtc 132960
accagacttc ttgtttcctc tccctccctg gctttctttg gtacccacct acacagaagc 133020
ctgagcacgg gttctcatgg ggacttttcc atgtggaccc tgctttacga tggagagggc 133080
cattctccta ggtatggttg tctggctcag cctctcagtg gccaaggaac ctggggacat 133140
gagctcaaaa acggacacta tgtccttaag ctgaattgtg ggggggctgt taggcccttc 133200
taaacactac ttcccagcag gtatttttgt tctttgtatg tgctttctgc attgcccaag 133260
atgcatctaa ttatttagca ggtctcaaag tctagacttg atctcatgag ttctcttaag 133320
tgattaaaaa taaatcagga gaaaaaagag gcaatcagaa aagggcatgg tttgacttag 133380
tttgaatgtg gtttcgttgg aagcaaatgt gtcttcactt tttcatgaaa aagtctgcaa 133440
gtgctctgcg acatccctgg gaaatgatcc taccctcact cttcagctca cagggaacct 133500
ttgctctttt tcagtgacca aaatcatctg tgcccagcag tgctccgggc gctgccgtgg 133560
caagtccccc agtgactgct gccacaacca gtgtgctgca ggctgcacag gcccccggga 133620
gagcgactgc ctggtaagat gcccctccag cagcctccct ggagcaggct ggggctgcac 133680
ccgccccacc cacaccagga cagaagactt cctgtggggg agctgtcaat tagcatttgt 133740
cataacagac aggatattgc cctctgcctg gtgacaaagt atctttagta tcctgcctcc 133800
accactcact gagaccttgg gaaaatgatg ggactaccat gcctccattt ccttacctga 133860
caatgatgca taacaaagtc tctcccagtt gaatgcttaa atgatgagat gcctgtgatg 133920
tccgtcatta ggacctgggc acagaacaag cactaaatac tacatgcaag tatttgtcat 133980

gaatgtgcct tgttgccagc agcacactct ctttattgtt tgacttcggc tatacctcta 134040
gagacttgac actgtgaggt ccctaagaga cccatggaga gccacacagg tcttgctggc 134100
tggggctggg ttagggcctc ctgacacgga tccctcggct cctccaccac tgctcaggca 134160
cctcctgagc tgcaccctgc cctcaagggg tcctgaagta ctcactgtcg ccccattgct 134220
ccagaaagtg ccagcagaag ccttgctgcc ccagcgggct ctgagcagca ctggagggta 134280
caggtcagaa gcgtcttgga agtcctggag acgccaaggc tggtggatgt gactcctgga 134340
gtgggagctg gtgtgacgaa gcccttccta agactaaatc cagagcactc tgtggtttca 134400
gagaagattc ctaaattcca gagtttggac ccagacccag gaattgtgac ttggttggcc 134460
tgagctgttt ctaatgtgag ccccagggag aagactgtgc gtggggttgg tcctaggaaa 134520
agccctcgct gtattgggtc tggctccttt acacggcatt gttctagcaa ggctttctgc 134580
cattcagcaa tacattataa aatataccct caattgtact ttataaggga agcccaatgt 134640
cctttataag ggaaattaaa cataatttca ttccatagtc accgctataa tgtgtgaact 134700
ccatcatcta tacgttagta aacagacgta tttttatcat aatccataaa ttatgatagg 134760
tgggacagtg cacctaagaa aaaaatggac tttttagaga agggtctttc tgactctgca 134820
gagggcgcca gctgggtttt cccacactag tggaacacta ggctgcaaag acagtaactt 134880
gggctttctg acgggagtca acaccgtgct gcgcttcctc cgtgtgtggc gctgagtgta 134940
cttacctcac ttgcccagcg tgtcctctct cctccatagg tctgccgcaa attccgagac 135000
gaagccacgt gcaaggacac ctgcccccca ctcatgctct acaaccccac cacgtaccag 135060
atggatgtga accccgaggg caaatacagc tttggtgcca cctgcgtgaa gaagtgtccc 135120
cgtgagtcct cctctgtggg ccctctaact ggtcaggcat ccttgtcccg ctctgtctcc 135180
tgctgagccc tggagtatcc catcttggag agtctttggg tggatgtgtt tgccttgctt 135240
ggaggaggcg accctgtgcc cgtccaggca cacaggcgag ggagggggct ggcttgctac 135300
cgaggagcgg gcaggtggtg gccatctcca cccatggggg ctgctcagtg cacagggcag 135360
atctgggtgg ccaggccacc tcacaggaga aacacctgct gctcagccct caccactcat 135420
ccagcagcca cagccgtggg tattcagttg tctgctgggc acaaagccgt gggcatgcca 135480
ctgtttagtg cttgtgccaa gcaggtattt aatacaccga aatcagagag tctatcagaa 135540
gacctgcctt cttgagtggt taaaattcta gtgaaagtta tgcctcttag gagtattgca 135600
gaggttttgt ttttgttttt attttgtttt gttttaatgg tttgggtttg agttttgctt 135660
gtttgtactt acatttgtac tggtggctcc agggtttagg gaaattgtga cataaaataa 135720
ttcctgacag agaaagcaaa actttgtcta atgaaagagt tttagaagcc actcttgatc 135780
tctagaaggg gagattaact gagaaaaaaa attgaaagaa caattatgag ggggagattt 135840
taccctgcca gatttgtgta catgaaaaat tttacattcc gtatggaaaa aaaaaacaca 135900
aaataataag ccattataag gtaaatgaca aacaaagcta aagaaaaatg tgccacagtg 135960
atgacacaga tatatctttg agatagggct taacagagct ttaaaatcca taggaaaaca 136020
cttcgagcct gagataccaa gagcagatgg ttcacagaag aatcatcaat gtcctataaa 136080

tatttttgag gatcttcttg gggaacttaa aacaggaaca ggccaggcac agtggctcat 136140
tggctcatgc ctttaatccc agcactttgg gagactgaag gggctggatt gtctgaggtc 136200
aggagtttgg gaccagcctg gccaacaggg tgaaacctcg tctctactaa aaatacaaaa 136260
attagccggg cgtggtggcg cacgcctgta atcacagccg ctcaggaggc tgaggcagga 136320
gaattgcttt aacccaggag gcggaggttg cagtgagctg agatcacacc actgcactcc 136380
agcctgggtg acagagcaag actccatctc agacaaacaa aaaaggaaga catagagctc 136440
ctaaaaataa cgcagaagtc tgctattaat acaaatgaat tactttaaag gtgagagcag 136500
gtggaggaga gggctgaggt gcctgctggg acgcaaaaca gctggcccct caagggaccc 136560
agtgtttcct gccatgatga aacacctgta ttgtccacat tgcggcctag aatgttatta 136620
aactcttgaa cgggattcct tctctatttg caacctttca ttctttgtcc ttaaagtaaa 136680
taaagccaaa ggaggatgga gcctttccat cacccctcaa gaggacctgg accgcctgtg 136740
tgaggcccga gcacctggtg ccaccgtcat caccttcctt tcatgctctc ttccccaggt 136800
aattatgtgg tgacagatca cggctcgtgc gtccgagcct gtggggccga cagctatgag 136860
atggaggaag acggcgtccg caagtgtaag aagtgcgaag ggccttgccg caaaggtagg 136920
aagcccgccg gtgtgcggac gaggcttgtt ctcggctgct gaggctgggc tctcatgcca 136980
cctccaaagg aacacatctt cctcttctca ttaaaaaaca actatacata tcgtttcttt 137040
aaaacagaag ataaagctgt aaagctaggt taggcaatgg gaaggcactg aaggttgtga 137100
cggggtgggg ggctctgatg agaacagtca cagagccagc cccgctcagc agctgccagg 137160
tgcccagccc tggggagaat ccagggaagg cagagctgga agcagtgcag ctccaagcgg 137220
cccatgggaa ataatgagga gaacgcaagg tcagtgtgag gtgacaggga tggcatctcc 137280
tacaccgccg tagccccaaa gtgtactata ggtcctggtg tccccccttc ccgcctgcac 137340
tctccccagc cccttcagtg tttgttgagt gaatgaagga tgatgtggca gtggcggttc 137400
cggtgaccgg aattccttcc tgcttccctc tgcctgtgga tccctagcta ttcttaatcc 137460
aacaaatgtg aacggaatac acgtctctct tatctctgca gtgtgtaacg gaataggtat 137520
tggtgaattt aaagactcac tctccataaa tgctacgaat attaaacact tcaaaaactg 137580
cacctccatc agtggcgatc tccacatcct gccggtggca tttagggggt gagtcacagg 137640
ttcagttgct tgtataaaga aaaacaaaat ctgccttttt aactggtaga gattggtgat 137700
caataatcac cctgttgttt gtttcagtga ctccttcaca catactcctc ctctggatcc 137760
acaggaactg gatattctga aaaccgtaaa ggaaatcaca ggtttgagct gaattatcac 137820
atgaatataa atgggaaatc agtgttttag agagagaact tttcgacata tttcctgttc 137880
ccttggaata aaaacatttc ttctgaaatt ttaccgttaa tggctgatgt tttgatattt 137940
ttcaaaagtg cagtttctcc tgcaggcaaa aggggacacg ttaagtccag gcttgggtca 138000
ttcactgcgg tgtaaacacg ctttctccct cccgcccggc cccagccagc tgccttggtg 138060
gcccataacc cctgagggta gagggagggg acaggggtag gtgacaggca gcctgggcct 138120

caggcttttg aaactggacg ccagagcctt gtggggccac gggcaagcct cgggtctatg 138180
actgccgcct gagctccgct tccttcctct ctaaaatggg aagattagac caaaataaca 138240
agactgtttt aaggttggaa tcaaataagg aaaatttgta aagctccttg tatgtgatac 138300
cagatccaca attggcagat aatcgcagca ggagcctctt cggggtaatc agatacgcgg 138360
cgcagcaggg gtctcagggc cacagccagg ggggcggcgg gagacatgcg gaatcgcagc 138420
ggaaggcggg aggcagctgt gaactgtggc tcggcctgcg tccgccctgc gcatgtacac 138480
tcagagaaga tgataatgaa aaagaaagca aatccaattt tcccacttac tgttcatata 138540
atacagagtc cctgagagtc tagagtaatg tctcatacaa aaaagaaact cctacgtggt 138600
gtgtgtctga agtctttcat ctgccttaca gggtttttgc tgattcaggc ttggcctgaa 138660
aacaggacgg acctccatgc ctttgagaac ctagaaatca tacgcggcag gaccaagcaa 138720
cagtaagttg accacagcca aagcctggta gattacattt gcctttttag ttggaaatta 138780
ggcttaacag gagagttgct aagatagggc acagagctcc tgcatctctc gccggcattc 138840
ccaaatgcta tctcacatga gcaggcacag ggagcaagac tgcacgacca ctggcacagg 138900
ctgtccgcta aaccacagac ttctcagcgc tcgccagtgc ttctgcttct gtgtccactc 138960
cagatcccac attgcactta gttgtcaaat cttttcagtc catttctaac ctatattagc 139020
tcctgtgtct ttccttgtct ttcacggcct tgacacttac aaaacgtgtg ggtcaggtac 139080
tttgcacact gtctaaccat gtctgttcag ctggtgtttt ctcaggatgc aattgaggtt 139140
atgcacatct tatcacaggg accagagaga ctttttagca ccactcttca agaatttcca 139200
ctttttcagc tttgacagtg gaatagacat gcaggtgctc acacacaagc atctttaata 139260
tggtaatggt aatcatcagt ttagtggtgt ggaggaggag atgggaatct cttagtgaaa 139320
cccgccttgg aagcagcctc gttatgagaa ctgctgcccc tacttgactc ttaaagcact 139380
agataatact gtgcaacatt aaagagaata agagtgcgtg aaatatgcat tgcctcccat 139440
aaactccctt ggctctgaat ctctgatact aaatatgtgg ctaccgttgc ttcccagaaa 139500
ggcctttttg ctctgaattc tctggaatgc tttctttgac caagattctt ataaaaataa 139560
gagatttaga gcaattttct tggatggctg gtatgagcca gttggcttag ttgtagggat 139620
ttaaacaaga taagggttac ttacttttca catttaatga gaagtctggt gattccagct 139680
cctactgaga cagggtggcc acacgttcca gggtgtgact cactgaggcc ccagacctgc 139740
cctgcaagga aaacctggct ctgccctggt gtcctggcct ccctgggcat atgtggggga 139800
gaattcctaa tggtattggt tacaggctcc tatgcgagac cactcatctg tgtaggagaa 139860
aggaaaaaga tgggggaaag aagagcagca gggagaggag aagcctctgg atgatactct 139920
aaccccctgc catccaacac ctgaacatca gtctcttcat ccagtgctct cagctggccc 139980
agcccccagc ctggggtcag atgagagctt cctgcaaatg cagatctctt tcctgtggct 140040
ccttctcaat tacagacagc tcctccacaa ggtgcactct ggccttgtgc tccctcccca 140100
aaccagccca gccctcccag cctgcatcat cgtggtcctg taggggctag aggttctcac 140160
acccatcgtg gtctggcaga ggctggtggt tctcacaccc atcgtggtcc ggcaggggct 140220

tagtggttct tatacccatc gtggttcagg aggggctagt ggttctcaca cccatcgtgg 140280
tctggctggg gctagtggtt ctcatgtcca ccgcgtgctt tcctgctcct ccaggtggct 140340
gaggacatcc ccccttcggt ctgaatgact tccatccagt catctgatat acacattgga 140400
ccacccaata gcatcctagt gtcatgttgg atggtgaaga aaatgccaca gttactgctt 140460
tcagggcctc acaaccttgg gcatagcttt ttggaggaag gccccacttc ccaggcatcc 140520
ctcccagacc tggtcagagg cccctgctct ttgcttccat gttgcccaca ctcactgtgc 140580
tcttcacacc ggctcaaaat gatctgctta cggggttgtg tcaccaccag atcaagcgtc 140640
ctggagagga ggaaacatat ttaacctgca cagaatttgg gacagagaac ctctagtgtt 140700
tgttcaataa atatatgaat ggatagaggg acaggttggg tggtggatag atggatgaac 140760
ccacaccttt gaagtgtatt tggctgtttg agaggttaga atatgttctc aatttccagg 140820
caaaatgaaa atggagaaaa tataatgaca ttaaggcatt ttattcatcc tccccatctg 140880
ccactgggtt aaagatacta aataaacaag gaactatctt ttgcctggag gaactttaaa 140940
aacacctgca gttttcaaaa ggtgcagtgt gtgcctccca cagcatgacc taccatcatt 141000
ggaaagcagt ttgtagtcaa tcaaaggtgg tctggagaaa caaagttttc agggatacat 141060
tgtttttata atttttcacc acatgatttt tcttctctcc aatgtagtgg tcagttttct 141120
cttgcagtcg tcagcctgaa cataacatcc ttgggattac gctccctcaa ggagataagt 141180
gatggagatg tgataatttc aggaaacaaa aatttgtgct atgcaaatac aataaactgg 141240
aaaaaactgt ttgggacctc cggtcagaaa accaaaatta taagcaacag aggtgaaaac 141300
agctgcagta agtcaccgct ttctgtttag tttatggagt tggttctaat gggtccttta 141360
tttgtattta gaatattgaa gggctattcc catttaaatt acttttttca gttccttaag 141420
aagcaaatta aaatcttaag attcctaact gtgaaattac catgtgaatt ccattaaaac 141480
tttttccaga tcattaccat tcaatgggat gaatttaccc tgaggtttag gctaccaatt 141540
atttgtaatg taagtaacta aatttagtat tagttatatt accttttagt tgtaggtcac 141600
tctctgctca tttcagcctg taaagactac agctacacac atacacacac agaggaatgg 141660
aatgagcact ttacatcaac acttcctgtt ctggctctag agcctcagct tttgaagctg 141720
gtgagagcct ggcctgtgct gggccttggc cacgggcagc gtcagctttg agtcaagtgc 141780
tggtctggcc tccctagctt tgagcctctg tcaattccct taatctgttt aggctttggc 141840
ttcctcatcc atagaatgga gatatgaatg attcctacgc cgtagtgctt tgagagaatt 141900
cagtgaaatt cctgtgtgta aaacccttcc atggtgccta gcacacagca cacagccaat 141960
ggcccaatgg ctcctatcag ctgtgggatt tgtcatcaga acaccaccag ctctgctcca 142020
ggctgccctg ggtaccatca aaacacaccc tgtgcccagc agcacctgct cctctgcaca 142080
cctggttcct tcagcagggg cagtggccgt gggagcacag aaaacatgga gtcccatctg 142140
gtttaattga tgccattgcc aaaggggagg actcacggca ccccctctcg ggtgccaggg 142200
tgcctggctc ccaccaggag gaagacctgt cctccactgt caggcacatt tcagtcttcc 142260

cagcagccag cacaactact ttgtccttcc agtcacggtc ggcctctggg aagcccagtc 142320
tgtgtcctcc tccttcaggg gtagccagca tgtctgtgtc acccaaggtc atggagcaca 142380
gggcccctcc cgggaaggtg ccgtctcctc cggcccctcg ggtccctgct ctgtcactga 142440
ctgctgtgac ccactctgtc tccgcagagg ccacaggcca ggtctgccat gccttgtgct 142500
cccccgaggg ctgctggggc ccggagccca gggactgcgt ctcttgccgg aatgtcagcc 142560
gaggcaggga atgcgtggac aagtgcaacc ttctggaggg gtaggaggtt atttctttaa 142620
tccccttgcg ttgatcaaaa ataaggctcc aggttgttgt tatagcttta caggcattct 142680
gtttgatttt ctcttccttt tattctttgc ccttggcttt tggaggtttt gggttttctg 142740
tggggagacg ggaagttgtt tgattgcgtt atttttggca aatttaagca caataggaaa 142800
taagcaagta ttattgccta atataatcca ataatttata gaatctcttt tcctggaagt 142860
atcttaaatt tttctaagct acaaaaagtt cctaagacaa atgagacagt catcaatggt 142920
tcatctagcc aacaccgtgg ccatttgggc ttttctttgt agtgcccgat tcctggtgtg 142980
tgaaaataaa ttaacacaaa ttatattgcc aagttaatat ctgttttatg tgcccccagc 143040
atgtgttgaa catcaaacag taccagggac tttaaatata cccacggaca aagaaataat 143100
tcataatgat gtttgttgaa tttagttgca atcaataaaa agtgcagttt gtgaatgctc 143160
tgaggttctt gatattgatg taaggctttg aacgacaaat gaggacaaaa cataaatagg 143220
aaagtaaaac tgaaggatag aggccaaggc catgttttag aagatttaaa gaaaaaggga 143280
aatttggtga gcaccatagg aattacagat ggctgtagga attcttcctg ttttactctc 143340
tgggcatgga ccacagcttg gatccagaaa tatttaggag caggataaga ggaccaagtt 143400
caattctata ggaatccttt agctgatagg ctcagaacaa atcacataat tgatagtgct 143460
gcttcaactt caagtaagga atattgatgc aatccttaca gctacaaatg gacagtggtc 143520
tcatgttttc agttttcaag tgtttcttaa gaggcaaggt gatgaaaacg cccacgtggg 143580
gagccccatg tccttccatt agtgtagaga aacctggtgt ccagcagcac ctgctccctc 143640
tgcaagccca gcccccttca gcaagggcag tgacccagag aagaagcaca gaagacacaa 143700
ccctgtatca cattttgttt aatggtgcca ttgaccaaag gggaggatga aaggcacaca 143760
cttttttgtt gttttttgag acagagtctc acgccatcac ccaggctgga gtgcagtgat 143820
gtgatctcaa ctcactgcaa cctctgcccc ctgagttcag gtgattctcc tgcctcagcc 143880
tcccaactag ctggaattac aggtgtgcac caccatgtcc agctaatttt ttgtagtttt 143940
agtagagacg gggtttcacc acgttggcca ggctggtctc aaactcctga cctcaagtga 144000
tctgcccgcc tcggcctccc aaagtgttgg gattataggc ataagccact gcacctagcc 144060
aaggcacaca ctttggagaa taaacactcc ttgttcgctg ctggagggta gaactatgct 144120
tgactactag gcagagtcca gtcttactga caaacagccg tacatctgtt ctgtcttttc 144180
aatcaaacat cagcttcttg cttaacattg atgtgtacat cttgagggat gtcaaaatat 144240
tgtaagctaa gtttttcata cctgtgttcc acactcacca tttttagtaa taaccattga 144300
gcgagttcat tctccctcct tcctttttct atcacttaat ctaaaattat catttttcca 144360

gcttaatttt gataaccatg aatctggtat tagaggcagg gaacacctcc tcaggactat 144420
cttttctttt atcatttggc ttgcttaccc aatatgcaaa aactatgctg tagaaaaagc 144480
agaaaagata tcttgattat gaatgaagct cctgtgttta ctcagagaga agatgaccca 144540
ggattcagtt aacaaaatca gctgattata ttactatata gtcctggagt cccaactcct 144600
tgaccattac ctcaagttat ttggaatttt gaagaggtga tttgtgttcc tgcaataatg 144660
tctcaggggt gggctgacgg gtttcctctt cctcctctca gtgagccaag ggagtttgtg 144720
gagaactctg agtgcataca gtgccaccca gagtgcctgc ctcaggccat gaacatcacc 144780
tgcacaggac gggtaagagc cccttgctgc tatccacgtc catttcatgg gaagggcctt 144840
cacagaagcc gaacagtgat gatggcccag ggcatcctgt gtgggcagga cggccatcag 144900
agccacttcc cagaggagac ggcaggcgct gacagcgctg tccgggcagg gtgtcggtga 144960
cattagcaca cacattagcc tgcgatgaac attcactctt tctgctgaca cccccaacct 145020
tatctaagct tatcaaatcc tcacatttaa cggaggctgt tttcacctgg tttcccccat 145080
ccctgaccta gtcagcattg ctttatcgct ttcatcaaac atcctcaaat tcttaacatt 145140
agcttgtaat taattgaaga atttttaaag aaattgctag caaaactttt taaactgcac 145200
aactttgtat ctatatgttc aataacatat agatacaata ttctttacaa taatctttta 145260
aagaatatga gtgagaattc gggcccctct cacaccaaat gtcctgatgt tgttaattct 145320
caatgttatt atatagggag ctctgttttc ttgtgagctt caacagccag ttctaaatct 145380
actaactgaa aacatttttt agacattctc taaattgggc agaagatgac aggactgtgt 145440
tttgagggat aggctgccag cgtggctgct tacaaagtaa agacttggtt tataggtttg 145500
catggtgttg ggttaaattt ctgtcattaa aataattggc gatattgaca tagtcatcta 145560
attatgctgg ctctgggcac acacagccct tgagtggaca aaaccaacat gagagaactt 145620
agccaagggg aaagcctttc cctgctggtt ttatttctgc tacttctgaa gtgtggggca 145680
cacaacctga gcagtgcttt tatttgagtc ccaatgcttt tatttgagtt ttgcaaggtt 145740
attccaagtt ttacaaatag aaggtagcgt atgactcagt ccttgatatg ccaaccactg 145800
cacagagact tgccaccttc ctgtcactgg agaaacactc atgtgggttt tcttaaattt 145860
gcctccctct gagcttccct ttaacttcaa ctataatatg caagaaagac tatctgacca 145920
taaatacaca tttgggccaa tcaagatggt tttgccaagg aaagatgccc acaatggtta 145980
agcagaatgc aataatgtag agaatatcat ttctttcatg ctggtgtata tcatatgcat 146040
tcaaaaacag ggagaacttc taagcaacta acagtgacca tatcaagcag gtgcaatcac 146100
agaataactg gttttctcct ttaagaattt ttctatcatt tggctttccc cactcacaca 146160
cactaaatat tttaagtaaa aagttacttc cattttgaaa gagaaaagaa agagacatgc 146220
atgaacattt ttctccacct tggtgcaggg accagacaac tgtatccagt gtgcccacta 146280
cattgacggc ccccactgcg tcaagacctg cccggcagga gtcatgggag aaaacaacac 146340
cctggtctgg aagtacgcag acgccggcca tgtgtgccac ctgtgccatc caaactgcac 146400

ctacgggtga gtggaaagtg aaggagaaca gaacatttcc tctcttgcaa attcagagat 146460
caaaaatgtc tcccaagttt tccggcaaca aattgccgag gtttgtattt gagtcagtta 146520
cttaaggtgt tttggtcccc acagccatgc cagtagcaac ttgcttgtga gcaggcctca 146580
gtgcagtggg aatgactctg ccatgcaccg tgtccccggc cgggcctgtg ttgtgcaatg 146640
ctgcacatca caacaggagg gtagggggac aaaagagcac aggtcctggc agctgccaca 146700
gtctccaggg gcttttgcgt ttctctccag atttctaagg ttaacatggg gattagctgt 146760
tttgcaatga ataaaaggta acattgcctg gaatgttgct taaagacact tttttaaagc 146820
tagttgattg ttaagctgtt gctacttaaa ttaaaactac tttgggccag acgcagtggc 146880
tcacgcctgt aattccagca ctttgggatt ccaaggcagg cagatcactt gaggtcagga 146940
gcttgagacc aggctggcca acatggtgaa accccacctc tactaaaaat acacctgtag 147000
tcccagctac tcaggaggct gaggcaggag aattgcttga acccgggagg cagaggttgc 147060
agtgagccaa gatctcgcca ctgcactcca gcctgagcac caagagcgaa actctgtcgc 147120
aaaaaacaaa aacaaaaaaa aaagctactt tgactggaat tagcagaagc actctgattg 147180
tgtgtatctt atttactgga ataataaagc tgtcaatcaa actggatccc actcaacaat 147240
cagaaagaga agttgagctg tcatatagta gttcacactt acttctgttt ctcaaaatcc 147300
tcagctttgt ttggaactgt tactcattct ttctctgaat ccatctgtat gagttgtgtg 147360
cccttgggca agggtcttac cttctctgtg cctcactttc ttttctgtaa attgggataa 147420
taatgctgca tagctcacag gatttttatg accatgagtt aagatatgtc atatacttaa 147480
aatggtgcct ggaaaatggt gaatactgag tcaatgatag catcattgat ggtgggatgg 147540
tgatgaggag gtgggagtca caatggtggt gttgatggtg gtgatggtgg tgaggaggtg 147600
ggagtcacag tggtggtggt gttgatggtg gtgaggaggt gggagtcaca atggtggtgg 147660
tgatggtgtt gatggtggtg aggaggtggg agtcacaatg gtggtagtga tgatggtgtt 147720
gatggtggtg aggaggtgag agtcacaatg ttggtggtgt tggtggtggt ggtggtgagg 147780
aggtgggagt cacaatggtg gcagtgttgg tggtgaggag gtgggagtca caatggtggt 147840
agtgatgatg gtgttgatgg tggtgaggag gtgagagtca caatgttggt ggtgttgatg 147900
gtggtgatgg tgatgaggag gtgggagtca caatggtggt gatgagggtg gtgatgatga 147960
tgaggaggtg ggagtcacaa tggtgtcagt gttgatggtc cgatggtgat gaggaggtgg 148020
gagtcacaat gttggtggtg ttgatggtgg tgatgatgat gaggaggtgg gagtcacaat 148080
ggtgtcagtg ttgatggtgg cgatggtgat gaggaggtgg gagtcacaat ggtggtggtg 148140
atgacggtgt tgacagtggt gacgaggcgg gagtcacaat ggtgtcggtg gtgatggtgg 148200
tgaggaggtg ggagtcacaa tggtggtggt ggtgatggtg gtgatggtgg tgaggaggtg 148260
ggagtcacaa tggtggtggt gttgatggtg gtgatggtgg tgaggaggtg ggagtcacaa 148320
tggtggtggt gttgatggtg gtgatggtgg tgaggaggtg ggagtcacag tggtggtggt 148380
gatgagggtg gtgatggtga tgaggaggtg ggagtcacaa cgttggtggt gatgatggtg 148440
ttactggtgg tgacgaggtg ggagtcacaa tggtggtggt ggtgatggtg gtgaggaggt 148500

gggagtcaca gtggtggtgg tgttgatggt ggtgatggtg gtgaggaggt gggagtcaca 148560
gtggtggtgg tgttgatggt ggtgatggtg gtgaggaggt gagagtcaca atggtagtgg 148620
cgatgatggt gttggtggtg aggaggtgga agtcacggtg gtggcgatga tggtggtgag 148680
gacgtgggag taacaacagt ggcagtgacg gtgattgaga catgatgatg atttgtcaac 148740
tttctaggaa aacaatcata taatctccaa cagtgatatc ttaatatctt ttccaaaagt 148800
atcagatcat attataaggg ccaagtttcc agaataatat cagacataat gacagtggac 148860
atcagagctt ggcatctaaa ggtaatggga atagctctaa tgtctcagcg tgaaaaacaa 148920
catttgctat tagtctgaga tactaattat ctagttaagg aagtactcac ctatacctag 148980
tttttaactg ttttttaaaa tctggaattg attttgaatt ttaacaaata tttccctggg 149040
aacaatgtaa gattcttcat attttcgcct ttgggtatac caacatgcca gctctgttgg 149100
ccactttgtg agctcgatga agcatggtat aaaagatgct ttgctagtgt ttcacgtaat 149160
ctatttctat aagcaatttt ggagctaagc ctctgaaaca gaattatatt atctgtatag 149220
aataaatgtt ttatcttccc ccttttcttt cttctggaat agatgtgcat cagtatctct 149280
gcatcaatat ctctatatca gtatctctgt gtcagtgagc atatgttgct gggcttaggg 149340
gaggtccaga aagtgattgg gttttggcat tttcaataca cttactttgt ataagaaata 149400
gtttgccaaa tatagaaaga ggggatttag tcaagattta aattaaaaat gttagtggtc 149460
atttttctaa tgtctttcta tttttcccca ggtcctaata aatcttcact gtctgacttt 149520
agtctcccac taaaactgca tttcctttct acaatttcaa tttctccctt tgcttcaaat 149580
aaagtcctga cactattcat ttgacatatg gaattttata aatattttct ttagtatgtg 149640
tgattacatt cctgattctg agccttttta gatgagtata tagtttgata taatcttgtt 149700
attgccacct gtgtcttctc ccaaagccat taattatata ggaattacac gatagaaatg 149760
ggtttaattt ttaaaatacg gccaagtgtt gatgagaggg aaaatttttt taatttcttt 149820
cactgagtat ttatgacgtg cacaacattc ctgaatatat tgtctctctc atttctcaga 149880
tgggatgtat tgccttctcc atttctattg ttaaagaaac acttacaggg gtttctttaa 149940
caacttgtga acagcagcat cagagcccag actacagcat aagcagctgc tgattccaaa 150000
agccctacct tccaaccggg caggtgcagc cacccagacg agggggagga accctggagg 150060
aatagctatt tctttttttt ttttgtcgag acggagtctt gttctgtcac cctggctgga 150120
gtgcagtgcc gtgatcttgg ctcactgcaa cctccacctc ccaggttcaa gcaattctcc 150180
tgcttcagcc tcccgagtag ctgggattac agacacctgc caccacgcct ggctaatttt 150240
tgtattttta gtacagacag ggtttcacca tgttggccag gcttgtcttg atctcctgac 150300
aagtgatcca cacaccttgg cctcccaaag tgctgagatt acaggcgtga gccactgcgc 150360
ccagcaggaa tatctatttt taaatggaac tgtgttttca tagtacacgg tgaggagaaa 150420
gttgctttga aatctttatc ctaataaacc aaataatatg aaaatttgcc tattttaatt 150480
atatgtaaca aagtttagtt actgctataa ttgcaaatat gtataaattc cttaccaaaa 150540

aaaaaagaat caagtgggag ccagagaata atttttctga cagaattaaa taacatgcta 150600
tagctgcttg agttcatact caatagtcat ttctgcagag ttaccgaggg cctcatcagc 150660
gtcagcagga gcccctcgcc ttctgacgct ctcacatcct tctctcctgc agccccgtcc 150720
tgccactgtc cttgtccagc ttctcttcaa gggtcaactg gtctaccttt ccctacaagt 150780
ctgtcacagc ttcttgttag caatccctat ggttgcccaa aagcattttc agagcctgca 150840
taagactgca tcttgtagaa aatttgcagt ttcaatctgc cctccctctg ccgggtgttc 150900
ccattgtatt gcattcagca ggcagggaga gactgctatt aggtctgttc ctgagtgact 150960
gctttctgtc tcagactgtt tggtgtctgt aggaggtagt ggggtgggca gtaacgaggt 151020
ctcctgtata ttccacccct acgaagcctg tgtgtttggt ttatgaacta agctcaaaag 151080
caccacaggg gtaagactgc agtacatgac accatggaaa agagggagca cccagacccc 151140
caaattaaga agagcagtgt agagaacaga gacctggaga gcagagatag aaactgttag 151200
gatcagatta tagtgttaca ccagggctcc ccaggcctct cacatattga aatgtacttg 151260
tccatctttc tccaggccag gaaatgagag tctcaaagcc atgttattct gcctttttaa 151320
actatcatcc tgtaatcaaa gtaatgatgg cagcgtgtcc caccagagcg ggagcccagc 151380
tgctcaggag tcatgcttag gatggatccc ttctcttctg ccgtcagagt ttcagctggg 151440
ttggggtgga tgcagccacc tccatgcctg gccttctgca tctgtgatca tcacggcctc 151500
ctcctgccac tgagcctcat gccttcacgt gtctgttccc cccgcttttc ctttctgcca 151560
cccctgcacg tgggccgcca ggttcccaag agtatcctac ccatttcctt ccttccactc 151620
cctttgccag tgcctctcac cccaactagt agctaaccat cacccccagg actgacctct 151680
tcctcctcgc tgccagatga ttgttcaaag cacagaattt gtcagaaacc tgcagggact 151740
ccatgctgcc agccttctcc gtaattagca tggccccagt ccatgcttct agccttggtt 151800
ccttctgccc ctctgtttga aattctagag ccagctgtgg gacaattatc tgtgtcaaaa 151860
gccagatgtg aaaacatctc aataacaaac tggctgcttt gttcaatgct agaacaacgc 151920
ctgtcacaga gtagaaactc aaaaatattt gctgagtgaa tgaacaaatg aataaatgca 151980
taataaataa ttaaccacca atccaacatc cagacacata gtgattttaa ttatttaaga 152040
gtagtttagc atatattgct ttatgattta attaaaaatc tccaaaatat atgccaaaga 152100
agtagaatga gaaaaatgta tatttctctt tcacttccta cagatgcact gggccaggtc 152160
ttgaaggctg tccaacgaat gggtaagtgt tcacagctct gtgtcacatg gacctcgtca 152220
agaatgacca cactgctgtg ggtgaagatg ctttcctgca tttctgactg tcctctgtcc 152280
tgatcaagtt tctatggctc tgggccagcc taccctcagc cagggtttct gcagagactg 152340
cccagctggt tccacgtggc tccacgtgcc aactttgtcc tcagtggagg gaaagttgga 152400
cacacagtgc tggggctgct ccctgctccg ccgttgctcg atgcatggcc tgcctctgaa 152460
ttccttggtt ccactggttt tgctgggtcc ttctgtgcct ctagctcctc tttttttctg 152520
tccacttacc ccattggtcc catcacaagc ctgtgtgtga gtggcctttc tgttcgatga 152580
caacctccag cataggggag tgtttctcct tgctttcttt cccagacaca ctgcccagca 152640

aaggcaaaag ggcttccttc aacatcagct ctggccagtt tgccagagca aagccctgag 152700
aaaagcaagg ttgaaaagtc ttattcaaac tcaccaggaa agagtggtgt tactctcgat 152760
ggcgtctagc caggaatcat ggaattatac accgagcacc tgtttgccat tttggatgtt 152820
tccaaacatg aaccaaactt ccaggcccct ctgccatctc tggtaacatt tacaaagtcc 152880
cttcctcacc actgcccttc cttcattttg gcatgctcct ccgcccccga gttgacagcc 152940
atagctctct ctcctgccac cagtgtcaca tgatcgagga agaaggcaac ttcaaaaaga 153000
ctgggtcccc ttccactccc atctcttcag tgagctgcta ggacacccag cagaacttcc 153060
ccactccaca ctgcaatctc agggatctta gtcacggggc tttccaccat gtctccacct 153120
ggaaaccagt catggccatt ccttcttaca tctgctcttt tccatctttt tcttctcctc 153180
ctgttcaccc gcccttactc ttgtggcgcc ctatggatat gcgctccata gcaaatgatt 153240
ctttatatct tacggtattc tagtgagctg gcacatgtgg cttctggttt cctctctctg 153300
gaactagaca tgacctctgt gggagggagg attaaatgca ccctacagtc tgaggctgca 153360
tgatgacatc actcatcaca atgatgcttt ctatgtctga atcctattcc tttataaccc 153420
ctttcaagct cgttcagaga gtatttcaca caatccatgt gctcatctta aaagccaagg 153480
acccagagga gtctcagcat tgccaaaaag tcccttcacc cagcctggcc agaggcagtg 153540
cctggtccat gtgtatggac tatggcactt caattgcatg gaaatactct tggaatgaac 153600
aaaataccaa tccatgaaaa agcattattg aagtctaagt tatttttgga atcatatttt 153660
gttaatcaac aaattgaaaa atactcatta tatggagagg tccagataaa gcctcaattt 153720
taaaaaatga ggaaaagtgt gcctggtagg ggactgggga gagcttgaga aagttggaaa 153780
cgttgcctta gaagcctgtt ttttctcctt ttagaagcta catagtgtct cactttccaa 153840
gatcattcta caagatgtca gtgcactgaa acatgcaggg gcgtgttgag tgccaaggcc 153900
atggaatctg tcagcaacct cacccttcct tgttcctcca cctcattcca ggcctaagat 153960
cccgtccatc gccactggga tggtgggggc cctcctcttg ctgctggtgg tggccctggg 154020
gatcggcctc ttcatgcgaa ggcgccacat cgttcggaag cgcacgctgc ggaggctgct 154080
gcaggagagg gaggtgagtg ccagtcctgg gtgggctcag gagccctcgc accccgacag 154140
gaacaagggc cagccccgag aacgggccat tagcagttgt gtatgttaga tacataattg 154200
tattatgatg cagaaagaat ctctgaatgt gcagttatac ccagttggtg acatgttggt 154260
acatccatcc gaggaaatgg caatgtttct aggctgcacc cttcaatgtc cacaaagctg 154320
tgtggcatct gcttaggacc cggtgcctgt gtgtgcatag gagggaggcc aggaagcctg 154380
gctgttgatc ccatgctggc actgtggcga aggcgagaga ttcctgcttt ggaaaacacc 154440
attgtccaca cagtggcttt gtccatgatg gacttcgcca cagcccagtc ctgtgctgga 154500
agccatgttc tctggaaaga gcaacccagc ggctcataag cataagcgcg tgtgatgtgc 154560
cccaaccaaa cgaccgccat gcacaacttc cctaccggag ttttcaatcc agttaatagg 154620
cgtggaaaca gacatagaaa ttgtgtttgt tgaaaggtag ctgttcagtt aaagaacacc 154680

tgtatcagag cctgtgtttc taccaacttc tgtcaagctc tgtagagaag gcgtacattt 154740
gtccttccaa atgagctggc aagtgccgtg tcctggcacc caagcccatg ccgtggctgc 154800
tggtccccct gctgggccat gtctggcact gctttccagc atggtgaggg ctgaggtgac 154860
ccttgtctct gtgttcttgt cccccccagc ttgtggagcc tcttacaccc agtggagaag 154920
ctcccaacca agctctcttg aggatcttga aggaaactga attcaaaaag atcaaagtgc 154980
tgggctccgg tgcgttcggc acggtgtata aggtaaggtc cctggcacag gcctctgggc 155040
tgggccgcag ggcctctcat ggtctggtgg ggagcccaga gtccttgcaa gctgtatatt 155100
tccatcatct actttactct ttgtttcact gagtgtttgg gaaactccag tgtttttccc 155160
aagttattga gaggaaatct tttataacca cagtaatcag tggtcctgtg agaccaattc 155220
acagaccaaa ggcattttta tgaaaggggc cattgacctt gccatggggt gcagcacagg 155280
gcgggaggag ggccgcctct caccgcacgg catcagaatg cagcccagct gaaatgggct 155340
catcttcgtt tgcttcttct agatcctctt tgcatgaaat ctgatttcag ttaggcctag 155400
acgcagcatc attaaattct ggatgaaatg atccacacgg actttataac aggctttaca 155460
agcttgagat tcttttatct aaataatcag tgtgattcgt ggagcccaac agctgcaggg 155520
ctgcgggggc gtcacagccc ccagcaatat cagccttagg tgcggctcca cagccccagt 155580
gtccctcacc ttcggggtgc atcgctggta acatccaccc agatcactgg gcagcatgtg 155640
gcaccatctc acaattgcca gttaacgtct tccttctctc tctgtcatag ggactctgga 155700
tcccagaagg tgagaaagtt aaaattcccg tcgctatcaa ggaattaaga gaagcaacat 155760
ctccgaaagc caacaaggaa atcctcgatg tgagtttctg ctttgctgtg tgggggtcca 155820
tggctctgaa cctcaggccc accttttctc atgtctggca gctgctctgc tctagaccct 155880
gctcatctcc acatcctaaa tgttcacttt ctatgtcttt ccctttctag ctctagtggg 155940
tataactccc tccccttaga gacagcactg gcctctccca tgctggtatc caccccaaaa 156000
ggctggaaac aggcaattac tggcatctac ccagcactag tttcttgaca cgcatgatga 156060
gtgagtgctc ttggtgagcc tggagcatgg gtattgtttt tggtattttt tggatgaaga 156120
aatggaggca taaagaaatt ggctgaccct tatatggctg ggatagggtt taagcccctt 156180
gttatttctg actctgaaac ttgcattcaa ttcactccac caagttatct catctttgaa 156240
atggcttttt ttaaaggtgc ctagaatatg atggcgtgca gtctataaac tgttgcccac 156300
cttctgtact ttctctcaga ataattcaca ttcttctcca gtgtctgttg attgttactt 156360
tgtggaataa gttcttggaa aattccacaa gattattgtt atcttcttac taccaattct 156420
attgaacttt ctccaccttc tctgggcctt ccccagccag tggtgggaag atgctggctg 156480
gagtctgaca gagcctcttc tacactggcc tgggcttgct gtgagttggt ggaaaccttt 156540
gctcttgtcc caacacagag caagtgaaag aggaggtcaa ggggctcagg cagcggacta 156600
gggaagcaga atcgaggaaa aggaaaaatg gctgacttat tacctcaaaa ctctagagaa 156660
tttagttgat cttacagcca agaaggacaa aagccagaga gtaatatcct ccgcctcatg 156720
tctaacccac agaatacata gcaagtaaag agaacatggg cctttataaa aatgtcttaa 156780

gatacaattt tttaattgga ggaaatctac agtttaattt tctctgggca gcttttcttc 156840
cttttattat agtaggggaa atcccatgtt gatatacttc taaatgaaag atgatgaatt 156900
gatataatac aataaaaaat ctgtaaaatt gatgatatac ttatcaagaa aaattagctt 156960
tcattttaac ggtttacaaa ttgagtcaag tcctagtaac aaaatgttaa gtctattaac 157020
ataaccacaa gaaatacagg aagacgggca atctgtgaag cctttcactt acaatctctg 157080
gcccctcacc tgtgctgtgt aggaaaatct ttgtgcacaa tttgcttcct taattcattt 157140
tttattcatt caacacattc taataaatta tacaaaatca tgttgaaatg tgaatttcag 157200
tggtatttat aaatgcagtg tgaggagggt ttggatgtat tctaagacaa tagttgtgct 157260
ttgggaagga agcagtgttc actgaaaagt gcccccagga ccttttaatt ggaggaaata 157320
tgcttctgtg gagttggaaa tggggtagaa gatagataag gtcaaggctt aaaagttaag 157380
tgcacccaac atctgaagcg tccatgggcc tggcatggtg gctttcgcct gtaatcccag 157440
cactttggga ggctgaggca ggaggatccc ttgagcttag gagtttgaga ccagcctggg 157500
caacatactg agacccagtc tctacaaaaa ataaaaaatt agctgggtgt ggtgtctcat 157560
gcctgtagtc ccagccactc aggagatggg aagatggctt gagtccagga gatctaggct 157620
gcagtgagct aaaatctcac cactgcactc cagcctgggt gacaaagcaa gaccctgctc 157680
aaaaaaatag ttagatataa atattaatat agatacctat atatatctga atatagatat 157740
ctatatatac tctgtatata gttatttaga tatataaata tatatgatat atatttagag 157800
agatatatat ttagagagat atatatttag agatttatat atattttata tatatttaga 157860
gatatatatc tctaaatata tatctctctc taaatatata tatatctctc tctaaatata 157920
tatatatccc taaatatatt aaataaataa aagaaataaa agaaagctca gtttggcctc 157980
ctgcttgtcc tgtctcctca tcccctcttc cccctccatc attttatttc cttgccccat 158040
gtttcttcac tgcggccatg tcccccctcc tctccaatga tggatgtcat gtctgctgca 158100
gtcagagggc gacaagcctg gagtgttccc tgaagcctgt ggtttgtggt ttgtcctgca 158160
gctcaggctg cccaggcctc accagcaatc ctggcgggca gggcaccaca ctgggatgga 158220
gagggggaag ctggaggagg cactttctgg taaagaaagc aaaagccagc agtgcccagg 158280
ccaatttcaa cagggagtta aatagcacct taatcctgtg gcaggacagc tcatggggcc 158340
atgtgtgctc ttagaaagac tcacatgcac gcatgcacgg cagcaatgac tccatactca 158400
cgttcccctg cagacaccag gcccccacag ccggcacaca cactgcagcc ccagttccat 158460
gttgctagca gtggcttagt gaatgagtaa agttcttaaa atgcagggga cacctgccct 158520
tcattcataa ggctggacgt acacctctcc ttaaggagtt caagagctag tggaatccca 158580
attcatacgg tagagccatt cacagatgag agagacaagc cagaaggaag gaaccaaaag 158640
tcatgtcagc agttaggaca aaataacagg ctttcaaggt cacaaagcct cagggacact 158700
cctgcggtgg gactgggcta ggagccatgg gggctccaac tgtgcgctct gcctgccagc 158760
ctgtgggtgc tggggctcca cgaagattgt tgtggaatac caagcatgct tgctgtaggt 158820

cacggtgcac gtttactact tccaagacaa acagccgaga acaaagctcg ctttagcttc 158880
tgcgtacacc gaacgggaca cacgactgaa cagcgttccc attgtgcctg ctgggtgggg 158940
aggaagtgat ggcccagtgg gtctatcaga tgttagtagg atggggcctg gcggggctcc 159000
aggctctgtg tggccgacac ccacgccccc cgctctgctc cccattccca gccccaggtc 159060
agccctgcga ggccctgcag cagatgggct gctcaaactg ctctggtttg cagatttttc 159120
ttccctctca aatgaataca atatgttttc aagtctcaac cagatcttga gaaaatagga 159180
agagccagag ggtttctttg gtgttatggt tgtacagctt cccagactcc gggggagaga 159240
tgtgatttgt gctttctggc aatcccatgg cgtattaaat tttcataggc tttccagttt 159300
aaatttaggg taggcaatgg aagggaacgc aaaacagatt tctaggtgta ctgtgtgtgt 159360
gtctcccacg tctaaagtct gttaactgga gcacccaaca ggccccacag gctgccttca 159420
cacagaggac ctggggcgcc tccgacccat tggggtgagc agtgggccat ggagggagcc 159480
agggtcagga gacctggttg tgggcctgac ctgaccctgc tcagggtggc ctcaggtggg 159540
ccgttcacct cgtcagcctc agcttaccct ctgactacag tgacctcaga caaaatacgc 159600
ttcctggccc tgtccagttc tgacttttta taaacaagca cttatccaag ttaaagggat 159660
attttcaata tctactgagt ccacagatat taaatatctc ctctcttctt taaaattgtg 159720
gcattatctt tagaatataa aaggaaaata acacacactc tccttgaaaa tagagagcct 159780
aaacactctg caggaaatat ttaaagctat agtttttgtt tgtttgtctt gaatgcaagt 159840
ggcctggact ttgacttgct ttgagtcttt gaccttcatg acttcagtac agttcaaccc 159900
tgacagtttt gaagtaggta tgtgcctaga tctgccctag tccctgctgg aatgttgaag 159960
aagcaaaggt ccaggccctc agagcacttg ccacgtactt gccaacagat acggggcgga 160020
gacttgagtc aacgtaagag caagtgtgtg ccgggtgatc cgacactgca gagcgccagc 160080
tagaccctaa gcgtgtgcta ggggctgacc aagccgttct ttcctcaaaa acttggtggg 160140
gagggtattt ttaaaatcac acaaatattt aagtacagat tatgatgact gcctcaaagc 160200
agtggctctt cagcttcatc aagcttcaga gtccagaggg tttgttcata tggaaggcta 160260
ggcctgtctc ctgcatttca ccctcttggc ctgggggcgg gacccaagaa tgtgtggctc 160320
taaaaggttc ccaggcaatg ctgaggctgc tttctgaagg aaaaactgca agataccagg 160380
agagtttcat ttagattgaa gagtcgagga aggctcctct gagaaagagt ctgctaagga 160440
aggaggaggt gggttctggg gacagaggtt ctcccgtggg taagggtgga gggaagctct 160500
cctggggaga aggtgggcag gaggaccaga ggctggaggg aggagggcag tcagcctcgg 160560
ggcttcccag gaacagggac ggccagggca gggtttaggg caaggaaagc gtgtgagcat 160620
atttgtattt tagtaaatat ttacagtttg ccctccatgt ctgcagtttc atatccatgg 160680
attcaatcaa ccacaatgaa aaacgttggg gaaaaaaatt gcatcggtac tgaacatata 160740
cggacttttt ttcttgtcat tattccctaa acaatacagc ataacaatta ttcacatagc 160800
atttgcactg tattaggtac tataggtaat caggagatgc tgtagatggg aggatgtctg 160860
taggttacac acaaatgctg tgccacttta tatcaggggc ttgagcatcc tcacattttg 160920

atatttaagg gaggtcctgg aaccaattcc ccagatactg agggtccact gtctgtgtcc 160980
cctcgcccca ccttgccttt gtctcctgtc tcctatctcc accctgcctc ccgccagcct 161040
gttgctcctg acctgcccgg gcaccctgga gcagcaccct atctcagagc ctggctcagt 161100
gtgttcactt ctgcagagaa actaacttgc ccaagtccac actcaaaaca taggcattgc 161160
tgagatgtga aaagcagctg tggatgcttt ctgctacagt ctgtgtgttc ttttccatat 161220
ctgaataaaa ggtcaccacc atttgtattt taaagagaaa gagaatttat gggtggaaat 161280
tggggattcc ctcattctca gtcagacaga aaagagggcc ccattgtgtg cctgattgca 161340
aataaattta gcttcctcag cccaagaata gcagaagggt taaaataaag tctgtattta 161400
tggctctgtc aaaggaaggc ccctgccttg gcagccagcc ggaattagca gggcagcaga 161460
tgcctgactc agtgcagcat ggatttccca tagggagcct gggggcacag cacagagaga 161520
ccacttctct ttagaaatgg gtcccgggca gccaggcagc ctttagtcac tgtagattga 161580
atgctctgtc catttcaaaa cctgggactg gtctattgaa agagcttatc cagctactct 161640
ttgcagaggt gctgtgggca gggtccccag cccaaatgcc cacccatttc ccagagcaca 161700
gtcagggcca agcctggcct gtggggaagg gaggcctttc tccctgctgg ctcggtgctc 161760
cccggatgcc ttctccatcg cttgtcctct gcagcaccca cagccagcgt tcctgatgtg 161820
cagggtcagt cattacccag ggtgttccgg accccacaca gattcctaca ggccctcatg 161880
atattttaaa acacagcatc ctcaaccttg aggcggaggt cttcataaca aagatactat 161940
cagttcccaa actcagagat caggtgactc cgactcctcc tttatccaat gtgctcctca 162000
tggccactgt tgcctgggcc tctctgtcat ggggaatccc cagatgcacc caggaggggc 162060
cctctcccac tgcatctgtc acttcacagc cctgcgtaaa cgtccctgtg ctaggtcttt 162120
tgcaggcaca gcttttcctc catgagtacg tattttgaaa ctcaagatcg cattcatgcg 162180
tcttcacctg gaaggggtcc atgtgcccct ccttctggcc accatgcgaa gccacactga 162240
cgtgcctctc cctccctcca ggaagcctac gtgatggcca gcgtggacaa cccccacgtg 162300
tgccgcctgc tgggcatctg cctcacctcc accgtgcagc tcatcacgca gctcatgccc 162360
ttcggctgcc tcctggacta tgtccgggaa cacaaagaca atattggctc ccagtacctg 162420
ctcaactggt gtgtgcagat cgcaaaggta atcagggaag ggagatacgg ggaggggaga 162480
taaggagcca ggatcctcac atgcggtctg cgctcctggg atagcaagag tttgccatgg 162540
ggatatgtgt gtgcgtgcat gcagcacaca cacattcctt tattttggat tcaatcaagt 162600
tgatcttctt gtgcacaaat cagtgcctgt cccatctgca tgtggaaact ctcatcaatc 162660
agctaccttt gaagaatttt ctctttattg agtgctcagt gtggtctgat gtctctgttc 162720
ttatttctct ggaattcttt gtgaatactg tggtgatttg tagtggagaa ggaatattgc 162780
ttcccccatt caggacttga taacaaggta agcaagccag gccaaggcca ggaggaccca 162840
ggtgatagtg gtggagtgga gcaggtgcct tgcaggaggc ccagtgagga ggtgcaagga 162900
gctgacagag ggcgcagctg ctgctgctat gtggctgggg ccttggctaa gtgtccccct 162960

ttccacaggc tcgctccaga gccagggcgg ggctgagaga gcagagtggt caggtagccc 163020
tgcctgggtg ctggagacag gcacagaaca acaagccagg tatttcacag ctggtgcgga 163080
cccagaaaga cttctgcttt tgccccaaac ccctcccatc tccatcccag tcttgcatca 163140
gttatttgca ctcaacttgc taagtcctat ttttttctaa caatgggtat acatttcatc 163200
ccattgactt taaaggattt gcaggcaggc cctgtctctg agaatacgcc gttgcccgtc 163260
atctctctcc gacagcaggg cagggggtcc agagatgtgc cagggaccag agggagggag 163320
cagacaccca cccggcctgg gcaggtcctc ctcattgctt gcatccgcct ggttagcagt 163380
ggcagtcagt cctgccgagt cattcgtgag gcgctcaccc aactccaggc agatgtaaaa 163440
ggtgacctac aagaagacaa acaaaaacat ctggagcgct cttatgccag catctgccct 163500
tgacaccacc aggcaggctg ttgctgggag ccgtggtgct tgggtaagct ccttcccatg 163560
gcagagctcc tgggacgcat tgtagaagca gggaccacct cccaggataa ccagatagca 163620
gcacaccctg cacagcccct tttactccag catcatcggg cattgatatc tcagctgcag 163680
ccacaggcgg cccccagcac cccaggaagt ggggagcgct catgcttctc tgagcacaaa 163740
aatcactgaa tatttttgcc attctcatgg tcataacccg ggccacagag tagaacactc 163800
ctatcactgt tgttagacag tggtcctggg agagggtctt gtgtgcctcg gatgccaggg 163860
cctcttttta ttgggaggtg cttgttattt ctgtgtgtgg ctgcatttgt ttcccaagac 163920
tgccacaaca aatcatcacc aacttggtag ctcaacatag cacagcttta ttccctcctg 163980
gctctggagg ccaggtgtct aaaaggccat gctcccacaa tggttctgag gaggatcctt 164040
cctgcctctc tggcttctgg tggctccagc atccctgggc tgtggctgca cctccccatg 164100
tcaacctccg tcttcacaag gccttttcct gtgtctctgc aaccacaggc ccctctcctt 164160
tctcttaata aagataccag tcattgagtt tgaaaattgc taagagagtc tgttgtaaat 164220
cttcttagca caaaaaaaaa tgacagatat gtgaagtggt agatatatta attagtttga 164280
tttgatcact ccgctatgtg tataaatgtc aaaacaaaca ttgcactcca taaatatata 164340
tattaaaaaa gatcccagtc attgcattta ggacccaccc taaatccagg atgatttcat 164400
ttcaagactt ttaactagat ttgcaaaacc ccatttccaa ataaggtcac attctgcagt 164460
tttgggtaga cgtgaaatgt ggagacactg tgcaacccac tgtcttgggg agggggtggt 164520
cagcctgggg cagatgttgc tgggtgtgga gctacatcca ctcatgccct gacctggaac 164580
ccagacctgc ttccccagct ctcctcctgg ttatctgaag cagggaatgg agagcactgc 164640
cctccttgcc caggcagtct ctatcacctg gttttagttt cttcttagca catattgccc 164700
cagaatatct ggttggttta tggcttactt gagtttgtgc ctacctgtcc caaccgggag 164760
gtgagccctg gctattcccc aaacccggcc ctgcatgtgg gagctgccct tcctccgttc 164820
atcagagggg gccaacagtc cacagctgtt cttaatcatc tcccagtaac ccccagctcc 164880
acaaaggtga ctccttacat ggtggagagg tggtcgggcc atccgtgtga aatgtgtatg 164940
tgaccgtttt ccttaagggg cacgtagtct tggcaggttt cgctcaatat aggatgagct 165000
caggactcca gtggactgtg gattcagatc tggattctgg cgcattcgcc gtgtgaacgg 165060

gggcacgttg ctggcctgtc tgcgcctcgt ctcccgactg tggagtgtgt tctgcccctt 165120
gtctttctgg gaggtaggga gggcagtgag ccccttcgca tcgcccacca caggcccagc 165180
acatggctga tccccactga gtgttctttt cctcctttga tcccctttgg ctgacctagg 165240
ttggagcagc cactaaaata tacccagaaa catcttccta atctacatct gtgccaaccc 165300
tcattccctg gcgcagcatg accatcacat gcccgccatt gttcctgatc tctgctgctc 165360
atgacctgct ctccagcgct ccttctcatg ctcacattcc agttggcctg acctagataa 165420
gtggaggttt atttgacccc aaaaattagc cttctacaaa cgaatataat agtgtccatt 165480
acagagaata aacttagtgc gtgtcccatt taagcagaag ttactgaaag cctgagttta 165540
agtttccagg gcctgaaagt tttccatgac agttttctgc ataatattac ctacaatttc 165600
aatctgttat ttaaagccat tcttgtgttt gttgtacttt gattagcttt attttgattt 165660
gaagtccttt tacattacgg gcagttaacg ctttgtctct gttagatttg ctttttagtt 165720
cacaagagaa acctcattcc tctgtatttg aatagttgca atgatggaac agctgtccct 165780
ggagggaaat gaaaacagtg attccccaaa ttgtgacaat agaaatttgc tcttgggtta 165840
cttacaatgt atctgagtat taaaaaattt tctttttaaa cgtttgaagt aaaactaccc 165900
agaaacactt agtggctgac cagaaactaa actcctggca tcctcaaaat gggatttatt 165960
ggcttataaa tgtcctgtgt tgactcacaa aggcacaaac tatctaggta agttttcttc 166020
taaatgttga tgggagagct ggccactgtt atgcaagttt cattgtcctg actaaactgc 166080
caaagagatt acataaaatt atatcaacta gacaaaagga aaaaggaaaa aaaacagagg 166140
tgtcttggga ggaatccata tgagaccagt agaccatgag agagacatcc cttgccatct 166200
acaaggaaaa tggattttgt tctccatatg caaaaccatc tcaggagctt gcggagacac 166260
cacttgctta ctagccagaa agagcaggtg cctcctaaat tccccacaca ggagctcaca 166320
gtggctttca tgcactggga ttaagttaga cttaagaaag cctgtctact cttcctggga 166380
tttacaagcc agctagtaaa tcccagaata aatcacacgg cacagtcatc caaagatccc 166440
gtcatccgtg ccgtttggaa agccctgctc ctgtgccacc ctctccccgt ggagcctccc 166500
atgcccagga ctgcagagtc ctgccattca gactgcaact catctcacat tcttccaaac 166560
tatttggaca acagagcttt ctcatcacct aatgcagatt acagtctcac agaattgagt 166620
gttcaggcag acactgatgt ggttctgtag tacagcaaac aatatcagtt tacagtcctg 166680
aggccaggcc tggtgaacaa cgcacggtag cggtggggca gggttctcag aatgaaactg 166740
gcttacacat ggcactctct gaccacaact gtataagcac caaactacac ttagttccat 166800
ctatgaggta aaatttaatg cagatgaaca tcaaagaaaa cgtcaaaggc tcctttttac 166860
aagtacgtgg gctacttaat ttggtccaag tccattttaa aaagccctag gtgctttcac 166920
ggctctgcta ctgacaagaa gccccagtgc ctgtgagctg ctaatgggag ggagaggaag 166980
atgagctgag tgggccgggc tatcccgtcc acaccgggag acagggaagg agactccaag 167040
ctggtggtgc cagcacattc caggccactc aggcctattc ctaggtgcca ggtcacgaaa 167100

accacgctga cagatcgtgc tgtgtgcgtg tcatagcaca caagcaggac tgtgagagag 167160
tgaaagtgac actgggtgga gcactgagga agggccacag tgtgttggtg gagataggct 167220
gtcatggaga agagaccctg gcttgctcta cattgcttcc aatgcaactg caaggcaggt 167280
cccagagggc tccggccttc gtcatccagg tttgctccct cccctcatgg ctttcccatc 167340
ctcagatgag gactcggcag agcctacccc tgctgactaa ctgtggcccc agggtggtga 167400
ctcagccctg cacctcctga tcccgtctgc actgggccag agaggatgac ttacccagca 167460
cgttcacatc acacagcttt gtggattcct aggtccaagg accagagatt tcagttatgt 167520
gagttatttt ttttatttgt tcttgcgtat tccacaaagg gtcgcagcta aacttaacct 167580
aatgatcact ttagtatatc actaaaaaga caaagctcac agtgctgttg aagcacattc 167640
atcatcttta gacattttga ctagttattt cttaagcatt tacctgctag tgttaagcat 167700
cacatgaaat acatatagaa gtaagacaaa atttcttatc tccccaagtt tgccaacaaa 167760
tacagagcag gaagggaagc aggtcagagc aggaggcgca gctatagtga ggccaccatg 167820
caaggcacag ggagggtgag ctccaagttt gaatggaatg ggtctgtcag ccaagccccc 167880
tggctctggg aagatagcag tgaacaagcc agatggcccc tcaccctcca gagccgtgag 167940
tcctgcagac caaacagcgt gacaggtcct ttccctgtcc aggaggcctc tgtgggtgag 168000
agttggctgc ggacagggcg tgaaggcact tgagggtggg gaagtgactc tgactgggag 168060
atgctgagga cagggaggaa accaccagat aagggacact ggggaggagg ggtggacccc 168120
tcagggccaa gcacatggag cctcatcaca aaggcaagat ggtggccaaa ttcaaggtcg 168180
ctgcaaaagg aatggagaag agagaataga tttggcattt ggaggaaatg gtgacaatca 168240
tgagcaccta cccgggactc tccatgggtg ctatctctac ataaactcat tccaccctct 168300
gattaatcca ttctacatat ggggaaacaa aggcatgcgg tgtttacgtc acttgccaag 168360
atctcaggat ttgatccagg tggcctggtt ccatggtgca gcctctcagc ctgcatggat 168420
gccccagctc agagcatgac tctcaggaca ggggtcccag cagccctccc tccctgagca 168480
gcagggtgcc cgtgctgcac cacttctgtc taggaatagg acattctgac actttcctgc 168540
ctcttccgag gtctagcact tactctatgc ctgcctggga aggtggcaag ctggcctgag 168600
gaacagactc ttccattttt tagggagctc aaggccacag atgctctgag atctggagtc 168660
cagagacagg agcggaggct tctcctggtg accactctgc ttaaaaactt catcagatcc 168720
gtagtttcag agcccccctg aaccccatcc cttacctcta ccagttgcag gtgggtctct 168780
ggggtggggc tgccctcccc accagcaccc caagggctaa aaggttgagg ggagaacacc 168840
atcatttgta cagggggatc ctggaagatg aggcctgaga aagccctgcg gggcccctca 168900
ccttctccct agctgtggcc aagagtgtct ggccttgcct gcctcaggac cagcccaaag 168960
tggaggtgag aggtgagccc cagcccccag gggaagggtg atggtggtct tggtctcagc 169020
atggttctgg tagaggtggg ttattttgaa gatgatgaac cttaagcctc tttctgatct 169080
tgctttaaat aaatacttct gaacaacagc aacaacagaa tagtgttgat aggaaagccc 169140
tccactccac cagaaccacg cggccttctc gtcctcccct cctccacttc cttcctaagt 169200

cactgctcca tgagctcttc cacaggagat ttacaaaata gaacacaaac aatccagttc 169260
ccgcctctca ctctgaactc ctcccaagac tcgtggggtg cggcagcccc tgggaacacc 169320
cagcccttca aggtcaaaca cagcccccgc ccctcactct ggggtaccct gccagaataa 169380
gccccgacag ccatgtggag cagagccttc ttttttgtaa gtggaagttc caggctggct 169440
tttcaaatcc ccttttaacc tcagtgctgt atttcaaaat tcattccagt tttcctgtag 169500
taattaacaa aaataaatat tttaatttca attaaagtga gggtctcgga gaagaagcag 169560
gaactgagtt tcctgagagg ccccgctgag gctttgttga tatttcttcc tgcgacctct 169620
gctcggaccc tgggagctca caggccgtat cgcagctctt atctttgggg accagttaaa 169680
gcataactgc gccaggcaca gagttgtcct ttcaaatgtg ccggcagtgg gacggagacc 169740
catgcgtcaa gtctcctcta agttcacatg ggattctctc cttgtcccaa agctgtctct 169800
gacttaaaac cctccaactg attacctgaa ttccagaata tgtcctgtgc tctctgccct 169860
ttcccacgcc tttggtgaag accggtgttc tgaggaaaca gacactgtgt agaaatggct 169920
caggtccttt aaagccctgg tgtgaggagt ggggaagggc tgggccagag gtcagctgga 169980
tttgttagat tgacagagtg acgcggactt ccccagaggc acgggaccaa ggtgcatgct 170040
cacgctgtct catgctctca cacataatgt gtgtgtgtgt gtgtgtgtat atatatatac 170100
acatatacat atatatatat acacacatat gcatatatat aaaaccccaa gcagcctctg 170160
gcttagcagg tgcatttccc agcagggcaa ttaaagccat ggtcccagta gtggtcttgg 170220
ggtctcaggg tatttggtct gtgcagccac atgcttcagt ctctggaccc caggtcatct 170280
aacgaggtgg tcgtgtgggg actgggatag aaaaggtgtc tgcacggacg tgtgtgaaag 170340
ggctggcaca tcgccagtgc tcagcactgt cagctgctat caccagtcat tcaatcattc 170400
attcattcag ttgttcattc ttcaacaggc cgttttaaaa atgtgcccag tataccaaaa 170460
tctccgctaa gcatttaaag aggcagaatg aaagttagca gtggtggtga aacgaagctg 170520
ggaatgtgct ctgagggcct ccttgtgggc ttaatgaata tgtagaaacc acgcatttta 170580
aatagagagg gagaaaggga gaggttcctg gtcctctgca tggggacttg tgtgtggctc 170640
tttactgtag gcctgtgcca ctcctgctca acagctacca cagaggacgc cttcaacaaa 170700
tgtgaagaac gaacaaaagg tacaaatgtg aagaacgaac agggtagaaa gaaaggagaa 170760
agcaagggtg agggtgagaa atcaagggac agagaagaga gaagaggaga tagcctggga 170820
gttcacacag ccaagaaggt agacactcag ttgaaccagc aagaggctga gcctaactct 170880
ccctttcgaa tgggcaggag ttcatgatat ttaataaaca gaggccttgc tctgtaagag 170940
acagggtacc aggcagagag caagtcagca tcgcaggagt caaacgaggc agacagcggg 171000
ggcagggagc ttgcctctga aggagaccca ggctgccaga gtagcaggga gtctgggcca 171060
gtcctctttt gggaagcgct tcctcggctt ctgccccccc tctcctctcc ctttccaccc 171120
accatcctga cataatactt cctaatctgg aagtgttgtc cagagaagaa cctgctcatt 171180
tcctcttaag taggcaggga agcactaacg tccagcagca tcggaaaccc gtaggagcgc 171240

tctcggcagt gcagggtgag gggacagtcc atgtagtcat gagacgtggg tgtcaggcaa 171300
gcgtctcttt tccaaaagag aaaaacatta aaggcctcac aaacggcgcc caaagactaa 171360
ttctgcatag catctttgcg agaccctagg ttcttatgat gactggtttt gcctgagaaa 171420
gaaaaaattt taattttgct ctgacatgcc aattcaacaa atcattttca cataatattc 171480
atgcaaaaaa aaaacaattt gccagaaaac ttgggaatcc atccacatct acagcttttc 171540
cctgcagtca cactacagtg ggatccctcc atacaggagc ggcagagtgg agcaggctag 171600
agatgcctgt ttgtttctgt ttgctgcacc gcagcaagca tttctgtcgt gcccactctg 171660
tactagaaag tacatgaaca tcagccataa agggaactag aaaggtggcc caccctcttg 171720
gtggagagag aagagagtgt ggtagaaaca ataataagaa gtctgcagaa cttgacccct 171780
cccagcctct cccacctgcc agcctggccc ttgcagagag atgcaggctg ccattcttag 171840
gccaaagcct gggacagttg ggctcagcaa ggtaggcatc cgtcaagcaa ggaggagcag 171900
gggtcagcag tgaccccagc agccagcagg gagaaaggtg catgtgacaa ggacaccaga 171960
ggccgtgggt caggatcagc cagggtcagg gtagcatttc taggaattca ctctgttggg 172020
cgctgtgctg gctgcttctc acatattatt cctttcttac tctcagagca gagatttcaa 172080
ttgcagcgag attgtggagg cagccaggga ggtggggagg gtggtgtctt ctaaaagcat 172140
tttcagtatc catgtggttt cagtaataat aataataata aaccagtgaa aagtaaaaca 172200
ggacaaaaat cttcataggc agtgaaccat atcagagagt ccaagaaagc acaatgagag 172260
tgtggcttaa aaaccctgaa cgacattcct ttgcaccagc ttggtgagga gggcatggtc 172320
cccgccaccc cccaccccca ctttgcagat aaaccacatg caggaaggtc agcctggcaa 172380
gtccagtaag ttcaagccca ggtctcaact gggcagcaga gctcctgctc ttctttgtcc 172440
tcatatacga gcacctctgg acttaaaact tgaggaactg gatggagaaa agttaatggt 172500
cagcagcggg ttacatcttc tttcatgcgc ctttccattc tttggatcag tagtcactaa 172560
cgttcgccag ccataagtcc tcgacgtgga gaggctcaga gcctggcatg aacatgaccc 172620
tgaattcgga tgcagagctt cttcccatga tgatctgtcc ctcacagcag ggtcttctct 172680
gtttcagggc atgaactact tggaggaccg tcgcttggtg caccgcgacc tggcagccag 172740
gaacgtactg gtgaaaacac cgcagcatgt caagatcaca gattttgggc tggccaaact 172800
gctgggtgcg gaagagaaag aataccatgc agaaggaggc aaagtaagga ggtggcttta 172860
ggtcagccag cattttcctg acaccaggga ccaggctgcc ttcccactag ctgtattgtt 172920
taacacatgc aggggaggat gctctccaga cattctgggt gagctcgcag cagctgctgc 172980
tggcagctgg gtccagccag ggtctcctgg tagtgtgagc cagagctgct ttgggaacag 173040
tacttgctgg gacagtgaat gaggatgtta tccccaggtg atcattagca aatgttaggt 173100
ttcagtctct ccctgcagga tatataagtc cccttcaata gcgcaattgg gaaaggtcac 173160
agctgccttg gtggtccact gctgtcaagg acacctaagg aacaggaaag gccccatgcg 173220
gacccgagct cccagggctg tctgtggctc gtggctggga caggcagcaa tggagtcctt 173280
ctctcccttc actggctcgg tttctcttag ggaccctcac agcactaagg ggtgcgcgtc 173340

ccctgtcagg ccctcgaatg ccctcccaca gccaggcccc tctgaggttt cactctggcc 173400
tgcttggctc ctagcagcca ccaacccatg atgctgggcc ctgaaaacac acgcagacct 173460
ggatgagtga ggccactggg cacaaccagg gctcccagct caccagagca gcctgggaca 173520
cagagggtgc tcagaaacct accagagcag ccctgaactc cgtcagactg aaatcccctg 173580
ttgccgggag gaggcgccgg gcctggggga cgggtcctgg ggtgatctgg ctcgtctgtg 173640
tgtgtcactc gtaattaggt ccagagtgag ttaacttttt ccaacagagg gaaactaata 173700
gttgtctcac tgcctcatct ctcaccatcc caaggtgcct atcaagtgga tggcattgga 173760
atcaatttta cacagaatct atacccacca gagtgatgtc tggagctacg gtgagtcata 173820
atcctgatgc taatgagttt gtactgaggc caagctggct tttattgtta gttaatttac 173880
attatatcct ctgacatgca agtattttct ttcgagataa tgactaatga taatgtaatc 173940
attgctgtct atctattgta ctgagaaaac acggcagagg aaatcgagtc cagctgccgt 174000
ccaaaagtca ctggagattg caatgagctc gtctggcagg gtggggggta tgggagggaa 174060
agagcttagg aaacggctct ccctgcaaag tccaaccaaa ctttaacgtt aaccaaacca 174120
ttaatgttgc catgaatttg aagtgaacca gagggaggtg gcagaagaag cttaatgggg 174180
aatagttccg gtagagaaat gaggcttaag atgaactacc ctggccctta tgtgtcagag 174240
agaacggctt gacaaacaca cactgaggat gtctgcaggg ataaaagaag aaagggagat 174300
gacccttgct tctcgctctc gggaggacca tctggtccgg ccctggggat tctctgtttc 174360
ctcttctgaa tcccagtgtt gcccagcact ggcctgtacc catcctcacg agggccgctc 174420
tcctcacccg gccctaggtc cctgccctgt cctgagccta caggggcctc ccatgttgag 174480
aaagtgttgc tgacacattg tctctgaccg ctgtgccagg cattttctgc tgaattaccg 174540
cacttggtcc ttgaatttca cccagcaact tactgaaagg ctggaaccca tgaacctacc 174600
ccttcactga ggaaaataag ttaccccagc catctacagc gacaggagca agggaggagt 174660
cgcctcacct ctctagaaat gtgtatttga ggagaacact attgaaatga atttccaaga 174720
ataatctagt cagtattaca aaagcaaaat tatttgggat atcgtccttt tttacttagt 174780
atttttcttt tttcctatag cattattaac tttctgattt tccaaataca tacacatttt 174840
taaatttcct gagtctttat ctcttctgtt aaaatgtaag atttatgata caaaggcaga 174900
gatttgtgtc catgaataag tgaagtttgg tgtgcacctg tgagctgagc cacctcaatt 174960
aatggaacag ataaggaaat aaaggtctgc tgatgcattg ttatttacag ccattttcag 175020
aatgtatctc ctctccacga gggaactgca gggtcctgcc ccaagccatt tattttgtcc 175080
tcaagcagcc cgcccctccc actccaggca cagcccggtc tcctgctggt ctcccctctt 175140
cccacttgct ccccctcatc tatgctccag acagaggcca catatatttt ttaacttttt 175200
tttttttttt tttgagacag agtcttgccc tgtcacccag gctggagtgc agtggtgcag 175260
tctcggctca ctgcaacctc cacctcccgg gttcaagtga ttctcctgcc tcagcctcct 175320
gagtagctgg gattacaggc gcacaccacc atgcccagct aattttttgt atctctagtt 175380

gagacagggt ttcactatgt tggccaggct ggtctcgaac tcctgacctc atgatctgcc 175440
cgcctcggcc tcccaaagtg catatttttt aactttatca gacttttcat tctctgctca 175500
acatctttct ttggtcctcc aggtatgttc agataaaacc tgagcacctg gccatgactg 175560
atgggttgct gggccatctg gccctggcaa ctctcccgtc caccaggtcc ccctcccgtc 175620
acgctccagg catagcctgt gtgtgccagc gcaatgccca cactccatgc acaagtggaa 175680
gccctctcaa agtcagtggc ttagtgcctt gatgtggtca cacccattct caggaagtcc 175740
gttcccactg aaaacattgt gtgttttcaa catcattgag gctgccacgg cagattataa 175800
tcactggcct aggcagccca ctggaactac cagaccatga gcctgaattt tttgtttaaa 175860
aatcatatcc tgttttctct actctctagt ctctagtcaa ggtgaattat tcaatttaat 175920
aaattagggg cctagtgtgt tgtaccaagg agctaaaaag agagaactcg caacaccttc 175980
cagcccattc tccacctaac actggctata ctggctctcc tctctctcgc tgtttgttcc 176040
aaaatctaat aacctgtctt cccactagaa ttcatcatac atgtttaaaa acctagttaa 176100
atagtagtta aactgactgc atagatctgg aaatgagaca gtctttcttt tacaaatcca 176160
tatagactat gagttggggg caggggatga cacaagaatc tattttcttg cccccaaacc 176220
attgctttcc ttccaatgtt aagcttgtat tctgtgtatt aattcaggtg gttccgtttg 176280
ggaatggcct ctgttaccca gagatgggag ggccatcaga actcggggtt gtctgaaaaa 176340
acactggttc taaaattatc actgctttca cttgttttta accatcatag ttgtttgatt 176400
ttgaaggaaa aacatgaggg tttttattct atgcttgtta tatctatatt gtggtttcgt 176460
attttttaga ttttagtacc tgacattttt ttaactttta ttttaggttc aggggtacat 176520
gtgcaggttt gttatatagg taaatttgtg tcatgggggt ttgttacaca gattatttta 176580
tcacccaggg attaagccta gtacccatta gttatttttc ctgatcctct ccctcctccc 176640
atcctccacc gtcctataga ccccagtgtg tgttgttccc ctctaagtgt ccatgtgttc 176700
tcatcattta gctcccactt ataagtaaga acatgcggta tttgattttc tgttcctgca 176760
ttagtttgct agggatgatg gcctctagct ccatccatgt tcttgcaaag tacatgatct 176820
cattctcttt tgtggctgcc tagtgttcca tggtgtatat gtaccacatt ttctttatcc 176880
agtctgtcat tgatgggcat ttaggttgat tccatgtctt tgctattgta aatagtgctg 176940
cagtgaaaat acgcatgcat atgtctttat ggtagaatga tttatattcc tttgagtaat 177000
gggattgccg ggtcaaatgg tagttctgtt tttagctatc tgagaaattg ccacactctt 177060
ttccacaata attgaactaa tttacattcc caccaacagt gtaaaagcat tcctttttct 177120
ccacaacctc accagcatgt gttgggattt tttttttttt ttacttttca ataatagcca 177180
tctgactggt atgagatggt atctcagtgt ggttttgatt tttatttctt taatgatcag 177240
tgatgttaag ctctttttca tatacttgtt ggctgcatgt atgtcttctt ctaaaaagtg 177300
tctgctcatg tcctttgccc actttttaat gggattgttt aattttttct tgtgaattta 177360
cttaagttcc ttatagatgc tggttattag acccttctca gatttgtagc ttgcaaaaat 177420
gttcacccat tctgtgggtt gtcttcactc tgatgatagt ttcttttgct gtgcagaaga 177480

tcttcagttt agttagatcc catttgtcaa tttttgcttt tgttgcaatt gcttgatgtg 177540
ttttcatcat gaaatcttag cccattccta tatccagaat ggtattacct aggttgtctt 177600
ccagggtttt tatagtttgg ggttttacat ttaagtcttt aatccatgtt gagtttattt 177660
ttgtgtatgg tgtaaggaag gagtccagtt tcaatcttct tcatggctag ctagtcatca 177720
tttattgagt agggagtcct ttattcattg cttttttttt tttgtcaact ttgtcaacga 177780
tcacatggtt gtaggtgtgc agccttattt ctgggctctc tattctgttt cattggtctg 177840
tatgtctgtt tctgtactag taccatgctg ttttggttac tgtatccctg tagtttaaag 177900
tcaggtagca tcatgcttcc agctttgttc tttttgctta ggattgcctt ggcaattcag 177960
gctctttttt ggttccatgt gaatttttaa attgtatttt ctagttctgt gaagaatctc 178020
attggtagtg tgataggagt aacattgaat ctataaaata ctttgggcag tatagtcatt 178080
ttaatgatat tgattctttc tatccatgag catggaatgt ttttccattt gtttgtgtca 178140
tctctgattt ctttaagcag tgttttgtgg ttcttattgt agagatcttt cactttcctg 178200
gtttactgta tttctaggta ttttattctt tttgtggcaa ttgtgaattg aattgcattc 178260
ctgatttggt tctcagcttg actgttgttg gcatattgga atgctaatta tttttgtaca 178320
ttgattttgt acaactgagt cttcactgaa gttgtttatc agcttaaggg gttttgggtc 178380
aagactatgg ggttttctag atataggatc atgtcatctg caaacagaga tagctgtttt 178440
cctctcttcc tgtttggatg tccattattt ctttctctca cctgatttat ctggccagga 178500
cttccaatac tatgttaaat aggagtgttg agagagggaa tccttgtctt gtgtcaattt 178560
tcaaggggaa tgttttcaac ttttgcccat tcaatatgat gttggctgtg ggtttgccat 178620
agatggctaa tatgttgagg tttgttcttt aaatacctag tttattgaga attttaaaca 178680
tgttgaattt tattgagagc cttttctgca tctattgaga tgatcatgtg gcttttgtcc 178740
ttagttctgt ttgtgtggtg aatcacattt attgatttgc atatgttgaa ccaatcttgc 178800
atcccaggga tgaagccgac ttgattgtgg tggcttaagc tttttgatgt gctgctggat 178860
tcgatttgcc agtattttgt tgaggatttt tatgtctatg ttcatcagag atattggcct 178920
gaagttttct ttttttgttg tatctctgcc aagctttggt atcaggatga cattggcctc 178980
atagaatgag ttaaggaaga gtccctcctt ctcaattttt ttggaatagt ttcagtagga 179040
atggtaccag ctttttttgt acatcttgta gaatttggct atgaatccat ctagtcttag 179100
gctttgtttt ggttggtagg ctatttatta ctgattcaat tttggagctc attattggtc 179160
tgttcaggga ttcagtttct tcctgaggtt tttattttta tcaaatggaa cttaagcttt 179220
ttcatttcca atttttttat gatctaaaaa tgtgcagttt acagccctgt tcagaatctg 179280
catcttcctc attctgcaga tacaggtccc tcagagcagg tgactgagtg tgtatcctgt 179340
ctggagcata atacttatgc tagtagagtt actgttgtct ttattgttaa ttaccaaagt 179400
ttaccactta tcagtcactt actacttgct gggcattgca ctaagcattt cagttgtatt 179460
atcttgttgg gtccttacag caatcctgtg aaacagatac tgctattacc ccactttata 179520

gagaggtaga ctgaggcttc cagcattgaa gcaaattgcc caagactaca gaaatgtagg 179580
tttctaaaca tcaagaaaca gtaaccagta atgatgacta aagcaaggga ttgtgattgt 179640
tcattcatga tcccactgcc ttcttttctt gcttcatcct ctcaggggtg accgtttggg 179700
agttgatgac ctttggatcc aagccatatg acggaatccc tgccagcgag atctcctcca 179760
tcctggagaa aggagaacgc ctccctcagc cacccatatg taccatcgat gtctacatga 179820
tcatggtcaa gtgtgagtga ctggtgggtc tgtccacact gcctagctga gccttggtgg 179880
ctgctcttag ccaaacagct gaggcctttg catccctgga gaaatgtcat cacattactt 179940
aaggcaggca cacaaatcca gaaacatctg taaatacccc ttcaagcatt cttttaaaga 180000
cacttcttga ctcattgggc agtatgacct gacatttgcc catgtttgca agcaaataaa 180060
taaaactaaa gtcttccgca agccattaca ccaaaatatt ctattcgctg agttactcaa 180120
tgaaataccg agttgcccta tattttgaag cctgttacca gagagactga atgtttttaa 180180
atgcatggca gtgagtaaca acataaggct aatagagtca acatttctgc tttgacttaa 180240
accttttaaa ccagtggatt tatgtgaagt ctctgcagtg tggcatttaa acatttcaat 180300
ctaaataaga gtgtgtaatt tgattgatgc tattattcta ccagattcac gagtgcagtg 180360
ggctctggag gtagcattac atgcatggga tgagcatttg caaaagaaag ttgtataggg 180420
aatatgacag agccaagtta atgtaaatat taatgccttt ctgaactcta ggccacagag 180480
ttgatctttt ttaacttcct tggtttgggc taaggaagct gtgatccaga gaagccacgt 180540
gatttgtcta aggtcacata gcagtctggc ctaaaatagc ttgatatgct gtggatggaa 180600
aataaatgtg atccctcaag aggcatgagg atttccaggc agtagccata cctccaaatt 180660
gtttaatctg gatttagatt gttgggtagt cacatgcagc agcacagtta acagtgtgtc 180720
ctcctgtgga agttgccagc acagccagcc ctctcacttg catgcatgcc caccagcctt 180780
ctcacttgca tgcatgccca ctgggtatgt gctgtactgg agacgccggg ggtaggggcc 180840
cagtcccaac cccaaattct ttaaagccta tttttctaag ttgcatctgg tttcctacct 180900
gaaggaatgc taagggtgga tgttgagtga ggaccttggt gcagggcacc ctgcagtcag 180960
gatagttcat ggagagcaat tgtacagacc cacactgctc catcccctca ggcgtaacac 181020
aggatgctga ccccaggaag agtgggcgta gaaaaactag agggcattat tgttattctg 181080
attcaaatgt acagtgctgg catggtcttt aaacagtaac cagtactagc tggccaagac 181140
agaaaagtct accacaaaga cttggttctt tcatcactta tttgactgga agtgtcgcat 181200
caccaatgcc ttctttaagc aatgccatct ttatcatttc ttccagtgtt ctaattgcac 181260
tgttttttct cattccttcc ccaggctgga tgatagacgc agatagtcgc ccaaagttcc 181320
gtgagttgat catcgaattc tccaaaatgg cccgagaccc ccagcgctac cttgtcattc 181380
aggtacaaat tgcagtctgt gcttccattg ggaagagtcc ctctaatgag catctcatgt 181440
cactgtgttc tgtcacatgc cagcctggcc tccctgtgtc ccagatcgca ttattaaacc 181500
ctccagcgca ttagagcaag cctcagtaag gcgcaggcca catcgtgaac taagcagcat 181560
ccgtgagtgg ggcccaccca actccatctc cccctccccg tctgaactct cctctggtgc 181620

tcgtcctcac tgtccggcta gccaaagcct cagctgggtc taagagagaa gcatggtcta 181680
ttgggctttg gtgtcaggca gacgtggctt cacacccctg actctccact tcttcgcatc 181740
acccaggcag ccgatccacc tatctccttc cataacacag gaataccaaa accaagctca 181800
caggattgtc tcaaagattc aataaaatat gttgcaaaat acgctcccta acacctcaca 181860
gcaaggtgca cactcgatga atgctgcagc ttcttccctt tctgtttcct cagaagctat 181920
ttgaatctca tgtaggggct ttcaagcatc aaaggatggt tcatgtttta ttttaaggca 181980
cccacatcat gtcatgaggg gaggcagcta taatttagag aaccaagggg gatttcatta 182040
taacaaaatt ggcaaacaca caggcacctg ctggcaatag acccctgctc ctatagccaa 182100
gaagtggaat agcatctcta cgggccattc taatagcctc aaaatctctg caccaggggg 182160
atgaaagaat gcatttgcca agtcctacag actccaactt ctaccgtgcc ctgatggatg 182220
aagaagacat ggacgacgtg gtggatgccg acgagtacct catcccacag cagggcttct 182280
tcagcagccc ctccacgtca cggactcccc tcctgagctc tctggtatga aatctctgtc 182340
tctctctctc tctcaagctg tgtctactca tttgaacaaa ttgaatttta gggaaaataa 182400
ccatctagtg aaactcacat ggatatgaag tcaattttaa ccaaatggta aaatcaaaat 182460
caaaataaat taagtgtatt aattattttg ttgcattgca acaacttgat tgtaagcctt 182520
ttaggtccac tatggaatgt aattaaatca aaactaaacc tagttgctct aaaactaacg 182580
attaagacaa aaattaaaca ccttcacaat ataccctcca tgaggcacac cacctgcatt 182640
caggaaaagt ggatgagatg tggtacaagc attccatggg caacttctct gtttcttttt 182700
cagagtgcaa ccagcaacaa ttccaccgtg gcttgcattg atagaaatgg ggtatgtatg 182760
aacaccttat aagccagaat ttacagctct ccactatggc tctattttac atggaaaatg 182820
ccttaaccta aataatttta acccagataa tcttgagttt tcttcctgtg tgggtttttc 182880
cctgcacggc tgtcacgcct cacagtgccg ttcaaagcgt gactcctgga ccagtagtag 182940
catcgcctgg ccttgttaga aacgccattt ttcaggccac tgccccagtt tgaccaaatc 183000
aggacctctg ggggtggcac ccagtagtct atgtttgagc cactttccag gtgatgctga 183060
tgtctgttga agtgtgaggc cgtggtctag accgcactgt gccatgcaga aaccactagc 183120
cacatgtggc tacttcaact taaatgttaa tgagttaaaa tgaaataaaa tataaaattc 183180
agtttctcac acatgtgaag tgtccagtag ccacacgtgg ctagtggtga ccgtattgaa 183240
gagcaccgct catagcacac ctccctcact gcggaaagtt ctgctgtaca gcacccagca 183300
cagccctgct gcccaccctg cagcctgtgg cccagtagca ccagcaccca ccagggtgca 183360
gactctcagg cctgcccaac ctactaatca gaaccagcat ctcaaggaga tctcgggtga 183420
tttttgcaaa cactgaagtt ggggcagccc tgaccggagt aaccttccct catttcctcc 183480
tgcagctgca aagctgtccc atcaaggaag acagcttctt gcagcgatac agctcagacc 183540
ccacaggcgc cttgactgag gacagcatag acgacacctt cctcccagtg cctggtgagt 183600
ggcttgtctg gaaacagtcc tgctcctcaa cctcctcgac ccactcagca gcagccagtc 183660

tccagtgtcc aagccaggtg ctccctccag catctccaga gggggaaaca gtggcagatt 183720
tgcagacaca gtgaagggcg taaggagcag ataaacacat gaccgagcct gcacaagctc 183780
tttgttgtgt ctggttgttt gctgtacctc tgttgtaaga atgaatctgc aaaatttcta 183840
gcttatgaag caaatcacgg acatacacat ctgtgtgtgt gagtgttcat gatgtgtgta 183900
catctgtgta tgtgtgtgtg tgtatgtgtg tgtttgtgac agatttgatc cctgttctct 183960
ctgctggctc tatcttgacc tgtgaaacgt atatttaact aattaaatat tagttaatat 184020
taataaattt taagctttat ccagatactc ataacctgct aacacacaca catatacaca 184080
cacatacaca tacacacata tacacacacc acacacatac acagacacca cacacatacc 184140
atacacagac acatacacat gcacacacat atacacacac acctcaaata catacacacc 184200
acacacacat acatgtatac acacatacac acaccacaca tacaccacaa aaaccccaca 184260
cacatacaca tatacacacc acacacacca catacacaca cgtatacaca catatataca 184320
cacatacacc atgcatacat acacaccaca catacataca gacacaccac acacacgtac 184380
acacaacaca caacacagac acgtacacac actacagaca tgtatgcaca catacacaca 184440
caccacacat acatacacac agacacatat acactacaca caccattaca tacacacgta 184500
cacatacacc acacacacca cacatacaca caccacacac acatacgcca cacacacacc 184560
acaaaaaccg cacacacata caaacatata cacactacac cacacataca cacacacacc 184620
acacaccaca cacacacata cacacaccac acacaccaca catacacgca ccacacatac 184680
acacacgtag acacaccaca cacaccacag aaacacacat taacacacca catacacata 184740
tgtatgtgca tatacacacc cacaccccac acacacatgt ataaagattt agatatatat 184800
aaaacatatg ttatatatat gttgatgtaa tatctaatat ctatatatct aatatgtagt 184860
ttattagcta tctaatatct atgtcatata tatcaaaatc tttatatata aaaatatgta 184920
gaaatcttta tacatatgtt atatgtatat aaagatttag atatataaca tatgtaagtt 184980
atatatatgt tagtgtaata tctaatatat agtttattgg ctatctaata taatataaac 185040
agattatcaa tattataagc tattagaaaa atgcaagtta aggcagatga tatacctctt 185100
tacacaccaa ctacacacac caactacaca cacacataca cacagacaca cacgacacac 185160
accatacaca tgtacacaca caccacatat acacaaacgt acacacacac cacacacaca 185220
tacacaccac acacacacca cacatacata cacatccaca caccacacat gtacacacgc 185280
cacacacaca catacacacc acatacacat atgtatgcac acatacacac caacaccaca 185340
cagacaccac acatgcataa acatatagac atatacacac cacacaccat atgtacacat 185400
gtacacacac accacatata cacacaacac acacaaatac acacaccaca cacacaccac 185460
aaaaacccca cacacacaca aacatataca ccccacacat acgcatatat acacacacac 185520
atacacacca cacacataca caccacacac acaccacaca tacacacacg tacacacacc 185580
acacacacac cacagacaca caccacacat acatacacat acacacacca cacacacgta 185640
cacacaccac acacaccaca gacacacata gacacaccac atacacaccc acaccacaca 185700
cacacaactc ataccacaca tacatacaca atagacacat acacaccaca cacaccatac 185760

atacacacgt atacacacac cacatataca cacacgtaca cacacaccac acacacccac 185820
atgcacacac cacacacaca tacaaatata caccacacac acatacacca cacacacggt 185880
gcacatacac acacatatac acacaccaga cacacatacc acatacacat cacacatata 185940
tgtatacatg catacacata cacacacaca tacacacact ctcctcaagg cagtttatcc 186000
tctgagaact ttaaatttac aaaagacaca tatgtccatt actttgagaa ggacaggaaa 186060
gaacccactt tcttttgcag caacagcaag agggccctcc cgaggctcct gctccctgtc 186120
ataagtctcc ttgttgagga cattcacagg gttcagaacc cagggatcct gcatgggatg 186180
gtgctttgct gattacttca cctctgattt ctttccactt tcagaataca taaaccagtc 186240
cgttcccaaa aggcccgctg gctctgtgca gaatcctgtc tatcacaatc agcctctgaa 186300
ccccgcgccc agcagagacc cacactacca ggacccccac agcactgcag tgggcaaccc 186360
cgagtatctc aacactgtcc agcccacctg tgtcaacagc acattcgaca gccctgccca 186420
ctgggcccag aaaggcagcc accaaattag cctggacaac cctgactacc agcaggactt 186480
ctttcccaag gaagccaagc caaatggcat ctttaagggc tccacagctg aaaatgcaga 186540
atacctaagg gtcgcgccac aaagcagtga atttattgga gcatgaccac ggaggatagt 186600
atgagcccta aaaatccaga ctctttcgat acccaggacc aagccacagc aggtcctcca 186660
tcccaacagc catgcccgca ttagctctta gacccacaga ctggttttgc aacgtttaca 186720
ccgactagcc aggaagtact tccacctcgg gcacattttg ggaagttgca ttcctttgtc 186780
ttcaaactgt gaagcattta cagaaacgca tccagcaaga atattgtccc tttgagcaga 186840
aatttatctt tcaaagaggt atatttgaaa aaaaaaaaaa gtatatgtga ggatttttat 186900
tgattgggga tcttggagtt tttcattgtc gctattgatt tttacttcaa tgggctcttc 186960
caacaaggaa gaagcttgct ggtagcactt gctaccctga gttcatccag gcccaactgt 187020
gagcaaggag cacaagccac aagtcttcca gaggatgctt gattccagtg gttctgcttc 187080
aaggcttcca ctgcaaaaca ctaaagatcc aagaaggcct tcatggcccc agcaggccgg 187140
atcggtactg tatcaagtca tggcaggtac agtaggataa gccactctgt cccttcctgg 187200
gcaaagaaga aacggagggg atggaattct tccttagact tacttttgta aaaatgtccc 187260
cacggtactt actccccact gatggaccag tggtttccag tcatgagcgt tagactgact 187320
tgtttgtctt ccattccatt gttttgaaac tcagtatgct gcccctgtct tgctgtcatg 187380
aaatcagcaa gagaggatga cacatcaaat aataactcgg attccagccc acattggatt 187440
catcagcatt tggaccaata gcccacagct gagaatgtgg aatacctaag gatagcaccg 187500
cttttgttct cgcaaaaacg tatctcctaa tttgaggctc agatgaaatg catcaggtcc 187560
tttggggcat agatcagaag actacaaaaa tgaagctgct ctgaaatctc ctttagccat 187620
caccccaacc ccccaaaatt agtttgtgtt acttatggaa gatagttttc tccttttact 187680
tcacttcaaa agctttttac tcaaagagta tatgttccct ccaggtcagc tgcccccaaa 187740
ccccctcctt acgctttgtc acacaaaaag tgtctctgcc ttgagtcatc tattcaagca 187800

cttacagctc tggccacaac agggcatttt acaggtgcga atgacagtag cattatgagt 187860

agtgtggaat tcaggtagta aatatgaaac tagggtttga aattgataat gctttcacaa 187920

catttgcaga tgttttagaa ggaaaaaagt tccttcctaa aataatttct ctacaattgg 187980

aagattggaa gattcagcta gttaggagcc cacctttttt cctaatctgt gtgtgccctg 188040

taacctgact ggttaacagc agtcctttgt aaacagtgtt ttaaactctc ctagtcaata 188100

tccaccccat ccaatttatc aaggaagaaa tggttcagaa aatattttca gcctacagtt 188160

atgttcagtc acacacacat acaaaatgtt ccttttgctt ttaaagtaat ttttgactcc 188220

cagatcagtc agagccccta cagcattgtt aagaaagtat ttgatttttg tctcaatgaa 188280

aataaaacta tattcatttc cactcta 188307

<210> 2
<211> 5616
<212> DNA
<213> Homo sapiens

<400> 2
ccccggcgca gcgcggccgc agcagcctcc gccccccgca cggtgtgagc gcccgacgcg 60

gccgaggcgg ccggagtccc gagctagccc cggcggccgc cgccgcccag accggacgac 120

aggccacctc gtcggcgtcc gcccgagtcc ccgcctcgcc gccaacgcca caaccaccgc 180

gcacggcccc ctgactccgt ccagtattga tcgggagagc cggagcgagc tcttcgggga 240

gcagcgatgc gaccctccgg gacggccggg gcagcgctcc tggcgctgct ggctgcgctc 300

tgcccggcga gtcgggctct ggaggaaaag aaagtttgcc aaggcacgag taacaagctc 360

acgcagttgg gcacttttga agatcatttt ctcagcctcc agaggatgtt caataactgt 420

gaggtggtcc ttgggaattt ggaaattacc tatgtgcaga ggaattatga tctttccttc 480

ttaaagacca tccaggaggt ggctggttat gtcctcattg ccctcaacac agtggagcga 540

attcctttgg aaaacctgca gatcatcaga ggaaatatgt actacgaaaa ttcctatgcc 600

ttagcagtct tatctaacta tgatgcaaat aaaaccggac tgaaggagct gcccatgaga 660

aatttacagg aaatcctgca tggcgccgtg cggttcagca acaaccctgc cctgtgcaac 720

gtggagagca tccagtggcg ggacatagtc agcagtgact ttctcagcaa catgtcgatg 780

gacttccaga accacctggg cagctgccaa aagtgtgatc caagctgtcc caatgggagc 840

tgctggggtg caggagagga gaactgccag aaactgacca aaatcatctg tgcccagcag 900

tgctccgggc gctgccgtgg caagtccccc agtgactgct gccacaacca gtgtgctgca 960

ggctgcacag gcccccggga gagcgactgc ctggtctgcc gcaaattccg agacgaagcc 1020

acgtgcaagg acacctgccc cccactcatg ctctacaacc ccaccacgta ccagatggat 1080

gtgaaccccg agggcaaata cagctttggt gccacctgcg tgaagaagtg tccccgtaat 1140

tatgtggtga cagatcacgg ctcgtgcgtc cgagcctgtg gggccgacag ctatgagatg 1200

gaggaagacg gcgtccgcaa gtgtaagaag tgcgaagggc cttgccgcaa agtgtgtaac 1260

ggaataggta ttggtgaatt taaagactca ctctccataa atgctacgaa tattaaacac 1320

ttcaaaaact gcacctccat cagtggcgat ctccacatcc tgccggtggc atttaggggt 1380
gactccttca cacatactcc tcctctggat ccacaggaac tggatattct gaaaaccgta 1440
aaggaaatca cagggttttt gctgattcag gcttggcctg aaaacaggac ggacctccat 1500
gcctttgaga acctagaaat catacgcggc aggaccaagc aacatggtca gttttctctt 1560
gcagtcgtca gcctgaacat aacatccttg ggattacgct ccctcaagga gataagtgat 1620
ggagatgtga taatttcagg aaacaaaaat ttgtgctatg caaatacaat aaactggaaa 1680
aaactgtttg ggacctccgg tcagaaaacc aaaattataa gcaacagagg tgaaaacagc 1740
tgcaaggcca caggccaggt ctgccatgcc ttgtgctccc ccgagggctg ctggggcccg 1800
gagcccaggg actgcgtctc ttgccggaat gtcagccgag gcagggaatg cgtggacaag 1860
tgcaaccttc tggagggtga gccaagggag tttgtggaga actctgagtg catacagtgc 1920
cacccagagt gcctgcctca ggccatgaac atcacctgca caggacgggg accagacaac 1980
tgtatccagt gtgcccacta cattgacggc ccccactgcg tcaagacctg cccggcagga 2040
gtcatgggag aaaacaacac cctggtctgg aagtacgcag acgccggcca tgtgtgccac 2100
ctgtgccatc caaactgcac ctacggatgc actgggccag gtcttgaagg ctgtccaacg 2160
aatgggccta agatcccgtc catcgccact gggatggtgg gggccctcct cttgctgctg 2220
gtggtggccc tggggatcgg cctcttcatg cgaaggcgcc acatcgttcg gaagcgcacg 2280
ctgcggaggc tgctgcagga gagggagctt gtggagcctc ttacacccag tggagaagct 2340
cccaaccaag ctctcttgag gatcttgaag gaaactgaat tcaaaaagat caaagtgctg 2400
ggctccggtg cgttcggcac ggtgtataag ggactctgga tcccagaagg tgagaaagtt 2460
aaaattcccg tcgctatcaa ggaattaaga gaagcaacat ctccgaaagc caacaaggaa 2520
atcctcgatg aagcctacgt gatggccagc gtggacaacc cccacgtgtg ccgcctgctg 2580
ggcatctgcc tcacctccac cgtgcagctc atcacgcagc tcatgccctt cggctgcctc 2640
ctggactatg tccgggaaca caaagacaat attggctccc agtacctgct caactggtgt 2700
gtgcagatcg caaagggcat gaactacttg gaggaccgtc gcttggtgca ccgcgacctg 2760
gcagccagga acgtactggt gaaaacaccg cagcatgtca agatcacaga ttttgggctg 2820
gccaaactgc tgggtgcgga agagaaagaa taccatgcag aaggaggcaa agtgcctatc 2880
aagtggatgg cattggaatc aattttacac agaatctata cccaccagag tgatgtctgg 2940
agctacgggg tgaccgtttg ggagttgatg acctttggat ccaagccata tgacggaatc 3000
cctgccagcg agatctcctc catcctggag aaaggagaac gcctccctca gccacccata 3060
tgtaccatcg atgtctacat gatcatggtc aagtgctgga tgatagacgc agatagtcgc 3120
ccaaagttcc gtgagttgat catcgaattc tccaaaatgg cccgagaccc ccagcgctac 3180
cttgtcattc agggggatga aagaatgcat ttgccaagtc ctacagactc caacttctac 3240
cgtgccctga tggatgaaga agacatggac gacgtggtgg atgccgacga gtacctcatc 3300
ccacagcagg gcttcttcag cagcccctcc acgtcacgga ctcccctcct gagctctctg 3360
agtgcaacca gcaacaattc caccgtggct tgcattgata gaaatgggct gcaaagctgt 3420

cccatcaagg aagacagctt cttgcagcga tacagctcag accccacagg cgccttgact 3480
gaggacagca tagacgacac cttcctccca gtgcctgaat acataaacca gtccgttccc 3540
aaaaggcccg ctggctctgt gcagaatcct gtctatcaca atcagcctct gaaccccgcg 3600
cccagcagag acccacacta ccaggacccc cacagcactg cagtgggcaa ccccgagtat 3660
ctcaacactg tccagcccac ctgtgtcaac agcacattcg acagccctgc ccactgggcc 3720
cagaaaggca gccaccaaat tagcctggac aaccctgact accagcagga cttctttccc 3780
aaggaagcca agccaaatgg catctttaag ggctccacag ctgaaaatgc agaataccta 3840
agggtcgcgc cacaaagcag tgaatttatt ggagcatgac cacggaggat agtatgagcc 3900
ctaaaaatcc agactctttc gatacccagg accaagccac agcaggtcct ccatcccaac 3960
agccatgccc gcattagctc ttagacccac agactggttt tgcaacgttt acaccgacta 4020
gccaggaagt acttccacct cgggcacatt ttgggaagtt gcattccttt gtcttcaaac 4080
tgtgaagcat ttacagaaac gcatccagca agaatattgt ccctttgagc agaaatttat 4140
ctttcaaaga ggtatatttg aaaaaaaaaa aaagtatatg tgaggatttt tattgattgg 4200
ggatcttgga gtttttcatt gtcgctattg atttttactt caatgggctc ttccaacaag 4260
gaagaagctt gctggtagca cttgctaccc tgagttcatc caggcccaac tgtgagcaag 4320
gagcacaagc cacaagtctt ccagaggatg cttgattcca gtggttctgc ttcaaggctt 4380
ccactgcaaa acactaaaga tccaagaagg ccttcatggc cccagcaggc cggatcggta 4440
ctgtatcaag tcatggcagg tacagtagga taagccactc tgtcccttcc tgggcaaaga 4500
agaaacggag gggatggaat tcttccttag acttactttt gtaaaaatgt ccccacggta 4560
cttactcccc actgatggac cagtggtttc cagtcatgag cgttagactg acttgtttgt 4620
cttccattcc attgttttga aactcagtat gctgcccctg tcttgctgtc atgaaatcag 4680
caagagagga tgacacatca aataataact cggattccag cccacattgg attcatcagc 4740
atttggacca atagcccaca gctgagaatg tggaatacct aaggatagca ccgcttttgt 4800
tctcgcaaaa acgtatctcc taatttgagg ctcagatgaa atgcatcagg tcctttgggg 4860
catagatcag aagactacaa aaatgaagct gctctgaaat ctcctttagc catcacccca 4920
accccccaaa attagtttgt gttacttatg gaagatagtt ttctcctttt acttcacttc 4980
aaaagctttt tactcaaaga gtatatgttc cctccaggtc agctgccccc aaaccccctc 5040
cttacgcttt gtcacacaaa aagtgtctct gccttgagtc atctattcaa gcacttacag 5100
ctctggccac aacagggcat tttacaggtg cgaatgacag tagcattatg agtagtgtgg 5160
aattcaggta gtaaatatga aactagggtt tgaaattgat aatgctttca caacatttgc 5220
agatgtttta gaaggaaaaa agttccttcc taaaataatt tctctacaat tggaagattg 5280
gaagattcag ctagttagga gcccaccttt tttcctaatc tgtgtgtgcc ctgtaacctg 5340
actggttaac agcagtcctt tgtaaacagt gttttaaact ctcctagtca atatccaccc 5400
catccaattt atcaaggaag aaatggttca gaaaatattt tcagcctaca gttatgttca 5460

gtcacacaca catacaaaat gttccttttg cttttaaagt aatttttgac tcccagatca 5520

gtcagagccc ctacagcatt gttaagaaag tatttgattt ttgtctcaat gaaaataaaa 5580

ctatattcat ttccactcta aaaaaaaaaa aaaaaa 5616

<210> 3
<211> 45675
<212> DNA
<213> Homo sapiens

<400> 3
ggccgcggcg gcggaggcag cagcggcggc ggcagtggcg gcggcgaagg tggcggcggc 60

tcggccagta ctcccggccc ccgccatttc ggactgggag cgagcgcggc gcaggcactg 120

aaggcggcgg cggggccaga ggctcagcgg ctcccaggtg cgggagagag gtacggagcg 180

gaccacccct cctgggcccc tgcccgggtc ccgaccctct ttgccggcgc cgggcggggc 240

cggcggcgag tgaatgaatt aggggtcccc ggaggggcgg gtggggggcg cgggcgcggg 300

gtcggggcgg gctgggtgag aggggtctgc aggggggagg cgcgcggacg cggcggcgcg 360

gggagtgagg aatgggcggt gcggggctga ggagggtgag gctggaggcg gtcgccgctg 420

gtgctgcttc ctggacgggg aaccccttcc ttcctcctcc ccgagagccg cggctggagg 480

cttctgggga gaaactcggg ccgggccggc tgcccctcgg agcggtgggg tgcggtggag 540

gttactcccg cggcgccccg gcctcccctc cccctctccc cgctcccgca cctcttgcct 600

ccctttccag cactcggctg cctcggtcca gccttccctg ctgcatttgg catctctagg 660

acgaaggtat aaacttctcc ctcgagcgca ggctggacgg atagtggtcc ttttccgtgt 720

gtaggggatg tgtgagtaag aggggaggtc acgttttgga agagcatagg aaagtgctta 780

gagaccactg tttgaggtta ttgtgtttgg aaaaaaatgc atctgcctcc gagttcctga 840

atgctcccct cccccatgta tgggctgtga cattgctgtg gccacaaagg aggaggtgga 900

ggtagagatg gtggaagaac aggtggccaa caccctacac gtagagcctg tgacctacag 960

tgaaaaggaa aaagttaatc ccagatggtc tgttttgctt ggtcaagtta aacccgaaga 1020

aaacccgcag agcagaagca aggctttttc cttgctagtt gagtgtagac agcaatagca 1080

aaaatagtac ttgaagttta atttacctgt tcttgtcctt tcccctattt cttatgtatt 1140

accctcatcc cctcgtctct tttatactac cctcattttg cagatgtgtt ctacatctca 1200

agagttatta cagtactcca aaacagcact tacatgattt tttaaactta cagaggaatt 1260

gtagcaatcc accagctaac cgcctgaaat agacttaaac atgtgcatct cctttttttt 1320

tttttttttg agacacagtc tcgctctgtt gcccaggctg gagtgcaatg gcgcggtatc 1380

ggctcactga aacctccgcc tcctgggttc aagcaattct cctgcctcag cctcccgagt 1440

agctgggact agtaggtgca cgccaccatg cccagctaat ttttgtattt ttagtagaga 1500

cagagtttca tcatgttggt caggatggtc tccatctgct ctgttgccca ggctggagtg 1560

cagtggcgcc gtctcggctc actgcaacct ctgcctcctg cattcaagca attctcctgc 1620

ctcagcctcc cgaataactg ggattacagg tgtctgctgc catgcccggc taattttttg 1680

tatttttagt agagacgggg gtttcaccat gttggtcagg ctggtctaga actcctgacc 1740
tcgtgatctg cccgcctcgg cctcccacag tggcatgtgc atcttatagc tgaagtctaa 1800
gccttcttaa atcttgagat ccatcaaaac agacaggttt tctaattgtt atacaatgta 1860
tatgttatgt ttataataga aatcatttta caaataagtt ataaatggga aaggtctatt 1920
tgtaattatc agctcagaat taaccataaa actggtgtca ctgaagtgac tgaggtccaa 1980
aatgctgact ctgcatgtta tagactacag atatcaaata tggttgctaa caatagttta 2040
ctttgagact gtagccatcc acagtatatt tgcttttaag agatggtaga tggtaattca 2100
gttttatgaa aaataaaaat gaattttctt ccattacaaa attgttggat tcgagtccag 2160
tccactcctt actagctttt ctaactctcg gtgagggatc ccctcccagc ccatgatctt 2220
catttggtaa gactcctttg gaacccagtt ctctctagtg gatttaaatg tgatttggtt 2280
ttaaaaatct cattcaagga attttttttt tttctggaaa caaccaccgc ataaacaagt 2340
aaaccggaag atacatgtgg ctctgaattc atatatatac acaaactcta atccaatgtc 2400
tgtccacagt atttcctagg ctagtaaact ttttggcctt aacgacccct ctaccctctt 2460
tgtttttttg agagagagag tctcactctg tcacccaggc cggaatgcag tggcgcgatc 2520
tcggcccgct actacctccg actctcaggc tcaagcgatt ctcccgcctc agcttcccga 2580
gtagccggga ttacaggctc ccgccaccgg gctaattgta tttttagata cgggatttca 2640
ccatgttggc caggctggtc tcgacctcct gacctcaggt gatccgcccg cctaagcctc 2700
ccaaagtgct gggattacag gccaccacac ccggcctaca ctcttaaaaa ttatcgaagg 2760
ggccgggcac attggctctt atctgtaatc ccagcacttt gggagactga ggcgggagga 2820
tcgcttgagg ccaggagttg gagaccagcg tactcaacat agtgagacct tgttataaag 2880
aaaaaaaaaa tccaggatta aaaaaaatct ttgatttgtt tgggatttat taatatttac 2940
cgtattggaa attaaaacaa ttttttaaaa tgtattcatt taaaaataat aagcccatta 3000
cttggtaaca tgaataaaat attttatgaa aaataactat tttccaaaac aaaaccaaaa 3060
cttagaaaag tggtattgtt tcacacttca gtaaatctct ttaatgatgt ggcttaatag 3120
aagatatgga ttcttatatc tgcatctgca ttcaatctat tatgatcaca catctggaaa 3180
acttgtgaaa gaatgggagt taaaagggta aaggacatct taatgttatt atgaaaacag 3240
ttttgacctc ttgcacacca gaaaagtctt agtaacctga ggggttccta gaccacattt 3300
tgagaactgt tttaggctat gcaaactggt tggggggagg ttggggtagg cagagagcta 3360
gaagatacat tttagtgtaa ttctcctcat ctattcctaa ttgctttggc ctacatttga 3420
aataaagcgt ggaggcaaac gggataagat acatgtttgt agtggttgtt aacttcaccc 3480
tagacaagca gccaataagt ctaggtagag cagagtaagg cggggaacta tgccgtgacc 3540
gtgtgtgata caatttttct agcctgtggt gctttttgcg gcagggctta ggagtaaggt 3600
tagtatgtta tcatttggga aaccaaatta ttattttggg tcttcagtca attatgatgc 3660
tgtgtatatt tagtgtttat ctacaatata tgcacattca ttaatttgga gctactcatc 3720
ctataataaa tagttgtgca tttactccca tttttttctg catttctctc cttatttata 3780

attatgtgtt acatgaggga aaggaggtga aattaaacat tcatattatt tcaaaaaatt 3840
tgaaacaact aactaaaaaa tatgttttat tttctgtatg gtgtttgtta tacaatctgt 3900
caatattcat gcacctcttg ggagacagtg tatgaaaagc aaagagtaac agtcacatgg 3960
attactgatt actgagatat attcacttgc atcttttttt ttttttgaga cggagtggct 4020
ctgtcgccca ggctggagtg cagtggcgtg atctcggctc actgcaagct ccgcctcctg 4080
ggttcacgcc attcttctgc ctcagcctcc caagtagctg ggactacagg cgcccgccac 4140
cacgcccggc taattttttt atatttttag tagagacggg gtttcaccgg gttagccagg 4200
atggtcttga tctcctgacc tcgtgatcca ccctcctcgg cctcccaaag tgctaggatt 4260
ataggcgtga gccaccgtgc ccggctcact tgcatctctt aacagctgtt ttcttactaa 4320
aaacagtgtt tatctctaat ctttttgttt gtttgtttgt tttgagatgg agtcttactc 4380
cgtcacccaa tctggagtgc agtggcgtga tctgggctca ctgcaacctc tgcctcccgg 4440
gttcaagtga ttctccttcc tcagcctccc cagtagctag gactacagga gagcgccacc 4500
acgcctgatt aatttttgta tttttagtag agagagggtt tcaccatatt ggccaggctg 4560
gtcttgaact cctggcctca ggtgatccac ccgccttggc ctctgaaagt gctgggatta 4620
caggcatgag ccgccgcacc cggctttcta atctttatct ttttttgtgc agcggtgata 4680
caggattatg tattgtactg aacagttaat tcggagttct cttggttttt agctttattt 4740
tccccagaga tttttttttt tttttttttt tttgagacgg agtcttgctc tatcgccagg 4800
ctggagtgca gtggcgccat ctcggctcat tgcaacctcg gactcctatt ttccccagag 4860
atatttcaca cattaaaatg tcgtcaaata ttgttcttct ttgcctcagt gtttaaattt 4920
ttatttcccc atgacacaat ccagctttat ttgacactca ttctctcaac tctcatctga 4980
ttcttactgt taatatttat ccaagagaac tactgccatg atgctttaaa agtttttctg 5040
tagctgttgc atattgactt ctaacactta gaggtggggg tccactagga aaactgtaac 5100
aataagagtg gagatagctg tcagcaactt ttgtgagggt gtgctacagg gtgtagagca 5160
ctgtgaagtc tctacatgag tgaagtcatg atatgatcct ttgagagcct ttagccgccg 5220
cagaacagca gtctggctat ttagatagaa caacttgatt ttaagataaa agaactgtct 5280
atgtagcatt tatgcatttt tcttaagcgt cgatggagga gtttgtaaat gaagtacagt 5340
tcattacgat acacgtctgc agtcaactgg aattttcatg attgaatttt gtaaggtatt 5400
ttgaaataat ttttcatata aaggtgagtt tgtattaaaa ggtactggtg gagtatttga 5460
tagtgtatta accttatgtg tgacatgttc taatatagtc acattttcat tatttttatt 5520
ataaggcctg ctgaaaatga ctgaatataa acttgtggta gttggagctg gtggcgtagg 5580
caagagtgcc ttgacgatac agctaattca gaatcatttt gtggacgaat atgatccaac 5640
aatagaggta aatcttgttt taatatgcat attactggtg caggaccatt ctttgataca 5700
gataaaggtt tctctgacca ttttcatgag tacttattac aagataatta tgctgaaagt 5760
taagttatct gaaatgtacc ttgggtttca agttatatgt aaccattaat atgggaactt 5820

tactttcctt gggagtatgt cagggtccat gatgttcact ctctgtgcat tttgattgga 5880
agtgtatttc agagtttcgt gagagggtag aaatttgtat cctatctgga cctaaaagac 5940
aatcttttta ttgtaacttt tatttttatg ggtttcttgg tattgtgaca tcatatgtaa 6000
aggttagatt taattgtact agtgaaatat aattgtttga tggttgattt ttttaaactt 6060
catcagcagt attttcctat cttcttctca acattagaga acctacaact accggataaa 6120
ttttacaaaa tgaattattt gcctaaggtg tggtttatat aaaggtacta ttaccaactt 6180
tacctttgct ttgttgtcat ttttaaattt actcaaggaa atactaggat ttaaaaaaaa 6240
attccttgag taaatttaaa ttgttatcat gtttttgagg attattttca gattttttta 6300
gtttaatgaa aatttaccaa agtaaagacc agcagcagaa tgataagtaa agacctgtaa 6360
gacaccttga aggtcatgga gtagaacttc catcccaagc agatgaggat ttatttaatc 6420
tcaaagacct ccaggagggg acattcccca actgtccttg ttaactcatt ttcagaacat 6480
atttattagc atattttaca tgtaatttgg atcttcatgt taaatttaac atcagtggag 6540
atggaaaata agcatatcgc cttgtctttg aaatagccct atattgttag attgtttctt 6600
aggcttcttt accctgggtt aagcagtcct aatactttag catttattct acatctagtg 6660
tactaattta aaaaaatcag ttctgaaaaa tttctaagaa ctttcttcaa gttccaagct 6720
gtgaaatcta gaacaggtca aagtgcctta ttaacgtact gtactgtgta gtgtcttgaa 6780
gagacacttt gcgctgaggc aagttctgag ggcattgggt ggccttggga agatatttat 6840
gcagtttaga acctggagaa ttgattagat aactaatcat aaggaaacgt cacatatttt 6900
tggtactata aaaaagtgga gaaataatgc ctatttgcaa agatttgatt taaacataga 6960
aacaacttta tttggcttcc aattttaaga atttacagca gtaaagggga acagtctaat 7020
tgaagtagac tgcctatgca atagtctctg tatatttact tttgacaagt taattcaatg 7080
tgtactatag ttttgtttct ttgaagaggt ttgaatagtg cacccatttt aatctgtatt 7140
gcaaattcag ggttacttgg cagactctac tatttaaatc agatgtaaaa ggaagtttta 7200
atataattca ctttatgcct gaaagttttc ctgggatttt ggaaggtgat tttactggaa 7260
atgctgtctg tcttccctga aaatctgaga aattccatta cactttgttt ccaatcagag 7320
gtcatgagtg ctatatgagt atatacagca tgacgtcatg aatgtgataa agtgggttag 7380
gaaacctttt gctaatgatt gttaaaatgc aatataaatg ttgaagaaat aaagctaaca 7440
gttaagcctt tatttgggcg gaaggctgaa aaagtttata aacttaaacc tataactctg 7500
cttatgattt ctgccaaacc agaagacttg actctgggaa gcattggtta cctgtgaact 7560
ttgaaactga cggtccctga cgtagtttag tcacctggga aaaggtatct gagattatct 7620
cttatctccc aagttacagt gagtctctga gggaactgac acattacatt aagttcttgg 7680
tgtagttaaa ctgtaagaaa ggcaggagaa cttagtagtt aaatagttgg ttaaatggaa 7740
atgctgactc catgttattg taaaaagtta aaaatttagg aggatatggg gatttcactg 7800
ccattgcagg ttttgattgg tatttaccaa tccgtgtggg tcagagagaa aattagaaag 7860
gatatgactg cacattttgg aattattagc agtttttcta catttaaaat ggaaataaat 7920

tttttaaaaa tttaaatcaa gtaatactgt attttttggt gatttagatt tttcaaaatt 7980
tacactaaga gatagtaagg agggtggcta ttgtttcttt caataatgtc tctgagaggt 8040
tgtaactcat ctaaggatac gtagctaata agtggtagga tttcaattta aattctctga 8100
gaccaagtta agtagaattt gcactgtact cttgtataac tttttaaaac tgaaaattag 8160
ctatctttca aattaagaaa atatttacta atggagacta attcagattt gtaagtatac 8220
caaaatttga acttagcctg ctatctaatg gcaacttagt ggcagaggta tgatgtaaaa 8280
tcattcaggt atgacacata gatggagtat gtttgtattc gaggctgtgc acataatcac 8340
ctttacttgt attgtgaagt atatattgtt atcttttatg aagcccacta aagagataat 8400
gaaatacctc gttattaggg caagattatt gaaaactcaa aatagccccc aaacacaata 8460
cttggctaga aatatatacc tttatagttc agagatcatt tattatcaaa accctgaagt 8520
tttttttcta aggtaaaatt tggtggaaga ggaaaagtct cgttttaaaa aaatgtaggt 8580
agttacagag atcagaatga ttagttgatc acttaccaaa tatatattaa gtatctactg 8640
tatataatat gctagtaaga ataaatatag caggaagtat tttttcccag gctctaattg 8700
tttgacatca gcatgctttt attgtggcac ttataattca gttcaagtat tatgcccctc 8760
tttgatggaa cagtttccta ttcagtaagg aagaccagat taatcattgg attggtttgt 8820
ttcatcttta gtgttctgag ctgtagagta tttatttacc aaggtttatt ttaattttta 8880
ttttattttt atttttccat gttcattgta gaattcattt tacctacgaa tgaagtatgt 8940
agattataga gagaaaattt gtaaaattaa actgatactg aagactggta taagaaaagc 9000
cttatgtaat ttgtaagctg ctattcttct gagtttatac atatatcttt agtaatcaat 9060
gagggatggt tgggtgactg ccctccaggg gacatttggc aacatctgga gatgtttttg 9120
gttgccacaa cttggggaga gagtactgct actggcatct attgagtaga tgctattact 9180
ttaaatggca aagctgcagt tacctttgca ccaacctaat attaaacttc ctgcagtgca 9240
cgggaaagcc cccacaacag ggttatctga ccccaaacct caatggtgtt aagatccaaa 9300
ccttgatatg ttaacctgta gctttaaaca tcctttaaat tgtcaaattc atgtccctga 9360
cataaggttt atgttagatt ttcaagtata acaaagattt aaactttaac ttttgtacgt 9420
taatgatatg ttagcttact ccagtcttct attaaaacat tctgttttta aaatcagaga 9480
cacacagcaa ttttataaat catttctctt caaggctgtg aagctctccc cacttttgtg 9540
agtgccctct actggtcaaa ttatttgctt tataacaagt aacagtgaaa tcctaagttt 9600
gtgtagtttc gctgtttaaa ttatgggtgg catcaattta taaatatatt cgttttattt 9660
aaaagtctta tatgattgat ttcgtatcat ttttgctctc tgctaatatt aatataaaga 9720
ttactgtctg tattagttag gcctaactaa gtaggtgagt atagtgaact aagaaaggaa 9780
acgaggcagt atataagaaa atagggtggt tcagttgtta acacttactg agcttacttt 9840
gttgaaggga ctaaaaggca gcagtgtggc tctctgagct tctttgcatg cactcaggag 9900
ctgcttaatg gagtccaagg cttggtggtg tgttacaggg gatgatagga gggtcctatt 9960

cagaagtggc aaattgtgaa agtgcacatt ttgtagagtt ttataggact gtagaatagt 10020
tgtgagcacc tgatttttag aataaacaga aaactcaggt actgtattta ggtcaaatta 10080
agaataagta tttattaaga cctgaatata aaactttact ggtcatggtt tttttctacc 10140
ttgggttttt ataaatccaa agatttaaaa acatacaaat ggaagttggt aatggaatta 10200
agtgaaagga aaaaatgatt ttatggtttg gaatctccta agattctggt tttaacaata 10260
caactaattc cttaatccta gaaatgttct tcactgccca ctttgtacca tgcagtcttc 10320
ctgtgggcta gagatacact gaggcgcaaa acagaccaga ttcctgcctt catggagctt 10380
attagtttta ggtatctcta gatttcttgt aatacctatt acaatgcctg cacatcagtt 10440
cattcatgtg ggttcaacgt agtactcagt acatggcaaa ttcaagtttt acttttcgga 10500
acttcatgga tttttttcct cagaatatct tttatccata attggttgaa tctgtagatg 10560
cagtacccat ggatatggat ggcccacttt attttgaaga gcagtgtttc taggcaatca 10620
tgctaattat atatgactta atttagaggc tttatactta agagcattac atttctggcg 10680
tctcttaacc attattattt cataatgtgt aggttatgga acagttaaat tattgggatc 10740
ttaatataga aattagtaga aataagccag atatggtggc tcatgcctgt aatcttagca 10800
ctttgggagg ctgaggctat tcgctgtact attttttact acttttctat aggtttgaaa 10860
ttttttcaaa ataaaacatt gaaaaaagta aggtaggtag tgtgtccctc cttaatcctt 10920
tcaaatattt tattttcact atttctatta attttttttt ttgtttttga gatggagtct 10980
cgctctgttg cccaggctgg agtgcagtgg cgcgatcttg gctcactgca gcctccacct 11040
cctgggttcc agccattctc ctgcctcagc ctcctgggta gctggtatta caggcatgca 11100
ccaccacacc caattacttt ttgtattttt agtagagacg gggtttcacc atgttggcca 11160
ggctagtctc gaactcctga cctcgtgatc tgcccgcctc agcagtgtca ctgcttctag 11220
accgttttca aggcacagag cttagaaatg catgttacta agaaatcaag agttaactat 11280
ttttcacctt ctttctcccg cagtgagaac cctggttcta ccctgtttct ccttgtgtaa 11340
attttaatgc taaactatac acttgtgaaa taaaaatgat aatgtcattc ttaaattatg 11400
gatcttgcag tgttatctaa gtaacataga ttgagtgatt taactttagg tttccttatt 11460
tgtggaattt ggataaatat ttttcaccct tgagaaaagt gagactcctt tctcatcatc 11520
agagtatcct taaaccatta aggcaaacat ttgggaaaaa actgagctat ctggctgcat 11580
aaaaattaag ttttctttaa caaagataga agacaaatga aaacctagaa aaaccatttg 11640
gttcaagtaa caggaagcta tcttatatat gaattagaga aaagcaaaca cacaaataga 11700
aaaaaaggga tggggggtac taaagatata aatagcttgt ctaccaaaaa agaaataaaa 11760
taaataacat gaacatataa aaagacactt acttcatgaa tgtgatgcaa gttcaaacaa 11820
taaataacat ttctgtactt tcatattggc taaggttaaa atgataactg ctaggaaggg 11880
tatggagaag tgtgcgcctt gcactgtagt gggagtatag accctcagac tttatggagg 11940
tcagtctgga aatatgtttc aaaatgtaaa ctacatgtcc tttgaccagg taattcaact 12000
tcttgaaatt tatccaagga tttaattgga taaatgttta agatgtatat ataagaatgt 12060

ttactgcagt gttgtttatg attttaaaaa aatggaaatc atcttcatgt ctaccaatag 12120
agaatgggtg aataaattat ggtatgtcca tatatacaaa ttacatagtt gttggaaata 12180
ttaggtagat ttagatatac tgatgttcaa aaatgtccat tatgtaagtg aagctgggtc 12240
acagcacctt gtgttgagta tgatttcatc tagaaacaaa attactccct catcctttgt 12300
tgtgttttag ttttttaaaa taagcttata ccattgggct gggggaaaag taaatactcg 12360
ttttggagag agaaaagggc actaaagttt cagataccgt tagattattt catgcttatt 12420
tttcaagcct caataaatta cataattcac atgtagtctt ggattaagga aattgctatt 12480
aaggctaaat aaataatatg agaggtatat aatataaaat atgaacatta tattggcatt 12540
aagattggat ccacggtcat tccagcctct cattcttacc tggacttcaa gtgatcactt 12600
gtgggcaaat gccatctgac ttgaacaggt tacacatgta tgctcattat atcgttattt 12660
tcaaaatttg tcatataaat tttccttgag ttcattcaga tttttgaact agttttttct 12720
cttgggagta gtacacactt aattctctct agtactaagc taatgttcac cattcttata 12780
attttaagta tccagcattt agtaaagaag tctttgtttt ctttatcctt acttttagtg 12840
aatgtcttag tttttaattg aaaattctgc catgaaaata agctctttaa catcttcact 12900
ccctaatcaa aacagaaatc cttcatagcc ttcagttgta gctatccttc cctgtgattt 12960
gtccagctcc attatattta ttttgaaata tggtgaccag ttttgcaaaa ttatttcaac 13020
tgtaggtgcc cagtgatttt gtaaggagaa gatactgttt ctgaacagtt ctcagtagcc 13080
agtggcctgc ccctactttt tggcctgcgt gtagtatata aaataatgca gttaactttt 13140
tatagcactt ttcattttat aaagagattt tcatggtctt taatattaat ctatgtataa 13200
agtcctgtat gcagttttac ctactttcac agctgaagga acaatagctt agagaagatg 13260
tgagataaag tagtttgccc aagcccatag cacaaataag tgaagttctt cggctgtcca 13320
tggatcgaag actcccaagt ctatctctag cctggacttc tgtcctgagc accagacatg 13380
tatgtatatc aagatgcctg caggtcatat ccaccaggac aacccatgag tacagggaat 13440
tcaacatgcc caatatcact catcttttcc ttcgccctcc cctttgtact catcccctgt 13500
cggtaagctc tgttatttta aaaaattgaa atgtattcac atagcataca atttacactt 13560
ttcaagtgta catggttttt agtatattca caagggttgt gcagtcatta ctactaattc 13620
cagaatgtta ttatcacccc aaaagtccca catccattag cagccactcc ccaatccctt 13680
ctcccaccag cctctaaaaa ctgctaattt ttccatctct gtggatttgt ccactctgat 13740
tatttcatat aaagagaatc gtacagacgt ggccttttgt gtctggcatc ctccacacag 13800
gatgatattt tcagagttcg tctatgtttt tgcttgttga tcattccttc attccttttt 13860
ctggctgaat aatactctgt tatatggata taccttattt tgtttatctg ttcatttgat 13920
gggcatttga gtgatttcct ctttttggca attttgaata atgccactat aaacatttat 13980
gtacacgttt ttgtgtgacc atatgttttc acttctctcg ggtgtatatc taaggtacag 14040
ttgctgggtt atatggtagc tctgtctttg actttttgag gaactgccaa gtggttttgg 14100

tagtgattgt actgtttaca ttcctaccaa caattttacc taagtatttc tcaaatctat 14160
ttaatctttt cggtccatac tgctgttgct gccttagttc agattttgtc atttcttgta 14220
ataattcgta gctcatctcc cagtctctgc tcccctctct ccctccctcc cccttcttct 14280
ctctcttatt tccacccatt tttaacattt atagaagtca aaagtctagt tcagaaagca 14340
gaaaccatac tagatatttc agcacagaga actaattagg tgttggaaga ctgaaaggca 14400
aaaaaacact gaagtaacac agtaacatca agaatgggca ctactcctaa gattcaggga 14460
atgctgggaa gatttggggt ttatcagaac tggaagctca gaggaggggc cccttgtcgc 14520
tgaggcttaa tccctgcaga ggtgcctttg gctgctactg gtgaatctga gtgggtatga 14580
tgagtcagtg tctgggaagg gccaaaacat tttgtccctt tctataattt gtcatgataa 14640
tgctagtaat gaatctgatc tcccttccta ttttaaaaac cttttagtga ttttgtatag 14700
gatgaagttt aaaactcctt acttaatata cacatgaccc tccgtaagct ggcccctgct 14760
tgattgtcca gtttcacttc ttggtgctta ttctaaggcc tctaagcctt agagatcctc 14820
taagcctttg agatccccaa accctggact gcggactggt acccacctgt gtggcctgtg 14880
aggaactggg ctgcacagcc ggaaggaggt gagcattact tgccttagct cctgtcagat 14940
cggcagcatt agattctaat aggagcgtga accgtgttgt gaactgccca tgcaaggatc 15000
taggttgcat actccttagg agaatctaac taatgcttga tggtctgagg tgaaacagtt 15060
tcatcctgaa atcaccccca actcggtcct tggaaaaatt gtcttccacg aaactggtcc 15120
ctgatgccgg aaaagttggg gaccgctgtt ctaagctaaa gttatatgga gctccttggt 15180
tctgtgtcct caacatgctg ttctatgttt tttacattct gtttgctcct tcctgcttgg 15240
aatgtccttc ccctccccgt ctttcttaat gcatacaaag ttgatctctc ctgtgtgcca 15300
ccattgtact tcgtcttgca tatggtgtta cattcatttt attttaatta tttatttacg 15360
ttcatgtctc ttccactcac cttagttgct tgaggtcaga aactatataa tgtgtgacac 15420
ggaatgtgac acctagattt tcaataagtg tttctatgat acaagggaga ctgatgtggg 15480
tagatgggaa tgaactcatc aacctctgtt tacataccct aaattccctg tttcttccct 15540
attataattc tgacagtcta caaccgtctt tgatggctta taaacggaaa gtgcggaaca 15600
catcattcta cagtgaattt aaataacctt tcggaagagt aacgtaaagt acttgagcat 15660
taattgagta aaagtttctc atcttttcct acaggtgtta ttaagcagta tgtaaaaagt 15720
ccttacaata cttaatacat taagaaaaca tacaatttca agaggaaatc cccgagtaat 15780
acattattga cattttcagc agttctagtt atattgagaa gagcatctca tggaattggc 15840
agaatgaaga tggagattaa atgagatgat gtttgtaata tgcttatgac agtatctggc 15900
atataagtaa gggctcagta aatgttgact gctgtaatta ctattaatag taatatgatt 15960
acctttagta aaagttatta gtttctttag gttttttgtt tactacaata tagtaaacaa 16020
aatctatact tggaatgtat atattgtttt gttttgatac atggaatatg tctctgtgtc 16080
agagtcactg cctgagttgg aaaacccata ctcgagtatg ttaaaaggtg aacacactga 16140
ataatttagt tattaattat aatggaaaaa tgacaaactt gatgttctgg ttaatgaggt 16200

tatcttatct tgaatgagtt agcttttaaa ttcctcaaaa taaaggcatt taataaacca 16260
ggaaacactt cattaaaaaa attatgcaag tcagtgtaaa agaagattaa aattccacat 16320
gggcaaagga cacacgttgg cgataaatat gcagataaga aaaaaaacct atataacatt 16380
attactcctc aaagaaattg gtatgaaaac aataaaaatg tgtagcttat caaaccaaca 16440
aaaatttaaa aatatgaaat ccattttaag taatgataaa atgggtgcac tcttagtgct 16500
ttatagaata gtagtataat gaacctcatg tgtgtaccaa ccagctcttt catatcttaa 16560
catttagcaa catttgattt agctctttct tttttccaag atagaaaagt taatattgtt 16620
gaagactcct gcattctttt ccctagtctt attttcttcc ctcccataaa tgtgttaaaa 16680
tctctgtgtg tattgttttg gttgtatttt tacataaaac tttacatatt atataaaatt 16740
taattgaagg taaaatttat taaattattc ttaatatata ttgtaattta aaaattaaca 16800
gcttcattgt cttgataaaa tttatggtat cttaaacatg tgcttgtttt tctaagagaa 16860
cattgaaaca tagattttaa aacaaattgt tgaaagatta aaaaatctgc ctttgcacac 16920
tgttacattg aaagtggggc atttgtcgtg aacattcatt tcaaatatgt agtatcttca 16980
gaatatttga gaaggatttg tattatataa ttgaaaaatc tgttaaattg tatttatgtt 17040
aactgcttaa ttctaataaa atttccattc attttttagt atctgcatat atttacatca 17100
aatggattca ttcacttatt taagaggcag tactaattac ctatagcgtt caagactgtt 17160
aggtagaggg tgtgtagtgg tgagtacaac aggcgtgagc cctaccaaca cggagtttaa 17220
agcctagtag aggatataga cttaaacaat ttcacaagta aatacataat tacaaattat 17280
aatacatgct atgaaggaaa cataggaggt accagagaag gaagagtgct ttgcattttt 17340
atttttaaga ccgaagagtg ctattggagg actttgagca agtgaatgac atgatctaac 17400
ctaccttcgt tcattcattc attcattcat tttcttcctt cctggctcaa gcagtcctcc 17460
cacctgagct ccccaaatag ctgggactac aggtacacac taccacacct aatttttttt 17520
tgtatttttt gtatttttga tgggatttta ccatgttggc caggctggtc ttgaactctt 17580
gacctcaggt gatccacctg tctcggcctc ccaaggtgtt gggattatag gtgcctagcc 17640
catggtgcct agccctaacc tacatttata aactatcact tgctgctgtg tggagactat 17700
attgtgagat taacagcagg gatacctgct aggaagcaat tgctgcagat tgcctgagac 17760
aaaatagtta tcatggacta gggggatggt ggtggtggtg gtggtaggtg gttggatgta 17820
ggatatattt tgaagatagg taaatggtgc aagattatgg gtcagtttta aatgcttaag 17880
taaattttct ttgtaagaca ttttaggatg ccatgttaag aatctcttta taactgtcat 17940
ttaaaaaaaa accacatatt ttcttagcat aatttcccat agtaacatta ctatgtcaaa 18000
ggctatgaac atttgaatga ctttagataa atactgtaat tgctttccaa aaatattgtg 18060
cttattatgt caccagaaat gtttgaattc tgtctacaat tcagtcttgc cagtatagta 18120
catttcattt agaaaaattt tttactatgt agatggaaaa aataatattt tagctgggag 18180
tggggggact atggggaata actttccttc atttaatatt ttattgtgag ttagtttaag 18240

ttactttatt ttatcgtagt ttcctaaggc tacaaattag taaccttggt aacttatgta 18300
cctaatttaa aagtttactt ttttgaaagg ctggaaatac taattaaaaa cgtaacacct 18360
tcatccttgt ctttgctcca ttattaacta gtttcattac agaatctctg tgttttaaaa 18420
tcagatgggt tttcataacc agtactttct cagagtggta aatttaaaaa aatatataaa 18480
gagaataaat aatatttgtt gagaatactt caaataatgt gaagagttat taacttacag 18540
caggagttgg caaacttttc tataaagggc catatgggtc tttgtcacaa agtcttgggt 18600
ttttgttttt gtttttttaa acagctattt aactattcct agctaatggg caatacaaaa 18660
acagtgggca agatttggcc tgtgggcagt agcttgctga aaccttattt agactctaaa 18720
ttttttgaaa gagtctacat tgatgcatat ttttttttct tcctccaaat acagttgacc 18780
cttgaacaac atgcgtttga gtgaccatgg gtccacttgt gatacacgtt tttttcccaa 18840
ccaaatgcag atatggaggg ctgacttttc atatacctgg atgttcctgg gccaactgta 18900
ggactagagg ctgggggggt cttggaacca atgccgtgtg tataccaggg atgactgttt 18960
cttatggcct gacctgaagt tggaacagaa tctttattaa tatataattt ttgttgcgtt 19020
tgttttctct ttatatttat ccattctttt tagatcgtat ttcatttaac actttttctt 19080
ctttagtttt taccaagttg cactgaaaat agctcagtga ctaattgcac ttctaagagt 19140
gaggacccta gttaaaatta actctaaaaa tactgaattt ttaacctaaa ccttttattt 19200
ctaatcaaca gtattattta tgagtaggtt atagattact ttgaaacgga atgtgtctca 19260
gaactttgct atcgatattt ttaaggtctg gtagggaaaa gataatagga atgagattta 19320
tcagtgaata ggggactgct ttcccagttt ctcggtcgca ctggtgtatt caccatggaa 19380
gcatcttatg aaatatgtac ataaactact aatatcccac attacaggtt gactattctt 19440
tatctgaaat gcttaggacc tagaagtatt tttggatttt ggtttttcag agtagggata 19500
ctcagcctac attggtaagt aaagaatgtg aggtgacagg ctgggcgcga tggttgacgc 19560
ctgtaatccc agcactttgg gaggccgagg cggatcacct gaggtcagga gttgaagacc 19620
agcctggcca atctgtacta aaaatacaaa aattagctgg acacagtggc acgtgccagt 19680
agtcccagct actcaggagg ctgaggtagg agaatcgctt gaacctggga ggcggaggtt 19740
gcagtgactc gagatcgtgt cactgccctc cagcctaggc aacagagcaa gactccatct 19800
caaaaaaaaa aaaaaaaaaa aaaaaaaaga atgtgaggtg gcagcaatag gtaggaagag 19860
tctttggtca gctttacatg ctctgtagcc atgcctgggt aatgggttga ctctaagact 19920
ctgtgctttg ctcccacctc ctgctttttc attactcttt agaatggttt ttaatttgtg 19980
atctatagga gttctttcaa gtatttaata agagaatagg ctaaattaag taaatgtcaa 20040
ctgaatgctc aaatctctac taaagagcct cttatttaga aaataaatat ccatcttttt 20100
tttctgactg gtgagataat taatttttat tacagatggt ttggaaaata ccatatgctt 20160
taaaagataa gcacaaaatt atagtctaat atgtaggttt tcatacttta aaaaattgaa 20220
aaccaaagaa aaacatttaa catagcatct agtacaaaga aaagagataa gcaagagata 20280
aatgtctttt ttgggacaga gttttgctgt tgttgcccag gctggagtgc aatggcacaa 20340

tctcagctca ccgtaacctc cacctcccgg gttcaagtga ttctcctgcc tcagcctccc 20400
gagtagctgg gattacagtc atgcaccacc aggcccaggt aattttgtat gtttagtaga 20460
gatggggttt ctccgtgttg gtcaggctga tctcaaactc ccgacctcag gtgatctgcc 20520
caccttggcc tcccaaagtg ctgggattac agacatgagc catcgcaccc ggccaagata 20580
aatgtctttt aaattatctc cattaaagac ataaccttta taacattttg atgtatatat 20640
taccagtttt taaacacata gtagatttgt ataaatacat aaacacatat tattgtgatc 20700
atgctgcact tagacatctt tatattctcc ttatactgta aacattttga aatactttac 20760
taacaacatt tgtaatgacc attctttctc tctttctccc tctgatagaa tggtctacag 20820
agtaattcat aaactaaaca tactttagag gctgggcgca gtggctcatg cctgtaatcc 20880
cagcactttg agaggctgag gcgtgcagat cacgaggtca ggagttagag accagcctga 20940
ctaacatggt gaaaccccat ctctactaaa aaaacagtac aaaaattagc cgggcgtggt 21000
ggcgtgcacc tagaatccca gctactcaag aggctgaggc aggagaatca ctcgagccca 21060
ggaggcagag gttgtagtga gccgagattg caccacagca ctccagcctg ggcgacagag 21120
cgagactcca tctcaaaaaa aaaaaaaaaa gatacattaa tactatagcc tacatgtgga 21180
acattaagaa aataattgct tttatgttta tgctttatac ctgttgttag ccctgcttct 21240
tatttcatga tttcatggct tcacattgta acatcccttt accatatttt ttgaggactg 21300
ttttggcaga atgtgtgaaa tcttgagcag aagtattacc caaaagtcag aagaaaatca 21360
gatttttatt tcaagattct gttaaagtta cccactccct tcttttactt aatcttatag 21420
ttgcagttct ctctcttttt agaaaagaaa aaagaggccc ctcaggattt gcagatgaaa 21480
caatattgct ctttagagat atccatctgg ctgttagatt atttttccac agttttcaga 21540
agtggatgag gccattagaa tcttgagtat tgcccatttc cttatgtgtg cctttgacta 21600
tagataaaat agatgcatga caattattta taagttgatt gatttttctt gtcatttaaa 21660
tcatcttgaa taatagagtt ggtagagcta tcccattttt gaaattattt tgttttgtca 21720
ataacttttt gttaccagca tgtacacttg cattgttgac tctccatata atacctttaa 21780
aaaatttttt tttgtggtaa aatatgcata acataaagtt taccatggta gttttctttc 21840
atttgttttg tttttgtttt tttgagacgg agccttgctc tgttgccagg ctggagtgca 21900
gtggagcgat cttggctcac tgcaacctcc gcctcccggg ttcaagcaat tcccctgcct 21960
cagcctcctg agtagctggg actacaggcg cccgccacca cgcccggcta atattttgta 22020
ttttaataga gatggggttt caccatgttg gccaggatgt tcttgatctc ctgacctcat 22080
gatccgccca cctcggcctc ccaaagtgtt gggattgcaa gtgtgagcca ccgcgcctag 22140
accatggtag ttaattttaa gtgttcaatt cagtgacctt aagtgtgttc ataatgttgt 22200
gcaaccatca ccatgttgtc taaccattag cactatctgt tttgagaact tttttttatc 22260
atcccaaatt agaattctgt acctgtcaaa tagtccccag taatcctccc tcccccagcc 22320
cctggtaatc tgtagtctac ttttcgtctt tttgaatttg cctattttag gttcctcata 22380

taagtggaat tatgtggtat ttgtcctttt gtgttggctt acttcattta gcataatgtt 22440
ttcaaggttc atctgtgttg tagcatgtat atacaggttg aagcatccgt tatccaaaat 22500
ggttgtgacc agaagtggtt tggatttcag attttttttt tggattttgg aatattcata 22560
gatacttaac tggttcagca tccctcgtcc aaaaatccaa aatcagatgg agctcagtgg 22620
ctcatgcttg taatcccaac acgttgggtg gccaaggcag gaggatcgct tgagcccagg 22680
agttcaacca gcctgagcaa cacaagaccc tatctctcca aaaaaaaaaa aaaaaaaaaa 22740
aagatgaaag aaaaaaaaat ccaaaatcaa atgctccagt gagcatttcc ttttagcatc 22800
atgtcaggct ctaaaagtta caggttttgg agcattttgg atttcagatt tttggattaa 22860
cctgcattaa tgctcaacct atatgaaatt ttattccttt ttatggctga ataatgttcc 22920
actgtatgta tatactacat tttgtttatc cattcatctg ttaacagaca cttaagttat 22980
ttccacattt tgggtattat aaatagtgct gctgcgaaca ttggtgtaca tgtatctgtt 23040
tgagtccctg tttttagtta ttttggttat atacctagga atggaattgc tgatcatatg 23100
gtaattctgt gtttaacttt ttgaggaact accactgttt tccacaatgg catcaccatt 23160
ttacattccc accagcaatg cacaaagatt tcagtgtctg tatccttgct aacacttatt 23220
ttccattttt tgagtttttt tgttttgttt ttttaataat agccaatcct aatgggtatg 23280
tggtagcatc tcatggtttt gattttattt tcctgactat tgatgatgtt gagcatcttt 23340
tcaggtgctt agtggccatt tgtccgtcat ctttggagca ggaacaatgt cttttcaagt 23400
cctttgccca tttttaaatt gaattttttg ttgttgagtt gtatataaca ccttttttga 23460
agtaaaaggt gcactgtaat aatccagact gtgtttctcc cttctcagga ttcctacagg 23520
aagcaagtag taattgatgg agaaacctgt ctcttggata ttctcgacac agcaggtcaa 23580
gaggagtaca gtgcaatgag ggaccagtac atgaggactg gggagggctt tctttgtgta 23640
tttgccataa ataatactaa atcatttgaa gatattcacc attataggtg ggtttaaatt 23700
gaatataata agctgacatt aaggagtaat tatagttttt attttttgag tctttgctaa 23760
tgccatgcat ataatattta ataaaaattt ttaaataatg tttatgaggt aggtaatatc 23820
cctgttttat aaatgaagtt cttgggggat tagagcagtg gagtaacttg ctccagactg 23880
catcggtagt ggtggtgctg ggattgaaac ctaggcctgt ttgactccac agccttctgt 23940
actcttgact attctacaaa agcaagactt taaacttttt agatacatca ttaaaaaaga 24000
aaaccataaa aaagaatatg aaaagatgat ttgagatggt gtcactttaa cagtcttaaa 24060
agcaatcgtg tgtatagcat agaattgctt ggattggata aacagtggca ttatatattt 24120
taaaaaataa aagttttgaa agattgaaga atttgggcat tacagttctc ttaaatctga 24180
caaagctgca taaaactatt aaaataatca ttattatact attttatatt ctatttcttt 24240
gagggtttag ttttccaaaa actacatatt aagcaaatga atcactcagt ggctatgtca 24300
tataataacg agttagccta gttataagaa gtttaacatt ttatttaaga acattgttac 24360
agcatgttta ctgtatagtc tagtaataga ggaaaagaca tttgggtggg tggtagtggt 24420
agtattttta tagaggagtt accaaatttc agctctatta tccaagttta cccagctaat 24480

ggtgttcgga accgggaatt tgagccaatt ctgactctgt tgtctgctct gctccttctt 24540
ttgtgctgtg tctttgaaag tcacctaaaa ttgtgaggga atgtaatttc accccaaatt 24600
tagagtttat gcacttgtta tattgaaaat gattaacatg tagaagggct tttaatggaa 24660
taagtggtgt agtaacttca gtgttgccta cctagaaatc aaaatctttc tagttgtcca 24720
ctttgttttt tgaaaaagta atatgaaaat tatgttaatg ctttaattca ggtttttgta 24780
aaatattttt tatctttaca catttaacat acgtttctaa aattatagtc tgttatatag 24840
cactttgggt ctagaatttt tcagtagttt ctgttttact attatgatct acctgcatat 24900
taacctatta ggttatagtt ttactatact tctaggtatt tgatcttttg agagagatac 24960
aaggtttctg tttaaaaagg taaagaaaca aaataactag tagaagaagg aaggaaaatt 25020
tggtgtagtg gaaactagga attacattgt tttctttcag ccaaatttta tgacaaaagt 25080
tgtggacagg ttttgaaaga tatttgtgtt actaatgact gtgctataac ttttttttct 25140
ttcccagaga acaaattaaa agagttaagg actctgaaga tgtacctatg gtcctagtag 25200
gaaataaatg tgatttgcct tctagaacag tagacacaaa acaggctcag gacttagcaa 25260
gaagttatgg aattcctttt attgaaacat cagcaaagac aagacaggta agtaacactg 25320
aaataaatac agatctgttt tctgcaaaat cataactgtt atgtcattta atatatcagt 25380
ttttctctca attatgctat actaggaaat aaaacaatat ttagtaaatg tttttgtctc 25440
ttgagagggc attgcttctt aatccagtgt ccatggtact gcttttggct ttggtttctt 25500
tctacattga aaatttctct tcaattctga gcacatgtta acatttagaa ttcaagaggt 25560
ggggattttt ttttcccatg gttacatata tatatatata tatatatata tatatatata 25620
tatatatata tataaagaac agggcaacaa atttttgcgt tttctatttc ggtagtactt 25680
ttaaaccatt atgtcatgtt tctaggttaa acgttgttgt atttgaagaa ttttactttg 25740
gcagaatttt tttgaggatg tgtttatttc tggagaaagg tctcattaaa gaaagacaat 25800
acccagaaag ccaacagaaa ttctgttact catttaatgc atttttctga caaaaattat 25860
tgccagagag aacctgaatt ttgtttcaaa aatcatcttt gttttaaaaa tgactttttc 25920
ttcaggtaaa ataaaataat ttcagttgct attatttaac ctgtttgtat gaagagttta 25980
acatatagga aatgaataca taaagatagg aaggaattaa ttgttatatg tagtcatatg 26040
tctcttaatg acagggatac tttctaagaa atacattgtt aggtgatttt gtcattgtgc 26100
aaacatcata gaatatactt acacaaacct tggtagtata acctactata cacctgggat 26160
atgtagtata gtctcttgcc ccagggatac aaacctgtac agtatgtaac tgtactaatg 26220
actataaggc aattgttaac acaatggtaa gttttgtgtg tctaaaccta cacttgggct 26280
accctaagtt tatatatttt tttaaatttc tgttcaataa taaattaacc ttactttact 26340
gtaacttttt aaacttttta attttcccta acattttgac ttttgtaata cagcttaaaa 26400
cacacattat acagctatac aaatttttct ttccttatat ctttattctg taagcttttt 26460
tccatattta aaattttttg tttgttttta cttattaaac ttttttgtta aaaactaaga 26520

catgcatgca cattaaccta ggcctacaca gggtcaggac catcaatatc attgtcttcc 26580
acttccacat cttgtcccac tggaagatct tcaggggcag taacacacgt ggagctgtca 26640
tctcctataa taacattgcc ttcttttgga atacctcctg aaggacctat ccaaggctgt 26700
ttatagttaa cttttttttt tttttttttt tttttttagt aaataggagg agtacactat 26760
aaaataacaa tataggtgct ataccattat acaactgaca gtgcagtagg tttgtttaca 26820
ccagcatcac cacaaacacg tgagcaatgt gtcgtactac agtgttagga tggctataac 26880
atcactaagc aataggaact tttaaactcc attataatct tatgggacca ctatcacata 26940
tgcaatctcc tgtggaccaa aatgtcatta tgtggtacat gactgtacta agaaattgat 27000
ccatctatat tccatcaatt tgtttagggc tttttctggt tacatttacc tgtgagccca 27060
gaaaaccagt tttgtagaaa ttaacttctg taatgctagg agttaaaaaa aattgctgaa 27120
caacttttac attgttaaac atttaaaaac aagcgttcta gaagtttatc aaatttcata 27180
aaggtgcaaa aatgtaaatg taaatcatta tccagctaat atatatgttg tatttcccta 27240
gtaggagagc atatgtacct cttcctagtt atacaaattt gatatatagt aaagaaacag 27300
taaattctac ttcaagtcat tttgggagga ttaaaaactg aatttctcta gtttgaccat 27360
tgtacagatt tatctggcaa ttttactaaa acctgattta taggttaaac ttggtgtata 27420
tcatatatca ctttacttta gaggaattaa gatttcacat aaatccattt ccaggttcca 27480
aagaccagga agaggcttgg tttttgtttt tctttttact gtctttacag tctccttgac 27540
ttttcttagg agagaaggta ctgagaaaac atgattctaa tatttattat tttttcttcc 27600
aacattttct tatgaaacat tttcaaatac aaaattgagt tttatttaaa acatttgcaa 27660
atatactacc tagattctac cattgttgtt ttatatttgc tttacttaca acttttaaaa 27720
gatgcttttt ataccactga acattttagc ttacatttca caaagaaaag aaaaaattta 27780
agagactttg cataatgttt taaggggttg cagtaaagaa gtgcttctta tattttctta 27840
tgcatacaaa tcagctgggc ttattaaaat ccagattcta attcagaagg tttaggtggg 27900
gaccgagtct gcatttctaa caaactccta ggtggtattt ttcttggtac ttggaccata 27960
ctttgagtag aaaagcagta gaggacataa aaagagtctt gttagtccca ctttgttgct 28020
gtccacttct catttgataa tatcctaaaa tagctgtgtc tcctttttgg tggttgtatg 28080
attactacct cagaagtact aattgattct tgctatttga ccttaatact ttaatataac 28140
acagcattca tatttgatca gaaaactatc tggcttcctt ttataagaga tttttaggtt 28200
ttatacagtt ttgtggcctt gggttttttt gtttgatttg tttttttgaa ggtatataat 28260
atgtaagtag ataaacaaat ttgatttgta gacattttta tgtggatcat ctaattaaaa 28320
atggagggat acagtatgaa agaatacttg tacttcttaa cagagcactc aacctttctt 28380
ttacatcctg tttcactgat gttattatgt aatttatgtt gctaaactat aaattagata 28440
tttaatttct gttctttgat ttccttttat tattaaatgg acttgttgat ttgcctagaa 28500
attaatttgc ctttcaaaag tcttattaat cttcctccgt tgaaattaat ttgatatttg 28560
catgcttctg gaagacttta aagagctatt ccgagtaact gtagagatta taaaatgaaa 28620

tatgggaatt ttaataaatt ttacatctcc agttactggt gaaaatgtca agtcctcctt 28680
tctgcagagt attttgttac tcatctgtta ttcagcttat ttatttattt atttatttat 28740
ttatttttct ttctttcttg tttttttttt ttgagacgga gtcttgcttt gtcgcccagg 28800
ctggagtaca gtggtgggat cttggctcac tgcaggctcc gcctcccggg ttcacaccat 28860
tcttctgcct cagcctccca agtagctggg actacaggca cccgccacca tgccttgcta 28920
aatttttgta tttttagtag agacgggttt cactgtgtta gccaggatgg tctcgatctc 28980
ttgacctcgt gatccacctg cctcggcctc ccaaagtgct gggattacag gcatgagcca 29040
ccgcgcctgg cccttatttg ttttttaaac aaaattagtg tgcatatcct tgttgtattt 29100
tatcggcaag ttgttttatg ccctaacttt tggggtcttg atcatgagcc taaaacacgt 29160
aaacacccaa aaagaattat attccggtta aaggaacaaa acattcattt agaagttctc 29220
atccatgtaa atcagaggct ggcaaatatt ttctgtaaag ggccaagata gtaaatgttt 29280
taggctttga gggccacaag tggtatctgt tgcatttttt tttaattatg accctttaaa 29340
atgcaaaaat cgttgttagc ttgtgcatag tataaaaata ggctggccgc atgctgtggc 29400
tcatgcctgt aatcccagaa atgaggtggg aagccgaggt gggcacacca cctgaggtca 29460
ggagttcgag gccagcctgg ccaacgtggt tgaaaccccg tctctactaa aaatacaaaa 29520
cttagccagg cgtggtggcg ggtgcctgtt atcctggcta ctcaaggggc tgaggcagta 29580
gaattgcttg aacctgagag gcagaggctg tagtgagccc agatcaagcc agtgcacacc 29640
agcctggacg accgagcgag actctgtctc aaaaaaaaaa aaaaaaggct gtggctgcat 29700
ttggtccatt ggctgtaata tgctgattcc taattctctg ggtaacttta gtgtttgatt 29760
agctactaga agttaggtta aacttttgta ttttacaggc taactttaat aatcttaaag 29820
taaaacttaa catagttcat ggaaaggaaa tagaaatttt accctagtac tctttttttt 29880
tttttttttt ttttttgagg cagagtctcc ctctgtcacc caggctggag tgcagtggtg 29940
ggatcttggc tgattgcaac ctcctcctcc tgggttcaag caattcttgt gcctcagcct 30000
cccgagcagc tgggactaca ggcacgcacc accacacctg actgattttt gtatttttag 30060
tagagacagg gtttcgccat gttggccagg ctggtcttga actcctggca tcaagtgatc 30120
ctcccatctg agcctcccag tgtgctggga ttacagacgt gagtcactgt gcctggtctc 30180
tagtattttt tttttttttg agacggtctc actgttgcca ggctggagtg cagtggcgcg 30240
atcctggctc actgcaacct ccgcttcccg gattcaagcg attttcctgc ctcagcctcc 30300
tgagtagctg ggactatggg tgcacaccac cacgcccagc taatttttgt atttttagta 30360
gagacggggt ttcaccatgt tggccaatat ggtctcaatc tcttgacctc gtgatctgcc 30420
cgtctcggcc tcccaaagtg ctgggattac aggcgtgagc cactgtgccc agctgtactt 30480
tttaagataa gaattgcagg gtatatattt ttaccaactt aataacttat aattttaaaa 30540
agctaattac ttggctagaa tataatgcgt tacatattct ttacactcag ttcagtccat 30600
atctgaaagg caaatagaat tattttctgc tagtacattg tgtagtccct atgttcctag 30660

tgtataagga ctgttaccta gttcacattt atctgggttg ttgacagatt ttcctggtcc 30720
ctttggacag tgcatggcca tgttggcaaa agctgtcaaa attgaaacat tgacaccatg 30780
agaattgtgt gttttccagt ctgctaaaat caaaagtggg agggttcagt aaggtgaata 30840
acagaagcag agttttcggg gtatctgtta ctcctcattc ggcttttctg ctctctgggg 30900
gtctcaattt aaatataatg tgaaaattag ttttacgaac ctaaaaatgt tgagtgattc 30960
atttcctggt tttgttgtta atttctagat atttaaatta attgttagaa gaaccccgtt 31020
aaagaatgct ttgcaaaaca acctccttat gtgctatgtc tctgtttaat agtagttgag 31080
tttgtgtaca tgagatcaat attttgaact atagcttttt atgagttaaa aattgacgga 31140
acagttactg tgcacttgct gtgcaccatg gtagtctccc aagtagtggt ttttctgcat 31200
ttcaatagta catgagatag gctgtgggtg gcaaggtttc ttgagaaagt gagggatgca 31260
cagttgggtt ttagaataca tcttgttcct ccatgccctt ccccaccaaa aggctggtag 31320
tcttgcattt gtatatagtt agggtatttg atgtgttgct tccttgacag agttttgcaa 31380
gaatttgcag atttaacagg aacaaaaact tacttaaaac aaaatctctt agtaaaagca 31440
tagtctagca agatttagaa tgatactttg gctaacagta ctttctctat atggagtgct 31500
ttgtttccat agcctcacaa gtatgttttc agataatagt tgagttgaaa atgttgtcaa 31560
tctcttgatt ttaaaaaatt tacatattta aagttgtata cttttgttcc tacgtatttt 31620
cagttgttct taaagtttaa taagtgacat ttgaaaatga gtatatgtgt ataaaaacaa 31680
aagtaggcta ggcacggtgg ctcatgccta taatcctagc actttgggag gctgaggcag 31740
gcggatcaca aggtcaggag tttgagacca gcctgggcaa tatggtgaaa cccccctcta 31800
ctaaaaatac aaaaattagc tgggtgtggt ggtgcatgcc tgtagtccca gctactcagg 31860
aggctgaggc aggagaatcg cttgaacccg gaggtggcgg ttgcagtgag ccgagattgc 31920
accactgcag tccagcctgg gcggcagagc gagactccat ctcaaaaaaa aaaaacaaaa 31980
aaagaaaaag ttaaaaaaaa acaaaaaacc cccacaaaat gagtatatgt ggcaacaagt 32040
cctattctca aaaaaattat tgtgtgctag ttaagagctt aatgagtagc cagtcggtat 32100
taaatatctg tttcagctat attttatctt taaaaattat ctacagattt tggaatgtga 32160
aaaactagtg ttttgtttca taggtatata ctgtaggcat tttaaaaata agagccagtg 32220
ccagtggttt acagtgtaca caaggataat gttctcatgt tctcttgatg tcagtatgac 32280
tttaaagcat attatcaaga aataactaag tctgaaaaac tgtggtaaat aactggtact 32340
ctaaaaccta agtttcttat tactaaaaat aagaaatggt aaaagtcacc ctgtgctgtt 32400
aattatatga gccactgagg tcctgacact gaattcttgg tggtggataa taatctcttc 32460
tttttaatta ttggcttcca attctctctg cattgctgga aacaaaaatc atatatttca 32520
ctattggtgg tggggatgct gtcactgaaa aagtagacac attcatattg attttagaaa 32580
taagttaaaa tcaaaatttg cttctgctaa attagtagag gaccaatact gtttttctcc 32640
ttcatagtat gttttggtac ttctacattg acattataac tttttttttt ttaaacagaa 32700
atagaagttt acattcttag aaaatttatg aaaatatgag cttttacctg gtttgtgtgt 32760

gtgcgtatat atatacacat atttttaaat ttcttacatt gattttcaaa ttgaaagaga 32820
accatttgtg aaagtatctt aacagagctc atgctttaca ttttacatgc tacaaagtta 32880
ttttagtgcc ttaaattatt tatgttgctt attaatgaaa attttggata cataattttt 32940
tcaagacaaa ggtaaaaata ataaaccctt tccttctgag gattaatgat aaatataaac 33000
tttaaaacga ttaaaaaaat ttttttagag acagggtctt gctctgttgc ccagactgaa 33060
gtgcagtggt gcagtcatag ctcaatgaag cctcaaactc ctgggcccag gcaaccctcc 33120
tgcctcagcc ttttgagtag ctgggacttc aggctcatgc caacatgcct aatttatctt 33180
atttttagta gagatgaggt ctcaaactcc tggcatctct tgccctctca aagtgctggt 33240
actacaggca ttagtcacca cacctgacac ttaaaatctt ttatatacag gtgtaagtgg 33300
gtatctaact taaagtgcca acgaatgtag ttgaaagttt gtagttggct tagctaacta 33360
gttaactaaa ttgattccat taaaaataag ataagactgc tcttagaata taatgatttt 33420
tgttattcgt taaatataaa tatatcactg gatagtatat gttaatgact tgagatacgc 33480
attttaacat ataatcacgt tacttaaatg cctgcctttg aactgaaact taacattatg 33540
aatttaaatt aaagtttgac tttagaggta aatttctgta ctttactaaa gcagttctta 33600
atataattct gagatttcta aaaattagtg tgccctaaag aattgaggtg tgtttttctt 33660
aactactgta ggcagtagat gtacagatga cttctgcatg caaaaattaa gccctagcca 33720
ttggtttact tcaactaata cttagttgcc aattctctgt gtgtgattga atttaaaact 33780
gcaaatggta ctggtgatac attaactttt taggtgctag gtccactttg ttacatttgg 33840
ttcagtagaa acattgatgt taccaatctc agaaagctaa aatatgtatg ccaatcccca 33900
aattaggtaa tttattctta attttaagat aaaagaatag aattccctta aaattaaatg 33960
tggagtaaaa tataccagct ttaaaaaata ttcacctttc tgttagaaga atgaacataa 34020
tattacatct tttaatttgc actatatata gattaatatt tctgtgtatt tctctgtgcc 34080
cctactttga tggtatgctt ttctgaacaa actagcagca cagttaacta agcactttgc 34140
cccgtttgat gactgcctaa ttttctagat tggaaaatat taaaaacttt tatctccata 34200
tggccaatat atgattgtac ctgttgtcat agctctctta tgtttaagca agaaaaaccc 34260
tattaagagt atttaaatta gaatggaagg cacacagcca gtatgattga acactgttct 34320
aaaaattatt tttaagactt gtagtaaggc caggtttggt ggctcatggc tgtaatccca 34380
gcccttagga ggccaaggtg ggcggatcac ttgtgctcag gagtttgaga ccagcccggg 34440
caacatggca aaaccctgtc tctacgaaaa atacaaaaat cagtcaggtg tggtggtgct 34500
tgcctgtagt cccagctatt tgagaggctg aggcaggggg atcacctagc ctgggaggtc 34560
gaggctgcag tcatgatcgt gccattgcac tccatcctgg gcaacccagt gagaccctgt 34620
ctctaaaaca aaaaaataaa aaaagaactt gtagtaagga tacaaaatgc tcctattttg 34680
tgtgtgtcct ttaattcatg atgtttttat attatggtaa gcagctctca tttaagattt 34740
taataatgta attaaacatg tacagaagac ccagtctcag cttcacttgt ataccctgga 34800

aatagactga aaggtgttaa aatttaagat aaaactcaag gttccagttt cttgactcac 34860
ctttgagatt cttttatgtt tttgttgttt tttaacaaag gtttcacgtc catattttac 34920
catttttctt ctcattctcc cctggaggag ggtgtgggaa tcgatagtat ataaatcact 34980
tttttcctaa gtcaaagaag taatttaaag ctaacttcag tttaggcttt aattccagga 35040
ctagcaaact aaaatggttg cattaattga caaacagatg ctaatacctg tgtttaggct 35100
tgtcataatc tctcctaatt cctaatttaa aaattttaaa atttaattcc attagaaaac 35160
aaaactgact tttaagaaca aaccaggatt ctagcccata ttttaaaact gcatcctcag 35220
ttttattcaa acagtctgat gtctgtttaa aaaaaaaaaa atctcaagct cataatctca 35280
aacttcttgc acatggcttt cccagtaaat tactcttacc aatgcaacag actttaaaga 35340
agttgtgttt tacaatgcag agagtggagg atgcttttta tacattggtg agggagatcc 35400
gacaatacag attgaaaaaa atcagcaaag aagaaaagac tcctggctgt gtgaaaatta 35460
aaaaatgcat tataatgtaa tctggtaagt ttaagttcag cacattaatt ttggcagaaa 35520
gcagatgtct tttaaaggta acaaggtggc aaccacttta gaactactta ggtgtagtat 35580
tctaacttga agtattaaaa gataagaaac ttgtttccat aattagtaca tttattttta 35640
atctagtggg aattaattat aattgagaca attttgatgg ctgtagtaga ctaatctata 35700
tttggcataa agtctaatga tttaatgagt cttaagtaaa ctaaatattt ggaaactgat 35760
atttaccttt atttttaagg gaaaagtttt gagataatca gcagcttttt tttttttttt 35820
ttttttttta gtagggagaa aaagatatga gctatagtag acagcagtaa tattgaatgg 35880
cccagaaggt gggaaaaagc cactcttaaa tgtatttttt cttttggata ttttacaagc 35940
aaataataac ttctgcctaa gttcgccatc tcagtggcat cagcagcaca gcactttctt 36000
atcccagtga gaaacctggg aattttagga tgactcctac cgccctcttt tccccctggt 36060
ttggaagtat ccacaaattc ctgtgacgtt acattctgtg tcttttatgt catcattagt 36120
tcaggcccct atcatttctt gttggactgt tagaacctcc tatttggttt accagttgct 36180
gccatcattc attgtgaaac cggagagata cactttaaag aaatgtcatt tttggccggg 36240
cgcggtggct cacgcctgta atcccagcac tttgggaggc ctaggcgggt gatcacctga 36300
ggtcaggagt tcaagaccag cctggctaac atggtgaaac cctatttcta ctaaaaatac 36360
aaaaaattag ccgggcgtgg tggcacgtgc ctgtaatccc agctacttgg gaggctgagg 36420
caggagaatt gcttgaacct gggaggcaga ggttgcagtg agctgagaat gcaccattgc 36480
actccagctt gagcaacaag agcgaaactc tgtctcaaaa aaaaaaaaaa aaagtcattt 36540
tagctataga ataaaatctc atgttccaca tgtgttgcag atagtcctta ctaccttccc 36600
accactccag ctcttttttg gtcttatatc taaaaacgtc atcttgcctg aatttctttt 36660
gttcttctat aaataaatac catgttattt cctaccttcc cttgagtctt ggctcttgtt 36720
tggaatgcca gtatttttat ccctagtctt actaattagc taacactctc atgattcccc 36780
agtctcctac tctctaaaaa cctttcttta aacccttaga ctaggcatgg agcccttcct 36840
gtgtattccc agaatactat tcttaactat tatatgcttc ccatgttatg ttgaaataac 36900

taacctcttc tgtttcattc ctatattact tgacagcaaa atcttagcca gaattacata 36960
tttttaatct ttgcacaccc attgcctagt aaggttcctg ggacatagta actacccagt 37020
aaatatttat tgcgtggaat tctcattttc gtttctaaac ccgtattaaa ctctgtcttg 37080
ctcagaaaat acttcactag gtatcataaa gttcatggca gagcttaagc tttggatgca 37140
tattgtttgt aatatatcat gttcttaaga ataggcaata aaattacagt tttcaaaaac 37200
tactacattt attatattta ttacaagttg gtgttcttta ttacatgaat tttaggtatt 37260
tcccaaaagt ataaaatata catttgaata gtagactcaa tcccaaaaga tactacgtgg 37320
tgtactaatc tactaaactc agaaacaaag catgactggc attaattttt gttgaaattt 37380
atgaactctg aatgtttttg aatatcattc tgtaaagcaa tattttgcaa ttaaagcaat 37440
tttgcatgtt aaattttacc acaacctcta aaatattgca aatttaacaa tacagtttga 37500
aaagttacac attttaaata acagtaccat gaccagattt aggtggtggt tttaattttt 37560
tattttctcc tcctattgtc tcaccattag atgattttaa aaatagaatt gtttagagta 37620
aaataagtgt tatgctctaa tttatattta aaatgaaggt ttaagcacgt actattctaa 37680
aatttctaat ttgtgcaaat tatgttttat acagtgactg taggtgaatg tcacaattgt 37740
ttgatgtgac gaatccttgt ttttcagtac acgtggaagt aattcatata aaagagaagt 37800
atacttggta attaaaaatt taaaattaaa tacaatttaa aaaaaaattt atttgacaag 37860
ctggctgtgg tgtgtgtgcc tgtagtatca gctgcttggg agcctgaggc aggaggattg 37920
cctgacccca ggagtttgag gttgaaggga gctatgatgg tgccatggca ctgtagccta 37980
ggcaacagaa agagactcca tctcttaaaa aaagtaaaaa taaaaaaatt ttggcacagg 38040
gacagtggct cacacttata atgccagaac tttaggagtc cacagcgcga ggactgcttg 38100
aggccaggag tttaagacca gactgggcaa cgtaatgaga ccccaccttt aggaaataaa 38160
tacataaata aaaatttgac aatgataaac atatataaat tagcttttct tagtcctgaa 38220
aaagataatg ttatgtgtat gtgtgagaat gattagttct catatgagaa aaaaagaatt 38280
cattgctctg tgtaggttgt gacatttcct tcacgattga aattaattaa ttttttttta 38340
ttacttattt atttttaaaa tagagacagg ttcttgctgt gttgcccagg ctggtctcaa 38400
actcctggcc tcaagcagtt ctcctgcctc agcctcccaa attgctgtga ctgtaggtgt 38460
gagccactgc actgggccaa aattacttaa ttttaacaag atgatgtaga gaggagagtt 38520
cattgcaaca taagcctaga atctttgtca gaatcttagg aagtaatgtt ttcaaattct 38580
gtgttttcac cataaaatgt gtcttctctg tgtccatcac atggtttttc attgttttct 38640
gctttaccat tttagtacca ttggcatttt tcttcattgt aaaagtagta gaaatggagt 38700
agattacata aggatgtgat cagagggaat ttattcattc agggtaaggg agttagatcc 38760
tcttttaaga ttctatcaca ttctaagggt ttatgattct aaactgtcaa gtaaattgtc 38820
aagtgctggc aagctacaga ataattttta ttgtatcatt ggaaattttc ccctctatat 38880
gtgttaaaga gtttagcctg aagggataca tacacataca tatatgtaat caaaccttga 38940

tggtattgta ttgctgataa attatttctt accacttttc ctttctcctg tgggagaaac 39000
aaaagcatat gtttgtgtag tatcagtaat gatattagag agtgggaaac atcagtgagt 39060
gcagtttggg gactttattg gagactttca ctagtgctca aataaataat gctggttttt 39120
atcctactgt ttgcttaatg tggactagcc tcttattccc attctatgtt tacctctctt 39180
aaaatattgg tcacgctttc ttgaattata gatctattag gaaaattcat gaactgtagc 39240
taattttcat tgttcatgct ccagatttat tttgaaatat cgttaatctt agtagtacag 39300
taaaggagaa ataccactta acattttttg tttttttttc tttgagacag agtcatgctc 39360
tgtcacccag tctggagtgc agtggtgcta tctcggctca ctgcaatgca cttcgcctct 39420
ccgggttcag caattctcct gcctcagcct cctgagtagc tgggattaca ggcacctgct 39480
accacaccca gctaattttt gtatttttag tagagacagg gtttcaccat gttggccagg 39540
ctggtctgaa actcctcacc tcaagtgatc cacccgtctt ggcctcccaa agtgctggga 39600
ttacaggctt gagccaccgc accccgccca cttaacattt taaattaatt tcaagataat 39660
atcacttgaa tatttttaca catataattt ttttaataca tttatttaca cagtttataa 39720
tatcctacaa agtgattaca atgagtaaaa acccagtttt cattgttcct aaagtggctt 39780
gatttataca acttaatgtg ttgggtattt gtttctaaga ctccctctgc tgtctaggtt 39840
tggaagtatt gtgaggttaa cagattttct ttttatagtt actactcagt tgaacaggct 39900
ttaaaataca gagagaatca tattttttct tcattttttg cttttattta tatttttctt 39960
ttaattggag acatgacaag aattgacttg tgtatggatc ttgcataatt taagtactgc 40020
aggtttaaaa tctactacca gtttgagagt gccatttttc acactgtaga ttattaggtt 40080
gaaaagtatt atggcttaaa atcgctttta gccattaaat ttaaataacc ttgctttaat 40140
cataaataga tggtggtcac aatgactaac tgttaaactc tttgaagaca ggatatttgg 40200
ctttatatgg caagcttttg aatacaacag aaattaaaac tttatgggat agaaagaatc 40260
tcctccaaat tggtaaacta taagaccttt caaatgattt agctaatttc tccacaaatc 40320
tgaggtatta gtgttttttt taaagtggta ttctcctgtg ttggggtcac tttaaacctt 40380
tttcttaatg ataaatatat gaattgaaac taatccctta atatatatca tttgaaaact 40440
gaaataatat gtttagatac tgtttacttg ttgataaatt attggaatag gatgttcgaa 40500
tactgtttac ttcttggtaa atttttaaat ccaatggatt ttacgtaagt atagaactgg 40560
agctcaaata ctgttactgt gtgtgaagat atatgaacat agtttacagt tgcatggctt 40620
atatctaaag tccagaaaca taaggacaat taagtgtaca cacacacaca tgcatttgga 40680
ttttgatgac ttaggtttgc caatgtggaa aaaatagtag caaattaagt tctcctgtga 40740
aaaagtcgtt accttattta aaattctgtg ccattggtta tccttgtctt ttgtgaaaat 40800
tagtgttcct gtttataata ttgacaaaac acctatgcgg atgacattta agaattctaa 40860
aagtcctaat atatgtaata tatattcagt tgcctgaaga gaaacataaa gaatcctttc 40920
ttaatatttt ttccattaat gaaatttgtt acctgtacac atgaagccat cgtatatatt 40980
cacattttaa tactttttat gtatttcagg gtgttgatga tgccttctat acattagttc 41040

gagaaattcg aaaacataaa gaaaagatga gcaaagatgg taaaaagaag aaaaagaagt 41100
caaagacaaa gtgtgtaatt atgtaaatac aatttgtact tttttcttaa ggcatactag 41160
tacaagtggt aatttttgta cattacacta aattattagc atttgtttta gcattaccta 41220
atttttttcc tgctccatgc agactgttag cttttacctt aaatgcttat tttaaaatga 41280
cagtggaagt ttttttttcc tctaagtgcc agtattccca gagttttggt ttttgaacta 41340
gcaatgcctg tgaaaaagaa actgaatacc taagatttct gtcttggggc ttttggtgca 41400
tgcagttgat tacttcttat ttttcttacc aattgtgaat gttggtgtga aacaaattaa 41460
tgaagctttt gaatcatccc tattctgtgt tttatctagt cacataaatg gattaattac 41520
taatttcagt tgagaccttc taattggttt ttactgaaac attgagggaa cacaaattta 41580
tgggcttcct gatgatgatt cttctaggca tcatgtccta tagtttgtca tccctgatga 41640
atgtaaagtt acactgttca caaaggtttt gtctcctttc cactgctatt agtcatggtc 41700
actctcccca aaatattata ttttttctat aaaaagaaaa aaatggaaaa aaattacaag 41760
gcaatggaaa ctattataag gccatttcct tttcacatta gataaattac tataaagact 41820
cctaatagct tttcctgtta aggcagaccc agtatgaaat ggggattatt atagcaacca 41880
ttttggggct atatttacat gctactaaat ttttataata attgaaaaga ttttaacaag 41940
tataaaaaat tctcatagga attaaatgta gtctccctgt gtcagactgc tctttcatag 42000
tataacttta aatcttttct tcaacttgag tctttgaaga tagttttaat tctgcttgtg 42060
acattaaaag attatttggg ccagttatag cttattaggt gttgaagaga ccaaggttgc 42120
aaggccaggc cctgtgtgaa cctttgagct ttcatagaga gtttcacagc atggactgtg 42180
tccccacggt catccagtgt tgtcatgcat tggttagtca aaatggggag ggactagggc 42240
agtttggata gctcaacaag atacaatctc actctgtggt ggtcctgctg acaaatcaag 42300
agcattgctt ttgtttctta agaaaacaaa ctctttttta aaaattactt ttaaatatta 42360
actcaaaagt tgagattttg gggtggtggt gtgccaagac attaattttt tttttaaaca 42420
atgaagtgaa aaagttttac aatctctagg tttggctagt tctcttaaca ctggttaaat 42480
taacattgca taaacacttt tcaagtctga tccatattta ataatgcttt aaaataaaaa 42540
taaaaacaat ccttttgata aatttaaaat gttacttatt ttaaaataaa tgaagtgaga 42600
tggcatggtg aggtgaaagt atcactggac taggaagaag gtgacttagg ttctagatag 42660
gtgtctttta ggactctgat tttgaggaca tcacttacta tccatttctt catgttaaaa 42720
gaagtcatct caaactctta gttttttttt tttacaacta tgtaatttat attccattta 42780
cataaggata cacttatttg tcaagctcag cacaatctgt aaatttttaa cctatgttac 42840
accatcttca gtgccagtct tgggcaaaat tgtgcaagag gtgaagttta tatttgaata 42900
tccattctcg ttttaggact cttcttccat attagtgtca tcttgcctcc ctaccttcca 42960
catgccccat gacttgatgc agttttaata cttgtaattc ccctaaccat aagatttact 43020
gctgctgtgg atatctccat gaagttttcc cactgagtca catcagaaat gccctacatc 43080

ttatttcctc agggctcaag agaatctgac agataccata aagggatttg acctaatcac 43140
taattttcag gtggtggctg atgctttgaa catctctttg ctgcccaatc cattagcgac 43200
agtaggattt ttcaaacctg gtatgaatag acagaaccct atccagtgga aggagaattt 43260
aataaagata gtgctgaaag aattccttag gtaatctata actaggacta ctcctggtaa 43320
cagtaataca ttccattgtt ttagtaacca gaaatcttca tgcaatgaaa aatactttaa 43380
ttcatgaagc ttactttttt tttttggtgt cagagtctcg ctcttgtcac ccaggctgga 43440
atgcagtggc gccatctcag ctcactgcaa cctccatctc ccaggttcaa gcgattctcg 43500
tgcctcggcc tcctgagtag ctgggattac aggcgtgtgc cactacactc aactaatttt 43560
tgtattttta ggagagacgg ggtttcaccc tgttggccag gctggtctcg aactcctgac 43620
ctcaagtgat tcacccacct tggcctcata aacctgtttt gcagaactca tttattcagc 43680
aaatatttat tgagtgccta ccagatgcca gtcaccacac aaggcactgg gtatatggta 43740
tccccaaaca agagacataa tcccggtcct taggtagtgc tagtgtggtc tgtaatatct 43800
tactaaggcc tttggtatac gacccagaga taacacgatg cgtattttag ttttgcaaag 43860
aaggggtttg gtctctgtgc cagctctata attgttttgc tacgattcca ctgaaactct 43920
tcgatcaagc tactttatgt aaatcacttc attgttttaa aggaataaac ttgattatat 43980
tgttttttta tttggcataa ctgtgattct tttaggacaa ttactgtaca cattaaggtg 44040
tatgtcagat attcatattg acccaaatgt gtaatattcc agttttctct gcataagtaa 44100
ttaaaatata cttaaaaatt aatagtttta tctgggtaca aataaacagg tgcctgaact 44160
agttcacaga caaggaaact tctatgtaaa aatcactatg atttctgaat tgctatgtga 44220
aactacagat ctttggaaca ctgtttaggt agggtgttaa gacttacaca gtacctcgtt 44280
tctacacaga gaaagaaatg gccatacttc aggaactgca gtgcttatga ggggatattt 44340
aggcctcttg aatttttgat gtagatgggc atttttttaa ggtagtggtt aattaccttt 44400
atgtgaactt tgaatggttt aacaaaagat ttgtttttgt agagatttta aagggggaga 44460
attctagaaa taaatgttac ctaattatta cagccttaaa gacaaaaatc cttgttgaag 44520
tttttttaaa aaaagctaaa ttacatagac ttaggcatta acatgtttgt ggaagaatat 44580
agcagacgta tattgtatca tttgagtgaa tgttcccaag taggcattct aggctctatt 44640
taactgagtc acactgcata ggaatttaga acctaacttt tataggttat caaaactgtt 44700
gtcaccattg cacaattttg tcctaatata tacatagaaa ctttgtgggg catgttaagt 44760
tacagtttgc acaagttcat ctcatttgta ttccattgat tttttttttc ttctaaacat 44820
tttttcttca aacagtatat aacttttttt aggggatttt tttttagaca gcaaaaacta 44880
tctgaagatt tccatttgtc aaaaagtaat gatttcttga taattgtgta gtaatgtttt 44940
ttagaaccca gcagttacct taaagctgaa tttatattta gtaacttctg tgttaatact 45000
ggatagcatg aattctgcat tgagaaactg aatagctgtc ataaaatgaa actttctttc 45060
taaagaaaga tactcacatg agttcttgaa gaatagtcat aactagatta agatctgtgt 45120
tttagtttaa tagtttgaag tgcctgtttg ggataatgat aggtaattta gatgaattta 45180

ggggaaaaaa aagttatctg cagatatgtt gagggcccat ctctcccccc acacccccac 45240
agagctaact gggttacagt gttttatccg aaagtttcca attccactgt cttgtgtttt 45300
catgttgaaa atacttttgc atttttcctt tgagtgccaa tttcttacta gtactatttc 45360
ttaatgtaac atgtttacct ggaatgtatt ttaactattt ttgtatagtg taaactgaaa 45420
catgcacatt ttgtacattg tgctttcttt tgtgggacat atgcagtgtg atccagttgt 45480
tttccatcat ttggttgcgc tgacctagga atgttggtca tatcaaacat taaaaatgac 45540
cactctttta attgaaatta acttttaaat gtttatagga gtatgtgctg tgaagtgatc 45600
taaaatttgt aatatttttg tcatgaactg tactactcct aattattgta atgtaataaa 45660
aatagttaca gtgac 45675

<210> 4
<211> 5436
<212> DNA
<213> Homo sapiens

<400> 4
ggccgcggcg gcggaggcag cagcggcggc ggcagtggcg gcggcgaagg tggcggcggc 60
tcggccagta ctcccggccc ccgccatttc ggactgggag cgagcgcggc gcaggcactg 120
aaggcggcgg cggggccaga ggctcagcgg ctcccaggtg cgggagagag gcctgctgaa 180
aatgactgaa tataaacttg tggtagttgg agctggtggc gtaggcaaga gtgccttgac 240
gatacagcta attcagaatc attttgtgga cgaatatgat ccaacaatag aggattccta 300
caggaagcaa gtagtaattg atggagaaac ctgtctcttg gatattctcg acacagcagg 360
tcaagaggag tacagtgcaa tgagggacca gtacatgagg actggggagg gctttctttg 420
tgtatttgcc ataaataata ctaaatcatt tgaagatatt caccattata gagaacaaat 480
taaaagagtt aaggactctg aagatgtacc tatggtccta gtaggaaata aatgtgattt 540
gccttctaga acagtagaca caaaacaggc tcaggactta gcaagaagtt atggaattcc 600
ttttattgaa acatcagcaa agacaagaca gagagtggag gatgcttttt atacattggt 660
gagggagatc cgacaataca gattgaaaaa aatcagcaaa gaagaaaaga ctcctggctg 720
tgtgaaaatt aaaaaatgca ttataatgta atctgggtgt tgatgatgcc ttctatacat 780
tagttcgaga aattcgaaaa cataaagaaa agatgagcaa agatggtaaa aagaagaaaa 840
agaagtcaaa gacaaagtgt gtaattatgt aaatacaatt tgtacttttt tcttaaggca 900
tactagtaca agtggtaatt tttgtacatt acactaaatt attagcattt gttttagcat 960
tacctaattt ttttcctgct ccatgcagac tgttagcttt taccttaaat gcttatttta 1020
aaatgacagt ggaagttttt ttttcctcta agtgccagta ttcccagagt tttggttttt 1080
gaactagcaa tgcctgtgaa aaagaaactg aatacctaag atttctgtct tggggttttt 1140
ggtgcatgca gttgattact tcttattttt cttaccaatt gtgaatgttg gtgtgaaaca 1200
aattaatgaa gcttttgaat catccctatt ctgtgtttta tctagtcaca taaatggatt 1260
aattactaat ttcagttgag accttctaat tggtttttac tgaaacattg agggaacaca 1320

aatttatggg cttcctgatg atgattcttc taggcatcat gtcctatagt ttgtcatccc 1380
tgatgaatgt aaagttacac tgttcacaaa ggttttgtct cctttccact gctattagtc 1440
atggtcactc tccccaaaat attatatttt ttctataaaa agaaaaaaat ggaaaaaaat 1500
tacaaggcaa tggaaactat tataaggcca tttccttttc acattagata aattactata 1560
aagactccta atagcttttc ctgttaaggc agacccagta tgaaatgggg attattatag 1620
caaccatttt ggggctatat ttacatgcta ctaaattttt ataataattg aaaagatttt 1680
aacaagtata aaaaattctc ataggaatta aatgtagtct ccctgtgtca gactgctctt 1740
tcatagtata actttaaatc ttttcttcaa cttgagtctt tgaagatagt tttaattctg 1800
cttgtgacat taaaagatta tttgggccag ttatagctta ttaggtgttg aagagaccaa 1860
ggttgcaagg ccaggccctg tgtgaacctt tgagctttca tagagagttt cacagcatgg 1920
actgtgtccc cacggtcatc cagtgttgtc atgcattggt tagtcaaaat ggggagggac 1980
tagggcagtt tggatagctc aacaagatac aatctcactc tgtggtggtc ctgctgacaa 2040
atcaagagca ttgcttttgt ttcttaagaa aacaaactct tttttaaaaa ttacttttaa 2100
atattaactc aaaagttgag attttggggt ggtggtgtgc caagacatta attttttttt 2160
taaacaatga agtgaaaaag ttttacaatc tctaggtttg gctagttctc ttaacactgg 2220
ttaaattaac attgcataaa cacttttcaa gtctgatcca tatttaataa tgctttaaaa 2280
taaaaataaa aacaatcctt ttgataaatt taaaatgtta cttattttaa aataaatgaa 2340
gtgagatggc atggtgaggt gaaagtatca ctggactagg aagaaggtga cttaggttct 2400
agataggtgt cttttaggac tctgattttg aggacatcac ttactatcca tttcttcatg 2460
ttaaaagaag tcatctcaaa ctcttagttt ttttttttta caactatgta atttatattc 2520
catttacata aggatacact tatttgtcaa gctcagcaca atctgtaaat ttttaaccta 2580
tgttacacca tcttcagtgc cagtcttggg caaaattgtg caagaggtga agtttatatt 2640
tgaatatcca ttctcgtttt aggactcttc ttccatatta gtgtcatctt gcctccctac 2700
cttccacatg ccccatgact tgatgcagtt ttaatacttg taattcccct aaccataaga 2760
tttactgctg ctgtggatat ctccatgaag ttttcccact gagtcacatc agaaatgccc 2820
tacatcttat ttcctcaggg ctcaagagaa tctgacagat accataaagg gatttgacct 2880
aatcactaat tttcaggtgg tggctgatgc tttgaacatc tctttgctgc ccaatccatt 2940
agcgacagta ggatttttca aacctggtat gaatagacag aaccctatcc agtggaagga 3000
gaatttaata aagatagtgc tgaaagaatt ccttaggtaa tctataacta ggactactcc 3060
tggtaacagt aatacattcc attgttttag taaccagaaa tcttcatgca atgaaaaata 3120
ctttaattca tgaagcttac tttttttttt tggtgtcaga gtctcgctct tgtcacccag 3180
gctggaatgc agtggcgcca tctcagctca ctgcaacctc catctcccag gttcaagcga 3240
ttctcgtgcc tcggcctcct gagtagctgg gattacaggc gtgtgccact acactcaact 3300
aatttttgta tttttaggag agacggggtt tcaccctgtt ggccaggctg gtctcgaact 3360

cctgacctca agtgattcac ccaccttggc ctcataaacc tgttttgcag aactcattta 3420
ttcagcaaat atttattgag tgcctaccag atgccagtca ccgcacaagg cactgggtat 3480
atggtatccc caaacaagag acataatccc ggtccttagg tagtgctagt gtggtctgta 3540
atatcttact aaggcctttg gtatacgacc cagagataac acgatgcgta ttttagtttt 3600
gcaaagaagg ggtttggtct ctgtgccagc tctataattg ttttgctacg attccactga 3660
aactcttcga tcaagctact ttatgtaaat cacttcattg ttttaaagga ataaacttga 3720
ttatattgtt tttttatttg gcataactgt gattctttta ggacaattac tgtacacatt 3780
aaggtgtatg tcagatattc atattgaccc aaatgtgtaa tattccagtt ttctctgcat 3840
aagtaattaa aatatactta aaaattaata gttttatctg ggtacaaata aacaggtgcc 3900
tgaactagtt cacagacaag gaaacttcta tgtaaaaatc actatgattt ctgaattgct 3960
atgtgaaact acagatcttt ggaacactgt ttaggtaggg tgttaagact tacacagtac 4020
ctcgtttcta cacagagaaa gaaatggcca tacttcagga actgcagtgc ttatgagggg 4080
atatttaggc ctcttgaatt tttgatgtag atgggcattt ttttaaggta gtggttaatt 4140
acctttatgt gaactttgaa tggtttaaca aaagatttgt ttttgtagag attttaaagg 4200
gggagaattc tagaaataaa tgttacctaa ttattacagc cttaaagaca aaaatccttg 4260
ttgaagtttt tttaaaaaaa gctaaattac atagacttag gcattaacat gtttgtggaa 4320
gaatatagca gacgtatatt gtatcatttg agtgaatgtt cccaagtagg cattctaggc 4380
tctatttaac tgagtcacac tgcataggaa tttagaacct aacttttata ggttatcaaa 4440
actgttgtca ccattgcaca attttgtcct aatatataca tagaaacttt gtggggcatg 4500
ttaagttaca gtttgcacaa gttcatctca tttgtattcc attgattttt tttttcttct 4560
aaacattttt tcttcaaaca gtatataact ttttttaggg gatttttttt tagacagcaa 4620
aaactatctg aagatttcca tttgtcaaaa agtaatgatt tcttgataat tgtgtagtaa 4680
tgttttttag aacccagcag ttaccttaaa gctgaattta tatttagtaa cttctgtgtt 4740
aatactggat agcatgaatt ctgcattgag aaactgaata gctgtcataa aatgaaactt 4800
tctttctaaa gaaagatact cacatgagtt cttgaagaat agtcataact agattaagat 4860
ctgtgtttta gtttaatagt ttgaagtgcc tgtttgggat aatgataggt aatttagatg 4920
aatttagggg aaaaaaaagt tatctgcaga tatgttgagg gcccatctct ccccccacac 4980
ccccacagag ctaactgggt tacagtgttt tatccgaaag tttccaattc cactgtcttg 5040
tgttttcatg ttgaaaatac ttttgcattt ttcctttgag tgccaatttc ttactagtac 5100
tatttcttaa tgtaacatgt ttacctggaa tgtattttaa ctatttttgt atagtgtaaa 5160
ctgaaacatg cacattttgt acattgtgct ttcttttgtg ggacatatgc agtgtgatcc 5220
agttgttttc catcatttgg ttgcgctgac ctaggaatgt tggtcatatc aaacattaaa 5280
aatgaccact cttttaattg aaattaactt ttaaatgttt ataggagtat gtgctgtgaa 5340
gtgatctaaa atttgtaata tttttgtcat gaactgtact actcctaatt attgtaatgt 5400
aataaaaata gttacagtga caaaaaaaaa aaaaaa 5436

<210> 5
<211> 5312
<212> DNA
<213> Homo sapiens

<400> 5
ggccgcggcg gcggaggcag cagcggcggc ggcagtggcg gcggcgaagg tggcggcggc 60

tcggccagta ctcccggccc ccgccatttc ggactgggag cgagcgcggc gcaggcactg 120

aaggcggcgg cggggccaga ggctcagcgg ctcccaggtg cgggagagag gcctgctgaa 180

aatgactgaa tataaacttg tggtagttgg agctggtggc gtaggcaaga gtgccttgac 240

gatacagcta attcagaatc attttgtgga cgaatatgat ccaacaatag aggattccta 300

caggaagcaa gtagtaattg atggagaaac ctgtctcttg gatattctcg acacagcagg 360

tcaagaggag tacagtgcaa tgagggacca gtacatgagg actggggagg gctttctttg 420

tgtatttgcc ataaataata ctaaatcatt tgaagatatt caccattata gagaacaaat 480

taaaagagtt aaggactctg aagatgtacc tatggtccta gtaggaaata aatgtgattt 540

gccttctaga acagtagaca caaaacaggc tcaggactta gcaagaagtt atggaattcc 600

ttttattgaa acatcagcaa agacaagaca gggtgttgat gatgccttct atacattagt 660

tcgagaaatt cgaaaacata aagaaaagat gagcaaagat ggtaaaaaga agaaaaagaa 720

gtcaaagaca aagtgtgtaa ttatgtaaat acaatttgta cttttttctt aaggcatact 780

agtacaagtg gtaatttttg tacattacac taaattatta gcatttgttt tagcattacc 840

taattttttt cctgctccat gcagactgtt agcttttacc ttaaatgctt attttaaaat 900

gacagtggaa gttttttttt cctctaagtg ccagtattcc cagagttttg gtttttgaac 960

tagcaatgcc tgtgaaaaag aaactgaata cctaagattt ctgtcttggg gtttttggtg 1020

catgcagttg attacttctt atttttctta ccaattgtga atgttggtgt gaaacaaatt 1080

aatgaagctt ttgaatcatc cctattctgt gttttatcta gtcacataaa tggattaatt 1140

actaatttca gttgagacct tctaattggt ttttactgaa acattgaggg aacacaaatt 1200

tatgggcttc ctgatgatga ttcttctagg catcatgtcc tatagtttgt catccctgat 1260

gaatgtaaag ttacactgtt cacaaaggtt ttgtctcctt tccactgcta ttagtcatgg 1320

tcactctccc caaaatatta tattttttct ataaaaagaa aaaaatggaa aaaaattaca 1380

aggcaatgga aactattata aggccatttc cttttcacat tagataaatt actataaaga 1440

ctcctaatag cttttcctgt taaggcagac ccagtatgaa atggggatta ttatagcaac 1500

cattttgggg ctatatttac atgctactaa atttttataa taattgaaaa gattttaaca 1560

agtataaaaa attctcatag gaattaaatg tagtctccct gtgtcagact gctctttcat 1620

agtataactt taaatctttt cttcaacttg agtctttgaa gatagtttta attctgcttg 1680

tgacattaaa agattatttg ggccagttat agcttattag gtgttgaaga gaccaaggtt 1740

gcaaggccag gccctgtgtg aacctttgag ctttcataga gagtttcaca gcatggactg 1800

tgtccccacg gtcatccagt gttgtcatgc attggttagt caaaatgggg agggactagg 1860

gcagtttgga tagctcaaca agatacaatc tcactctgtg gtggtcctgc tgacaaatca 1920
agagcattgc ttttgtttct taagaaaaca aactcttttt taaaaattac ttttaaatat 1980
taactcaaaa gttgagattt tggggtggtg gtgtgccaag acattaattt tttttttaaa 2040
caatgaagtg aaaaagtttt acaatctcta ggtttggcta gttctcttaa cactggttaa 2100
attaacattg cataaacact tttcaagtct gatccatatt taataatgct ttaaaataaa 2160
aataaaaaca atccttttga taaatttaaa atgttactta ttttaaaata aatgaagtga 2220
gatggcatgg tgaggtgaaa gtatcactgg actaggaaga aggtgactta ggttctagat 2280
aggtgtcttt taggactctg attttgagga catcacttac tatccatttc ttcatgttaa 2340
aagaagtcat ctcaaactct tagttttttt tttttacaac tatgtaattt atattccatt 2400
tacataagga tacacttatt tgtcaagctc agcacaatct gtaaattttt aacctatgtt 2460
acaccatctt cagtgccagt cttgggcaaa attgtgcaag aggtgaagtt tatatttgaa 2520
tatccattct cgttttagga ctcttcttcc atattagtgt catcttgcct ccctaccttc 2580
cacatgcccc atgacttgat gcagttttaa tacttgtaat tcccctaacc ataagattta 2640
ctgctgctgt ggatatctcc atgaagtttt cccactgagt cacatcagaa atgccctaca 2700
tcttatttcc tcagggctca agagaatctg acagatacca taaagggatt tgacctaatc 2760
actaattttc aggtggtggc tgatgctttg aacatctctt tgctgcccaa tccattagcg 2820
acagtaggat ttttcaaacc tggtatgaat agacagaacc ctatccagtg gaaggagaat 2880
ttaataaaga tagtgctgaa agaattcctt aggtaatcta taactaggac tactcctggt 2940
aacagtaata cattccattg ttttagtaac cagaaatctt catgcaatga aaaatacttt 3000
aattcatgaa gcttactttt tttttttggt gtcagagtct cgctcttgtc acccaggctg 3060
gaatgcagtg gcgccatctc agctcactgc aacctccatc tcccaggttc aagcgattct 3120
cgtgcctcgg cctcctgagt agctgggatt acaggcgtgt gccactacac tcaactaatt 3180
tttgtatttt taggagagac ggggtttcac cctgttggcc aggctggtct cgaactcctg 3240
acctcaagtg attcacccac cttggcctca taaacctgtt ttgcagaact catttattca 3300
gcaaatattt attgagtgcc taccagatgc cagtcaccgc acaaggcact gggtatatgg 3360
tatccccaaa caagagacat aatcccggtc cttaggtagt gctagtgtgg tctgtaatat 3420
cttactaagg cctttggtat acgacccaga gataacacga tgcgtatttt agttttgcaa 3480
agaaggggtt tggtctctgt gccagctcta taattgtttt gctacgattc cactgaaact 3540
cttcgatcaa gctactttat gtaaatcact tcattgtttt aaaggaataa acttgattat 3600
attgtttttt tatttggcat aactgtgatt cttttaggac aattactgta cacattaagg 3660
tgtatgtcag atattcatat tgacccaaat gtgtaatatt ccagttttct ctgcataagt 3720
aattaaaata tacttaaaaa ttaatagttt tatctgggta caaataaaca ggtgcctgaa 3780
ctagttcaca gacaaggaaa cttctatgta aaaatcacta tgatttctga attgctatgt 3840
gaaactacag atctttggaa cactgtttag gtagggtgtt aagacttaca cagtacctcg 3900

tttctacaca gagaaagaaa tggccatact tcaggaactg cagtgcttat gaggggatat 3960

ttaggcctct tgaatttttg atgtagatgg gcattttttt aaggtagtgg ttaattacct 4020

ttatgtgaac tttgaatggt ttaacaaaag atttgttttt gtagagattt taaaggggga 4080

gaattctaga aataaatgtt acctaattat tacagcctta aagacaaaaa tccttgttga 4140

agttttttta aaaaaagcta aattacatag acttaggcat taacatgttt gtggaagaat 4200

atagcagacg tatattgtat catttgagtg aatgttccca agtaggcatt ctaggctcta 4260

tttaactgag tcacactgca taggaattta gaacctaact tttataggtt atcaaaactg 4320

ttgtcaccat tgcacaattt tgtcctaata tatacataga aactttgtgg ggcatgttaa 4380

gttacagttt gcacaagttc atctcatttg tattccattg attttttttt tcttctaaac 4440

atttttcttt caaacagtat ataacttttt ttaggggatt tttttttaga cagcaaaaac 4500

tatctgaaga tttccatttg tcaaaaagta atgatttctt gataattgtg tagtaatgtt 4560

ttttagaacc cagcagttac cttaaagctg aatttatatt tagtaacttc tgtgttaata 4620

ctggatagca tgaattctgc attgagaaac tgaatagctg tcataaaatg aaactttctt 4680

tctaaagaaa gatactcaca tgagttcttg aagaatagtc ataactagat taagatctgt 4740

gttttagttt aatagtttga agtgcctgtt tgggataatg ataggtaatt tagatgaatt 4800

taggggaaaa aaaagttatc tgcagatatg ttgagggccc atctctcccc ccacaccccc 4860

acagagctaa ctgggttaca gtgttttatc cgaaagtttc caattccact gtcttgtgtt 4920

ttcatgttga aaatactttt gcatttttcc tttgagtgcc aatttcttac tagtactatt 4980

tcttaatgta acatgtttac ctggaatgta ttttaactat ttttgtatag tgtaaactga 5040

aacatgcaca ttttgtacat tgtgctttct tttgtgggac atatgcagtg tgatccagtt 5100

gttttccatc atttggttgc gctgacctag gaatgttggt catatcaaac attaaaaatg 5160

accactcttt taattgaaat taacttttaa atgtttatag gagtatgtgc tgtgaagtga 5220

tctaaaattt gtaatatttt tgtcatgaac tgtactactc ctaattattg taatgtaata 5280

aaaatagtta cagtgacaaa aaaaaaaaaa aa 5312

<210> 6
<211> 99
<212> DNA
<213> Homo sapiens

<400> 6
ggactctgga tcccagaagg tgagaaagtt aaaattcccg tcgctatcaa ggaattaaga 60

gaagcaacat ctccgaaagc caacaaggaa atcctcgat 99

<210> 7
<211> 165
<212> DNA
<213> Homo sapiens

<400> 7
ggcatgaact acttggagga ccgtcgcttg gtgcaccgcg acctggcagc caggaacgta 60

ctggtgaaaa caccgcagca tgtcaagatc acagattttg ggctggccaa actgctgggt 120

gcggaagaga aagaatacca tgcagaagga ggcaaagtgc ctatc                165

<210>  8
<211>  195
<212>  DNA
<213>  Homo sapiens

<400>  8
atcctcgatg aagcctacgt gatggccagc gtggacaacc cccacgtgtg ccgcctgctg      60

ggcatctgcc tcacctccac cgtgcagctc atcacgcagc tcatgccctt cggctgcctc     120

ctggactatg tccgggaaca caaagacaat attggctccc agtacctgct caactggtgt     180

gtgcagatcg caaag                                                      195

<210>  9
<211>  170
<212>  DNA
<213>  Homo sapiens

<400>  9
atgactgaat ataaacttgt ggtagttgga gctggtggcg taggcaagag tgccttgacg      60

atacagctaa ttcagaatca ttttgtggac gaatatgatc caacaataga ggattcctac     120

aggaagcaag tagtaattga tggagaaacc tgtctcttgg atattctcga                170

<210>  10
<211>  189
<212>  DNA
<213>  Homo sapiens

<400>  10
cacagcaggt caagaggagt acagtgcaat gagggaccag tacatgagga ctggggaggg      60

ctttctttgt gtatttgcca taaataatac taaatcattt gaagatattc accattatag     120

agaacaaatt aaaagagtta aggactctga agatgtacct atggtcctag taggaaataa     180

atgtgattt                                                             189

<210>  11
<211>  1210
<212>  PRT
<213>  Homo sapiens

<400>  11

<210>  12
<211>  189
<212>  PRT
<213>  Homo sapiens

<400>  12

<210>  13
<211>  189
<212>  PRT
<213>  Homo sapiens

<400>  13

<210>  14
<211>  21
<212>  DNA
<213>  Artificial

<220>
<223>  Forward Primer EGFR exon 19

<400>  14
taaaattccc gtcgctatca a                                                21

<210>  15
<211>  21
<212>  DNA
<213>  Artificial

<220>
<223>  Reverse Primer EGFR exon 19

<400>  15
agctgccaga catgagaaaa g                                                21


<210>  16
<211>  19
<212>  DNA
<213>  Artificial

<220>
<223>  Forward Primer EGFR L858R, L861Q

<400>  16
aaacaccgca gcatgtcaa                                                   19


<210>  17
<211>  19
<212>  DNA
<213>  Artificial

<220>
<223>  Reverse Primer EGFR L858R, L861Q

<400>  17
tctttctctt ccgcaccca                                                   19


<210>  18
<211>  19
<212>  DNA
<213>  Artificial

<220>
<223>  Forward Primer EGFR T790M

<400>  18
ctgggcatct gcctcacct                                                   19


<210>  19
<211>  21
<212>  DNA
<213>  Artificial

<220>
<223>  Reverse Primer EGFR T790M

<400>  19
tgtgttcccg gacatagtcc a                                                21


<210>  20
<211>  24
<212>  DNA
<213>  Artificial

<220>
<223>  Forward Primer KRAS exon 2

<400>  20
ctgaatataa acttgtggta gttg                                              24

<210>  21
<211>  19
<212>  DNA
<213>  Artificial

<220>
<223>  Reverse Primer KRAS exon 2

<400>  21
tgtatcgtca aggcactct                                                    19

<210>  22
<211>  22
<212>  DNA
<213>  Artificial

<220>
<223>  Forward Primer KRAS Q61H/K/L/R

<400>  22
gatggagaaa cctgtctctt gg                                                22

<210>  23
<211>  20
<212>  DNA
<213>  Artificial

<220>
<223>  Reverse Primer KRAS Q61H/K/L/R

<400>  23
ccctccccag tcctcatgta                                                   20

<210>  24
<211>  16
<212>  DNA
<213>  Artificial

<220>
<223>  Pyrosequencing Primer EGFR exon 19

<400>  24
ttcccgtcgc tatcaa                                                       16

<210>  25
<211>  19
<212>  DNA
<213>  Artificial

<220>
<223>  Pyrosequencing Primer EGFR exon 21

<400>  25
catgtcaaga tcacagatt                                                    19

<210>  26
<211>  16
<212>  DNA
<213>  Artificial

<220>
<223>  Pyrosequencing Primer EGFR exon 20

<400>  26
accgtgcagc tcatca                                                        16


<210>  27
<211>  18
<212>  DNA
<213>  Artificial

<220>
<223>  Pyrosequencing  Primer KRAS exon 2

<400>  27
aaacttgtgg tagttgga                                                      18


<210>  28
<211>  16
<212>  DNA
<213>  Artificial

<220>
<223>  Pyrosequencing  Primer KRAS exon 3

<400>  28
tctcgacaca gcaggt                                                        16


<210>  29
<211>  22
<212>  DNA
<213>  Artificial

<220>
<223>  EGFR exon 19 dispensing order 1

<400>  29
agatagagtc agcacatctc ga                                                 22


<210>  30
<211>  20
<212>  DNA
<213>  Artificial

<220>
<223>  EGFR exon 19 dispensing order 2

<400>  30
agatagagag cacatctcga                                                    20


<210>  31
<211>  15
<212>  DNA
<213>  Artificial

<220>
<223>  EGFR exon 21 dispensing order

<400>  31
ctgcgtgtca ctacg                                                         15


<210>  32
<211>  9

<212>  DNA
<213>  Artificial

<220>
<223>  EGFR exon 20 dispensing order

<400>  32
actagcagc                                                                9


<210>  33
<211>  17
<212>  DNA
<213>  Artificial

<220>
<223>  KRAS exon 2 dispensing order

<400>  33
gctgacgatg agtcgta                                                      17


<210>  34
<211>  11
<212>  DNA
<213>  Artificial

<220>
<223>  KRAS exon 3 dispensing order

<400>  34
cgatgcatga g                                                            11